# EUROPEAN PATENT APPLICATION

(11) **EP 3 118 215 A1**
(43) Date of publication of application: **18.01.2017**
(21) Application number: 15177013.8
(22) Date of filing: 16.07.2015
(51) Int. Cl.: C07K 14/415, A61K 38/01

(54) **ANTI-INFLAMMATORY PEPTIDES, AND USES THEREOF**

(71) Applicant: Nuritas Limited, Co. Dublin (IE)
(72) Inventor: KHALDI, Nora, Co. Dublin (IE); LOPEZ, Cyril, Dublin, 6W (IE)
(74) Representative: Purdy, Hugh Barry

(57) **Abstract**

An anti-inflammatory peptide comprises an anti-inflammatory fragment of a protein selected from SEQUENCE ID NO's: 1 to 16, the anti-inflammatory fragment being 7 to 37 amino acids in length and having a charge of between -9 and +3; wherein the c-terminal amino acid is not cysteine (C) or methionine (M), and the n-terminal amino acid is not cysteine (C), histidine (H), proline (P) or threonine (T). The anti-inflammatory fragment does not contain cysteine (C). The anti-inflammatory fragment is from a region of the proteins of SEQUENCE ID NO's: 1 to 16, which region is characterised by being 17 to 109 amino acids in length and having a charge of between -6 and +4, wherein the c-terminal amino acid of the region is not aspartic acid (D), phenylalanine (F), methionine (M) or tryptophan (W), and the n-terminal amino acid of the region is not aspartic acid (D), histidine (H), methionine (M), proline (P) or tryptophan (W). Examples of peptides are provided in SEQUENCE ID NO's: 71 to 221.

SEQUENCE LISTING

<110>  NURITAS LIMITED

<120>  Anti-inflammatory peptides, and uses thereof

<130>  P11683EP00

<160>  267

<170>  PatentIn version 3.5

<210>  1
<211>  459
<212>  PRT
<213>  Pisum sativum

<400>  1

Met Ala Ala Thr Thr Met Lys Ala Ser Phe Pro Leu Leu Met Leu Met
1               5                   10                  15

Gly Ile Ser Phe Leu Ala Ser Val Cys Val Ser Ser Arg Ser Asp Pro
            20                  25                  30

Gln Asn Pro Phe Ile Phe Lys Ser Asn Lys Phe Gln Thr Leu Phe Glu
        35                  40                  45

Asn Glu Asn Gly His Ile Arg Leu Leu Gln Lys Phe Asp Gln Arg Ser
    50                  55                  60

Lys Ile Phe Glu Asn Leu Gln Asn Tyr Arg Leu Leu Glu Tyr Lys Ser
65                  70                  75                  80

Lys Pro His Thr Ile Phe Leu Pro Gln His Thr Asp Ala Asp Tyr Ile
                85                  90                  95

Leu Val Val Leu Ser Gly Lys Ala Ile Leu Thr Val Leu Lys Pro Asp
            100                 105                 110

Asp Arg Asn Ser Phe Asn Leu Glu Arg Gly Asp Thr Ile Lys Leu Pro
        115                 120                 125

Ala Gly Thr Ile Ala Tyr Leu Val Asn Arg Asp Asp Asn Glu Glu Leu
    130                 135                 140

Arg Val Leu Asp Leu Ala Ile Pro Val Asn Arg Pro Gly Gln Leu Gln
145                 150                 155                 160

Ser Phe Leu Leu Ser Gly Asn Gln Asn Gln Gln Asn Tyr Leu Ser Gly
                165                 170                 175

Phe Ser Lys Asn Ile Leu Glu Ala Ser Phe Asn Thr Asp Tyr Glu Glu
            180                 185                 190

Ile Glu Lys Val Leu Leu Glu Glu His Glu Lys Glu Thr Gln His Arg
        195                 200                 205

Arg Ser Leu Lys Asp Lys Arg Gln Gln Ser Gln Glu Glu Asn Val Ile
    210                 215                 220

Val Lys Leu Ser Arg Gly Gln Ile Glu Glu Leu Ser Lys Asn Ala Lys
225                 230                 235                 240

Ser Thr Ser Lys Lys Ser Val Ser Ser Glu Ser Glu Pro Phe Asn Leu
                245                 250                 255

Arg Ser Arg Gly Pro Ile Tyr Ser Asn Glu Phe Gly Lys Phe Phe Glu
            260                 265                 270

Ile Thr Pro Glu Lys Asn Pro Gln Leu Gln Asp Leu Asp Ile Phe Val
        275                 280                 285

Asn Ser Val Glu Ile Lys Glu Gly Ser Leu Leu Leu Pro His Tyr Asn
    290                 295                 300

Ser Arg Ala Ile Val Ile Val Thr Val Asn Glu Gly Lys Gly Asp Phe
305                 310                 315                 320

Glu Leu Val Gly Gln Arg Asn Glu Asn Gln Gln Glu Gln Arg Lys Glu
                325                 330                 335

Asp Asp Glu Glu Glu Glu Gln Gly Glu Glu Glu Ile Asn Lys Gln Val
            340                 345                 350

Gln Asn Tyr Lys Ala Lys Leu Ser Ser Gly Asp Val Phe Val Ile Pro
        355                 360                 365

Ala Gly His Pro Val Ala Val Lys Ala Ser Ser Asn Leu Asp Leu Leu
    370                 375                 380

Gly Phe Gly Ile Asn Ala Glu Asn Asn Gln Arg Asn Phe Leu Ala Gly
385                 390                 395                 400

Asp Glu Asp Asn Val Ile Ser Gln Ile Gln Arg Pro Val Lys Glu Leu
                405                 410                 415

Ala Phe Pro Gly Ser Ala Gln Glu Val Asp Arg Ile Leu Glu Asn Gln
            420                 425                 430

Lys Gln Ser His Phe Ala Asp Ala Gln Pro Gln Gln Arg Glu Arg Gly
        435                 440                 445

Ser Arg Glu Thr Arg Asp Arg Leu Ser Ser Val
    450                 455

<210>  2
<211>  124
<212>  PRT
<213>  Pisum sativum

<400>  2

Asp Arg Arg Gln Glu Leu Ser Asn Glu Asn Val Leu Val Lys Val Ser
1               5                   10                  15

Arg Arg Gln Leu Glu Glu Leu Ser Lys Asn Ala Lys Ser Ser Ser Arg
            20                  25                  30

Arg Ser Val Ser Ser Glu Ser Gly Pro Phe Asn Leu Arg Ser Glu Asp
        35                  40                  45

Pro Leu Tyr Ser Asn Asn Ser Gly Lys Phe Phe Glu Leu Thr Pro Glu
    50                  55                  60

Lys Asn Gln Gln Leu Gln Asp Leu Asp Leu Phe Val Asn Ser Val Asp
65                  70                  75                  80

Leu Lys Glu Gly Ser Leu Leu Leu Pro Asn Tyr Asn Ser Arg Ala Leu
                85                  90                  95

Leu Val Leu Val Leu Val Val Asn Glu Gly Lys Gly Asp Phe Glu Leu
            100                 105                 110

Val Gly Gln Arg Asn Glu Asn Gln Gly Lys Glu Asn
        115                 120

<210>  3
<211>  520
<212>  PRT
<213>  Pisum sativum

<400>  3

Met Ala Thr Lys Leu Leu Ala Leu Ser Leu Ser Phe Cys Phe Leu Leu
1               5                   10                  15

Leu Gly Gly Cys Phe Ala Leu Arg Glu Gln Pro Glu Gln Asn Glu Cys
            20                  25                  30

Gln Leu Glu Arg Leu Asn Ala Leu Glu Pro Asp Asn Arg Ile Glu Ser
        35                  40                  45

Glu Gly Gly Leu Ile Glu Thr Trp Asn Pro Asn Asn Lys Gln Phe Arg
    50                  55                  60

Cys Ala Gly Val Ala Leu Ser Arg Ala Thr Leu Gln His Asn Ala Leu
65                  70                  75                  80

Arg Arg Pro Tyr Tyr Ser Asn Ala Pro Gln Glu Ile Phe Ile Gln Gln
                85                  90                  95

Gly Asn Gly Tyr Phe Gly Met Val Phe Pro Gly Cys Pro Glu Thr Phe
            100                 105                 110

Glu Glu Pro Gln Glu Ser Glu Gln Gly Glu Gly Arg Arg Tyr Arg Asp
        115                 120                 125

Arg His Gln Lys Val Asn Arg Phe Arg Glu Gly Asp Ile Ile Ala Val
    130                 135                 140

Pro Thr Gly Ile Val Phe Trp Met Tyr Asn Asp Gln Asp Thr Pro Val
145                 150                 155                 160

Ile Ala Val Ser Leu Thr Asp Ile Arg Ser Ser Asn Asn Gln Leu Asp
                165                 170                 175

Gln Met Pro Arg Arg Phe Tyr Leu Ala Gly Asn His Glu Gln Glu Phe
            180                 185                 190

Leu Arg Tyr Gln His Gln Gln Gly Gly Lys Gln Glu Gln Glu Asn Glu
        195                 200                 205

Gly Asn Asn Ile Phe Ser Gly Phe Lys Arg Asp Phe Leu Glu Asp Ala
    210                 215                 220

Phe Asn Val Asn Arg His Ile Val Asp Arg Leu Gln Gly Arg Asn Glu
225                 230                 235                 240

Asp Glu Glu Lys Gly Ala Ile Val Lys Val Lys Gly Gly Leu Ser Ile
                245                 250                 255

Ile Ser Pro Pro Glu Lys Gln Ala Arg His Gln Arg Gly Ser Arg Gln
            260                 265                 270

Glu Glu Asp Glu Asp Glu Asp Glu Glu Arg Gln Pro Arg His Gln Arg
        275                 280                 285

Gly Ser Arg Gln Glu Glu Glu Glu Asp Glu Asp Glu Glu Arg Gln Pro
    290                 295                 300

Arg His Gln Arg Arg Arg Gly Glu Glu Glu Glu Glu Asp Lys Lys Glu
305                 310                 315                 320

Arg Arg Gly Ser Gln Lys Gly Lys Ser Arg Arg Gln Gly Asp Asn Gly
                325                 330                 335

Leu Glu Glu Thr Val Cys Thr Ala Lys Leu Arg Leu Asn Ile Gly Pro
            340                 345                 350

Ser Ser Ser Pro Asp Ile Tyr Asn Pro Glu Ala Gly Arg Ile Lys Thr
        355                 360                 365

Val Thr Ser Leu Asp Leu Pro Val Leu Arg Trp Leu Lys Leu Ser Ala
    370                 375                 380

Glu His Gly Ser Leu His Lys Asn Ala Met Phe Val Pro His Tyr Asn
385                 390                 395                 400

Leu Asn Ala Asn Ser Ile Ile Tyr Ala Leu Lys Gly Arg Ala Arg Leu
                405                 410                 415

Gln Val Val Asn Cys Asn Gly Asn Thr Val Phe Asp Gly Glu Leu Glu
            420                 425                 430

Ala Gly Arg Ala Leu Thr Val Pro Gln Asn Tyr Ala Val Ala Ala Lys
        435                 440                 445

Ser Leu Ser Asp Arg Phe Ser Tyr Val Ala Phe Lys Thr Asn Asp Arg
    450                 455                 460

Ala Gly Ile Ala Arg Leu Ala Gly Thr Ser Ser Val Ile Asn Asn Leu
465                 470                 475                 480

Pro Leu Asp Val Val Ala Ala Thr Phe Asn Leu Gln Arg Asn Glu Ala
                485                 490                 495

Arg Gln Leu Lys Ser Asn Asn Pro Phe Lys Phe Leu Val Pro Ala Arg
            500                 505                 510

Gln Ser Glu Asn Arg Ala Ser Ala
        515                 520

<210>  4
<211>  613

<212>  PRT
<213>  Pisum sativum

<400>  4

Met Ala Thr Thr Ile Lys Ser Arg Phe Pro Leu Leu Leu Leu Leu Gly
1               5                   10                  15

Ile Ile Phe Leu Ala Ser Val Val Cys Val Thr Tyr Ala Asn Tyr Asp
            20                  25                  30

Glu Gly Ser Glu Pro Arg Val Pro Ala Gln Arg Glu Arg Gly Arg Gln
        35                  40                  45

Glu Gly Glu Lys Glu Glu Lys Arg His Gly Glu Trp Arg Pro Ser Tyr
    50                  55                  60

Glu Lys Glu Glu Asp Glu Glu Glu Gly Gln Arg Glu Arg Gly Arg Gln
65                  70                  75                  80

Glu Gly Glu Lys Glu Glu Lys Arg His Gly Glu Trp Arg Pro Ser Tyr
                85                  90                  95

Glu Lys Gln Glu Asp Glu Glu Glu Lys Gln Lys Tyr Arg Tyr Gln Arg
            100                 105                 110

Glu Lys Glu Asp Glu Glu Glu Lys Gln Lys Tyr Gln Tyr Gln Arg Glu
        115                 120                 125

Lys Lys Glu Gln Lys Glu Val Gln Pro Gly Arg Glu Arg Trp Glu Arg
    130                 135                 140

Glu Glu Asp Glu Glu Gln Val Asp Glu Glu Trp Arg Gly Ser Gln Arg
145                 150                 155                 160

Arg Glu Asp Pro Glu Glu Arg Ala Arg Leu Arg His Arg Glu Glu Arg
                165                 170                 175

Thr Lys Arg Asp Arg Arg His Gln Arg Glu Gly Glu Glu Glu Glu Arg
            180                 185                 190

Ser Ser Glu Ser Gln Glu Arg Arg Asn Pro Phe Leu Phe Lys Ser Asn
        195                 200                 205

Lys Phe Leu Thr Leu Phe Glu Asn Glu Asn Gly His Ile Arg Leu Leu
    210                 215                 220

Gln Arg Phe Asp Lys Arg Ser Asp Leu Phe Glu Asn Leu Gln Asn Tyr
225                 230                 235                 240

Arg Leu Val Glu Tyr Arg Ala Lys Pro His Thr Ile Phe Leu Pro Gln
                245                 250                 255

His Ile Asp Ala Asp Leu Ile Leu Val Val Leu Ser Gly Lys Ala Ile
            260                 265                 270

Leu Thr Val Leu Ser Pro Asn Asp Arg Asn Ser Tyr Asn Leu Glu Arg
        275                 280                 285

Gly Asp Thr Ile Lys Leu Pro Ala Gly Thr Thr Ser Tyr Leu Val Asn
    290                 295                 300

Gln Asp Asp Glu Glu Asp Leu Arg Leu Val Asp Leu Val Ile Pro Val
305                 310                 315                 320

Asn Gly Pro Gly Lys Phe Glu Ala Phe Asp Leu Ala Lys Asn Lys Asn
                325                 330                 335

Gln Tyr Leu Arg Gly Phe Ser Lys Asn Ile Leu Glu Ala Ser Tyr Asn
            340                 345                 350

Thr Arg Tyr Glu Thr Ile Glu Lys Val Leu Leu Glu Glu Gln Glu Lys
        355                 360                 365

Asp Arg Lys Arg Arg Gln Gln Gly Glu Glu Thr Asp Ala Ile Val Lys
    370                 375                 380

Val Ser Arg Glu Gln Ile Glu Glu Leu Lys Lys Leu Ala Lys Ser Ser
385                 390                 395                 400

Ser Lys Lys Ser Leu Pro Ser Glu Phe Glu Pro Ile Asn Leu Arg Ser
                405                 410                 415

His Lys Pro Glu Tyr Ser Asn Lys Phe Gly Lys Leu Phe Glu Ile Thr
            420                 425                 430

Pro Glu Lys Lys Tyr Pro Gln Leu Gln Asp Leu Asp Leu Phe Val Ser
        435                 440                 445

Cys Val Glu Ile Asn Glu Gly Ala Leu Met Leu Pro His Tyr Asn Ser
    450                 455                 460

Arg Ala Ile Val Val Leu Leu Val Asn Glu Gly Lys Gly Asn Leu Glu
465                 470                 475                 480

Leu Leu Gly Leu Lys Asn Glu Gln Gln Glu Arg Glu Asp Arg Lys Glu

485                 490                 495

Arg Asn Asn Glu Val Gln Arg Tyr Glu Ala Arg Leu Ser Pro Gly Asp
            500                 505                 510

Val Val Ile Ile Pro Ala Gly His Pro Val Ala Ile Thr Ala Ser Ser
        515                 520                 525

Asn Leu Asn Leu Leu Gly Phe Gly Ile Asn Ala Glu Asn Asn Glu Arg
    530                 535                 540

Asn Phe Leu Ser Gly Ser Asp Asp Asn Val Ile Ser Gln Ile Glu Asn
545                 550                 555                 560

Pro Val Lys Glu Leu Thr Phe Pro Gly Ser Val Gln Glu Ile Asn Arg
                565                 570                 575

Leu Ile Lys Asn Gln Lys Gln Ser His Phe Ala Asn Ala Glu Pro Glu
            580                 585                 590

Gln Lys Glu Gln Gly Ser Gln Gly Lys Arg Ser Pro Leu Ser Ser Ile
        595                 600                 605

Leu Gly Thr Phe Tyr
    610

<210>  5
<211>  438
<212>  PRT
<213>  Pisum sativum

<400>  5

Met Ala Ala Thr Pro Ile Lys Pro Leu Met Leu Leu Ala Ile Ala Phe
1               5                   10                  15

Leu Ala Ser Val Cys Val Ser Ser Arg Ser Asp Gln Glu Asn Pro Phe
            20                  25                  30

Ile Phe Lys Ser Asn Arg Phe Gln Thr Leu Tyr Glu Asn Glu Asn Gly
        35                  40                  45

His Ile Arg Leu Leu Gln Lys Phe Asp Lys Arg Ser Lys Ile Phe Glu
    50                  55                  60

Asn Leu Gln Asn Tyr Arg Leu Leu Glu Tyr Lys Ser Lys Pro Arg Thr
65                  70                  75                  80

Leu Phe Leu Pro Gln Tyr Thr Asp Ala Asp Phe Ile Leu Val Val Leu

85                  90                  95

Ser Gly Lys Ala Thr Leu Thr Val Leu Lys Ser Asn Asp Arg Asn Ser
            100                 105                 110

Phe Asn Leu Glu Arg Gly Asp Thr Ile Lys Leu Pro Ala Gly Thr Ile
        115                 120                 125

Ala Tyr Leu Ala Asn Arg Asp Asp Asn Glu Asp Leu Arg Val Leu Asp
    130                 135                 140

Leu Thr Ile Pro Val Asn Lys Pro Gly Gln Leu Gln Ser Phe Leu Leu
145                 150                 155                 160

Ser Gly Thr Gln Asn Gln Pro Ser Leu Leu Ser Gly Phe Ser Lys Asn
                165                 170                 175

Ile Leu Glu Ala Ala Phe Asn Thr Asn Tyr Glu Glu Ile Glu Lys Val
            180                 185                 190

Leu Leu Glu Gln Gln Glu Gln Glu Pro Gln His Arg Arg Ser Leu Lys
        195                 200                 205

Asp Arg Arg Gln Glu Ile Asn Glu Glu Asn Val Ile Val Lys Val Ser
    210                 215                 220

Arg Glu Gln Ile Glu Glu Leu Ser Lys Asn Ala Lys Ser Ser Ser Lys
225                 230                 235                 240

Lys Ser Val Ser Ser Glu Ser Gly Pro Phe Asn Leu Arg Ser Arg Asn
                245                 250                 255

Pro Ile Tyr Ser Asn Lys Phe Gly Lys Phe Phe Glu Ile Thr Pro Glu
            260                 265                 270

Lys Asn Gln Gln Leu Gln Asp Leu Asp Ile Phe Val Asn Ser Val Asp
        275                 280                 285

Ile Lys Glu Gly Ser Leu Leu Leu Pro Asn Tyr Asn Ser Arg Ala Ile
    290                 295                 300

Val Ile Val Thr Val Thr Glu Gly Lys Gly Asp Phe Glu Leu Val Gly
305                 310                 315                 320

Gln Arg Asn Glu Asn Gln Gly Lys Glu Asn Asp Lys Glu Glu Glu Gln
                325                 330                 335

Glu Glu Glu Thr Ser Lys Gln Val Gln Leu Tyr Arg Ala Lys Leu Ser
            340                 345                 350

Pro Gly Asp Val Phe Val Ile Pro Ala Gly His Pro Val Ala Ile Asn
        355                 360                 365

Ala Ser Ser Asp Leu Asn Leu Ile Gly Phe Gly Ile Asn Ala Glu Asn
    370                 375                 380

Asn Glu Arg Asn Phe Leu Ala Gly Glu Glu Asp Asn Val Ile Ser Gln
385                 390                 395                 400

Val Glu Arg Pro Val Lys Glu Leu Ala Phe Pro Gly Ser Ser His Glu
                405                 410                 415

Val Asp Arg Leu Leu Lys Asn Gln Lys Gln Ser Tyr Phe Ala Asn Ala
            420                 425                 430

Gln Pro Leu Gln Arg Glu
        435

<210>  6
<211>  484
<212>  PRT
<213>  Oryza sativa

<400>  6

Met Ala Thr Thr Thr Ser Leu Leu Ser Ser Cys Leu Cys Ala Leu Leu
1               5                   10                  15

Leu Ala Pro Leu Phe Ser Gln Gly Val Asp Ala Trp Glu Ser Arg Gln
            20                  25                  30

Gly Ala Ser Arg Gln Cys Arg Phe Asp Arg Leu Gln Ala Phe Glu Pro
        35                  40                  45

Leu Arg Lys Val Arg Ser Glu Ala Gly Asp Thr Glu Tyr Phe Asp Glu
    50                  55                  60

Arg Asn Glu Gln Phe Arg Cys Ala Gly Val Phe Val Ile Arg Arg Val
65                  70                  75                  80

Ile Glu Pro Gln Gly Leu Val Val Pro Arg Tyr Ser Asn Thr Pro Ala
                85                  90                  95

Leu Ala Tyr Ile Ile Gln Gly Lys Gly Tyr Val Gly Leu Thr Phe Pro
            100                 105                 110

Gly Cys Pro Ala Thr His Gln Gln Gln Phe Gln Leu Phe Glu Gln Arg
        115                 120                 125

Gln Ser Asp Gln Ala His Lys Phe Arg Asp Glu His Gln Lys Ile His
    130                 135                 140

Glu Phe Arg Gln Gly Asp Val Val Ala Leu Pro Ala Ser Val Ala His
145                 150                 155                 160

Trp Phe Tyr Asn Gly Gly Asp Thr Pro Ala Val Val Val Tyr Val Tyr
                165                 170                 175

Asp Ile Lys Ser Phe Ala Asn Gln Leu Glu Pro Arg Gln Lys Glu Phe
            180                 185                 190

Leu Leu Ala Gly Asn Asn Gln Arg Gly Gln Gln Ile Phe Glu His Ser
        195                 200                 205

Ile Phe Gln His Ser Gly Gln Asn Ile Phe Ser Gly Phe Asn Thr Glu
    210                 215                 220

Val Leu Ser Glu Ala Leu Gly Ile Asn Thr Glu Ala Ser Lys Arg Leu
225                 230                 235                 240

Gln Ser Gln Asn Asp Gln Arg Gly Asp Ile Ile Arg Val Lys His Gly
                245                 250                 255

Leu Gln Leu Leu Lys Pro Thr Leu Thr Gln Arg Gln Glu Glu His Arg
            260                 265                 270

Gln Tyr Gln Gln Val Gln Tyr Arg Glu Gly Gln Tyr Asn Gly Leu Asp
        275                 280                 285

Glu Asn Phe Cys Thr Ile Lys Ala Arg Val Asn Ile Glu Asn Pro Ser
    290                 295                 300

Arg Ala Asp Tyr Tyr Asn Pro Arg Ala Gly Arg Ile Thr Leu Leu Asn
305                 310                 315                 320

Asn Gln Lys Phe Pro Ile Leu Asn Leu Ile Gly Met Gly Ala Ala Arg
                325                 330                 335

Val Asn Leu Tyr Gln Asn Ala Leu Leu Ser Pro Phe Trp Asn Ile Asn
            340                 345                 350

Ala His Ser Val Val Tyr Ile Ile Gln Gly Ser Val Arg Val Gln Val
        355                 360                 365

Ala Asn Asn Gln Gly Arg Ser Val Phe Asn Gly Val Leu His Gln Gly
    370                 375                 380

Gln Leu Leu Ile Ile Pro Gln Asn His Ala Val Ile Lys Lys Ala Glu
385                 390                 395                 400

His Asn Gly Cys Gln Tyr Val Ala Ile Lys Thr Ile Ser Asp Pro Thr
                405                 410                 415

Val Ser Trp Val Ala Gly Lys Asn Ser Ile Leu Arg Ala Leu Pro Val
            420                 425                 430

Asp Val Ile Ala Asn Ala Tyr Arg Ile Ser Arg Asp Glu Ala Arg Arg
        435                 440                 445

Leu Lys Asn Asn Arg Ala Asp Glu Ile Gly Pro Phe Thr Pro Arg Phe
    450                 455                 460

Pro Gln Lys Ser Gln Arg Gly Tyr Gln Phe Leu Thr Glu Gly Leu Ser
465                 470                 475                 480

Leu Ile Gly Met

<210>  7
<211>  510
<212>  PRT
<213>  Oryza sativa

<400>  7

Met Ala Ser Met Ser Thr Ile Leu Pro Leu Cys Leu Gly Leu Leu Leu
1               5                   10                  15

Phe Phe Gln Val Ser Met Ala Gln Phe Ser Phe Gly Gly Ser Pro Leu
            20                  25                  30

Gln Ser Pro Arg Gly Phe Arg Gly Asp Gln Asp Ser Arg His Gln Cys
        35                  40                  45

Arg Phe Glu His Leu Thr Ala Leu Glu Ala Thr His Gln Gln Arg Ser
    50                  55                  60

Glu Ala Gly Phe Thr Glu Tyr Tyr Asn Ile Glu Ala Arg Asn Glu Phe
65                  70                  75                  80

Arg Cys Ala Gly Val Ser Val Arg Arg Leu Val Val Glu Ser Lys Gly
                85                  90                  95

Leu Val Leu Pro Met Tyr Ala Asn Ala His Lys Leu Val Tyr Ile Val
            100                 105                 110

Gln Gly Arg Gly Val Phe Gly Met Ala Leu Pro Gly Cys Pro Glu Thr
        115                 120                 125

Phe Gln Ser Val Arg Ser Pro Phe Glu Gln Glu Val Ala Thr Ala Gly
    130                 135                 140

Glu Ala Gln Ser Ser Ile Gln Lys Met Arg Asp Glu His Gln Gln Leu
145                 150                 155                 160

His Gln Phe His Gln Gly Asp Val Ile Ala Val Pro Ala Gly Val Ala
                165                 170                 175

His Trp Leu Tyr Asn Asn Gly Asp Ser Pro Val Val Ala Phe Thr Val
            180                 185                 190

Ile Asp Thr Ser Asn Asn Ala Asn Gln Leu Asp Pro Lys Arg Arg Glu
        195                 200                 205

Phe Phe Leu Ala Gly Lys Pro Arg Ser Ser Trp Gln Gln Gln Ser Tyr
    210                 215                 220

Ser Tyr Gln Thr Glu Gln Leu Ser Arg Asn Gln Asn Ile Phe Ala Gly
225                 230                 235                 240

Phe Ser Pro Asp Leu Leu Ser Glu Ala Leu Ser Val Ser Lys Gln Thr
                245                 250                 255

Val Leu Arg Leu Gln Gly Leu Ser Asp Pro Arg Gly Ala Ile Ile Arg
            260                 265                 270

Val Glu Asn Gly Leu Gln Ala Leu Gln Pro Ser Leu Gln Val Glu Pro
        275                 280                 285

Val Lys Glu Glu Gln Thr Gln Ala Tyr Leu Pro Thr Lys Gln Leu Gln
    290                 295                 300

Pro Thr Trp Leu Arg Ser Gly Gly Ala Cys Gly Gln Gln Asn Val Leu
305                 310                 315                 320

Asp Glu Ile Met Cys Ala Phe Lys Leu Arg Lys Asn Ile Asp Asn Pro
                325                 330                 335

Gln Ser Ser Asp Ile Phe Asn Pro His Gly Gly Arg Ile Thr Arg Ala
            340                 345                 350

Asn Ser Gln Asn Phe Pro Ile Leu Asn Ile Ile Gln Met Ser Ala Thr
        355                 360                 365

Arg Ile Val Leu Gln Asn Asn Ala Leu Leu Thr Pro His Trp Thr Val
    370                 375                 380

Asn Ala His Thr Val Met Tyr Val Thr Ala Gly Gln Gly His Ile Gln
385                 390                 395                 400

Val Val Asp His Arg Gly Arg Ser Val Phe Asp Gly Glu Leu His Gln
                405                 410                 415

Gln Gln Ile Leu Leu Ile Pro Gln Asn Phe Ala Val Val Val Lys Ala
            420                 425                 430

Arg Arg Glu Gly Phe Ala Trp Val Ser Phe Lys Thr Asn His Asn Ala
        435                 440                 445

Val Asp Ser Gln Ile Ala Gly Lys Ala Ser Ile Leu Arg Ala Leu Pro
    450                 455                 460

Val Asp Val Val Ala Asn Ala Tyr Arg Leu Ser Arg Glu Asp Ser Arg
465                 470                 475                 480

His Val Lys Phe Asn Arg Gly Asp Glu Met Ala Val Phe Ala Pro Arg
                485                 490                 495

Arg Gly Pro Gln Gln Tyr Ala Glu Trp Gln Ile Asn Glu Lys
            500                 505                 510


<210>  8
<211>  386
<212>  PRT
<213>  Pisum sativum

<400>  8

Met Ala Thr Thr Ile Lys Ser Arg Phe Pro Leu Leu Leu Leu Leu Gly
1               5                   10                  15

Ile Ile Phe Leu Ala Ser Val Val Ser Val Thr Tyr Ala Asn Tyr Asp
            20                  25                  30

Glu Gly Ser Glu Pro Arg Val Pro Ala Gln Arg Glu Arg Gly Arg Gln
        35                  40                  45

Glu Gly Glu Lys Glu Glu Lys Arg His Gly Glu Trp Arg Pro Ser Tyr
    50                  55                  60

Glu Lys Glu Glu Asp Glu Glu Glu Gly Gln Arg Glu Arg Gly Arg Gln
65                  70                  75                  80

Glu Gly Glu Lys Glu Glu Lys Arg His Gly Glu Trp Gly Pro Ser Tyr
                85                  90                  95

Glu Lys Gln Glu Asp Glu Glu Glu Lys Gln Lys Tyr Arg Tyr Gln Arg
            100                 105                 110

Glu Lys Glu Asp Glu Glu Glu Lys Gln Lys Tyr Gln Tyr Gln Arg Glu
        115                 120                 125

Lys Lys Glu Gln Lys Glu Val Gln Pro Gly Arg Glu Arg Trp Glu Arg
    130                 135                 140

Glu Glu Asp Glu Glu Gln Val Asp Glu Glu Trp Arg Gly Ser Gln Arg
145                 150                 155                 160

Arg Glu Asp Pro Glu Glu Arg Ala Arg Leu Arg His Arg Glu Glu Arg
                165                 170                 175

Thr Lys Arg Asp Arg Arg His Gln Arg Glu Gly Glu Glu Glu Glu Arg
            180                 185                 190

Ser Ser Glu Ser Gln Glu Arg Arg Asn Pro Phe Leu Phe Lys Ser Asn
        195                 200                 205

Lys Phe Leu Thr Leu Phe Glu Asn Glu Asn Gly His Ile Arg Leu Leu
    210                 215                 220

Gln Arg Phe Asp Lys Arg Ser Asp Leu Phe Glu Asn Leu Gln Asn Tyr
225                 230                 235                 240

Arg Leu Val Glu Tyr Arg Ala Lys Pro His Thr Ile Phe Leu Pro Gln
                245                 250                 255

His Ile Asp Ala Asp Leu Ile Leu Val Val Leu Ser Gly Lys Ala Ile
            260                 265                 270

Leu Thr Val Leu Ser Pro Asn Asp Arg Asn Ser Tyr Asn Leu Glu Arg
        275                 280                 285

Gly Asp Thr Ile Lys Leu Pro Ala Gly Thr Thr Ser Tyr Leu Val Asn
    290                 295                 300

Gln Asp Asp Glu Glu Asp Leu Arg Leu Val Asp Leu Val Ile Pro Val

305                 310                 315                 320

Asn Gly Pro Gly Lys Phe Glu Ala Phe Asp Leu Ala Lys Asn Lys Asn
                325                 330                 335

Gln Tyr Leu Arg Gly Phe Ser Lys Asn Ile Leu Glu Ala Ser Tyr Asn
            340                 345                 350

Thr Arg Tyr Glu Thr Ile Glu Lys Val Leu Leu Glu Glu Gln Glu Lys
        355                 360                 365

Asp Arg Lys Arg Arg Gln Gln Gly Glu Glu Thr Asp Ala Ile Val Lys
    370                 375                 380

Val Ser
385

<210>  9
<211>  386
<212>  PRT
<213>  Pisum sativum

<400>  9

Met Ala Thr Thr Ile Lys Ser Arg Phe Pro Leu Leu Leu Leu Leu Gly
1               5                   10                  15

Ile Ile Phe Leu Ala Ser Val Val Ser Val Thr Tyr Ala Asn Tyr Asp
            20                  25                  30

Glu Gly Ser Glu Pro Arg Val Pro Ala Gln Arg Glu Arg Gly Arg Gln
        35                  40                  45

Glu Gly Glu Lys Glu Glu Lys Arg His Gly Glu Trp Arg Pro Ser Tyr
    50                  55                  60

Glu Lys Glu Glu Asp Glu Glu Glu Gly Gln Arg Glu Arg Gly Arg Gln
65                  70                  75                  80

Glu Gly Glu Lys Glu Glu Lys Arg His Gly Glu Trp Gly Pro Ser Tyr
                85                  90                  95

Glu Lys Gln Glu Asp Glu Glu Glu Lys Gln Lys Tyr Arg Tyr Gln Arg
            100                 105                 110

Glu Lys Glu Asp Glu Glu Glu Lys Gln Lys Tyr Gln Tyr Gln Arg Glu
        115                 120                 125

Lys Lys Glu Gln Lys Glu Val Gln Pro Gly Arg Glu Arg Trp Glu Arg

130                 135                 140

Glu Glu Asp Glu Glu Gln Val Asp Glu Glu Trp Arg Gly Ser Gln Arg
145                 150                 155                 160

Arg Glu Asp Pro Glu Glu Arg Ala Arg Leu Arg His Arg Glu Glu Arg
                165                 170                 175

Thr Lys Arg Asp Arg Arg His Gln Arg Glu Gly Glu Glu Glu Glu Arg
            180                 185                 190

Ser Ser Glu Ser Gln Glu Arg Arg Asn Pro Phe Leu Phe Lys Ser Asn
        195                 200                 205

Lys Phe Leu Thr Leu Phe Glu Asn Glu Asn Gly His Ile Arg Leu Leu
    210                 215                 220

Gln Arg Phe Asp Lys Arg Ser Asp Leu Phe Glu Asn Leu Gln Asn Tyr
225                 230                 235                 240

Arg Leu Val Glu Tyr Arg Ala Lys Pro His Thr Ile Phe Leu Pro Gln
                245                 250                 255

His Ile Asp Ala Asp Leu Ile Leu Val Val Leu Ser Gly Lys Ala Ile
            260                 265                 270

Leu Thr Val Leu Ser Pro Asn Asp Arg Asn Ser Tyr Asn Leu Glu Arg
        275                 280                 285

Gly Asp Thr Ile Lys Leu Pro Ala Gly Thr Thr Ser Tyr Leu Val Asn
    290                 295                 300

Gln Asp Asp Glu Glu Asp Leu Arg Leu Val Asp Leu Val Ile Pro Val
305                 310                 315                 320

Asn Gly Pro Gly Lys Phe Glu Ala Phe Asp Leu Ala Lys Asn Lys Asn
                325                 330                 335

Gln Tyr Leu Arg Gly Phe Ser Lys Asn Ile Leu Glu Ala Ser Tyr Asn
            340                 345                 350

Thr Arg Tyr Glu Thr Ile Glu Lys Val Leu Leu Glu Glu Gln Glu Lys
        355                 360                 365

Asp Arg Lys Arg Arg Gln Gln Gly Glu Glu Thr Asp Ala Ile Val Lys
    370                 375                 380

Val Ser
385

<210> 10
<211> 499
<212> PRT
<213> Oryza sativa

<400> 10

Met Ala Ser Ser Val Phe Ser Arg Phe Ser Ile Tyr Phe Cys Val Leu
1               5                   10                  15

Leu Leu Cys His Gly Ser Met Ala Gln Leu Phe Asn Pro Ser Thr Asn
            20                  25                  30

Pro Trp His Ser Pro Arg Gln Gly Ser Phe Arg Glu Cys Arg Phe Asp
        35                  40                  45

Arg Leu Gln Ala Phe Glu Pro Leu Arg Lys Val Arg Ser Glu Ala Gly
    50                  55                  60

Val Thr Glu Tyr Phe Asp Glu Lys Asn Glu Leu Phe Gln Cys Thr Gly
65                  70                  75                  80

Thr Phe Val Ile Arg Arg Val Ile Gln Pro Gln Gly Leu Leu Val Pro
                85                  90                  95

Arg Tyr Thr Asn Ile Pro Gly Val Val Tyr Ile Ile Gln Gly Arg Gly
            100                 105                 110

Ser Met Gly Leu Thr Phe Pro Gly Cys Pro Ala Thr Tyr Gln Gln Gln
        115                 120                 125

Phe Gln Gln Phe Ser Ser Gln Gly Gln Ser Gln Ser Gln Lys Phe Arg
    130                 135                 140

Asp Glu His Gln Lys Ile His Gln Phe Arg Gln Gly Asp Ile Val Ala
145                 150                 155                 160

Leu Pro Ala Gly Val Ala His Trp Phe Tyr Asn Asp Gly Asp Ala Pro
                165                 170                 175

Ile Val Ala Val Tyr Val Tyr Asp Val Asn Asn Asn Ala Asn Gln Leu
            180                 185                 190

Glu Pro Arg Gln Lys Glu Phe Leu Leu Ala Gly Asn Asn Asn Arg Ala
        195                 200                 205

Gln Gln Gln Gln Val Tyr Gly Ser Ser Ile Glu Gln His Ser Gly Gln
    210                 215                 220

Asn Ile Phe Ser Gly Phe Gly Val Glu Met Leu Ser Glu Ala Leu Gly
225                 230                 235                 240

Ile Asn Ala Val Ala Ala Lys Arg Leu Gln Ser Gln Asn Asp Gln Arg
                245                 250                 255

Gly Glu Ile Ile His Val Lys Asn Gly Leu Gln Leu Leu Lys Pro Thr
            260                 265                 270

Leu Thr Gln Gln Gln Glu Gln Ala Gln Ala Gln Asp Gln Tyr Gln Gln
        275                 280                 285

Val Gln Tyr Ser Glu Arg Gln Gln Thr Ser Ser Arg Trp Asn Gly Leu
    290                 295                 300

Glu Glu Asn Phe Cys Thr Ile Lys Val Arg Val Asn Ile Glu Asn Pro
305                 310                 315                 320

Ser Arg Ala Asp Ser Tyr Asn Pro Arg Ala Gly Arg Ile Thr Ser Val
                325                 330                 335

Asn Ser Gln Lys Phe Pro Ile Leu Asn Leu Ile Gln Met Ser Ala Thr
            340                 345                 350

Arg Val Asn Leu Tyr Gln Asn Ala Ile Leu Ser Pro Phe Trp Asn Val
        355                 360                 365

Asn Ala His Ser Leu Val Tyr Met Ile Gln Gly Arg Ser Arg Val Gln
    370                 375                 380

Val Val Ser Asn Phe Gly Lys Thr Val Phe Asp Gly Val Leu Arg Pro
385                 390                 395                 400

Gly Gln Leu Leu Ile Ile Pro Gln His Tyr Ala Val Leu Lys Lys Ala
                405                 410                 415

Glu Arg Glu Gly Cys Gln Tyr Ile Ala Ile Lys Thr Asn Ala Asn Ala
            420                 425                 430

Phe Val Ser His Leu Ala Gly Lys Asn Ser Val Phe Arg Ala Leu Pro
        435                 440                 445

Val Asp Val Val Ala Asn Ala Tyr Arg Ile Ser Arg Glu Gln Ala Arg
    450                 455                 460

Ser Leu Lys Asn Asn Arg Gly Glu Glu His Gly Ala Phe Thr Pro Arg
465                 470                 475                 480

Phe Gln Gln Gln Tyr Tyr Pro Gly Leu Ser Asn Glu Ser Glu Ser Glu
                485                 490                 495

Thr Ser Glu

<210>  11
<211>  499
<212>  PRT
<213>  Oryza sativa

<400>  11

Met Ala Ser Ile Asn Arg Pro Ile Val Phe Phe Thr Val Cys Leu Phe
1               5                   10                  15

Leu Leu Cys Asn Gly Ser Leu Ala Gln Gln Leu Leu Gly Gln Ser Thr
            20                  25                  30

Ser Gln Trp Gln Ser Ser Arg Arg Gly Ser Pro Arg Glu Cys Arg Phe
        35                  40                  45

Asp Arg Leu Gln Ala Phe Glu Pro Ile Arg Ser Val Arg Ser Gln Ala
    50                  55                  60

Gly Thr Thr Glu Phe Phe Asp Val Ser Asn Glu Gln Phe Gln Cys Thr
65                  70                  75                  80

Gly Val Ser Val Val Arg Arg Val Ile Glu Pro Arg Gly Leu Leu Leu
                85                  90                  95

Pro His Tyr Thr Asn Gly Ala Ser Leu Val Tyr Ile Ile Gln Gly Arg
            100                 105                 110

Gly Ile Thr Gly Pro Thr Phe Pro Gly Cys Pro Glu Ser Tyr Gln Gln
        115                 120                 125

Gln Phe Gln Gln Ser Gly Gln Ala Gln Leu Thr Glu Ser Gln Ser Gln
    130                 135                 140

Ser Gln Lys Phe Lys Asp Glu His Gln Lys Ile His Arg Phe Arg Gln
145                 150                 155                 160

Gly Asp Val Ile Ala Leu Pro Ala Gly Val Ala His Trp Cys Tyr Asn
                165                 170                 175

Asp Gly Glu Val Pro Val Val Ala Ile Tyr Val Thr Asp Leu Asn Asn
            180                 185                 190

Gly Ala Asn Gln Leu Asp Pro Arg Gln Arg Asp Phe Leu Leu Ala Gly
        195                 200                 205

Asn Lys Arg Asn Pro Gln Ala Tyr Arg Arg Glu Val Glu Glu Arg Ser
    210                 215                 220

Gln Asn Ile Phe Ser Gly Phe Ser Thr Glu Leu Leu Ser Glu Ala Leu
225                 230                 235                 240

Gly Val Ser Ser Gln Val Ala Arg Gln Leu Gln Cys Gln Asn Asp Gln
                245                 250                 255

Arg Gly Glu Ile Val Arg Val Glu His Gly Leu Ser Leu Leu Gln Pro
            260                 265                 270

Tyr Ala Ser Leu Gln Glu Gln Glu Gln Gly Gln Val Gln Ser Arg Glu
        275                 280                 285

Arg Tyr Gln Glu Gly Gln Tyr Gln Gln Ser Gln Tyr Gly Ser Gly Cys
    290                 295                 300

Ser Asn Gly Leu Asp Glu Thr Phe Cys Thr Leu Arg Val Arg Gln Asn
305                 310                 315                 320

Ile Asp Asn Pro Asn Arg Ala Asp Thr Tyr Asn Pro Arg Ala Gly Arg
                325                 330                 335

Val Thr Asn Leu Asn Thr Gln Asn Phe Pro Ile Leu Ser Leu Val Gln
            340                 345                 350

Met Ser Ala Val Lys Val Asn Leu Tyr Gln Asn Ala Leu Leu Ser Pro
        355                 360                 365

Phe Trp Asn Ile Asn Ala His Ser Val Val Tyr Ile Thr Gln Gly Arg
    370                 375                 380

Ala Arg Val Gln Val Val Asn Asn Asn Gly Lys Thr Val Phe Asn Gly
385                 390                 395                 400

Glu Leu Arg Arg Gly Gln Leu Leu Ile Ile Pro Gln His Tyr Ala Val
                405                 410                 415

Val Lys Lys Ala Gln Arg Glu Gly Cys Ala Tyr Ile Ala Phe Lys Thr
            420                 425                 430

Asn Pro Asn Ser Met Val Ser His Ile Ala Gly Lys Ser Ser Ile Phe
        435                 440                 445

Arg Ala Leu Pro Asn Asp Val Leu Ala Asn Ala Tyr Arg Ile Ser Arg
    450                 455                 460

Glu Glu Ala Gln Arg Leu Lys His Asn Arg Gly Asp Glu Phe Gly Ala
465                 470                 475                 480

Phe Thr Pro Ile Gln Tyr Lys Ser Tyr Gln Asp Val Tyr Asn Ala Ala
                485                 490                 495

Glu Ser Ser

<210>  12
<211>  499
<212>  PRT
<213>  Oryza sativa

<400>  12

Met Ala Ser Ile Asn Arg Pro Ile Val Phe Phe Thr Val Cys Leu Phe
1               5                   10                  15

Leu Leu Cys Asp Gly Ser Leu Ala Gln Gln Leu Leu Gly Gln Ser Thr
            20                  25                  30

Ser Gln Trp Gln Ser Ser Arg Arg Gly Ser Pro Arg Gly Cys Arg Phe
        35                  40                  45

Asp Arg Leu Gln Ala Phe Glu Pro Ile Arg Ser Val Arg Ser Gln Ala
    50                  55                  60

Gly Thr Thr Glu Phe Phe Asp Val Ser Asn Glu Leu Phe Gln Cys Thr
65                  70                  75                  80

Gly Val Ser Val Val Arg Arg Val Ile Glu Pro Arg Gly Leu Leu Leu
                85                  90                  95

Pro His Tyr Thr Asn Gly Ala Ser Leu Val Tyr Ile Ile Gln Gly Arg
            100                 105                 110

Gly Ile Thr Gly Pro Thr Phe Pro Gly Cys Pro Glu Thr Tyr Gln Gln
        115                 120                 125

Gln Phe Gln Gln Ser Gly Gln Ala Gln Leu Thr Glu Ser Gln Ser Gln
    130                 135                 140

Ser His Lys Phe Lys Asp Glu His Gln Lys Ile His Arg Phe Arg Gln
145                 150                 155                 160

Gly Asp Val Ile Ala Leu Pro Ala Gly Val Ala His Trp Cys Tyr Asn
                165                 170                 175

Asp Gly Glu Val Pro Val Val Ala Ile Tyr Val Thr Asp Ile Asn Asn
            180                 185                 190

Gly Ala Asn Gln Leu Asp Pro Arg Gln Arg Asp Phe Leu Leu Ala Gly
        195                 200                 205

Asn Lys Arg Asn Pro Gln Ala Tyr Arg Arg Glu Val Glu Glu Trp Ser
    210                 215                 220

Gln Asn Ile Phe Ser Gly Phe Ser Thr Glu Leu Leu Ser Glu Ala Phe
225                 230                 235                 240

Gly Ile Ser Asn Gln Val Ala Arg Gln Leu Gln Cys Gln Asn Asp Gln
                245                 250                 255

Arg Gly Glu Ile Val Arg Val Glu Arg Gly Leu Ser Leu Leu Gln Pro
            260                 265                 270

Tyr Ala Ser Leu Gln Glu Gln Glu Gln Gly Gln Met Gln Ser Arg Glu
        275                 280                 285

His Tyr Gln Glu Gly Gly Tyr Gln Gln Ser Gln Tyr Gly Ser Gly Cys
    290                 295                 300

Pro Asn Gly Leu Asp Glu Thr Phe Cys Thr Met Arg Val Arg Gln Asn
305                 310                 315                 320

Ile Asp Asn Pro Asn Arg Ala Asp Thr Tyr Asn Pro Arg Ala Gly Arg
                325                 330                 335

Val Thr Asn Leu Asn Ser Gln Asn Phe Pro Ile Leu Asn Leu Val Gln
            340                 345                 350

Met Ser Ala Val Lys Val Asn Leu Tyr Gln Asn Ala Leu Leu Ser Pro
        355                 360                 365

Phe Trp Asn Ile Asn Ala His Ser Ile Val Tyr Ile Thr Gln Gly Arg
    370                 375                 380

Ala Gln Val Gln Val Val Asn Asn Asn Gly Lys Thr Val Phe Asn Gly

385                 390                 395                 400

Glu Leu Arg Arg Gly Gln Leu Leu Ile Val Pro Gln His Tyr Val Val
                405                 410                 415

Val Lys Lys Ala Gln Arg Glu Gly Cys Ala Tyr Ile Ala Phe Lys Thr
            420                 425                 430

Asn Pro Asn Ser Met Val Ser His Ile Ala Gly Lys Ser Ser Ile Phe
        435                 440                 445

Arg Ala Leu Pro Thr Asp Val Leu Ala Asn Ala Tyr Arg Ile Ser Arg
    450                 455                 460

Glu Glu Ala Gln Arg Leu Lys His Asn Arg Gly Asp Glu Phe Gly Ala
465                 470                 475                 480

Phe Thr Pro Leu Gln Tyr Lys Ser Tyr Gln Asp Val Tyr Asn Val Ala
                485                 490                 495

Glu Ser Ser

<210>  13
<211>  160
<212>  PRT
<213>  Oryza sativa

<400>  13

Met Ala Ser Asn Lys Val Val Phe Ser Val Leu Leu Leu Ala Val Val
1               5                   10                  15

Ser Val Leu Ala Ala Thr Ala Thr Met Ala Glu Tyr His His Gln Asp
            20                  25                  30

Gln Val Val Tyr Thr Pro Gly Pro Leu Cys Gln Pro Gly Met Gly Tyr
        35                  40                  45

Pro Met Tyr Pro Leu Pro Arg Cys Arg Ala Leu Val Lys Arg Gln Cys
    50                  55                  60

Val Gly Arg Gly Thr Ala Ala Ala Ala Glu Gln Val Arg Arg Asp Cys
65                  70                  75                  80

Cys Arg Gln Leu Ala Ala Val Asp Asp Ser Trp Cys Arg Cys Glu Ala
                85                  90                  95

Ile Ser His Met Leu Gly Gly Ile Tyr Arg Glu Leu Gly Ala Pro Asp

            100                 105                 110

Val Gly His Pro Met Ser Glu Val Phe Arg Gly Cys Arg Arg Gly Asp
        115                 120                 125

Leu Glu Arg Ala Ala Ala Ser Leu Pro Ala Phe Cys Asn Val Asp Ile
    130                 135                 140

Pro Asn Gly Gly Gly Gly Val Cys Tyr Trp Leu Ala Arg Ser Gly Tyr
145                 150                 155                 160

<210> 14
<211> 500
<212> PRT
<213> Oryza sativa

<400> 14
   Met Ala Thr Ile Ala Phe Ser Arg Leu Ser Ile Tyr Phe Cys Val Leu
   1 5 10 15
   Leu Leu Cys His Gly Ser Met Ala Gln Leu Phe Gly Pro Asn Val Asn
   20 25 30
   Pro Trp His Asn Pro Arg Gln Gly Gly Phe Arg Glu Cys Arg Phe Asp
   35 40 45
   Arg Leu Gln Ala Phe Glu Pro Leu Arg Arg Val Arg Ser Glu Ala Gly
   50 55 60
   Val Thr Glu Tyr Phe Asp Glu Lys Asn Glu Gln Phe Gln Cys Thr Gly
   65 70 75 80
   Thr Phe Val Ile Arg Arg Val Ile Glu Pro Gln Gly Leu Leu Val Pro
   85 90 95
   Arg Tyr Ser Asn Thr Pro Gly Met Val Tyr Ile Ile Gln Gly Arg Gly
   100 105 110
   Ser Met Gly Leu Thr Phe Pro Gly Cys Pro Ala Thr Tyr Gln Gln Gln
   115 120 125
   Phe Gln Gln Phe Leu Pro Glu Gly Gln Ser Gln Ser Gln Lys Phe Arg
   130 135 140
   Asp Glu His Gln Lys Ile His Gln Phe Arg Gln Gly Asp Ile Val Ala
   145 150 155 160
   Leu Pro Ala Gly Val Ala His Trp Phe Tyr Asn Glu Gly Asp Ala Pro
   165 170 175
   Val Val Ala Leu Tyr Val Phe Asp Leu Asn Asn Asn Ala Asn Gln Leu
   180 185 190
   Glu Pro Arg Gln Lys Glu Phe Leu Leu Ala Gly Asn Asn Asn Arg Glu
   195 200 205
   Gln Gln Met Tyr Gly Arg Ser Ile Glu Gln His Ser Gly Gln Asn Ile
   210 215 220
   Phe Ser Gly Phe Asn Asn Glu Leu Leu Ser Glu Ala Leu Gly Val Asn
   225 230 235 240
   Ala Leu Val Ala Lys Arg Leu Gln Gly Gln Asn Asp Gln Arg Gly Glu
   245 250 255
   Ile Ile Arg Val Lys Asn Gly Leu Lys Leu Leu Arg Pro Ala Phe Ala
   260 265 270
   Gln Gln Gln Glu Gln Ala Gln Gln Gln Glu Gln Ala Gln Ala Gln Tyr
   275 280 285
   Gln Val Gln Tyr Ser Glu Glu Gln Gln Pro Ser Thr Arg Cys Asn Gly
   290 295 300
   Leu Asp Glu Asn Phe Cys Thr Ile Lys Ala Arg Leu Asn Ile Glu Asn
   305 310 315 320
   Pro Ser His Ala Asp Thr Tyr Asn Pro Arg Ala Gly Arg Ile Thr Arg
   325 330 335
   Leu Asn Ser Gln Lys Phe Pro Ile Leu Asn Leu Val Gln Leu Ser Ala
   340 345 350
   Thr Arg Val Asn Leu Tyr Gln Asn Ala Ile Leu Ser Pro Phe Trp Asn
   355 360 365
   Val Asn Ala His Ser Leu Val Tyr Ile Val Gln Gly His Ala Arg Val
   370 375 380
   Gln Val Val Ser Asn Leu Gly Lys Thr Val Phe Asn Gly Val Leu Arg
   385 390 395 400
   Pro Gly Gln Leu Leu Ile Ile Pro Gln His Tyr Val Val Leu Lys Lys
   405 410 415
   Ala Glu His Glu Gly Cys Gln Tyr Ile Ser Phe Lys Thr Asn Ala Asn
               420                 425                 430
   Ser Met Val Ser His Leu Ala Gly Lys Asn Ser Ile Phe Arg Ala Met
           435                 440                 445
   Pro Val Asp Val Ile Ala Asn Ala Tyr Arg Ile Ser Arg Glu Gln Ala
       450                 455                 460
   Arg Ser Leu Lys Asn Asn Arg Gly Glu Glu Leu Gly Ala Phe Thr Pro
   465                 470                 475                 480
   Arg Tyr Gln Gln Gln Thr Tyr Pro Gly Phe Ser Asn Glu Ser Glu Asn
                   485                 490                 495
   Glu Ala Leu Glu
               500

<210> 15
<211> 609
<212> PRT
<213> Oryza sativa

<400> 15

Met Ser Ala Leu Thr Thr Ser Gln Leu Ala Thr Ser Ala Thr Gly Phe
1               5                   10                  15

Gly Ile Ala Asp Arg Ser Ala Pro Ser Ser Leu Leu Arg His Gly Phe
            20                  25                  30

Gln Gly Leu Lys Pro Arg Ser Pro Ala Gly Gly Asp Ala Thr Ser Leu
        35                  40                  45

Ser Val Thr Thr Ser Ala Arg Ala Thr Pro Lys Gln Gln Arg Ser Val
    50                  55                  60

Gln Arg Gly Ser Arg Arg Phe Pro Ser Val Val Val Tyr Ala Thr Gly
65                  70                  75                  80

Ala Gly Met Asn Val Val Phe Val Gly Ala Glu Met Ala Pro Trp Ser
                85                  90                  95

Lys Thr Gly Gly Leu Gly Asp Val Leu Gly Gly Leu Pro Pro Ala Met
            100                 105                 110

Ala Ala Asn Gly His Arg Val Met Val Ile Ser Pro Arg Tyr Asp Gln
        115                 120                 125

Tyr Lys Asp Ala Trp Asp Thr Ser Val Val Ala Glu Ile Lys Val Ala
    130                 135                 140

Asp Arg Tyr Glu Arg Val Arg Phe Phe His Cys Tyr Lys Arg Gly Val
145                 150                 155                 160

Asp Arg Val Phe Ile Asp His Pro Ser Phe Leu Glu Lys Val Trp Gly
                165                 170                 175

Lys Thr Gly Glu Lys Ile Tyr Gly Pro Asp Thr Gly Val Asp Tyr Lys
            180                 185                 190

Asp Asn Gln Met Arg Phe Ser Leu Leu Cys Gln Ala Ala Leu Glu Ala
        195                 200                 205

Pro Arg Ile Leu Asn Leu Asn Asn Asn Pro Tyr Phe Lys Gly Thr Tyr
    210                 215                 220

Gly Glu Asp Val Val Phe Val Cys Asn Asp Trp His Thr Gly Pro Leu
225                 230                 235                 240

Ala Ser Tyr Leu Lys Asn Asn Tyr Gln Pro Asn Gly Ile Tyr Arg Asn
                245                 250                 255

Ala Lys Val Ala Phe Cys Ile His Asn Ile Ser Tyr Gln Gly Arg Phe
            260                 265                 270

Ala Phe Glu Asp Tyr Pro Glu Leu Asn Leu Ser Glu Arg Phe Arg Ser
        275                 280                 285

Ser Phe Asp Phe Ile Asp Gly Tyr Asp Thr Pro Val Glu Gly Arg Lys
    290                 295                 300

Ile Asn Trp Met Lys Ala Gly Ile Leu Glu Ala Asp Arg Val Leu Thr
305                 310                 315                 320

Val Ser Pro Tyr Tyr Ala Glu Glu Leu Ile Ser Gly Ile Ala Arg Gly
                325                 330                 335

Cys Glu Leu Asp Asn Ile Met Arg Leu Thr Gly Ile Thr Gly Ile Val
            340                 345                 350

Asn Gly Met Asp Val Ser Glu Trp Asp Pro Ser Lys Asp Lys Tyr Ile
        355                 360                 365

Thr Ala Lys Tyr Asp Ala Thr Thr Ala Ile Glu Ala Lys Ala Leu Asn
    370                 375                 380

Lys Glu Ala Leu Gln Ala Glu Ala Gly Leu Pro Val Asp Arg Lys Ile
385                 390                 395                 400

Pro Leu Ile Ala Phe Ile Gly Arg Leu Glu Glu Gln Lys Gly Pro Asp
                405                 410                 415

Val Met Ala Ala Ala Ile Pro Glu Leu Met Gln Glu Asp Val Gln Ile
            420                 425                 430

Val Leu Leu Gly Thr Gly Lys Lys Lys Phe Glu Lys Leu Leu Lys Ser
        435                 440                 445

Met Glu Glu Lys Tyr Pro Gly Lys Val Arg Ala Val Val Lys Phe Asn
    450                 455                 460

Ala Pro Leu Ala His Leu Ile Met Ala Gly Ala Asp Val Leu Ala Val
465                 470                 475                 480

Pro Ser Arg Phe Glu Pro Cys Gly Leu Ile Gln Leu Gln Gly Met Arg
                485                 490                 495

Tyr Gly Thr Pro Cys Ala Cys Ala Ser Thr Gly Gly Leu Val Asp Thr
            500                 505                 510

Val Ile Glu Gly Lys Thr Gly Phe His Met Gly Arg Leu Ser Val Asp
        515                 520                 525

Cys Lys Val Val Glu Pro Ser Asp Val Lys Lys Val Ala Ala Thr Leu
    530                 535                 540

Lys Arg Ala Ile Lys Val Val Gly Thr Pro Ala Tyr Glu Glu Met Val
545                 550                 555                 560

Arg Asn Cys Met Asn Gln Asp Leu Ser Trp Lys Gly Pro Ala Lys Asn
                565                 570                 575

Trp Glu Asn Val Leu Leu Gly Leu Gly Val Ala Gly Ser Ala Pro Gly
            580                 585                 590

Ile Glu Gly Asp Glu Ile Ala Pro Leu Ala Lys Glu Asn Val Ala Ala
        595                 600                 605

Pro

<210>  16
<211>  336

<212>  PRT
<213>  Staphylococcus aureus

<400>  16

Met Lys Gly Lys Phe Leu Lys Val Ser Ser Leu Phe Val Ala Thr Leu
1               5                   10                  15

Thr Thr Ala Thr Leu Val Ser Ser Pro Ala Ala Asn Ala Leu Ser Ser
            20                  25                  30

Lys Ala Met Asp Asn His Pro Gln Gln Thr Gln Ser Ser Lys Gln Gln
        35                  40                  45

Thr Pro Lys Ile Gln Lys Gly Gly Asn Leu Lys Pro Leu Glu Gln Arg
    50                  55                  60

Glu His Ala Asn Val Ile Leu Pro Asn Asn Asp Arg His Gln Ile Thr
65                  70                  75                  80

Asp Thr Thr Asn Gly His Tyr Ala Pro Val Thr Tyr Ile Gln Val Glu
                85                  90                  95

Ala Pro Thr Gly Thr Phe Ile Ala Ser Gly Val Val Val Gly Lys Asp
            100                 105                 110

Thr Leu Leu Thr Asn Lys His Val Val Asp Ala Thr His Gly Asp Pro
        115                 120                 125

His Ala Leu Lys Ala Phe Pro Ser Ala Ile Asn Gln Asp Asn Tyr Pro
    130                 135                 140

Asn Gly Gly Phe Thr Ala Glu Gln Ile Thr Lys Tyr Ser Gly Glu Gly
145                 150                 155                 160

Asp Leu Ala Ile Val Lys Phe Ser Pro Asn Glu Gln Asn Lys His Ile
                165                 170                 175

Gly Glu Val Val Lys Pro Ala Thr Met Ser Asn Asn Ala Glu Thr Gln
            180                 185                 190

Val Asn Gln Asn Ile Thr Val Thr Gly Tyr Pro Gly Asp Lys Pro Val
        195                 200                 205

Ala Thr Met Trp Glu Ser Lys Gly Lys Ile Thr Tyr Leu Lys Gly Glu
    210                 215                 220

Ala Met Gln Tyr Asp Leu Ser Thr Thr Gly Gly Asn Ser Gly Ser Pro
225                 230                 235                 240

Val Phe Asn Glu Lys Asn Glu Val Ile Gly Ile His Trp Gly Gly Val
                245                 250                 255

Pro Asn Glu Phe Asn Gly Ala Val Phe Ile Asn Glu Asn Val Arg Asn
            260                 265                 270

Phe Leu Lys Gln Asn Ile Glu Asp Ile His Phe Ala Asn Asp Asp Gln
        275                 280                 285

Pro Asn Asn Pro Asp Asn Pro Asp Asn Pro Asn Asn Pro Asp Asn Pro
    290                 295                 300

Asn Asn Pro Asp Glu Pro Asn Asn Pro Asp Asn Pro Asn Asn Pro Asp
305                 310                 315                 320

Asn Pro Asp Asn Gly Asp Asn Asn Asn Ser Asp Asn Pro Asp Ala Ala
                325                 330                 335

<210>  17
<211>  22
<212>  PRT
<213>  Artificial Sequence

<220>
<223>  FRAGMENT OF SEQ ID 1

<400>  17

Pro Phe Asn Leu Arg Ser Arg Gly Pro Ile Tyr Ser Asn Glu Phe Gly
1               5                   10                  15

Lys Phe Phe Glu Ile Thr
            20

<210>  18
<211>  17
<212>  PRT
<213>  Artificial Sequence

<220>
<223>  FRAGMENT OF SEQ ID 1

<400>  18

Lys Pro Asp Asp Arg Asn Ser Phe Asn Leu Glu Arg Gly Asp Thr Ile
1               5                   10                  15

Lys

<210>  19

<211>  27
<212>  PRT
<213>  Artificial Sequence

<220>
<223>  FRAGMENT OF SEQ ID 1

<400>  19

Tyr Leu Val Asn Arg Asp Asp Asn Glu Glu Leu Arg Val Leu Asp Leu
1               5                   10                  15

Ala Ile Pro Val Asn Arg Pro Gly Gln Leu Gln
            20                  25

<210>  20
<211>  51
<212>  PRT
<213>  Artificial Sequence

<220>
<223>  FRAGMENT OF SEQ ID 1

<400>  20

Gln Glu Gln Arg Lys Glu Asp Asp Glu Glu Glu Glu Gln Gly Glu Glu
1               5                   10                  15

Glu Ile Asn Lys Gln Val Gln Asn Tyr Lys Ala Lys Leu Ser Ser Gly
            20                  25                  30

Asp Val Phe Val Ile Pro Ala Gly His Pro Val Ala Val Lys Ala Ser
        35                  40                  45

Ser Asn Leu
    50

<210>  21
<211>  68
<212>  PRT
<213>  Artificial Sequence

<220>
<223>  FRAGMENT OF SEQ ID 1

<400>  21

Ser Gly Phe Ser Lys Asn Ile Leu Glu Ala Ser Phe Asn Thr Asp Tyr
1               5                   10                  15

Glu Glu Ile Glu Lys Val Leu Leu Glu Glu His Glu Lys Glu Thr Gln
            20                  25                  30

His Arg Arg Ser Leu Lys Asp Lys Arg Gln Gln Ser Gln Glu Glu Asn
        35                  40                  45

Val Ile Val Lys Leu Ser Arg Gly Gln Ile Glu Glu Leu Ser Lys Asn
    50                  55                  60

Ala Lys Ser Thr
65

<210>  22
<211>  74
<212>  PRT
<213>  Artificial Sequence

<220>
<223>  FRAGMENT OF SEQ ID 1

<400>  22

Pro Phe Ile Phe Lys Ser Asn Lys Phe Gln Thr Leu Phe Glu Asn Glu
1               5                   10                  15

Asn Gly His Ile Arg Leu Leu Gln Lys Phe Asp Gln Arg Ser Lys Ile
            20                  25                  30

Phe Glu Asn Leu Gln Asn Tyr Arg Leu Leu Glu Tyr Lys Ser Lys Pro
        35                  40                  45

His Thr Ile Phe Leu Pro Gln His Thr Asp Ala Asp Tyr Ile Leu Val
    50                  55                  60

Val Leu Ser Gly Lys Ala Ile Leu Thr Val
65                  70

<210>  23
<211>  28
<212>  PRT
<213>  Artificial Sequence

<220>
<223>  FRAGMENT OF SEQ ID 1

<400>  23

Gln Glu Val Asp Arg Ile Leu Glu Asn Gln Lys Gln Ser His Phe Ala
1               5                   10                  15

Asp Ala Gln Pro Gln Gln Arg Glu Arg Gly Ser Arg
            20                  25

<210>  24
<211>  35
<212>  PRT
<213>  Artificial Sequence

<220>
<223>  FRAGMENT OF SEQ ID 2

<400>  24

Asn Asn Ser Gly Lys Phe Phe Glu Leu Thr Pro Glu Lys Asn Gln Gln
1               5                   10                  15

Leu Gln Asp Leu Asp Leu Phe Val Asn Ser Val Asp Leu Lys Glu Gly
            20                  25                  30

Ser Leu Leu
        35

<210>  25
<211>  17
<212>  PRT
<213>  Artificial Sequence

<220>
<223>  FRAGMENT OF SEQ ID 2

<400>  25

Lys Val Ser Arg Arg Gln Leu Glu Glu Leu Ser Lys Asn Ala Lys Ser
1               5                   10                  15

Ser

<210>  26
<211>  21
<212>  PRT
<213>  Artificial Sequence

<220>
<223>  FRAGMENT OF SEQ ID 3

<400>  26

Gln Arg Gly Ser Arg Gln Glu Glu Asp Glu Asp Glu Asp Glu Glu Arg
1               5                   10                  15

Gln Pro Arg His Gln
            20

<210>  27
<211>  33
<212>  PRT
<213>  Artificial Sequence

<220>
<223>  FRAGMENT OF SEQ ID 3

<400>  27

Gln Glu Phe Leu Arg Tyr Gln His Gln Gln Gly Gly Lys Gln Glu Gln
1               5                   10                  15

Glu Asn Glu Gly Asn Asn Ile Phe Ser Gly Phe Lys Arg Asp Phe Leu
            20                  25                  30

Glu

<210>  28
<211>  34
<212>  PRT
<213>  Artificial Sequence

<220>
<223>  FRAGMENT OF SEQ ID 4

<400>  28

Tyr Asn Leu Glu Arg Gly Asp Thr Ile Lys Leu Pro Ala Gly Thr Thr
1               5                   10                  15

Ser Tyr Leu Val Asn Gln Asp Asp Glu Glu Asp Leu Arg Leu Val Asp
            20                  25                  30

Leu Val

<210>  29
<211>  80
<212>  PRT
<213>  Artificial Sequence

<220>
<223>  FRAGMENT OF SEQ ID 4

<400>  29

Asn Gly Pro Gly Lys Phe Glu Ala Phe Asp Leu Ala Lys Asn Lys Asn
1               5                   10                  15

Gln Tyr Leu Arg Gly Phe Ser Lys Asn Ile Leu Glu Ala Ser Tyr Asn
            20                  25                  30

Thr Arg Tyr Glu Thr Ile Glu Lys Val Leu Leu Glu Glu Gln Glu Lys
        35                  40                  45

Asp Arg Lys Arg Arg Gln Gln Gly Glu Glu Thr Asp Ala Ile Val Lys
    50                  55                  60

Val Ser Arg Glu Gln Ile Glu Glu Leu Lys Lys Leu Ala Lys Ser Ser
65                  70                  75                  80

<210>  30
<211>  47
<212>  PRT
<213>  Artificial Sequence

<220>
<223>  FRAGMENT OF SEQ ID 4

<400>  30

Arg Tyr Glu Ala Arg Leu Ser Pro Gly Asp Val Val Ile Ile Pro Ala
1               5                   10                  15

Gly His Pro Val Ala Ile Thr Ala Ser Ser Asn Leu Asn Leu Leu Gly
            20                  25                  30

Phe Gly Ile Asn Ala Glu Asn Asn Glu Arg Asn Phe Leu Ser Gly
        35                  40                  45

<210>  31
<211>  24
<212>  PRT
<213>  Artificial Sequence

<220>
<223>  FRAGMENT OF SEQ ID 4

<400>  31

His Gly Glu Trp Arg Pro Ser Tyr Glu Lys Gln Glu Asp Glu Glu Glu
1               5                   10                  15

Lys Gln Lys Tyr Arg Tyr Gln Arg
            20

<210>  32
<211>  19
<212>  PRT
<213>  Artificial Sequence

<220>
<223>  FRAGMENT OF SEQ ID 4

<400>  32

Pro Ser Tyr Glu Lys Glu Glu Asp Glu Glu Glu Gly Gln Arg Glu Arg
1               5                   10                  15

Gly Arg Gln

<210>  33
<211>  30
<212>  PRT
<213>  Artificial Sequence

<220>
<223>  FRAGMENT OF SEQ ID 4

<400>  33

Lys Ser Lys Pro Arg Thr Leu Phe Leu Pro Gln Tyr Thr Asp Ala Asp
1               5                   10                  15

Phe Ile Leu Val Val Leu Ser Gly Lys Ala Thr Leu Thr Val
            20                  25                  30

<210>  34
<211>  22
<212>  PRT
<213>  Artificial Sequence

<220>
<223>  FRAGMENT OF SEQ ID 10

<400>  34

Ser Gln Ser Gln Lys Phe Arg Asp Glu His Gln Lys Ile His Gln Phe
1               5                   10                  15

Arg Gln Gly Asp Ile Val
            20

<210>  35
<211>  23
<212>  PRT
<213>  Artificial Sequence

<220>
<223>  FRAGMENT OF SEQ ID 10

<400>  35

Val Asn Ser Gln Lys Phe Pro Ile Leu Asn Leu Ile Gln Met Ser Ala
1               5                   10                  15

Thr Arg Val Asn Leu Tyr Gln
            20

<210>  36
<211>  40
<212>  PRT
<213>  Artificial Sequence

<220>
<223>  FRAGMENT OF SEQ ID 10

<400>  36

Tyr Ile Ala Ile Lys Thr Asn Ala Asn Ala Phe Val Ser His Leu Ala
1               5                   10                  15

Gly Lys Asn Ser Val Phe Arg Ala Leu Pro Val Asp Val Val Ala Asn
            20                  25                  30

Ala Tyr Arg Ile Ser Arg Glu Gln
        35                  40

<210>  37
<211>  31
<212>  PRT
<213>  Artificial Sequence

<220>
<223>  FRAGMENT OF SEQ ID 10

<400>  37

Ala Pro Ile Val Ala Val Tyr Val Tyr Asp Val Asn Asn Asn Ala Asn
1               5                   10                  15

Gln Leu Glu Pro Arg Gln Lys Glu Phe Leu Leu Ala Gly Asn Asn
            20                  25                  30

<210>  38
<211>  17
<212>  PRT
<213>  Artificial Sequence

<220>
<223>  FRAGMENT OF SEQ ID 10

<400>  38

Glu Asn Pro Ser Arg Ala Asp Ser Tyr Asn Pro Arg Ala Gly Arg Ile
1               5                   10                  15

Thr

<210>  39
<211>  32
<212>  PRT
<213>  Artificial Sequence

<220>
<223>  FRAGMENT OF SEQ ID 10

<400>  39

Gly Leu Gln Leu Leu Lys Pro Thr Leu Thr Gln Gln Gln Glu Gln Ala
1               5                   10                  15

Gln Ala Gln Asp Gln Tyr Gln Gln Val Gln Tyr Ser Glu Arg Gln Gln
            20                  25                  30

<210>  40
<211>  18
<212>  PRT
<213>  Artificial Sequence

<220>
<223>  FRAGMENT OF SEQ ID 10

<400>  40

Cys Arg Phe Asp Arg Leu Gln Ala Phe Glu Pro Leu Arg Lys Val Arg
1               5                   10                  15

Ser Glu

<210>  41
<211>  48
<212>  PRT
<213>  Artificial Sequence

<220>
<223>  FRAGMENT OF SEQ ID 10

<400>  41

Ile Leu Ser Pro Phe Trp Asn Val Asn Ala His Ser Leu Val Tyr Met
1               5                   10                  15

Ile Gln Gly Arg Ser Arg Val Gln Val Val Ser Asn Phe Gly Lys Thr
            20                  25                  30

Val Phe Asp Gly Val Leu Arg Pro Gly Gln Leu Leu Ile Ile Pro Gln
        35                  40                  45

<210>  42
<211>  24
<212>  PRT
<213>  Artificial Sequence

<220>
<223>  FRAGMENT OF SEQ ID 10

<400>  42

Ala Phe Thr Pro Arg Phe Gln Gln Gln Tyr Tyr Pro Gly Leu Ser Asn
1               5                   10                  15

Glu Ser Glu Ser Glu Thr Ser Glu
            20

<210>  43
<211>  31
<212>  PRT
<213>  Artificial Sequence

<220>
<223>  FRAGMENT OF SEQ ID 11

<400>  43

Pro Arg Ala Gly Arg Val Thr Asn Leu Asn Thr Gln Asn Phe Pro Ile
1               5                   10                  15

Leu Ser Leu Val Gln Met Ser Ala Val Lys Val Asn Leu Tyr Gln
            20                  25                  30

<210>  44
<211>  32
<212>  PRT
<213>  Artificial Sequence

<220>
<223>  FRAGEMENT OF SEQ ID 11

<400>  44

His Ser Val Val Tyr Ile Thr Gln Gly Arg Ala Arg Val Gln Val Val
1               5                   10                  15

Asn Asn Asn Gly Lys Thr Val Phe Asn Gly Glu Leu Arg Arg Gly Gln
            20                  25                  30

<210>  45
<211>  32
<212>  PRT
<213>  Artificial Sequence

<220>
<223>  FRAGMENT OF SEQ ID 11

<400>  45

His Ile Ala Gly Lys Ser Ser Ile Phe Arg Ala Leu Pro Asn Asp Val
1               5                   10                  15

Leu Ala Asn Ala Tyr Arg Ile Ser Arg Glu Glu Ala Gln Arg Leu Lys
            20                  25                  30

<210>  46
<211>  29
<212>  PRT
<213>  Artificial Sequence

<220>
<223>  FRAGMENT OF SEQ ID 11

<400>  46

Val Pro Val Val Ala Ile Tyr Val Thr Asp Leu Asn Asn Gly Ala Asn
1               5                   10                  15

Gln Leu Asp Pro Arg Gln Arg Asp Phe Leu Leu Ala Gly
            20                  25

<210>  47
<211>  52
<212>  PRT
<213>  Artificial Sequence

<220>
<223>  FRAGMENT OF SEQ ID 12

<400>  47

Thr Phe Cys Thr Met Arg Val Arg Gln Asn Ile Asp Asn Pro Asn Arg
1               5                   10                  15

Ala Asp Thr Tyr Asn Pro Arg Ala Gly Arg Val Thr Asn Leu Asn Ser
            20                  25                  30

Gln Asn Phe Pro Ile Leu Asn Leu Val Gln Met Ser Ala Val Lys Val
        35                  40                  45

Asn Leu Tyr Gln
    50

<210>  48
<211>  73
<212>  PRT
<213>  Artificial Sequence

<220>
<223>  FRAGMENT OF SEQ ID 12

<400>  48

Tyr Ile Ala Phe Lys Thr Asn Pro Asn Ser Met Val Ser His Ile Ala
1               5                   10                  15

Gly Lys Ser Ser Ile Phe Arg Ala Leu Pro Thr Asp Val Leu Ala Asn
            20                  25                  30

Ala Tyr Arg Ile Ser Arg Glu Glu Ala Gln Arg Leu Lys His Asn Arg
        35                  40                  45

Gly Asp Glu Phe Gly Ala Phe Thr Pro Leu Gln Tyr Lys Ser Tyr Gln
    50                  55                  60

Asp Val Tyr Asn Val Ala Glu Ser Ser
65                  70

<210>  49
<211>  36
<212>  PRT

<213>  Artificial Sequence

<220>
<223>  FRAGMENT OF SEQ ID 12

<400>  49

Ser Gln Ser His Lys Phe Lys Asp Glu His Gln Lys Ile His Arg Phe
1               5                   10                  15

Arg Gln Gly Asp Val Ile Ala Leu Pro Ala Gly Val Ala His Trp Cys
            20                  25                  30

Tyr Asn Asp Gly
        35

<210>  50
<211>  29
<212>  PRT
<213>  Artificial Sequence

<220>
<223>  FRAGMENT OF SEQ ID 12

<400>  50

Thr Gln Gly Arg Ala Gln Val Gln Val Val Asn Asn Asn Gly Lys Thr
1               5                   10                  15

Val Phe Asn Gly Glu Leu Arg Arg Gly Gln Leu Leu Ile
            20                  25

<210>  51
<211>  24
<212>  PRT
<213>  Artificial Sequence

<220>
<223>  FRAGMENT OF SEQ ID 12

<400>  51

Asn Gly Ala Ser Leu Val Tyr Ile Ile Gln Gly Arg Gly Ile Thr Gly
1               5                   10                  15

Pro Thr Phe Pro Gly Cys Pro Glu
            20

<210>  52
<211>  21
<212>  PRT
<213>  Artificial Sequence

<220>
<223>  FRAGMENT OF SEQ ID 12

<400>  52

Cys Arg Phe Asp Arg Leu Gln Ala Phe Glu Pro Ile Arg Ser Val Arg
1               5                   10                  15

Ser Gln Ala Gly Thr
            20

<210>  53
<211>  40
<212>  PRT
<213>  Artificial Sequence

<220>
<223>  FRAGMENT OF SEQ ID 12

<400>  53

Gln Asn Asp Gln Arg Gly Glu Ile Val Arg Val Glu Arg Gly Leu Ser
1               5                   10                  15

Leu Leu Gln Pro Tyr Ala Ser Leu Gln Glu Gln Glu Gln Gly Gln Met
            20                  25                  30

Gln Ser Arg Glu His Tyr Gln Glu
        35                  40

<210>  54
<211>  35
<212>  PRT
<213>  Artificial Sequence

<220>
<223>  FRAGMENT OF SEQ ID 12

<400>  54

Pro Arg Gln Arg Asp Phe Leu Leu Ala Gly Asn Lys Arg Asn Pro Gln
1               5                   10                  15

Ala Tyr Arg Arg Glu Val Glu Glu Trp Ser Gln Asn Ile Phe Ser Gly
            20                  25                  30

Phe Ser Thr
        35

<210>  55
<211>  23
<212>  PRT
<213>  Artificial Sequence

<220>
<223>  FRAGMENT OF SEQ ID 13

<400>  55

Gly Gly Ile Tyr Arg Glu Leu Gly Ala Pro Asp Val Gly His Pro Met
1               5                   10                  15

Ser Glu Val Phe Arg Gly Cys
            20

<210>  56
<211>  26
<212>  PRT
<213>  Artificial Sequence

<220>
<223>  FRAGMENT OF SEQ ID 14

<400>  56

His Ser Leu Val Tyr Ile Val Gln Gly His Ala Arg Val Gln Val Val
1               5                   10                  15

Ser Asn Leu Gly Lys Thr Val Phe Asn Gly
            20                  25

<210>  57
<211>  23
<212>  PRT
<213>  Artificial Sequence

<220>
<223>  FRAGMENT OF SEQ ID 14

<400>  57

Ala Arg Ser Leu Lys Asn Asn Arg Gly Glu Glu Leu Gly Ala Phe Thr
1               5                   10                  15

Pro Arg Tyr Gln Gln Gln Thr
            20

<210>  58
<211>  23
<212>  PRT
<213>  Artificial Sequence

<220>
<223>  FRAGMENT OF SEQ ID 14

<400>  58

Leu Asn Ser Gln Lys Phe Pro Ile Leu Asn Leu Val Gln Leu Ser Ala
1               5                   10                  15

Thr Arg Val Asn Leu Tyr Gln
            20

<210>  59
<211>  84
<212>  PRT
<213>  Artificial Sequence

<220>
<223>  FRAGMENT OF SEQ ID 14

<400>  59
   Gln Met Tyr Gly Arg Ser Ile Glu Gln His Ser Gly Gln Asn Ile Phe
   1               5                   10                  15
   Ser Gly Phe Asn Asn Glu Leu Leu Ser Glu Ala Leu Gly Val Asn Ala
               20                  25                  30
   Leu Val Ala Lys Arg Leu Gln Gly Gln Asn Asp Gln Arg Gly Glu Ile
           35                  40                  45
   Ile Arg Val Lys Asn Gly Leu Lys Leu Leu Arg Pro Ala Phe Ala Gln
       50                  55                  60
   Gln Gln Glu Gln Ala Gln Gln Gln Glu Gln Ala Gln Ala Gln Tyr Gln
   65                  70                  75                  80
   Val Gln Tyr Ser

<210>  60
<211>  24
<212>  PRT
<213>  Artificial Sequence

<220>
<223>  FRAGMENT IF SEQ ID 14

<400>  60

Ala Gln Leu Phe Gly Pro Asn Val Asn Pro Trp His Asn Pro Arg Gln
1               5                   10                  15

Gly Gly Phe Arg Glu Cys Arg Phe
            20

<210>  61
<211>  23
<212>  PRT
<213>  Artificial Sequence

<220>
<223>  FRAGMENT OF SEQ ID 14

<400>  61

Gly Val Ala His Trp Phe Tyr Asn Glu Gly Asp Ala Pro Val Val Ala

1               5                   10                  15

Leu Tyr Val Phe Asp Leu Asn
            20

<210>  62
<211>  40
<212>  PRT
<213>  Artificial Sequence

<220>
<223>  FRAGMENT OF SEQ ID 14

<400>  62

Tyr Ile Ser Phe Lys Thr Asn Ala Asn Ser Met Val Ser His Leu Ala
1               5                   10                  15

Gly Lys Asn Ser Ile Phe Arg Ala Met Pro Val Asp Val Ile Ala Asn
            20                  25                  30

Ala Tyr Arg Ile Ser Arg Glu Gln
        35                  40

<210>  63
<211>  38
<212>  PRT
<213>  Artificial Sequence

<220>
<223>  FRAGMENT OF SEQ ID 15

<400>  63

Lys Gly Pro Ala Lys Asn Trp Glu Asn Val Leu Leu Gly Leu Gly Val
1               5                   10                  15

Ala Gly Ser Ala Pro Gly Ile Glu Gly Asp Glu Ile Ala Pro Leu Ala
            20                  25                  30

Lys Glu Asn Val Ala Ala
        35

<210>  64
<211>  31
<212>  PRT
<213>  Artificial Sequence

<220>
<223>  FRAGMENT OF SEQ ID 15

<400>  64

Arg Ala Val Val Lys Phe Asn Ala Pro Leu Ala His Leu Ile Met Ala
1               5                   10                  15

Gly Ala Asp Val Leu Ala Val Pro Ser Arg Phe Glu Pro Cys Gly
            20                  25                  30

<210>  65
<211>  22
<212>  PRT
<213>  Artificial Sequence

<220>
<223>  FRAGMENT OF SEQ ID 15

<400>  65

Lys Arg Ala Ile Lys Val Val Gly Thr Pro Ala Tyr Glu Glu Met Val
1               5                   10                  15

Arg Asn Cys Met Asn Gln
            20

<210>  66
<211>  55
<212>  PRT
<213>  Artificial Sequence

<220>
<223>  FRAGMENT OF SEQ ID 15

<400>  66

Ala Pro Trp Ser Lys Thr Gly Gly Leu Gly Asp Val Leu Gly Gly Leu
1               5                   10                  15

Pro Pro Ala Met Ala Ala Asn Gly His Arg Val Met Val Ile Ser Pro
            20                  25                  30

Arg Tyr Asp Gln Tyr Lys Asp Ala Trp Asp Thr Ser Val Val Ala Glu
        35                  40                  45

Ile Lys Val Ala Asp Arg Tyr
    50                  55

<210>  67
<211>  109
<212>  PRT
<213>  Artificial Sequence

<220>
<223>  FRAGMENT OF SEQ ID 15

<400>  67

Ile Asn Trp Met Lys Ala Gly Ile Leu Glu Ala Asp Arg Val Leu Thr
1               5                   10                  15

Val Ser Pro Tyr Tyr Ala Glu Glu Leu Ile Ser Gly Ile Ala Arg Gly
            20                  25                  30

Cys Glu Leu Asp Asn Ile Met Arg Leu Thr Gly Ile Thr Gly Ile Val
        35                  40                  45

Asn Gly Met Asp Val Ser Glu Trp Asp Pro Ser Lys Asp Lys Tyr Ile
    50                  55                  60

Thr Ala Lys Tyr Asp Ala Thr Thr Ala Ile Glu Ala Lys Ala Leu Asn
65                  70                  75                  80

Lys Glu Ala Leu Gln Ala Glu Ala Gly Leu Pro Val Asp Arg Lys Ile
                85                  90                  95

Pro Leu Ile Ala Phe Ile Gly Arg Leu Glu Glu Gln Lys
            100                 105

<210> 68
<211> 45
<212> PRT
<213> Artificial Sequence

<220>
<223> FRAGMENT OF SEQ ID 15

<400> 68

Arg Gly Val Asp Arg Val Phe Ile Asp His Pro Ser Phe Leu Glu Lys
1               5                   10                  15

Val Trp Gly Lys Thr Gly Glu Lys Ile Tyr Gly Pro Asp Thr Gly Val
            20                  25                  30

Asp Tyr Lys Asp Asn Gln Met Arg Phe Ser Leu Leu Cys
        35                  40                  45

<210> 69
<211> 21
<212> PRT
<213> Artificial Sequence

<220>
<223> FRAGMENT OF SEQ ID 15

<400> 69

Leu Glu Ala Pro Arg Ile Leu Asn Leu Asn Asn Asn Pro Tyr Phe Lys
1               5                   10                  15

Gly Thr Tyr Gly Glu
            20

<210>  70
<211>  26
<212>  PRT
<213>  Artificial Sequence

<220>
<223>  FRAGMENT OF SEQ ID 16

<400>  70

Tyr Ile Gln Val Glu Ala Pro Thr Gly Thr Phe Ile Ala Ser Gly Val
1               5                   10                  15

Val Val Gly Lys Asp Thr Leu Leu Thr Asn
            20                  25

<210>  71
<211>  12
<212>  PRT
<213>  Artificial Sequence

<220>
<223>  FRAGMENT OF SEQ ID 1

<400>  71

Ser Arg Gly Pro Ile Tyr Ser Asn Glu Phe Gly Lys
1               5                   10

<210>  72
<211>  7
<212>  PRT
<213>  Artificial Sequence

<220>
<223>  FRAGMENT OF SEQ ID 1

<400>  72

Asn Ser Phe Asn Leu Glu Arg
1               5

<210>  73
<211>  10
<212>  PRT
<213>  Artificial Sequence

<220>
<223>  FRAGMENT OF SEQ ID 1

<400>  73

Val Leu Asp Leu Ala Ile Pro Val Asn Arg
1               5                   10

<210>  74

<211>  7
<212>  PRT
<213>  Artificial Sequence

<220>
<223>  FRAGMENT OF SEQ ID 1

<400>  74

Asp Asp Asn Glu Glu Leu Arg
1               5


<210>  75
<211>  18
<212>  PRT
<213>  Artificial Sequence

<220>
<223>  FRAGMENT OF SEQ ID 1

<400>  75

Leu Ser Ser Gly Asp Val Phe Val Ile Pro Ala Gly His Pro Val Ala
1               5                   10                  15


Val Lys



<210>  76
<211>  15
<212>  PRT
<213>  Artificial Sequence

<220>
<223>  FRAGMENT OF SEQ ID 1

<400>  76

Glu Asp Asp Glu Glu Glu Glu Gln Gly Glu Glu Glu Ile Asn Lys
1               5                   10                  15


<210>  77
<211>  29
<212>  PRT
<213>  Artificial Sequence

<220>
<223>  FRAGMENT OF SEQ ID 1

<400>  77

Asn Ile Leu Glu Ala Ser Phe Asn Thr Asp Tyr Glu Glu Ile Glu Lys
1               5                   10                  15


Val Leu Leu Glu Glu His Glu Lys Glu Thr Gln His Arg
            20                  25

<210>  78
<211>  24
<212>  PRT
<213>  Artificial Sequence

<220>
<223>  FRAGMENT OF SEQ ID 1

<400>  78

Asn Ile Leu Glu Ala Ser Phe Asn Thr Asp Tyr Glu Glu Ile Glu Lys
1               5                   10                  15

Val Leu Leu Glu Glu His Glu Lys
            20

<210>  79
<211>  16
<212>  PRT
<213>  Artificial Sequence

<220>
<223>  FRAGMENT OF SEQ ID 1

<400>  79

Asn Ile Leu Glu Ala Ser Phe Asn Thr Asp Tyr Glu Glu Ile Glu Lys
1               5                   10                  15

<210>  80
<211>  12
<212>  PRT
<213>  Artificial Sequence

<220>
<223>  FRAGMENT OF SEQ ID 1

<400>  80

Arg Gln Gln Ser Gln Glu Glu Asn Val Ile Val Lys
1               5                   10

<210>  81
<211>  11
<212>  PRT
<213>  Artificial Sequence

<220>
<223>  FRAGMENT OF SEQ ID 1

<400>  81

Gln Gln Ser Gln Glu Glu Asn Val Ile Val Lys
1               5                   10

<210>  82
<211>  11
<212>  PRT

<213>  Artificial Sequence

<220>
<223>  FRAGMENT OF SEQ ID 1

<400>  82

Leu Ser Arg Gly Gln Ile Glu Glu Leu Ser Lys
1               5                   10


<210>  83
<211>  8
<212>  PRT
<213>  Artificial Sequence

<220>
<223>  FRAGMENT OF SEQ ID 1

<400>  83

Gly Gln Ile Glu Glu Leu Ser Lys
1               5


<210>  84
<211>  8
<212>  PRT
<213>  Artificial Sequence

<220>
<223>  FRAGMENT OF SEQ ID 1

<400>  84

Val Leu Leu Glu Glu His Glu Lys
1               5


<210>  85
<211>  24
<212>  PRT
<213>  Artificial Sequence

<220>
<223>  FRAGMENT OF SEQ ID 1

<400>  85

Ser Lys Pro His Thr Ile Phe Leu Pro Gln His Thr Asp Ala Asp Tyr
1               5                   10                  15


Ile Leu Val Val Leu Ser Gly Lys
            20


<210>  86
<211>  22
<212>  PRT
<213>  Artificial Sequence

<220>

<223>  FRAGMENT OF SEQ ID 1

<400>  86

Pro His Thr Ile Phe Leu Pro Gln His Thr Asp Ala Asp Tyr Ile Leu
1               5                   10                  15

Val Val Leu Ser Gly Lys
            20

<210>  87
<211>  16
<212>  PRT
<213>  Artificial Sequence

<220>
<223>  FRAGMENT OF SEQ ID 1

<400>  87

Ser Asn Lys Phe Gln Thr Leu Phe Glu Asn Glu Asn Gly His Ile Arg
1               5                   10                  15

<210>  88
<211>  11
<212>  PRT
<213>  Artificial Sequence

<220>
<223>  FRAGMENT OF SEQ ID 1

<400>  88

Ser Lys Ile Phe Glu Asn Leu Gln Asn Tyr Arg
1               5                   10

<210>  89
<211>  9
<212>  PRT
<213>  Artificial Sequence

<220>
<223>  FRAGMENT OF SEQ ID 1

<400>  89

Ile Phe Glu Asn Leu Gln Asn Tyr Arg
1               5

<210>  90
<211>  18
<212>  PRT
<213>  Artificial Sequence

<220>
<223>  FRAGMENT OF SEQ ID 1

<400>  90

Ile Leu Glu Asn Gln Lys Gln Ser His Phe Ala Asp Ala Gln Pro Gln
1               5                   10                  15

Gln Arg

<210>  91
<211>  24
<212>  PRT
<213>  Artificial Sequence

<220>
<223>  FRAGMENT OF SEQ ID 1

<400>  91

Pro Gly Gln Leu Gln Ser Phe Leu Leu Ser Gly Asn Gln Asn Gln Gln
1               5                   10                  15

Asn Tyr Leu Ser Gly Phe Ser Lys
            20

<210>  92
<211>  25
<212>  PRT
<213>  Artificial Sequence

<220>
<223>  FRAGMENT OF SEQ ID 2

<400>  92

Phe Phe Glu Leu Thr Pro Glu Lys Asn Gln Gln Leu Gln Asp Leu Asp
1               5                   10                  15

Leu Phe Val Asn Ser Val Asp Leu Lys
            20                  25

<210>  93
<211>  7
<212>  PRT
<213>  Artificial Sequence

<220>
<223>  FRAGMENT OF SEQ ID 2

<400>  93

Gln Leu Glu Glu Leu Ser Lys
1               5

<210>  94
<211>  11
<212>  PRT
<213>  Artificial Sequence

<220>
<223>  FRAGMENT OF SE QID 3

<400>  94

Gln Glu Glu Asp Glu Asp Glu Asp Glu Glu Arg
1               5                   10


<210>  95
<211>  23
<212>  PRT
<213>  Artificial Sequence

<220>
<223>  FRAGMENT OF SEQ 3

<400>  95

Tyr Gln His Gln Gln Gly Gly Lys Gln Glu Gln Glu Asn Glu Gly Asn
1               5                   10                  15


Asn Ile Phe Ser Gly Phe Lys
            20


<210>  96
<211>  24
<212>  PRT
<213>  Artificial Sequence

<220>
<223>  FRAGMENT OF SEQ ID 4

<400>  96

Gly Asp Thr Ile Lys Leu Pro Ala Gly Thr Thr Ser Tyr Leu Val Asn
1               5                   10                  15


Gln Asp Asp Glu Glu Asp Leu Arg
            20


<210>  97
<211>  12
<212>  PRT
<213>  Artificial Sequence

<220>
<223>  FRAGMENT OF SEQ ID 4

<400>  97

Arg Gln Gln Gly Glu Glu Thr Asp Ala Ile Val Lys
1               5                   10


<210>  98
<211>  11
<212>  PRT

<213>  Artificial Sequence

<220>
<223>  FRAGMENT OF SEQ ID 4

<400>  98

Val Leu Leu Glu Glu Gln Glu Lys Asp Arg Lys
1               5                   10


<210>  99
<211>  10
<212>  PRT
<213>  Artificial Sequence

<220>
<223>  FRAGMENT OF SEQ ID 4

<400>  99

Asn Ile Leu Glu Ala Ser Tyr Asn Thr Arg
1               5                   10


<210>  100
<211>  8
<212>  PRT
<213>  Artificial Sequence

<220>
<223>  FRAGMENT OF SEQ ID 4

<400>  100

Phe Glu Ala Phe Asp Leu Ala Lys
1               5


<210>  101
<211>  8
<212>  PRT
<213>  Artificial Sequence

<220>
<223>  FRAGMENT OF SEQ ID 4

<400>  101

Glu Gln Ile Glu Glu Leu Lys Lys
1               5


<210>  102
<211>  7
<212>  PRT
<213>  Artificial Sequence

<220>
<223>  FRAGMENT OF SEQ ID 4

<400>  102

Glu Gln Ile Glu Glu Leu Lys
1               5


<210>  103
<211>  7
<212>  PRT
<213>  Artificial Sequence

<220>
<223>  FRAGMENT OF SEQ ID 4

<400>  103

Asn Lys Asn Gln Tyr Leu Arg
1               5


<210>  104
<211>  37
<212>  PRT
<213>  Artificial Sequence

<220>
<223>  FRAGMENT OF SEQ ID 4

<400>  104

Leu Ser Pro Gly Asp Val Val Ile Ile Pro Ala Gly His Pro Val Ala
1               5                   10                  15


Ile Thr Ala Ser Ser Asn Leu Asn Leu Leu Gly Phe Gly Ile Asn Ala
            20                  25                  30


Glu Asn Asn Glu Arg
        35


<210>  105
<211>  14
<212>  PRT
<213>  Artificial Sequence

<220>
<223>  FRAGMENT OF SEQ ID 4

<400>  105

Pro Ser Tyr Glu Lys Gln Glu Asp Glu Glu Glu Lys Gln Lys
1               5                   10


<210>  106
<211>  9
<212>  PRT
<213>  Artificial Sequence

<220>
<223>  FRAGMENT OF SEQ ID 4

<400>  106

Glu Glu Asp Glu Glu Glu Gly Gln Arg
1               5

<210>  107
<211>  20
<212>  PRT
<213>  Artificial Sequence

<220>
<223>  FRAGMENT OF SEQ ID 5

<400>  107

Thr Leu Phe Leu Pro Gln Tyr Thr Asp Ala Asp Phe Ile Leu Val Val
1               5                   10                  15

Leu Ser Gly Lys
            20

<210>  108
<211>  24
<212>  PRT
<213>  Artificial Sequence

<220>
<223>  FRAGMENT OF SEQ ID 6

<400>  108

Gly Tyr Val Gly Leu Thr Phe Pro Gly Cys Pro Ala Thr His Gln Gln
1               5                   10                  15

Gln Phe Gln Leu Phe Glu Gln Arg
            20

<210>  109
<211>  14
<212>  PRT
<213>  Artificial Sequence

<220>
<223>  FRAGMENT OF SEQ ID 7

<400>  109

Arg Gly Pro Gln Gln Tyr Ala Glu Trp Gln Ile Asn Glu Lys
1               5                   10

<210>  110
<211>  21
<212>  PRT
<213>  Artificial Sequence

<220>
<223>  FRAGMENT OF SEQ ID 8

<400>  110

Leu Val Asp Leu Val Ile Pro Val Asn Gly Pro Gly Lys Phe Glu Ala
1               5                   10                  15

Phe Asp Leu Ala Lys
            20

<210>  111
<211>  18
<212>  PRT
<213>  Artificial Sequence

<220>
<223>  FRAGMENT OF SEQ ID 9

<400>  111

Lys Asn Pro Gln Leu Gln Asp Leu Asp Ile Phe Val Asn Tyr Val Glu
1               5                   10                  15

Ile Lys

<210>  112
<211>  10
<212>  PRT
<213>  Artificial Sequence

<220>
<223>  FRAGMENT OF SEQ ID 10

<400>  112

Asp Glu His Gln Lys Ile His Gln Phe Arg
1               5                   10

<210>  113
<211>  7
<212>  PRT
<213>  Artificial Sequence

<220>
<223>  FRAGMENT OF SEQ ID 10

<400>  113

Phe Arg Asp Glu His Gln Lys
1               5

<210>  114
<211>  13
<212>  PRT
<213>  Artificial Sequence

<220>
<223>  FRAGMENT OF SEQ ID 10

<400>  114

Phe Pro Ile Leu Asn Leu Ile Gln Met Ser Ala Thr Arg
1               5                   10


<210>  115
<211>  13
<212>  PRT
<213>  Artificial Sequence

<220>
<223>  FRAGMENT OF SEQ ID 10

<400>  115

Thr Asn Ala Asn Ala Phe Val Ser His Leu Ala Gly Lys
1               5                   10


<210>  116
<211>  12
<212>  PRT
<213>  Artificial Sequence

<220>
<223>  FRAGMENT OF SEQ ID 10

<400>  116

Ala Leu Pro Val Asp Val Val Ala Asn Ala Tyr Arg
1               5                   10


<210>  117
<211>  20
<212>  PRT
<213>  Artificial Sequence

<220>
<223>  FRAGMENT OF SEQ ID 10

<400>  117

Tyr Val Tyr Asp Val Asn Asn Asn Ala Asn Gln Leu Glu Pro Arg Gln
1               5                   10                  15


Lys Glu Phe Leu
            20


<210>  118
<211>  21
<212>  PRT
<213>  Artificial Sequence

<220>
<223>  FRAGMENT OF SEQ ID 10

<400>  118

Val Tyr Val Tyr Asp Val Asn Asn Asn Ala Asn Gln Leu Glu Pro Arg
1               5                   10                  15

Gln Lys Glu Phe Leu
            20

<210>  119
<211>  7
<212>  PRT
<213>  Artificial Sequence

<220>
<223>  FRAGMENT OF SEQ ID 10

<400>  119

Ala Asp Ser Tyr Asn Pro Arg
1               5

<210>  120
<211>  16
<212>  PRT
<213>  Artificial Sequence

<220>
<223>  FRAGMENT OF SEQ ID 10

<400>  120

Lys Pro Thr Leu Thr Gln Gln Gln Glu Gln Ala Gln Ala Gln Asp Gln
1               5                   10                  15

<210>  121
<211>  13
<212>  PRT
<213>  Artificial Sequence

<220>
<223>  FRAGMENT OF SEQ ID 10

<400>  121

Gln Ala Gln Ala Gln Asp Gln Tyr Gln Gln Val Gln Tyr
1               5                   10

<210>  122
<211>  11
<212>  PRT
<213>  Artificial Sequence

<220>
<223>  FRAGMENT OF SEQ ID 10

<400>  122

Gln Ala Gln Asp Gln Tyr Gln Gln Val Gln Tyr
1               5                   10

<210>  123
<211>  8
<212>  PRT
<213>  Artificial Sequence

<220>
<223>  FRAGMENT OF SEQ ID 10

<400>  123

Leu Gln Ala Phe Glu Pro Leu Arg
1               5


<210>  124
<211>  11
<212>  PRT
<213>  Artificial Sequence

<220>
<223>  FRAGMENT OF SEQ ID 10

<400>  124

Ser Arg Val Gln Val Val Ser Asn Phe Gly Lys
1               5                   10


<210>  125
<211>  10
<212>  PRT
<213>  Artificial Sequence

<220>
<223>  FAGMENT OF SEQ ID 10

<400>  125

Trp Asn Val Asn Ala His Ser Leu Val Tyr
1               5                   10


<210>  126
<211>  9
<212>  PRT
<213>  Artificial Sequence

<220>
<223>  FRAGMENT OF SEQ ID 10

<400>  126

Asn Val Asn Ala His Ser Leu Val Tyr
1               5


<210>  127
<211>  15
<212>  PRT
<213>  Artificial Sequence

<220>

<223>  FRAGMENT OF SEQ ID 10

<400>  127

Ile Gln Gly Arg Ser Arg Val Gln Val Val Ser Asn Phe Gly Lys
1               5                   10                  15


<210>  128
<211>  14
<212>  PRT
<213>  Artificial Sequence

<220>
<223>  FRAGMENT OF SEQ ID 10

<400>  128

Gly Lys Thr Val Phe Asp Gly Val Leu Arg Pro Gly Gln Leu
1               5                   10


<210>  129
<211>  15
<212>  PRT
<213>  Artificial Sequence

<220>
<223>  FRAGMENT OF SEQ ID 10

<400>  129

Phe Gly Lys Thr Val Phe Asp Gly Val Leu Arg Pro Gly Gln Leu
1               5                   10                  15


<210>  130
<211>  19
<212>  PRT
<213>  Artificial Sequence

<220>
<223>  FRAGMENT OF SEQ ID 10

<400>  130

Phe Gln Gln Gln Tyr Tyr Pro Gly Leu Ser Asn Glu Ser Glu Ser Glu
1               5                   10                  15


Thr Ser Glu


<210>  131
<211>  9
<212>  PRT
<213>  Artificial Sequence

<220>
<223>  FRAGMENT OF SEQ ID 10

<400>  131

Gln Gln Tyr Tyr Pro Gly Leu Ser Asn
1               5


<210>  132
<211>  10
<212>  PRT
<213>  Artificial Sequence

<220>
<223>  FRAGMENT OF SEQ ID 10

<400>  132

Gln Gln Gln Tyr Tyr Pro Gly Leu Ser Asn
1               5                   10


<210>  133
<211>  21
<212>  PRT
<213>  Artificial Sequence

<220>
<223>  FRAGMENT SEQ ID 11

<400>  133

Val Thr Asn Leu Asn Thr Gln Asn Phe Pro Ile Leu Ser Leu Val Gln
1               5                   10                  15


Met Ser Ala Val Lys
            20


<210>  134
<211>  19
<212>  PRT
<213>  Artificial Sequence

<220>
<223>  FRAGMENT OF SEQ ID 11

<400>  134

Ile Thr Gln Gly Arg Ala Arg Val Gln Val Val Asn Asn Asn Gly Lys
1               5                   10                  15


Thr Val Phe



<210>  135
<211>  22
<212>  PRT
<213>  Artificial Sequence

<220>
<223>  FRAGMENT OF SEQ ID 11

<400>  135

Ile Thr Gln Gly Arg Ala Arg Val Gln Val Val Asn Asn Asn Gly Lys
1               5                   10                  15

Thr Val Phe Asn Gly Glu
            20

<210>  136
<211>  21
<212>  PRT
<213>  Artificial Sequence

<220>
<223>  FRAGMENT OF SEQ ID 11

<400>  136

Ile Thr Gln Gly Arg Ala Arg Val Gln Val Val Asn Asn Asn Gly Lys
1               5                   10                  15

Thr Val Phe Asn Gly
            20

<210>  137
<211>  13
<212>  PRT
<213>  Artificial Sequence

<220>
<223>  FRAGMENT OF SEQ ID 11

<400>  137

Arg Val Gln Val Val Asn Asn Asn Gly Lys Thr Val Phe
1               5                   10

<210>  138
<211>  18
<212>  PRT
<213>  Artificial Sequence

<220>
<223>  FRAGMENT OF SEQ ID 11

<400>  138

Arg Ala Leu Pro Asn Asp Val Leu Ala Asn Ala Tyr Arg Ile Ser Arg
1               5                   10                  15

Glu Glu

<210>  139
<211>  16
<212>  PRT

<213>  Artificial Sequence

<220>
<223>  FRAGMENT OF SEQ ID 11

<400>  139

Ser Ile Phe Arg Ala Leu Pro Asn Asp Val Leu Ala Asn Ala Tyr Arg
1               5                   10                  15


<210>  140
<211>  15
<212>  PRT
<213>  Artificial Sequence

<220>
<223>  FRAGMENT OF SEQ ID 11

<400>  140

Ser Ile Phe Arg Ala Leu Pro Asn Asp Val Leu Ala Asn Ala Tyr
1               5                   10                  15


<210>  141
<211>  13
<212>  PRT
<213>  Artificial Sequence

<220>
<223>  FRAGMENT OF SEQ ID 11

<400>  141

Ser Ile Phe Arg Ala Leu Pro Asn Asp Val Leu Ala Asn
1               5                   10


<210>  142
<211>  17
<212>  PRT
<213>  Artificial Sequence

<220>
<223>  FRAGMNET OF SEQ ID 11

<400>  142

Ser Ser Ile Phe Arg Ala Leu Pro Asn Asp Val Leu Ala Asn Ala Tyr
1               5                   10                  15


Arg


<210>  143
<211>  21
<212>  PRT
<213>  Artificial Sequence

<220>

<223>  FRAGMENT OF SEQ ID 11

<400>  143

Ser Ile Phe Arg Ala Leu Pro Asn Asp Val Leu Ala Asn Ala Tyr Arg
1               5                   10                  15

Ile Ser Arg Glu Glu
            20

<210>  144
<211>  9
<212>  PRT
<213>  Artificial Sequence

<220>
<223>  FRAGMENT OF SEQ ID 11

<400>  144

Ser Ile Phe Arg Ala Leu Pro Asn Asp
1               5

<210>  145
<211>  19
<212>  PRT
<213>  Artificial Sequence

<220>
<223>  FRAGMENT OF SEQ ID 11

<400>  145

Ile Tyr Val Thr Asp Leu Asn Asn Gly Ala Asn Gln Leu Asp Pro Arg
1               5                   10                  15

Gln Arg Asp

<210>  146
<211>  21
<212>  PRT
<213>  Artificial Sequence

<220>
<223>  FRAGMENT OF SEQ ID 12

<400>  146

Val Thr Asn Leu Asn Ser Gln Asn Phe Pro Ile Leu Asn Leu Val Gln
1               5                   10                  15

Met Ser Ala Val Lys
            20

<210>  147

<211>  8
<212>  PRT
<213>  Artificial Sequence

<220>
<223>  FRAGMENT OF SEQ ID 12

<400>  147

Gln Asn Ile Asp Asn Pro Asn Arg
1               5


<210>  148
<211>  7
<212>  PRT
<213>  Artificial Sequence

<220>
<223>  FRAGMENT OF SEQ ID 12

<400>  148

Ala Asp Thr Tyr Asn Pro Arg
1               5


<210>  149
<211>  21
<212>  PRT
<213>  Artificial Sequence

<220>
<223>  FRAGMENT OF SEQ ID 12

<400>  149

Asn Ile Asp Asn Pro Asn Arg Ala Asp Thr Tyr Asn Pro Arg Ala Gly
1               5                   10                  15


Arg Val Thr Asn Leu
            20


<210>  150
<211>  25
<212>  PRT
<213>  Artificial Sequence

<220>
<223>  FRAGMENT OF SEQ ID 12

<400>  150

Arg Val Arg Gln Asn Ile Asp Asn Pro Asn Arg Ala Asp Thr Tyr Asn
1               5                   10                  15


Pro Arg Ala Gly Arg Val Thr Asn Leu
            20                  25

<210>  151
<211>  18
<212>  PRT
<213>  Artificial Sequence

<220>
<223>  FRAGMENT OF SEQ ID 12

<400>  151

Thr Asn Pro Asn Ser Met Val Ser His Ile Ala Gly Lys Ser Ser Ile
1               5                   10                  15

Phe Arg

<210>  152
<211>  16
<212>  PRT
<213>  Artificial Sequence

<220>
<223>  FRAGMENT OF SEQ ID 12

<400>  152

His Asn Arg Gly Asp Glu Phe Gly Ala Phe Thr Pro Leu Gln Tyr Lys
1               5                   10                  15

<210>  153
<211>  12
<212>  PRT
<213>  Artificial Sequence

<220>
<223>  FRAGMENT OF SEQ ID 12

<400>  153

Ser Tyr Gln Asp Val Tyr Asn Val Ala Glu Ser Ser
1               5                   10

<210>  154
<211>  8
<212>  PRT
<213>  Artificial Sequence

<220>
<223>  FRAGMENT OF SEQ ID 12

<400>  154

Ile Ser Arg Glu Glu Ala Gln Arg
1               5

<210>  155
<211>  21
<212>  PRT

<213>  Artificial Sequence

<220>
<223>  FRAGMENT OF SEQ ID 12

<400>  155

Ser Ile Phe Arg Ala Leu Pro Thr Asp Val Leu Ala Asn Ala Tyr Arg
1               5                   10                  15

Ile Ser Arg Glu Glu
            20

<210>  156
<211>  19
<212>  PRT
<213>  Artificial Sequence

<220>
<223>  FRAGMENT OF SEQ ID 12

<400>  156

Tyr Arg Ile Ser Arg Glu Glu Ala Gln Arg Leu Lys His Asn Arg Gly
1               5                   10                  15

Asp Glu Phe

<210>  157
<211>  18
<212>  PRT
<213>  Artificial Sequence

<220>
<223>  FRAGMENT OF SEQ ID 12

<400>  157

Tyr Arg Ile Ser Arg Glu Glu Ala Gln Arg Leu Lys His Asn Arg Gly
1               5                   10                  15

Asp Glu

<210>  158
<211>  10
<212>  PRT
<213>  Artificial Sequence

<220>
<223>  FRAGMENT OF SEQ ID 12

<400>  158

Phe Lys Asp Glu His Gln Lys Ile His Arg
1               5                   10

<210>  159
<211>  14
<212>  PRT
<213>  Artificial Sequence

<220>
<223>  FRAGMENT OF SEQ ID 12

<400>  159

Gln Gly Asp Val Ile Ala Leu Pro Ala Gly Val Ala His Trp
1               5                   10


<210>  160
<211>  9
<212>  PRT
<213>  Artificial Sequence

<220>
<223>  FRAGMENT OF SEQ ID 12

<400>  160

Thr Val Phe Asn Gly Glu Leu Arg Arg
1               5


<210>  161
<211>  8
<212>  PRT
<213>  Artificial Sequence

<220>
<223>  FRAGMENT OF SEQ ID 12

<400>  161

Thr Val Phe Asn Gly Glu Leu Arg
1               5


<210>  162
<211>  13
<212>  PRT
<213>  Artificial Sequence

<220>
<223>  FRAGMENT OF SEQ ID 12

<400>  162

Gln Val Gln Val Val Asn Asn Asn Gly Lys Thr Val Phe
1               5                   10


<210>  163
<211>  13
<212>  PRT
<213>  Artificial Sequence

<220>
<223>  FRAGMENT OF SEQ ID 12

<400>  163

Tyr Ile Ile Gln Gly Arg Gly Ile Thr Gly Pro Thr Phe
1               5                   10


<210>  164
<211>  14
<212>  PRT
<213>  Artificial Sequence

<220>
<223>  FRAGMENT OF SEQ ID 12

<400>  164

Val Tyr Ile Ile Gln Gly Arg Gly Ile Thr Gly Pro Thr Phe
1               5                   10


<210>  165
<211>  11
<212>  PRT
<213>  Artificial Sequence

<220>
<223>  FRAGMENT OF SEQ ID 12

<400>  165

Leu Gln Ala Phe Glu Pro Ile Arg Ser Val Arg
1               5                   10


<210>  166
<211>  22
<212>  PRT
<213>  Artificial Sequence

<220>
<223>  FRAGMENT OF SEQ ID 12

<400>  166

Gly Leu Ser Leu Leu Gln Pro Tyr Ala Ser Leu Gln Glu Gln Glu Gln
1               5                   10                  15


Gly Gln Met Gln Ser Arg
            20


<210>  167
<211>  8
<212>  PRT
<213>  Artificial Sequence

<220>
<223>  FRAGMENT OF SEQ ID 12

<400>  167

Gly Glu Ile Val Arg Val Glu Arg
1               5


<210>  168
<211>  13
<212>  PRT
<213>  Artificial Sequence

<220>
<223>  FRAGMENT OF SEQ ID 12

<400>  168

Arg Gly Leu Ser Leu Leu Gln Pro Tyr Ala Ser Leu Gln
1               5                   10


<210>  169
<211>  15
<212>  PRT
<213>  Artificial Sequence

<220>
<223>  FRAGMENT OF SEQ ID 12

<400>  169

Arg Gly Leu Ser Leu Leu Gln Pro Tyr Ala Ser Leu Gln Glu Gln
1               5                   10                  15


<210>  170
<211>  16
<212>  PRT
<213>  Artificial Sequence

<220>
<223>  FRAGMENT OF SEQ ID 12

<400>  170

Arg Gly Leu Ser Leu Leu Gln Pro Tyr Ala Ser Leu Gln Glu Gln Glu
1               5                   10                  15


<210>  171
<211>  14
<212>  PRT
<213>  Artificial Sequence

<220>
<223>  FRAGMENT OF SEQ ID 12

<400>  171

Arg Gly Leu Ser Leu Leu Gln Pro Tyr Ala Ser Leu Gln Glu
1               5                   10


<210>  172

<211>  7
<212>  PRT
<213>  Artificial Sequence

<220>
<223>  FRAGMENT OF SEQ ID 12

<400>  172

Arg Asn Pro Gln Ala Tyr Arg
1               5


<210>  173
<211>  13
<212>  PRT
<213>  Artificial Sequence

<220>
<223>  FRAGMENT OF SEQ ID 12

<400>  173

Phe Leu Leu Ala Gly Asn Lys Arg Asn Pro Gln Ala Tyr
1               5                   10


<210>  174
<211>  10
<212>  PRT
<213>  Artificial Sequence

<220>
<223>  FRAGMENT OF SEQ ID 12

<400>  174

Glu Val Glu Glu Trp Ser Gln Asn Ile Phe
1               5                   10


<210>  175
<211>  12
<212>  PRT
<213>  Artificial Sequence

<220>
<223>  FRAGMENT OF SEQ ID 12

<400>  175

Leu Ala Gly Asn Lys Arg Asn Pro Gln Ala Tyr Arg
1               5                   10


<210>  176
<211>  12
<212>  PRT
<213>  Artificial Sequence

<220>
<223>  FRAGMENT OF SEQ ID 12

<400>  176

Phe Leu Leu Ala Gly Asn Lys Arg Asn Pro Gln Ala
1               5                   10


<210>  177
<211>  13
<212>  PRT
<213>  Artificial Sequence

<220>
<223>  FRAGMENT OF SEQ ID 13

<400>  177

Glu Leu Gly Ala Pro Asp Val Gly His Pro Met Ser Glu
1               5                   10


<210>  178
<211>  16
<212>  PRT
<213>  Artificial Sequence

<220>
<223>  FRAGMENT OF SEQ ID 14

<400>  178

Ile Val Gln Gly His Ala Arg Val Gln Val Val Ser Asn Leu Gly Lys
1               5                   10                  15


<210>  179
<211>  14
<212>  PRT
<213>  Artificial Sequence

<220>
<223>  FRAGMENT OF SEQ ID 14

<400>  179

Ile Val Gln Gly His Ala Arg Val Gln Val Val Ser Asn Leu
1               5                   10


<210>  180
<211>  13
<212>  PRT
<213>  Artificial Sequence

<220>
<223>  FRAGMENT OF SEQ ID 14

<400>  180

Ile Val Gln Gly His Ala Arg Val Gln Val Val Ser Asn
1               5                   10


<210>  181

<211>  13
<212>  PRT
<213>  Artificial Sequence

<220>
<223>  FRAGMENT OF SEQ ID 14

<400>  181

Asn Asn Arg Gly Glu Glu Leu Gly Ala Phe Thr Pro Arg
1               5                   10


<210>  182
<211>  10
<212>  PRT
<213>  Artificial Sequence

<220>
<223>  FRAGMENT OF SEQ ID 14

<400>  182

Gly Glu Glu Leu Gly Ala Phe Thr Pro Arg
1               5                   10


<210>  183
<211>  13
<212>  PRT
<213>  Artificial Sequence

<220>
<223>  FRAGMENT OF SEQ ID 14

<400>  183

Phe Pro Ile Leu Asn Leu Val Gln Leu Ser Ala Thr Arg
1               5                   10


<210>  184
<211>  31
<212>  PRT
<213>  Artificial Sequence

<220>
<223>  FRAGMENT OF SE QID 14

<400>  184

Ser Ile Glu Gln His Ser Gly Gln Asn Ile Phe Ser Gly Phe Asn Asn
1               5                   10                  15


Glu Leu Leu Ser Glu Ala Leu Gly Val Asn Ala Leu Val Ala Lys
            20                  25                  30


<210>  185
<211>  8
<212>  PRT
<213>  Artificial Sequence

<220>
<223>  FRAGMENT OF SEQ ID 14

<400>  185

Leu Gln Gly Gln Asn Asp Gln Arg
1               5


<210>  186
<211>  20
<212>  PRT
<213>  Artificial Sequence

<220>
<223>  FRAGMENT OF SEQ ID 14

<400>  186

Ser Gly Phe Asn Asn Glu Leu Leu Ser Glu Ala Leu Gly Val Asn Ala
1               5                   10                  15


Leu Val Ala Lys
            20


<210>  187
<211>  20
<212>  PRT
<213>  Artificial Sequence

<220>
<223>  FRAGMENT OF SEQ ID 14

<400>  187

Pro Ala Phe Ala Gln Gln Gln Glu Gln Ala Gln Gln Gln Glu Gln Ala
1               5                   10                  15


Gln Ala Gln Tyr
            20


<210>  188
<211>  15
<212>  PRT
<213>  Artificial Sequence

<220>
<223>  FRAGMENT OF SEQ ID 14

<400>  188

Val Ala Lys Arg Leu Gln Gly Gln Asn Asp Gln Arg Gly Glu Ile
1               5                   10                  15


<210>  189
<211>  17
<212>  PRT

<213>  Artificial Sequence

<220>
<223>  FRAGMENT OF SEQ ID 14

<400>  189

Ala Leu Val Ala Lys Arg Leu Gln Gly Gln Asn Asp Gln Arg Gly Glu
1               5                   10                  15

Ile

<210>  190
<211>  13
<212>  PRT
<213>  Artificial Sequence

<220>
<223>  FRAGMENT OF SEQ ID 14

<400>  190

Leu Gln Gly Gln Asn Asp Gln Arg Gly Glu Ile Ile Arg
1               5                   10

<210>  191
<211>  14
<212>  PRT
<213>  Artificial Sequence

<220>
<223>  FRAGMENT OF SEQ ID 14

<400>  191

Pro Asn Val Asn Pro Trp His Asn Pro Arg Gln Gly Gly Phe
1               5                   10

<210>  192
<211>  13
<212>  PRT
<213>  Artificial Sequence

<220>
<223>  FRAGMENT OF SEQ ID 14

<400>  192

Phe Tyr Asn Glu Gly Asp Ala Pro Val Val Ala Leu Tyr
1               5                   10

<210>  193
<211>  10
<212>  PRT
<213>  Artificial Sequence

<220>

<223>  FRAGMENT OF SEQ ID 14

<400>  193

Phe Tyr Asn Glu Gly Asp Ala Pro Val Val
1               5                   10


<210>  194
<211>  12
<212>  PRT
<213>  Artificial Sequence

<220>
<223>  FRAGMENT OF SEQ ID 14

<400>  194

Phe Tyr Asn Glu Gly Asp Ala Pro Val Val Ala Leu
1               5                   10


<210>  195
<211>  11
<212>  PRT
<213>  Artificial Sequence

<220>
<223>  FRAGMENT OF SEQ ID 14

<400>  195

Phe Tyr Asn Glu Gly Asp Ala Pro Val Val Ala
1               5                   10


<210>  196
<211>  13
<212>  PRT
<213>  Artificial Sequence

<220>
<223>  FRAGMENT OF SEQ ID 14

<400>  196

Thr Asn Ala Asn Ser Met Val Ser His Leu Ala Gly Lys
1               5                   10


<210>  197
<211>  12
<212>  PRT
<213>  Artificial Sequence

<220>
<223>  FRAGMENT OF SEQ ID 14

<400>  197

Ala Met Pro Val Asp Val Ile Ala Asn Ala Tyr Arg
1               5                   10

<210>  198
<211>  28
<212>  PRT
<213>  Artificial Sequence

<220>
<223>  FRAGMENT OF SEQ ID 15

<400>  198

Asn Trp Glu Asn Val Leu Leu Gly Leu Gly Val Ala Gly Ser Ala Pro
1               5                   10                  15

Gly Ile Glu Gly Asp Glu Ile Ala Pro Leu Ala Lys
            20                  25

<210>  199
<211>  19
<212>  PRT
<213>  Artificial Sequence

<220>
<223>  FRAGMENT OF SEQ ID 15

<400>  199

Asn Val Leu Leu Gly Leu Gly Val Ala Gly Ser Ala Pro Gly Ile Glu
1               5                   10                  15

Gly Asp Glu

<210>  200
<211>  28
<212>  PRT
<213>  Artificial Sequence

<220>
<223>  FRAGMENT OF SEQ ID 15

<400>  200

Asn Trp Glu Asn Val Leu Leu Gly Leu Gly Val Ala Gly Ser Ala Pro
1               5                   10                  15

Gly Ile Glu Gly Asp Glu Ile Ala Pro Leu Ala Lys
            20                  25

<210>  201
<211>  21
<212>  PRT
<213>  Artificial Sequence

<220>
<223>  FRAGMENT OF SEQ ID 15

<400>  201

Phe Asn Ala Pro Leu Ala His Leu Ile Met Ala Gly Ala Asp Val Leu
1               5                   10                  15

Ala Val Pro Ser Arg
            20

<210>  202
<211>  10
<212>  PRT
<213>  Artificial Sequence

<220>
<223>  FRAGMENT OF SEQ ID 15

<400>  202

Phe Asn Ala Pro Leu Ala His Leu Ile Met
1               5                   10

<210>  203
<211>  21
<212>  PRT
<213>  Artificial Sequence

<220>
<223>  FRAGMENT OF SEQ ID 15

<400>  203

Phe Asn Ala Pro Leu Ala His Leu Ile Met Ala Gly Ala Asp Val Leu
1               5                   10                  15

Ala Val Pro Ser Arg
            20

<210>  204
<211>  12
<212>  PRT
<213>  Artificial Sequence

<220>
<223>  FRAGMENT OF SEQ ID 15

<400>  204

Val Val Gly Thr Pro Ala Tyr Glu Glu Met Val Arg
1               5                   10

<210>  205
<211>  21
<212>  PRT
<213>  Artificial Sequence

<220>
<223>  FRAGMENT OD SEQ ID 15

<400>  205

Thr Gly Gly Leu Gly Asp Val Leu Gly Gly Leu Pro Pro Ala Met Ala
1               5                   10                  15

Ala Asn Gly His Arg
            20

<210>  206
<211>  17
<212>  PRT
<213>  Artificial Sequence

<220>
<223>  FRAGMENT OF SEQ ID 15

<400>  206

Tyr Asp Gln Tyr Lys Asp Ala Trp Asp Thr Ser Val Val Ala Glu Ile
1               5                   10                  15

Lys

<210>  207
<211>  12
<212>  PRT
<213>  Artificial Sequence

<220>
<223>  FRAGMENT OF SEQ ID 15

<400>  207

Asp Ala Trp Asp Thr Ser Val Val Ala Glu Ile Lys
1               5                   10

<210>  208
<211>  7
<212>  PRT
<213>  Artificial Sequence

<220>
<223>  FRAGMENT OF SEQ ID 15

<400>  208

Val Met Val Ile Ser Pro Arg
1               5

<210>  209
<211>  22
<212>  PRT
<213>  Artificial Sequence

<220>

<223>  FRAGMENT OF SEQ ID 15

<400>  209

Leu Thr Gly Ile Thr Gly Ile Val Asn Gly Met Asp Val Ser Glu Trp
1               5                   10                  15

Asp Pro Ser Lys Asp Lys
            20

<210>  210
<211>  18
<212>  PRT
<213>  Artificial Sequence

<220>
<223>  FRAGMENT OF SEQ ID 15

<400>  210

Val Leu Thr Val Ser Pro Tyr Tyr Ala Glu Glu Leu Ile Ser Gly Ile
1               5                   10                  15

Ala Arg

<210>  211
<211>  14
<212>  PRT
<213>  Artificial Sequence

<220>
<223>  FRAGMENT OF SEQ ID 15

<400>  211

Glu Ala Leu Gln Ala Glu Ala Gly Leu Pro Val Asp Arg Lys
1               5                   10

<210>  212
<211>  10
<212>  PRT
<213>  Artificial Sequence

<220>
<223>  FRAGMENT OF SEQ ID 15

<400>  212

Tyr Asp Ala Thr Thr Ala Ile Glu Ala Lys
1               5                   10

<210>  213
<211>  9
<212>  PRT
<213>  Artificial Sequence

<220>
<223>  FRAGMENT OF SEQ ID 15

<400>  213

Ile Pro Leu Ile Ala Phe Ile Gly Arg
1               5


<210>  214
<211>  8
<212>  PRT
<213>  Artificial Sequence

<220>
<223>  FRAGMENT OF SEQ ID 15

<400>  214

Ala Gly Ile Leu Glu Ala Asp Arg
1               5


<210>  215
<211>  9
<212>  PRT
<213>  Artificial Sequence

<220>
<223>  FRAGMENT OF SEQ ID 15

<400>  215

Ile Pro Leu Ile Ala Phe Ile Gly Arg
1               5


<210>  216
<211>  11
<212>  PRT
<213>  Artificial Sequence

<220>
<223>  FRAGMENT OF SEQ ID 15

<400>  216

Val Phe Ile Asp His Pro Ser Phe Leu Glu Lys
1               5                   10


<210>  217
<211>  13
<212>  PRT
<213>  Artificial Sequence

<220>
<223>  FRAGMENT OF SEQ ID 15

<400>  217

Gly Pro Asp Thr Gly Val Asp Tyr Lys Asp Asn Gln Met
1               5                   10

<210>  218
<211>  16
<212>  PRT
<213>  Artificial Sequence

<220>
<223>  FRAGMENT OF SEQ ID 15

<400>  218

Ile Tyr Gly Pro Asp Thr Gly Val Asp Tyr Lys Asp Asn Gln Met Arg
1               5                   10                  15


<210>  219
<211>  11
<212>  PRT
<213>  Artificial Sequence

<220>
<223>  FRAGMENT OF SEQ ID 15

<400>  219

Ile Tyr Gly Pro Asp Thr Gly Val Asp Tyr Lys
1               5                   10


<210>  220
<211>  11
<212>  PRT
<213>  Artificial Sequence

<220>
<223>  FRAGMENT OF SEQ ID 15

<400>  220

Ile Leu Asn Leu Asn Asn Asn Pro Tyr Phe Lys
1               5                   10


<210>  221
<211>  16
<212>  PRT
<213>  Artificial Sequence

<220>
<223>  FRAGMENT OF SEQ ID 16

<400>  221

Ala Pro Thr Gly Thr Phe Ile Ala Ser Gly Val Val Val Gly Lys Asp
1               5                   10                  15


<210>  222
<211>  463
<212>  PRT
<213>  Vicia faba

<400> 222

Met Ala Ala Thr Thr Leu Lys Asp Ser Phe Pro Leu Leu Thr Leu Leu
1               5                   10                  15

Gly Ile Ala Phe Leu Ala Ser Val Cys Leu Ser Ser Arg Ser Asp Gln
            20                  25                  30

Asp Asn Pro Phe Val Phe Glu Ser Asn Arg Phe Gln Thr Leu Phe Glu
        35                  40                  45

Asn Glu Asn Gly His Ile Arg Leu Leu Gln Lys Phe Asp Gln His Ser
    50                  55                  60

Lys Leu Leu Glu Asn Leu Gln Asn Tyr Arg Leu Leu Glu Tyr Lys Ser
65                  70                  75                  80

Lys Pro His Thr Ile Phe Leu Pro Gln Gln Thr Asp Ala Asp Phe Ile
                85                  90                  95

Leu Val Val Leu Ser Gly Lys Ala Ile Leu Thr Val Leu Leu Pro Asn
            100                 105                 110

Asp Arg Asn Ser Phe Ser Leu Glu Arg Gly Asp Thr Ile Lys Leu Pro
        115                 120                 125

Ala Gly Thr Ile Gly Tyr Leu Val Asn Arg Asp Asp Glu Glu Asp Leu
    130                 135                 140

Arg Val Leu Asp Leu Val Ile Pro Val Asn Arg Pro Gly Glu Pro Gln
145                 150                 155                 160

Ser Phe Leu Leu Ser Gly Asn Gln Asn Gln Pro Ser Ile Leu Ser Gly
                165                 170                 175

Phe Ser Lys Asn Ile Leu Glu Ala Ser Phe Asn Thr Asp Tyr Lys Glu
            180                 185                 190

Ile Glu Lys Val Leu Leu Glu Glu His Gly Lys Glu Lys Tyr His Arg
        195                 200                 205

Arg Gly Leu Lys Asp Arg Arg Gln Arg Gly Gln Glu Glu Asn Val Ile
    210                 215                 220

Val Lys Ile Ser Arg Lys Gln Ile Glu Glu Leu Asn Lys Asn Ala Lys
225                 230                 235                 240

Ser Ser Ser Lys Lys Ser Thr Ser Ser Glu Ser Glu Pro Phe Asn Leu

245                 250                 255

Arg Ser Arg Glu Pro Ile Tyr Ser Asn Lys Phe Gly Lys Phe Phe Glu
            260                 265                 270

Ile Thr Pro Lys Arg Asn Pro Gln Leu Gln Asp Leu Asn Ile Phe Val
        275                 280                 285

Asn Tyr Val Glu Ile Asn Glu Gly Ser Leu Leu Leu Pro His Tyr Asn
    290                 295                 300

Ser Arg Ala Ile Val Ile Val Thr Val Asn Glu Gly Lys Gly Asp Phe
305                 310                 315                 320

Glu Leu Val Gly Gln Arg Asn Glu Asn Gln Gln Gly Leu Arg Glu Glu
                325                 330                 335

Tyr Asp Glu Glu Lys Glu Gln Gly Glu Glu Glu Ile Arg Lys Gln Val
            340                 345                 350

Gln Asn Tyr Lys Ala Lys Leu Ser Pro Gly Asp Val Leu Val Ile Pro
        355                 360                 365

Ala Gly Tyr Pro Val Ala Ile Lys Ala Ser Ser Asn Leu Asn Leu Val
    370                 375                 380

Gly Phe Gly Ile Asn Ala Glu Asn Asn Gln Arg Tyr Phe Leu Ala Gly
385                 390                 395                 400

Glu Glu Asp Asn Val Ile Ser Gln Ile His Lys Pro Val Lys Glu Leu
                405                 410                 415

Ala Phe Pro Gly Ser Ala Gln Glu Val Asp Thr Leu Leu Glu Asn Gln
            420                 425                 430

Lys Gln Ser His Phe Ala Asn Ala Gln Pro Arg Glu Arg Glu Arg Gly
        435                 440                 445

Ser Gln Glu Ile Lys Asp His Leu Tyr Ser Ile Leu Gly Ser Phe
    450                 455                 460

<210>  223
<211>  446
<212>  PRT
<213>  Cicer arietinum

<400>  223

Met Ala Ile Lys Ala Arg Phe Pro Leu Leu Val Leu Leu Gly Ile Val

1               5                   10                  15

Phe Leu Ala Ser Val Cys Ala Lys Ser Asp Lys Glu Asn Pro Phe Phe
            20                  25                  30

Phe Lys Ser Asn Asn Cys Gln Thr Leu Phe Glu Asn Glu Asn Gly His
        35                  40                  45

Val Arg Leu Leu Gln Arg Phe Asp Lys Arg Ser Gln Leu Phe Glu Asn
    50                  55                  60

Leu Gln Asn Tyr Arg Leu Met Glu Tyr Asn Ser Lys Pro His Thr Leu
65                  70                  75                  80

Phe Leu Pro Gln His Asn Asp Ala Asp Phe Ile Leu Val Val Leu Arg
                85                  90                  95

Gly Arg Ala Ile Leu Thr Val Leu Asn Pro Asn Asp Arg Asn Thr Phe
            100                 105                 110

Lys Leu Glu Arg Gly Asp Thr Ile Lys Leu Pro Ala Gly Thr Ile Ala
        115                 120                 125

Tyr Leu Ala Asn Arg Asp Asp Asn Glu Asp Leu Arg Val Leu Asp Leu
    130                 135                 140

Ala Ile Pro Val Asn Arg Pro Gly Gln Phe Gln Ser Phe Ser Leu Ser
145                 150                 155                 160

Gly Asn Glu Asn Gln Gln Ser Tyr Phe Gln Gly Phe Ser Lys Lys Ile
                165                 170                 175

Leu Glu Ala Ser Phe Asn Ser Asp Tyr Glu Glu Ile Glu Arg Val Leu
            180                 185                 190

Leu Glu Glu Gln Glu Gln Lys Pro Glu Gln Arg Arg Gly His Lys Gly
        195                 200                 205

Arg Gln Gln Ser Gln Glu Thr Asp Val Ile Val Lys Ile Ser Arg Glu
    210                 215                 220

Gln Ile Glu Glu Leu Ser Lys Asn Ala Lys Ser Asn Cys Lys Lys Ser
225                 230                 235                 240

Val Ser Ser Glu Ser Glu Pro Phe Asn Leu Arg Ser Arg Ser Pro Ile
                245                 250                 255

Tyr Ser Asn Arg Phe Gly Asn Phe Phe Glu Ile Thr Pro Glu Lys Asn
            260                 265                 270

Pro Gln Leu Lys Asp Leu Asp Ile Phe Val Asn Ser Val Glu Ile Lys
        275                 280                 285

Glu Gly Ser Leu Leu Leu Pro His Phe Asn Ser Arg Ala Thr Val Ile
    290                 295                 300

Leu Val Val Asn Glu Gly Lys Gly Glu Val Glu Leu Val Gly Leu Arg
305                 310                 315                 320

Asn Glu Asn Glu Gln Glu Asn Lys Lys Glu Asp Glu Glu Glu Glu Glu
                325                 330                 335

Asp Arg Asn Val Gln Val Gln Arg Phe Gln Ser Lys Leu Ser Ser Gly
            340                 345                 350

Asp Val Val Val Ile Pro Ala Ser His Pro Phe Ser Ile Asn Ala Ser
        355                 360                 365

Ser Asp Leu Phe Leu Leu Gly Phe Gly Ile Asn Ala Gln Asn Asn Gln
    370                 375                 380

Arg Asn Phe Leu Ala Gly Glu Glu Asp Asn Val Ile Ser Gln Ile Gln
385                 390                 395                 400

Arg Pro Val Lys Glu Val Ala Phe Pro Gly Ser Ala Glu Glu Val Asp
                405                 410                 415

Arg Leu Leu Lys Asn Gln Arg Gln Ser His Phe Ala Asn Ala Gln Pro
            420                 425                 430

Gln Gln Lys Arg Lys Gly Ser Gln Arg Ile Arg Ser Pro Phe
        435                 440                 445

<210>  224
<211>  418
<212>  PRT
<213>  Lens culinaris

<400>  224

Ser Arg Ser Asp Gln Glu Asn Pro Phe Ile Phe Lys Ser Asn Arg Phe
1               5                   10                  15

Gln Thr Ile Tyr Glu Asn Glu Asn Gly His Ile Arg Leu Leu Gln Arg
            20                  25                  30

Phe Asp Lys Arg Ser Lys Ile Phe Glu Asn Leu Gln Asn Tyr Arg Leu
        35                  40                  45

Leu Glu Tyr Lys Ser Lys Pro His Thr Ile Phe Leu Pro Gln Phe Thr
    50                  55                  60

Asp Ala Asp Phe Ile Leu Val Val Leu Ser Gly Lys Ala Ile Leu Thr
65                  70                  75                  80

Val Leu Asn Ser Asn Asp Arg Asn Ser Phe Asn Leu Glu Arg Gly Asp
                85                  90                  95

Thr Ile Lys Leu Pro Ala Gly Thr Ile Ala Tyr Leu Ala Asn Arg Asp
            100                 105                 110

Asp Asn Glu Asp Leu Arg Val Leu Asp Leu Ala Ile Pro Val Asn Arg
        115                 120                 125

Pro Gly Gln Leu Gln Ser Phe Leu Leu Ser Gly Thr Gln Asn Gln Pro
    130                 135                 140

Ser Phe Leu Ser Gly Phe Ser Lys Asn Ile Leu Glu Ala Ala Phe Asn
145                 150                 155                 160

Thr Glu Tyr Glu Glu Ile Glu Lys Val Leu Leu Glu Glu Gln Glu Gln
                165                 170                 175

Lys Ser Gln His Arg Arg Ser Leu Arg Asp Lys Arg Gln Glu Ile Thr
            180                 185                 190

Asn Glu Asp Val Ile Val Lys Val Ser Arg Glu Gln Ile Glu Glu Leu
        195                 200                 205

Ser Lys Asn Ala Lys Ser Ser Ser Lys Lys Ser Val Ser Ser Glu Ser
    210                 215                 220

Glu Pro Phe Asn Leu Arg Ser Arg Asn Pro Ile Tyr Ser Asn Lys Phe
225                 230                 235                 240

Gly Lys Phe Phe Glu Ile Thr Pro Glu Lys Asn Pro Gln Leu Gln Asp
                245                 250                 255

Leu Asp Ile Phe Val Asn Ser Val Glu Ile Lys Glu Gly Ser Leu Leu
            260                 265                 270

Leu Pro Asn Tyr Asn Ser Arg Ala Ile Val Ile Val Thr Val Asn Glu
        275                 280                 285

Gly Lys Gly Asp Phe Glu Leu Val Gly Gln Arg Asn Glu Asn Gln Gln
    290                 295                 300

Glu Gln Arg Glu Glu Asn Asp Glu Glu Glu Gly Gln Glu Glu Glu Thr
305                 310                 315                 320

Thr Lys Gln Val Gln Arg Tyr Arg Ala Arg Leu Ser Pro Gly Asp Val
                325                 330                 335

Leu Val Ile Pro Ala Gly His Pro Val Ala Ile Asn Ala Ser Ser Asp
            340                 345                 350

Leu Asn Leu Ile Gly Phe Gly Ile Asn Ala Lys Asn Asn Gln Arg Asn
        355                 360                 365

Phe Leu Ala Gly Glu Glu Asp Asn Val Ile Ser Gln Ile Gln Arg Pro
    370                 375                 380

Val Lys Glu Leu Ala Phe Pro Gly Ser Ser Arg Glu Val Asp Arg Leu
385                 390                 395                 400

Leu Thr Asn Gln Lys Gln Ser His Phe Ala Asn Ala Gln Pro Leu Gln
                405                 410                 415

Ile Glu

<210>  225
<211>  415
<212>  PRT
<213>  Lens culinaris

<400>  225

Ser Arg Ser Asp Gln Glu Asn Pro Phe Ile Phe Lys Ser Asn Arg Phe
1               5                   10                  15

Gln Thr Ile Tyr Glu Asn Glu Asn Gly His Ile Arg Leu Leu Gln Lys
            20                  25                  30

Phe Asp Lys Arg Ser Lys Ile Phe Glu Asn Leu Gln Asn Tyr Arg Leu
        35                  40                  45

Leu Glu Tyr Lys Ser Lys Pro His Thr Leu Phe Leu Pro Gln Tyr Thr
    50                  55                  60

Asp Ala Asp Phe Ile Leu Val Val Leu Ser Gly Lys Ala Val Leu Thr
65                  70                  75                  80

Val Leu Asn Ser Asn Asp Arg Asn Ser Phe Asn Leu Glu Arg Gly Asp
                85                  90                  95

Thr Ile Lys Leu Pro Ala Gly Thr Ile Ala Tyr Leu Ala Asn Arg Asp
            100                 105                 110

Asp Asn Glu Asp Leu Arg Val Leu Asp Leu Ala Ile Pro Val Asn Asn
        115                 120                 125

Pro Gly Gln Leu Glu Ser Phe Leu Leu Ser Gly Thr Gln Asn Gln Pro
    130                 135                 140

Ser Phe Leu Ser Gly Phe Asn Lys Ser Ile Leu Glu Ala Ala Phe Asn
145                 150                 155                 160

Thr Asp Tyr Glu Glu Ile Glu Lys Val Leu Leu Glu Asp Gln Glu Gln
                165                 170                 175

Glu Pro Gln His Arg Arg Ser Leu Arg Asp Arg Arg Gln Glu Ile Asn
            180                 185                 190

Lys Glu Asn Val Ile Val Lys Val Ser Arg Glu Gln Ile Lys Glu Leu
        195                 200                 205

Ser Lys Asn Ala Lys Ser Ser Ser Lys Lys Ser Val Ser Ser Glu Ser
    210                 215                 220

Glu Pro Phe Asn Leu Arg Ser Arg Asn Pro Ile Tyr Ser Asn Lys Phe
225                 230                 235                 240

Gly Lys Phe Phe Glu Ile Thr Pro Glu Lys Asn Pro Gln Leu Gln Asp
                245                 250                 255

Leu Asp Ile Phe Val Asn Ser Val Glu Ile Lys Glu Gly Ser Leu Leu
            260                 265                 270

Leu Pro Asn Tyr Asn Ser Arg Ala Ile Val Ile Val Thr Val Asn Glu
        275                 280                 285

Gly Lys Gly Tyr Phe Glu Leu Val Gly Gln Arg Asn Glu Asn Gln Arg
    290                 295                 300

Glu Glu Asn Asp Asp Glu Glu Glu Gln Glu Glu Glu Thr Ser Thr Gln
305                 310                 315                 320

Val Gln Arg Tyr Arg Ala Lys Leu Ser Pro Gly Asp Val Phe Val Val
                325                 330                 335

Pro Ala Gly His Pro Val Ala Ile Asn Ala Ser Ser Asp Leu Asn Leu
            340                 345                 350

Ile Gly Phe Gly Ile Asn Ala Lys Asn Asn Gln Arg Asn Phe Leu Ala
        355                 360                 365

Gly Glu Glu Asp Asn Val Ile Ser Gln Ile Gln Arg Pro Val Lys Glu
    370                 375                 380

Leu Ala Phe Pro Gly Ser Ser Arg Glu Val Asp Arg Leu Leu Thr Asn
385                 390                 395                 400

Gln Lys Gln Ser His Phe Ala Asn Ala Gln Pro Leu Gln Ile Glu
                405                 410                 415

<210> 226
<211> 463
<212> PRT
<213> Vicia narbonensis

<400> 226

Met Ala Ala Ile Thr Met Lys Val Ser Phe Pro Leu Leu Met Leu Leu
1               5                   10                  15

Gly Ile Ser Phe Leu Ala Ser Val Cys Val Ser Ser Arg Ser Asp Gln
            20                  25                  30

Glu Asn Pro Phe Ile Phe Lys Ser Asn Lys Phe Gln Thr Leu Phe Glu
        35                  40                  45

Asn Asp Asn Gly His Ile Arg Leu Leu Gln Lys Phe Asp Glu Arg Ser
    50                  55                  60

Lys Ile Leu Glu Asn Leu Gln Asn Tyr Arg Leu Leu Glu Tyr Lys Ser
65                  70                  75                  80

Lys Pro Arg Thr Ile Phe Leu Pro Gln Gln Thr Asn Ala Asp Phe Ile
                85                  90                  95

Leu Val Val Leu Ser Gly Lys Ala Ile Leu Thr Val Leu Lys Pro Asp
            100                 105                 110

Asp Arg Asn Ser Phe Asn Leu Glu Arg Gly Asp Thr Ile Lys Leu Pro
        115                 120                 125

Ala Gly Thr Ile Ala Tyr Leu Val Asn Lys Asp Asp Asn Glu Asp Leu
    130                 135                 140

Arg Val Leu Asp Leu Ala Ile Pro Val Asn Gly Pro Asp Gln Leu Gln
145                 150                 155                 160

Ser Phe Leu Leu Ser Gly Ser Glu Asn Gln Gln Ser Ile Leu Ser Gly
                165                 170                 175

Phe Ser Lys Ser Val Leu Glu Ala Ser Phe Asn Thr Gly Tyr Glu Glu
            180                 185                 190

Ile Glu Lys Val Leu Leu Glu Glu Arg Glu Lys Glu Thr Gln His Arg
        195                 200                 205

Arg Ser Leu Arg Asp Lys Arg Gln His Ser Gln Asp Glu Asp Val Ile
    210                 215                 220

Val Lys Leu Ser Arg Gly Gln Ile Glu Glu Leu Ser Arg Asn Ala Lys
225                 230                 235                 240

Ser Ser Ser Lys Lys Ser Val Ser Ser Glu Ser Glu Pro Phe Asn Leu
                245                 250                 255

Arg Ser Arg Asn Pro Ile Tyr Ser Asn Lys Phe Gly Lys Phe Phe Glu
            260                 265                 270

Ile Thr Pro Glu Lys Asn Pro Gln Leu Gln Asp Leu Asp Val Leu Val
        275                 280                 285

Asn Ser Val Glu Ile Lys Glu Gly Ser Leu Leu Leu Pro His Tyr Asn
    290                 295                 300

Ser Arg Ala Ile Val Ile Val Thr Val Asn Asp Gly Lys Gly Asp Phe
305                 310                 315                 320

Glu Ile Val Gly Gln Arg Asn Glu Asn Arg Gln Gly Gln Arg Lys Glu
                325                 330                 335

Asp Asp Glu Glu Glu Glu Gln Gly Asp Glu Asn Thr Asn Thr Gln Val
            340                 345                 350

Gln Asn Tyr Lys Ala Lys Leu Ser Arg Gly Asp Val Phe Val Ile Pro
        355                 360                 365

Ala Gly His Pro Val Ser Ile Lys Ala Ser Ser Asn Leu Asp Leu Leu
    370                 375                 380

Gly Phe Gly Ile Asn Ala Lys Asn Asn Gln Arg Asn Phe Leu Ala Gly

385                 390                 395                 400

Glu Glu Asp Asn Val Ile Ser Gln Ile Asp Arg Pro Val Lys Glu Leu
                405                 410                 415

Ala Phe Pro Gly Ser Ala Gln Glu Val Asp Arg Leu Leu Glu Asn Gln
            420                 425                 430

Lys Gln Ser His Phe Ala Asn Ala Gln Pro Gln Gln Arg Glu Arg Gly
        435                 440                 445

Ser His Glu Thr Arg Asp His Leu Ser Ser Ile Leu Asp Ala Phe
    450                 455                 460

<210>  227
<211>  396
<212>  PRT
<213>  Glycine max

<400>  227
   Gln Tyr Gly His Val Arg Val Leu Gln Arg Phe Asn Lys Arg Ser Gln
   1               5                   10                  15
   Gln Leu Gln Asn Leu Arg Asp Tyr Arg Ile Leu Glu Phe Asn Ser Lys
               20                  25                  30
   Pro Asn Thr Leu Leu Leu Pro His His Ala Asp Ala Asp Tyr Leu Ile
           35                  40                  45
   Val Ile Leu Asn Gly Thr Ala Ile Leu Thr Leu Val Asn Asn Asp Asp
       50                  55                  60
   Arg Asp Ser Tyr Asn Leu Gln Ser Gly Asp Ala Leu Arg Val Pro Ala
   65                  70                  75                  80
   Gly Thr Thr Tyr Tyr Val Val Asn Pro Asp Asn Asp Glu Asn Leu Arg
                   85                  90                  95
   Met Ile Thr Leu Ala Ile Pro Val Asn Lys Pro Gly Arg Phe Glu Ser
               100                 105                 110
   Phe Phe Leu Ser Ser Thr Gln Ala Gln Gln Ser Tyr Leu Gln Gly Phe
           115                 120                 125
   Ser Lys Asn Ile Leu Glu Ala Ser Tyr Asp Thr Lys Phe Glu Glu Ile
       130                 135                 140
   Asn Lys Val Leu Phe Gly Arg Glu Glu Gly Gln Gln Gln Gly Glu Glu
   145                 150                 155                 160
   Arg Leu Gln Glu Ser Val Ile Val Glu Ile Ser Lys Lys Gln Ile Arg
               165                 170                 175
   Glu Leu Ser Lys His Ala Lys Ser Ser Ser Arg Lys Thr Ile Ser Ser
           180                 185                 190
   Glu Asp Lys Pro Phe Asn Leu Arg Ser Arg Asp Pro Ile Tyr Ser Asn
       195                 200                 205
   Lys Leu Gly Lys Leu Phe Glu Ile Thr Pro Glu Lys Asn Pro Gln Leu
       210                 215                 220
   Arg Asp Leu Asp Val Phe Leu Ser Val Val Asp Met Asn Glu Gly Ala
   225                 230                 235                 240
   Leu Phe Leu Pro His Phe Asn Ser Lys Ala Ile Val Val Leu Val Ile
               245                 250                 255
   Asn Glu Gly Glu Ala Asn Ile Glu Leu Val Gly Ile Lys Glu Gln Gln
           260                 265                 270
   Gln Arg Gln Gln Gln Glu Glu Gln Pro Leu Glu Val Arg Lys Tyr Arg
       275                 280                 285
   Ala Glu Leu Ser Glu Gln Asp Ile Phe Val Ile Pro Ala Gly Tyr Pro
       290                 295                 300
   Val Val Val Asn Ala Thr Ser Asp Leu Asn Phe Phe Ala Phe Gly Ile
   305                 310                 315                 320
   Asn Ala Glu Asn Asn Gln Arg Asn Phe Leu Ala Gly Ser Lys Asp Asn
               325                 330                 335
   Val Ile Ser Gln Ile Pro Ser Gln Val Gln Glu Leu Ala Phe Pro Gly
           340                 345                 350
   Ser Ala Lys Asp Ile Glu Asn Leu Ile Lys Ser Gln Ser Glu Ser Tyr
       355                 360                 365
   Phe Val Asp Ala Gln Pro Gln Gln Lys Glu Glu Gly Asn Lys Gly Arg
       370                 375                 380
   Lys Gly Pro Leu Ser Ser Ile Leu Arg Ala Phe Tyr
   385                 390                 395

<210>  228
<211>  498
<212>  PRT
<213>  Vicia sativa

<400>  228

Met Ala Lys Leu Leu Ala Leu Ser Leu Ser Phe Cys Phe Leu Leu Phe
1               5                   10                  15

Ser Ser Cys Phe Ala Leu Arg Glu Gln Ser Gln Gln Asn Glu Cys Gln
            20                  25                  30

Leu Glu Arg Ile Asn Ala Leu Glu Pro Asp Asn Arg Ile Glu Ser Glu
        35                  40                  45

Gly Gly Leu Ile Glu Thr Trp Asn Pro Asn Asn Arg Gln Phe Arg Cys
    50                  55                  60

Ala Arg Val Ala Leu Ser Arg Ala Thr Leu Gln Arg Asn Ala Leu Arg
65                  70                  75                  80

Arg Pro Tyr Tyr Ser Asn Ala Pro Gln Glu Ile Tyr Ile Gln Gln Gly
                85                  90                  95

Asn Gly Tyr Phe Gly Met Val Phe Pro Gly Cys Pro Glu Thr His Glu
            100                 105                 110

Glu Pro Gln Gln Ser Glu Gln Gly Glu Gly Arg Arg Tyr Arg Asp Ser
        115                 120                 125

His Gln Lys Val Asn Arg Phe Arg Glu Gly Asp Ile Ile Ala Val Pro
    130                 135                 140

Thr Gly Ile Ala Phe Trp Met Tyr Asn Asp Gln Asp Thr Pro Val Ile
145                 150                 155                 160

Ala Ile Ser Leu Thr Asp Thr Gly Ser Ser Asn Asn Gln Leu Asp Gln
                165                 170                 175

Met Pro Arg Arg Phe Tyr Leu Ala Gly Asn Gln Glu Gln Glu Phe Leu
            180                 185                 190

Arg Tyr Gln His Gln Gln Gly Gly Lys Gln Glu Gln Asp Asn Asp Gly
        195                 200                 205

Asn Asn Ile Phe Ser Gly Phe Lys Arg Asp Phe Leu Glu Asp Ala Phe
    210                 215                 220

Asn Val Asn Arg His Ile Val Asp Arg Leu Gln Gly Arg Asn Glu Asp
225                 230                 235                 240

Glu Glu Lys Gly Ala Ile Val Lys Val Lys Gly Gly Leu Ser Ile Ile
                245                 250                 255

Ala Pro Pro Glu Arg Gln Ala Arg His Glu Arg Gly Ser Arg Gln Glu
            260                 265                 270

Glu Asp Glu Asp Glu Lys Glu Glu Arg Gln Pro Ser His His Lys Ser
        275                 280                 285

Arg Arg Asp Glu Asp Glu Asp Asp Lys Glu Lys Arg His Ser Gln Lys
    290                 295                 300

Gly Gln Ser Arg Arg Gln Gly Asp Asn Gly Leu Glu Glu Thr Val Cys
305                 310                 315                 320

Thr Ala Lys Leu Arg Ala Asn Ile Gly Ser Ser Pro Ser Pro Asp Ile
                325                 330                 335

Tyr Asn Pro Gln Ala Gly Arg Ile Lys Thr Val Thr Ser Leu Asp Leu
            340                 345                 350

Pro Val Leu Arg Trp Leu Lys Leu Ser Ala Glu His Gly Ser Leu His
        355                 360                 365

Lys Asn Ala Met Phe Val Pro His Tyr Asn Leu Asn Ala Asn Ser Val
    370                 375                 380

Ile Tyr Ala Leu Lys Gly Arg Ala Arg Leu Gln Val Val Asn Cys Asn
385                 390                 395                 400

Gly Asn Thr Val Phe Asp Gly Glu Leu Glu Ala Gly Arg Ala Leu Thr
                405                 410                 415

Val Pro Gln Asn Tyr Ala Val Ala Ala Lys Ser Leu Ser Glu Arg Phe
            420                 425                 430

Thr Tyr Val Ala Phe Lys Thr Asp Asp Arg Ala Ser Ile Ala Arg Leu
        435                 440                 445

Ala Gly Thr Ser Ser Val Ile Asp Asp Leu Pro Leu Asp Val Val Ala
    450                 455                 460

Ala Thr Phe Asn Met Gln Arg Asn Glu Ala Arg Gln Leu Lys Ser Asn
465                 470                 475                 480

Asn Pro Phe Lys Phe Leu Val Pro Pro Arg Gln Ser Glu Met Arg Ala
                485                 490                 495

Ser Ala

<210>  229
<211>  531
<212>  PRT
<213>  Medicago truncatula

<400>  229

Met Ala Lys Leu Leu Ala Leu Ser Leu Ser Leu Cys Phe Leu Leu Phe
1               5                   10                  15

Ser Gly Cys Phe Ala Ile Arg Glu His Gln Pro His Gln Lys Gln Gln
            20                  25                  30

Pro Gln Gln Asn Glu Cys Gln Leu Glu Gln Leu Asn Ala Leu Glu Pro
        35                  40                  45

Asp Asn Arg Ile Glu Ser Glu Gly Gly Ile Ile Glu Thr Trp Asn Pro
    50                  55                  60

Asn Asn Arg Gln Phe Arg Cys Ala Gly Val Ala Leu Ser Arg Cys Thr
65                  70                  75                  80

Leu Gln Arg Asn Ser Leu Arg Arg Pro Phe Tyr Ser Asn Ala Pro Gln
                85                  90                  95

Glu Ile Phe Ile Gln Gln Gly Ser Gly Tyr Phe Gly Met Val Phe Pro
            100                 105                 110

Gly Cys Pro Glu Thr Phe Glu Glu Pro Gln Glu Ser Glu Gln Arg Glu
        115                 120                 125

Ser Arg Arg Ile Arg Glu Ser Glu Gln Gly Glu Ser Arg Arg Ile Arg
    130                 135                 140

Glu Ser Glu Gln Gly Glu Gly Arg Arg Phe Arg Asp Ser His Gln Lys
145                 150                 155                 160

Val Asn Arg Phe Arg Glu Gly Asp Leu Ile Ala Val Pro Thr Gly Thr
                165                 170                 175

Val Phe Trp Met Tyr Asn Asp Gln Asp Thr Pro Val Ile Ala Val Ser
            180                 185                 190

Leu Ile Asp Thr Gly Ser Phe Gln Asn Gln Leu Asp Glu Met Pro Arg
        195                 200                 205

Arg Phe Tyr Leu Ala Gly Asn Gln Glu Gln Glu Phe Leu Gln Tyr Gln
    210                 215                 220

Gln Gln Gln Val Arg Gly Arg Gly Glu Gln Arg Arg Gly Arg Glu Gln
225                 230                 235                 240

Gln Glu Asn Glu Gly Gly Asn Ile Phe Ser Gly Phe Lys Arg Asp Phe
                245                 250                 255

Leu Glu Asp Ala Leu Asn Val Asn Arg His Ile Val Asp Arg Leu Gln
            260                 265                 270

Gly Arg Asn Glu Asp Glu Glu Lys Gly Ala Ile Val Lys Val Arg Gly
        275                 280                 285

Gly Leu Ser Phe Val Thr Pro Pro Glu Arg Gln Ser Arg His Gln Gly
    290                 295                 300

Gly Ser Ile Ile Glu Glu Asp Glu Asp Glu Glu Asp Glu Trp Arg Arg
305                 310                 315                 320

Pro His His Gln Lys Ser Arg Arg Gly Glu Glu Glu Glu Arg Pro Cys
                325                 330                 335

Arg Arg Gly Gln Lys Cys Glu Arg Ser Asn Gly Leu Glu Glu Thr Ile
            340                 345                 350

Cys Thr Ala Arg Leu Arg Gln Asn Ile Gly Ser Ser Ser Ser Pro Asp
        355                 360                 365

Ile Tyr Asn Pro Glu Ala Gly Arg Ile Lys Thr Val Thr Ser Phe Asp
    370                 375                 380

Leu Pro Ala Leu Arg Trp Leu Arg Leu Ser Ala Glu His Gly Thr Leu
385                 390                 395                 400

His Arg Asn Ala Met Phe Val Pro His Tyr Asn Leu Asn Ala Asn Ser
                405                 410                 415

Ala Ile Tyr Ala Leu Arg Gly Arg Ala Arg Leu Gln Val Val Asn Cys
            420                 425                 430

Asn Gly Asn Thr Val Phe Asp Gly Glu Leu Glu Ala Gly Arg Val Leu
        435                 440                 445

Ile Val Pro Gln Asn Phe Ala Val Ala Ala Lys Ser Met Ser Asp Arg
    450                 455                 460

Phe Gln Tyr Val Ser Phe Lys Thr Asn Asp Asn Ala Ala Ile Ala Arg
465                 470                 475                 480

Leu Ala Gly Thr Gln Ser Thr Leu Ser Gly Val Pro Met Asp Val Leu
                485                 490                 495

Ala Ala Thr Tyr Asn Met Asp Arg Asn Glu Ala Arg Gln Leu Lys Asn
            500                 505                 510

Asn Asn Leu Tyr Lys Phe Leu Val Pro Pro Arg Glu Ser Glu Arg Arg
        515                 520                 525

Ala Ala Ala
    530

<210>  230
<211>  476
<212>  PRT
<213>  Lotus japonicus

<400>  230

Met Ala Tyr Lys Leu Phe Ala Leu Ser Leu Ser Phe Cys Phe Leu Leu
1               5                   10                  15

Phe Gly Gly Cys Phe Ala Ile Arg Gln Gln Ser Gln Gln Gln Asn Glu
            20                  25                  30

Cys Gln Leu Glu Arg Leu Asn Ala Leu Lys Pro Asp Asn Arg Ile Glu
        35                  40                  45

Ser Glu Ala Gly Tyr Ile Glu Thr Trp Asn Pro Thr Asn Asn Gln Phe
    50                  55                  60

Arg Cys Ala Gly Val Ala Leu Ser Arg Cys Thr Leu Arg Arg Asn Gly
65                  70                  75                  80

Leu Lys Arg Pro Ser Tyr Ser Asn Ala Pro Gln Glu Ile Phe Ile Gln
                85                  90                  95

Gln Gly Ser Gly Ile Phe Gly Met Ile Phe Pro Gly Cys Pro Glu Thr
            100                 105                 110

Val Glu Glu Pro Phe Glu Ser Asp Gln Gln Gly Arg Arg Asp Arg His
        115                 120                 125

Gln Lys Val Asn Arg Phe Arg Glu Gly Asp Val Ile Ala Val Pro Pro
    130                 135                 140

Gly Val Val Phe Trp Met Tyr Asn Glu Glu Glu Thr Pro Val Ile Ala
145                 150                 155                 160

Val Ser Leu Ile Asp Thr Gly Ser Tyr Leu Asn Gln Leu Asp Gln Met
                165                 170                 175

Pro Arg Arg Phe Tyr Leu Ser Gly Asn Gln Glu Gln Glu Phe Leu Gln
            180                 185                 190

Tyr Gln Arg Gln Glu Val Arg Gly Arg Glu Glu Glu Asn Gln Gly Gly
        195                 200                 205

Asn Ile Phe Ser Gly Phe Gly Gly Glu Phe Leu Glu Asp Ala Leu Asn
    210                 215                 220

Ile Asp Arg Asn Ile Val His Lys Leu Gln Gly Arg Asp Glu Glu Gln
225                 230                 235                 240

Asp Lys Gly Ala Ile Val Arg Val Lys Gly Gly Leu Ser Val Ile Thr
                245                 250                 255

Pro Pro Glu Arg Gln Ser His Arg Arg Gly Ser Glu Glu Glu Glu Asp
            260                 265                 270

Glu Glu Glu Asp Arg Pro Ser Arg His Gln Ser Arg Gly Gly Ser Arg
        275                 280                 285

Arg Asn Gly Leu Glu Glu Thr Ile Cys Thr Val Arg Leu Arg Met Asn
    290                 295                 300

Ile Gly Lys Ser Ser Ser Pro Asp Ile Phe Asn Pro Gln Ala Gly Arg
305                 310                 315                 320

Ile Lys Thr Ala Thr Gly Phe Asp Phe Pro Ala Leu Arg Phe Leu Lys
                325                 330                 335

Leu Ser Ala Glu His Gly Ser Leu Asn Arg Asn Ala Met Val Val Pro
            340                 345                 350

His Tyr Asn Leu Asn Ala Asn Ser Ile Ile Tyr Ala Leu Arg Gly Arg
        355                 360                 365

Ala Trp Ile Gln Val Val Asn Cys Lys Gly Asn Arg Ile Phe Asp Gly

370                 375                 380

Glu Leu Glu Glu Gly Gln Val Leu Ile Val Pro Gln Asn Phe Val Val
385                 390                 395                 400

Ala Ala Arg Ser Met Ser Asp Lys Phe Asn Tyr Val Ala Phe Lys Thr
                405                 410                 415

Asn Asp Met Pro Thr Met Ala Lys Leu Ala Gly Ala Thr Ser Glu Ile
            420                 425                 430

Gln Ala Met Pro Leu Glu Val Ile Gln Asn Ala Phe Asn Leu Glu Arg
        435                 440                 445

Glu Gln Ala Lys Gln Val Lys Phe Asn Asn Arg Phe Asn Phe Leu Val
    450                 455                 460

Pro Pro Arg Glu Gln Ser Gln Arg Arg Ala Ser Ala
465                 470                 475

<210>  231
<211>  526
<212>  PRT
<213>  PISUM ABYSSINICUM

<400>  231

Met Ala Thr Thr Val Glu Ser Arg Phe Pro Leu Leu Leu Phe Pro Gly
1               5                   10                  15

Ile Ile Phe Leu Ala Ser Val Cys Val Thr Tyr Ala Asn Tyr Asp Glu
            20                  25                  30

Gly Ser Glu Thr Arg Val Pro Gly Gln Arg Glu Arg Gly Arg Gln Glu
        35                  40                  45

Gly Glu Lys Glu Glu Lys Arg His Gly Glu Trp Arg Pro Ser Tyr Glu
    50                  55                  60

Lys Glu Glu Asp Glu Glu Glu Lys Gln Lys Tyr Arg Tyr Gln Arg Glu
65                  70                  75                  80

Lys Glu Asp Glu Glu Glu Lys Gln Lys Tyr Arg Tyr Gln Arg Glu Lys
                85                  90                  95

Lys Glu Glu Lys Glu Val Gln Pro Gly Arg Glu Arg Trp Glu Arg Glu
            100                 105                 110

Glu Asp Glu Glu Gln Val Asp Glu Glu Trp Arg Gly Ser Gln Arg Arg

115                 120                 125

Gln Asp Pro Glu Glu Arg Ala Arg Leu Arg His Arg Glu Glu Arg Thr
    130                 135                 140

Lys Arg Asp Arg Arg His Lys Arg Glu Gly Glu Glu Glu Glu Arg Ser
145                 150                 155                 160

Ser Glu Ser Gln Glu Gln Arg Asn Pro Phe Leu Phe Lys Ser Asn Lys
                165                 170                 175

Phe Leu Thr Leu Phe Glu Asn Glu Asn Gly His Ile Arg Arg Leu Gln
            180                 185                 190

Arg Phe Asp Lys Arg Ser Asp Leu Phe Glu Asn Leu Gln Asn Tyr Arg
        195                 200                 205

Leu Val Glu Tyr Arg Ala Lys Pro His Thr Ile Phe Leu Pro Gln His
    210                 215                 220

Ile Asp Ala Asp Leu Ile Leu Val Val Leu Asn Gly Lys Ala Ile Leu
225                 230                 235                 240

Thr Val Leu Ser Pro Asn Asp Arg Asn Ser Tyr Asn Leu Glu Arg Gly
                245                 250                 255

Asp Thr Ile Lys Ile Pro Ala Gly Thr Thr Ser Tyr Leu Val Asn Gln
            260                 265                 270

Asp Asp Glu Glu Asp Leu Arg Val Val Asp Phe Val Ile Pro Val Asn
        275                 280                 285

Arg Pro Gly Lys Phe Glu Ala Phe Gly Leu Ser Glu Asn Lys Asn Gln
    290                 295                 300

Tyr Leu Arg Gly Phe Ser Lys Asn Ile Leu Glu Ala Ser Leu Asn Thr
305                 310                 315                 320

Lys Tyr Glu Thr Ile Glu Lys Val Leu Leu Glu Glu Gln Glu Lys Lys
                325                 330                 335

Pro Gln Gln Leu Arg Asp Arg Lys Arg Arg Gln Gln Gly Gly Glu Arg
            340                 345                 350

Asp Ala Ile Ile Lys Val Ser Arg Glu Gln Ile Glu Glu Leu Arg Lys
        355                 360                 365

Leu Ala Lys Ser Ser Ser Lys Lys Ser Leu Pro Ser Glu Phe Glu Pro
    370                 375                 380

Phe Asn Leu Arg Ser His Lys Pro Glu Tyr Ser Asn Lys Phe Gly Lys
385                 390                 395                 400

Leu Phe Glu Ile Thr Pro Glu Lys Lys Tyr Pro Gln Leu Gln Asp Leu
                405                 410                 415

Asp Ile Leu Val Ser Cys Val Glu Ile Asn Lys Gly Ala Leu Met Leu
            420                 425                 430

Pro His Tyr Asn Ser Arg Ala Ile Val Val Leu Leu Val Asn Glu Gly
        435                 440                 445

Lys Gly Asn Leu Glu Leu Leu Gly Leu Lys Asn Glu Gln Gln Glu Arg
    450                 455                 460

Glu Asp Arg Lys Glu Arg Asn Asn Glu Val Gln Arg Tyr Glu Ala Arg
465                 470                 475                 480

Leu Ser Pro Gly Asp Val Val Ile Ile Pro Ala Gly His Pro Val Ala
                485                 490                 495

Ile Ser Ala Ser Ser Asn Leu Asn Leu Leu Gly Phe Gly Thr Asn Ala
            500                 505                 510

Glu Asn Asn Gln Arg Asn Phe Leu Ser Gly Ser Asp Asp Asn
        515                 520                 525

<210>  232
<211>  541
<212>  PRT
<213>  LATHYRUS ANNUUS

<400>  232

Met Ala Thr Thr Ile Lys Ser Arg Phe Pro Leu Leu Leu Leu Leu Gly
1               5                   10                  15

Ile Ile Phe Leu Ala Ser Val Cys Val Thr Trp Ala Asn Tyr Asp Glu
            20                  25                  30

Gly Ser Glu Pro Arg Val Pro Gly Gln Arg Glu Arg Gly Arg Gln Glu
        35                  40                  45

Gly Glu Lys Glu Glu Lys Arg His Gly Glu Trp Arg Pro Ser Tyr Glu
    50                  55                  60

Glu Glu Tyr Asp Glu Gly Leu Glu Pro Lys Val Pro Gly Lys Arg Glu
65                  70                  75                  80

Arg Gly Arg Gln Glu Gly Glu Lys Glu Glu Lys Arg His Glu Glu Trp
                85                  90                  95

Arg Pro Ser Tyr Glu Lys Glu Glu Asp Glu Glu Glu Lys Gln Lys Tyr
            100                 105                 110

Asn Tyr Gln Arg Glu Lys Lys Glu His Lys Glu Val Gln Pro Gly Arg
        115                 120                 125

Glu Arg Trp Glu Arg Lys Gln Asp Glu Lys Gln Val Glu Glu Asp Glu
    130                 135                 140

Glu Pro Gly Glu Glu Gln Trp Arg Gly Ser Lys Arg His Glu Asp Pro
145                 150                 155                 160

Glu Glu Arg Ala Arg Leu Arg His Arg Glu Glu Lys Thr Lys Ser Tyr
                165                 170                 175

Val Glu Asp Asn Glu Glu Thr Ser Ser Lys Glu Gly Arg Asn Pro Phe
            180                 185                 190

Leu Phe Lys Ser Asn Lys Phe Leu Thr Leu Phe Glu Asn Glu Asn Gly
        195                 200                 205

His Ile Arg Arg Leu Gln Arg Phe Asp Glu Arg Ser Asp Ile Phe Glu
    210                 215                 220

Asn Leu Gln Asn Tyr Arg Leu Val Glu Tyr Arg Ala Lys Pro His Thr
225                 230                 235                 240

Met Phe Leu Pro Gln His Ile Asp Ala Asp Leu Ile Leu Val Val Leu
                245                 250                 255

Asn Gly Lys Ala Ile Leu Thr Val Leu Ser Pro Asn Asp Arg Asn Ser
            260                 265                 270

Tyr Asn Leu Glu Arg Gly Asp Thr Val Lys Leu Pro Ala Gly Thr Thr
        275                 280                 285

Ser Tyr Leu Val Asn Gln Asp Asp Glu Glu Asp Leu Arg Val Val Asp
    290                 295                 300

Leu Ala Ile Pro Val Asn Arg Pro Gly Lys Phe Glu Ala Phe Gly Leu
305                 310                 315                 320

Ser Ala Asn Lys Asn Gln Tyr Leu Arg Gly Phe Ser Lys Asn Ile Leu
                325                 330                 335

Glu Ala Ser Leu Asn Thr Lys Tyr Glu Thr Ile Glu Lys Val Leu Leu
            340                 345                 350

Glu Glu Arg Arg Asp Gln Lys Gly Arg Gln Gln Gly Gln Glu Thr Asn
        355                 360                 365

Ala Ile Val Lys Val Ser Arg Glu Gln Ile Glu Glu Leu Arg Lys Leu
    370                 375                 380

Ala Lys Ser Ser Ser Lys Lys Ser Leu Leu Ser Glu Ser Glu Pro Leu
385                 390                 395                 400

Asn Leu Arg Ser Gln Asn Pro Lys Tyr Ser Asn Lys Phe Gly Lys Phe
                405                 410                 415

Phe Glu Ile Thr Pro Gln Lys Lys Tyr Pro Gln Leu Gln Asp Leu Asp
            420                 425                 430

Val Ser Ile Ser Cys Val Glu Ile Asn Lys Gly Ala Leu Leu Leu Pro
        435                 440                 445

His Tyr Asn Ser Arg Ser Ile Gly Ile Leu Leu Val Asn Glu Gly Lys
    450                 455                 460

Gly Asn Leu Glu Leu Val Gly Phe Lys Asn Glu Gln Gln Arg Gln Arg
465                 470                 475                 480

Glu Asn Glu Glu Thr Asn Lys Lys Leu Gln Arg Tyr Glu Ala Arg Leu
                485                 490                 495

Ser Ser Gly Asp Val Val Val Ile Pro Glu Gly His Pro Val Ala Ile
            500                 505                 510

Ser Ala Ser Ser Asn Leu Asn Leu Leu Gly Phe Gly Ile Asn Ala Ala
        515                 520                 525

Asn Asn Gln Arg Asn Phe Leu Thr Gly Ser Asp Asp Asn
    530                 535                 540

<210>  233
<211>  589
<212>  PRT
<213>  Vicia villosa

<400>  233

Met Ala Thr Thr Ile Lys Ser Arg Phe Pro Val Leu Leu Leu Leu Gly
1               5                   10                  15

Ile Ile Phe Leu Thr Ser Val Cys Val Thr Tyr Ala Asn Tyr Asp Glu
            20                  25                  30

Gly Arg Glu Pro Ser Val Pro Gly Gln Arg Glu Arg Gly Arg Gln Glu
        35                  40                  45

Gly Glu Lys Glu Glu Lys Arg His Gly Glu Trp Arg Pro Ser Glu Glu
    50                  55                  60

Asp Glu Glu Glu Lys Tyr Lys Tyr Glu Glu Gly Arg Val Pro Gly Gln
65                  70                  75                  80

Arg Glu Arg Gly Arg Gln Glu Gly Glu Lys Glu Glu Lys Arg His Gly
                85                  90                  95

Lys Trp Arg Pro Ser Glu Glu Glu Asp Glu Glu Glu Lys Tyr Arg Tyr
            100                 105                 110

Glu Glu Gly Ser Glu Pro Arg Gly Pro Gly Gln Arg Glu Thr Gly Arg
        115                 120                 125

Gln Glu Gly Glu Lys Glu Lys Gln Arg Pro Glu Arg Glu Pro Ser Tyr
    130                 135                 140

Glu Lys Glu Glu Asp Glu Glu Glu Lys Gln Lys Tyr Gln Tyr His Arg
145                 150                 155                 160

Glu Lys Lys Glu Gln Arg Glu Val Arg Pro Gly Arg Glu Arg Phe Glu
                165                 170                 175

Arg His Glu Asp Glu Glu Gln Trp Arg Gly Ile Gln Arg His Glu Asp
            180                 185                 190

Pro Glu Glu Arg Ala Arg Glu Arg Tyr Arg Ala Glu Ile Ala Lys Arg
        195                 200                 205

Gln Val Glu Glu Glu Arg Glu Glu Arg Asp Ile Pro His Glu Arg Glu
    210                 215                 220

Gln Arg Asn Pro Phe Leu Phe Lys Ser Asn Lys Phe Gln Thr Leu Phe
225                 230                 235                 240

Gln Asn Glu Asn Gly Tyr Ile Arg Arg Leu Gln Arg Phe Asp Lys Arg
                245                 250                 255

Ser Asp Leu Phe Glu Asn Leu Gln Asn Tyr Arg Leu Val Glu Tyr Arg
            260                 265                 270

Ala Lys Pro His Thr Ile Phe Leu Pro Gln His Ile Asp Ala Asp Leu
        275                 280                 285

Ile Ile Val Val Leu Ser Gly Arg Ala Ile Leu Thr Val Leu Ser Pro
    290                 295                 300

Asp Asp Arg Asn Ser Tyr Asn Leu Glu Arg Gly Asp Thr Ile Lys Leu
305                 310                 315                 320

Pro Ala Gly Thr Thr Ser Tyr Leu Val Asn Gln Asp Asp Glu Glu Asp
                325                 330                 335

Leu Arg Val Val Asp Leu Ala Ile Pro Val Asn Arg Pro Gly Lys Val
            340                 345                 350

Glu Ser Phe Leu Leu Ser Gly Asn Lys Asn Gln Tyr Leu Arg Gly Phe
        355                 360                 365

Ser Lys Asn Ile Leu Glu Ala Ser Phe Asn Thr Asn Tyr Glu Thr Ile
    370                 375                 380

Glu Arg Val Leu Leu Glu Glu Gln Asp Lys Glu Ser Gln Gln Ser Ile
385                 390                 395                 400

Gly Gln Lys Arg Arg Ser Gln Arg Gln Glu Thr Asn Ala Leu Val Lys
                405                 410                 415

Val Ser Arg Glu Gln Leu Glu Asp Leu Lys Arg Leu Ala Lys Ser Ser
            420                 425                 430

Ser Gln Glu Gly Leu Ser Ser Gln Phe Glu Pro Ile Asn Leu Arg Ser
        435                 440                 445

Gln Asn Pro Lys Tyr Ser Asn Lys Phe Gly Lys Val Phe Glu Ile Thr
    450                 455                 460

Pro Glu Lys Lys Tyr Pro Gln Leu Gln Asp Leu Asp Leu Phe Val Ser
465                 470                 475                 480

Ser Val Asp Ile Lys Glu Gly Ala Leu Met Leu Pro His Tyr Asn Ser
                485                 490                 495

Arg Ala Ile Val Val Leu Leu Val Asn Glu Gly Arg Gly Asn Leu Glu

500                 505                 510

Leu Val Gly Leu Lys Asn Glu Gln Gln Glu Gln Arg Glu Lys Glu Asp
        515                 520                 525

Glu Gln Gln Glu Arg Asn Asn Gln Val Gln Arg Tyr Glu Ala Arg Leu
    530                 535                 540

Ser Pro Gly Asp Val Val Ile Ile Pro Ala Gly His Pro Val Ala Val
545                 550                 555                 560

Arg Ala Ser Ser Asp Leu Asn Leu Leu Ala Phe Gly Ile Asn Ala Glu
                565                 570                 575

Asn Asn Gln Arg Asn Phe Leu Ala Gly Ser Asp Asp Asn
            580                 585

<210>  234
<211>  463
<212>  PRT
<213>  Medicago truncatula

<400>  234

Met Ala Ile Lys Ala Pro Phe Gln Leu Leu Met Leu Leu Gly Ile Phe
1               5                   10                  15

Phe Leu Ala Ser Val Cys Val Ser Ser Arg Asp Asp Arg His Asp Gln
            20                  25                  30

Glu Asn Pro Phe Phe Phe Asn Ala Asn His Phe Gln Thr Leu Phe Glu
        35                  40                  45

Asn Glu Asn Gly His Ile Arg Leu Leu Gln Arg Phe Asp Lys Arg Ser
    50                  55                  60

Lys Ile Phe Glu Asn Leu Gln Asn Tyr Arg Leu Leu Glu Tyr His Ser
65                  70                  75                  80

Lys Pro His Thr Leu Phe Leu Pro Gln His Asn Asp Ala Asp Phe Ile
                85                  90                  95

Leu Ala Val Leu Ser Gly Lys Ala Ile Leu Thr Val Leu Asn Pro Asp
            100                 105                 110

Asn Arg Asn Ser Phe Asn Leu Glu Arg Gly Asp Thr Ile Lys Leu Pro
        115                 120                 125

Ala Gly Ser Ile Ala Tyr Leu Ala Asn Arg Asp Asp Asn Glu Asp Leu

130                 135                 140

Arg Val Leu Asp Leu Ala Ile Pro Val Asn Arg Pro Gly Lys Phe Gln
145                 150                 155                 160

Ser Phe Ser Leu Ser Gly Ser Gln Asn Gln Gln Ser Phe Phe Ser Gly
                165                 170                 175

Phe Ser Lys Asn Ile Leu Glu Ala Ala Phe Asn Ala Asn Tyr Glu Glu
            180                 185                 190

Ile Glu Arg Val Leu Ile Glu Glu His Glu Gln Glu Pro Gln His Arg
        195                 200                 205

Arg Gly Leu Arg Lys Asp Arg Arg Gln Gln Ser Gln Asp Ser Asn Val
    210                 215                 220

Ile Val Lys Val Ser Arg Glu Gln Ile Glu Glu Leu Ser Arg His Ala
225                 230                 235                 240

Lys Ser Ser Ser Arg Arg Ser Gly Ser Ser Glu Ser Ala Pro Phe Asn
                245                 250                 255

Leu Arg Ser Arg Glu Pro Ile Tyr Ser Asn Glu Phe Gly Asn Phe Phe
            260                 265                 270

Glu Ile Thr Pro Glu Lys Asn Pro Gln Leu Lys Asp Leu Asp Ile Leu
        275                 280                 285

Val Asn Tyr Ala Glu Ile Arg Glu Gly Ser Leu Leu Leu Pro His Phe
    290                 295                 300

Asn Ser Arg Ala Thr Val Ile Val Val Val Asp Glu Gly Lys Gly Glu
305                 310                 315                 320

Phe Glu Leu Val Gly Gln Arg Asn Glu Asn Gln Gln Glu Gln Arg Glu
                325                 330                 335

Glu Asp Glu Gln Gln Glu Glu Glu Arg Ser Gln Gln Val Gln Arg Tyr
            340                 345                 350

Arg Ala Arg Leu Ser Pro Gly Asp Val Tyr Val Ile Pro Ala Gly His
        355                 360                 365

Pro Thr Val Val Ser Ala Ser Ser Asp Leu Ser Leu Leu Gly Phe Gly
    370                 375                 380

Ile Asn Ala Glu Asn Asn Glu Arg Asn Phe Leu Ala Gly Glu Glu Asp
385                 390                 395                 400

Asn Val Ile Ser Gln Ile Glu Arg Pro Val Lys Glu Val Ala Phe Pro
                405                 410                 415

Gly Ser Ala Gln Asp Val Glu Ser Leu Leu Lys Asn Gln Arg Gln Ser
            420                 425                 430

Tyr Phe Ala Asn Ala Gln Pro Gln Gln Arg Glu Arg Glu Glu Gly Arg
        435                 440                 445

Ser Gln Arg Gln Arg Glu Leu Ile Ser Ser Ile Leu Gly Val Phe
    450                 455                 460

<210>  235
<211>  499
<212>  PRT
<213>  VICIA PEREGRINA

<400>  235

Met Ala Thr Thr Phe Lys Ser Arg Phe Ser Leu Leu Leu Leu Leu Gly
1               5                   10                  15

Ile Ile Phe Leu Ala Phe Val Cys Val Thr Cys Ala Asn Tyr Asp Glu
            20                  25                  30

Gly Ser Glu Pro Arg Val Pro Gly Gln Arg Glu Arg Gly Arg Gln Glu
        35                  40                  45

Gly Glu Lys Glu Glu Gln Ser Arg Glu Arg His Pro Gln Arg Glu Pro
    50                  55                  60

Ser Arg Glu Lys Glu Glu Asp Glu Glu Glu Lys Gln Lys Tyr Asp Glu
65                  70                  75                  80

Gly Thr Glu Pro Arg Val Pro Gly Gln Arg Glu Arg Gly Arg Gln Glu
                85                  90                  95

Gly Glu Lys Glu Glu Gln Arg Arg Glu Arg His Pro Gly Gln Arg Glu
            100                 105                 110

Pro Ser Gln Glu Glu Asp Glu Glu Arg Glu Glu Ser Asp Arg Arg Gln
        115                 120                 125

Glu Gly Ser Ser Lys Ser Glu Glu Gln Arg Asn Pro Phe Leu Phe Lys
    130                 135                 140

Ser Asn Lys Phe Leu Thr Leu Phe Gln Asn Gly Asn Gly His Ile Arg
145                 150                 155                 160

Leu Leu Gln Arg Phe Asp Lys Arg Ser Asp Leu Phe Glu Asn Leu Gln
                165                 170                 175

Asn Tyr Arg Leu Leu Glu Tyr Arg Ala Lys Pro His Thr Ile Phe Leu
            180                 185                 190

Pro Gln His Ile Asp Ala Asp Leu Ile Leu Val Val Leu Ser Gly Arg
        195                 200                 205

Ala Ile Leu Thr Val Leu Ser Pro Asp Asp Arg Asn Ser Tyr Asn Leu
    210                 215                 220

Glu Arg Gly Asp Thr Ile Lys Leu Pro Ala Gly Thr Thr Ser Tyr Pro
225                 230                 235                 240

Leu Asn Gln Asp Asp Glu Glu Asp Leu Arg Val Val Asp Leu Ala Ile
                245                 250                 255

Ser Val Asn Arg Pro Gly Lys Val Glu Ser Phe Asn Leu Ser Gly Asn
            260                 265                 270

Lys Asn Gln Tyr Leu Arg Gly Phe Ser Glu Asn Ile Leu Glu Ala Ser
        275                 280                 285

Phe Asn Thr Lys Tyr Glu Thr Ile Glu Lys Val Leu Leu Glu Glu Gln
    290                 295                 300

Asp Lys Glu Ser Gln Gln Pro Arg Gly Gln Arg Leu Gln Arg Gln Glu
305                 310                 315                 320

Thr Asn Ala Leu Val Lys Val Ser Arg Glu Gln Val Glu Glu Leu Lys
                325                 330                 335

Arg Leu Ala Arg Thr Ser Ser Lys Lys Gly Val Ser Ser Glu Phe Glu
            340                 345                 350

Pro Phe Asn Leu Arg Ser His Gly Pro Lys Tyr Ser Asn Lys Phe Gly
        355                 360                 365

Lys Phe Phe Glu Ile Thr Pro Glu Lys Lys Tyr Pro Gln Leu Gln Asp
    370                 375                 380

Leu Asp Ile Ser Val Ser Ser Val Glu Ile Asn Glu Gly Ala Leu Phe
385                 390                 395                 400

Leu Pro His Tyr Asn Ser Arg Ala Ile Val Val Val Leu Val Asp Glu
                405                 410                 415

Gly Lys Gly Asn Leu Glu Leu Val Gly Phe Lys Asn Glu Gln Gln Glu
            420                 425                 430

Gln Arg Glu Lys Glu Asp Glu Gln Glu Glu Arg Asn Lys Gln Val Gln
        435                 440                 445

Arg Tyr Glu Ala Lys Leu Ser Pro Gly Asp Val Val Ile Ile Pro Ala
    450                 455                 460

Gly His Pro Val Ala Val Ser Ala Ser Ser Asn Leu Asn Leu Leu Gly
465                 470                 475                 480

Phe Gly Ile Asn Ala Glu Asn Asn Gln Arg Asn Phe Leu Thr Gly Ser
                485                 490                 495

Asp Asp Asn

<210>  236
<211>  515
<212>  PRT
<213>  VICIA LUTEA

<400>  236

Met Ala Thr Thr Ile Lys Leu Arg Phe Pro Leu Leu Leu Leu Leu Gly
1               5                   10                  15

Val Ile Leu Leu Ala Ser Val Cys Val Thr Cys Ala Asn Tyr Asp Glu
            20                  25                  30

Gly Ser Glu Pro Arg Val Pro Gly Arg Pro Glu Gly Glu Lys Glu Glu
        35                  40                  45

Lys His Arg Gly Lys Leu Arg Pro Ser Tyr Glu Lys Glu Glu Asp Glu
    50                  55                  60

Gly Glu Lys Gln Arg Tyr His Tyr Glu Lys Lys Glu Gln Lys Glu Ala
65                  70                  75                  80

Gln Pro Arg Arg Glu Lys Lys Glu Gln Lys Glu Glu Glu Lys Gln Val
                85                  90                  95

Glu Glu Glu Ser Arg Glu Ser Gln Arg Tyr Glu Asp Pro Gly Glu Arg
            100                 105                 110

Ala Arg Glu Arg Tyr Arg Ala Glu Ile Ile Lys Arg Gln Val Glu Lys
        115                 120                 125

Glu Arg Glu Glu Arg Asp Arg Arg His Gln Arg Glu Gly Glu Glu Glu
    130                 135                 140

Glu Gly Ser Ser Lys Ser Arg Asn Pro Phe Leu Phe Lys Ser Asn Asn
145                 150                 155                 160

Phe Leu Thr Leu Phe Glu Asn Glu Asn Gly His Ile Arg Leu Leu Gln
                165                 170                 175

Arg Phe Asp Lys Arg Ser Asp Leu Phe Glu Asn Leu Gln Asn Tyr Arg
            180                 185                 190

Leu Val Glu Tyr Arg Ala Lys Pro His Thr Ile Phe Leu Pro Gln His
        195                 200                 205

Ile Asp Ala Asp Leu Ile Leu Val Val Leu Ser Gly Lys Ala Ile Leu
    210                 215                 220

Thr Val Leu Ser Pro Asn Asn Arg Asn Ser Tyr Asn Leu Lys Arg Gly
225                 230                 235                 240

Asp Thr Ile Lys Leu Pro Ala Gly Thr Thr Ser Tyr Leu Leu Asn Ser
                245                 250                 255

Asp Asp Glu Glu Asp Leu Arg Met Val Asp Leu Ala Ile Ser Val Asn
            260                 265                 270

Arg Pro Gly Lys Val Glu Ser Phe Asn Leu Ser Gly Asn Lys Asn Gln
        275                 280                 285

Tyr Leu Arg Gly Phe Ser Lys Asn Ile Leu Glu Ala Ser Phe Asn Thr
    290                 295                 300

Lys Tyr Glu Thr Ile Glu Lys Val Leu Leu Glu Glu Gln Asp Lys Glu
305                 310                 315                 320

Ser Gln Gln Ser Ile Gly Gln Lys Arg Ile Ser Gln Arg Gln Glu Thr
                325                 330                 335

Asn Ala Leu Val Lys Val Ser Arg Glu Gln Ile Glu Glu Pro Lys Arg
            340                 345                 350

Leu Ala Arg Ser Ser Ser Arg Lys Gly Val Ser Ser Glu Phe Glu Pro
        355                 360                 365

Ile Asn Leu Arg Ser Gln Arg Pro Lys Tyr Ser Asn Lys Phe Gly Lys
    370                 375                 380

Phe Tyr Glu Ile Ser Pro Glu Lys Lys Tyr Pro Gln Leu Gln Asp Leu
385                 390                 395                 400

Asp Val Ser Val Ser Ser Val Glu Ile Asn Glu Gly Ala Leu Leu Leu
                405                 410                 415

Pro His Tyr Asn Ser Arg Ala Ile Val Thr Val Leu Val Asn Glu Gly
            420                 425                 430

Lys Gly Asn Leu Glu Leu Ile Gly Phe Gln Asn Glu Gln Gln Gly Gln
        435                 440                 445

Arg Glu Lys Glu Asp Glu Gln Gln His Glu Arg Asn Lys Gln Val Gln
    450                 455                 460

Arg Tyr Asp Ala Arg Leu Ser Ser Gly Asp Val Val Ile Ile Pro Ala
465                 470                 475                 480

Gly His Pro Val Ala Val Ser Ala Ser Ser Asn Leu Asp Leu Leu Gly
                485                 490                 495

Phe Gly Ile Asn Ala Glu Asn Ser Gln Arg Asn Phe Leu Thr Gly Ser
            500                 505                 510

Asp Asp Asn
        515

<210>  237
<211>  494
<212>  PRT
<213>  Oryza brachyantha

<400>  237

Met Ala Thr Thr Thr Phe Ser Arg Phe Ser Ile Tyr Phe Cys Val Leu
1               5                   10                  15

Leu Leu Cys His Gly Ser Met Ala Gln Leu Phe Ser Pro Thr Leu Asn
            20                  25                  30

Pro Trp His Ser Ser Arg Arg Gly Gly Ser Arg Asp Cys Arg Phe Asp
        35                  40                  45

Arg Leu Gln Ala Phe Glu Pro Leu Arg Arg Val Arg Ser Glu Ala Gly
    50                  55                  60

Val Thr Glu Tyr Phe Asp Glu Arg Asn Glu Gln Phe Gln Cys Thr Gly
65                  70                  75                  80

Thr Phe Val Ile Arg Arg Val Ile Glu Pro Gln Gly Leu Leu Val Pro
                85                  90                  95

Arg Tyr Thr Asn Thr Pro Gly Val Val Tyr Ile Met Gln Gly Arg Gly
            100                 105                 110

Ser Met Gly Leu Thr Phe Pro Gly Cys Pro Ala Thr Tyr Gln Gln Gln
        115                 120                 125

Phe Gln Gln Phe Leu Pro Glu Gly Gln Ser Gln Ser Gln Lys Phe Arg
    130                 135                 140

Asp Glu His Gln Lys Ile His Gln Phe Arg Gln Gly Asp Ile Val Ala
145                 150                 155                 160

Leu Pro Ala Gly Val Ala His Trp Phe Tyr Asn Glu Gly Asp Thr Pro
                165                 170                 175

Val Val Ala Leu Tyr Val Phe Asp Ile Asn Asn Ser Ala Asn Gln Leu
            180                 185                 190

Glu Pro Arg Gln Lys Asp Phe Leu Leu Ala Gly Asn Asn Asn Arg Glu
        195                 200                 205

Gln Gln Val Tyr Gly Arg Ser Ile Glu Lys His Ser Gly Gln Asn Ile
    210                 215                 220

Phe Ser Gly Phe Asn His Glu Leu Leu Ser Glu Ala Leu Gly Ile Ser
225                 230                 235                 240

Thr Leu Ala Ala Lys Arg Leu Gln Gly Gln Asn Asp His Arg Gly Glu
                245                 250                 255

Ile Ile Arg Val Arg Asn Gly Leu Gln Leu Leu Lys Pro Thr Phe Thr
            260                 265                 270

Gln Gln Gln Glu Gln Ala Gln Ser Gln Tyr Gln Val Gln Tyr Ser Glu
        275                 280                 285

Lys Gln Gln Glu Ser Thr Arg Cys Asn Gly Leu Asp Glu Asn Phe Cys
    290                 295                 300

Thr Ile Asn Ala Arg Leu Asn Ile Glu Asn Pro Ser Arg Ala Asp Thr

305                 310                 315                 320

Tyr Asn Pro Arg Ala Gly Arg Ile Thr His Leu Asn Asn Gln Lys Phe
                325                 330                 335

Pro Ile Leu Asn Leu Val Gln Met Ser Ala Thr Arg Val Asn Leu Tyr
            340                 345                 350

Gln Asn Ala Ile Leu Ser Pro Tyr Trp Asn Val Asn Ala His Ser Leu
        355                 360                 365

Val Tyr Met Val Gln Gly His Ala Arg Val Gln Val Val Ser Asn Leu
    370                 375                 380

Gly Lys Thr Val Phe Asn Ser Val Leu Arg Pro Gly Gln Leu Leu Ile
385                 390                 395                 400

Ile Pro Gln His Tyr Val Val Leu Lys Lys Ala Glu Arg Glu Gly Cys
                405                 410                 415

Gln Tyr Ile Ala Phe Lys Thr Asn Ala Asn Ser Ile Val Ser Gln Leu
            420                 425                 430

Ala Gly Lys Asn Ser Ile Leu Arg Ala Met Pro Val Asp Val Val Ala
        435                 440                 445

Asn Ala Tyr Arg Ile Ser Arg Glu Gln Ala Arg Asp Leu Lys Asn Asn
    450                 455                 460

Arg Gly Glu Glu Leu Gly Ala Phe Thr Pro Lys Phe Glu Gln Gln Ser
465                 470                 475                 480

Tyr Pro Gly Leu Ser Asn Glu Ser Glu Ser Glu Ala Ser Glu
                485                 490

<210>  238
<211>  515
<212>  PRT
<213>  Avena sativa

<400>  238

Met Ala Thr Thr Ser Phe Pro Ser Val Leu Phe Tyr Ser Cys Ile Phe
1               5                   10                  15

Leu Leu Tyr Asn Gly Ser Met Ala Gln Leu Phe Gly Gln Ser Phe Thr
            20                  25                  30

Pro Trp Gln Ser Ser Arg Gln Gly Gly Leu Lys Gly Cys Lys Phe Asp

35                  40                  45

Arg Leu Gln Ala Phe Glu Pro Leu Arg Gln Val Arg Ser Gln Ala Gly
    50                  55                  60

Val Thr Glu Tyr Phe Asp Glu Gln Asn Glu Gln Phe Arg Cys Thr Gly
65                  70                  75                  80

Val Phe Val Ile Arg Arg Val Ile Glu Pro Gln Gly Leu Leu Leu Pro
                85                  90                  95

Gln Tyr His Asn Ala Pro Gly Leu Val Tyr Ile Leu Gln Gly Arg Gly
            100                 105                 110

Tyr Thr Gly Leu Thr Phe Pro Gly Cys Pro Ala Thr Phe Gln Gln Gln
        115                 120                 125

Phe Gln Pro Phe Asp Gln Ala Gln Asp Gln Ser Gln Ser His Leu Lys
    130                 135                 140

Asp Glu His Gln Arg Val His Arg Phe Lys Gln Gly Asp Val Ile Ala
145                 150                 155                 160

Leu Pro Ala Gly Ile Val His Trp Gly Tyr Asn Asp Gly Asp Ala Pro
                165                 170                 175

Val Val Ala Ile Tyr Val Phe Asp Val Asn Asn Asn Ala Asn Gln Leu
            180                 185                 190

Glu Pro Arg Gln Lys Glu Phe Leu Leu Ala Gly Asn Asn Lys Glu Asp
        195                 200                 205

Gln Gln Phe Gly Gln Asn Ile Phe Ser Gly Phe Asn Ile Gln Leu Leu
    210                 215                 220

Ser Glu Ala Leu Gly Ile Ser Gln Gln Ala Ala Gln Arg Ile Gln Ser
225                 230                 235                 240

Gln Lys Glu Gln Arg Gly Glu Ile Ile Arg Val Thr Gln Ala Leu Gln
                245                 250                 255

Phe Leu Lys Pro Thr Met Ser Gln Gln Glu Leu Val Glu His Gln Ala
            260                 265                 270

Tyr Gln Pro Ile Gln Ser Gln Glu Gly Gln Ser Thr Gln Tyr Gln Val
        275                 280                 285

Gly Gln Ser Thr Gln Tyr Gln Glu Gly Gln Ser Thr Gln Tyr Gln Ala
    290                 295                 300

Gly Gln Ser Gln Asp Arg Ser Phe Asn Gly Leu Glu Glu Asn Phe Cys
305                 310                 315                 320

Ser Leu Glu Ala Arg Gln Asn Ile Gly Asn Pro Lys Arg Ala Asp Thr
                325                 330                 335

His Asn Pro Arg Ala Gly Arg Ile Thr Arg Leu His Gly Gln Asn Phe
            340                 345                 350

Pro Ile Leu Asn Leu Val Gln Met Ser Ala Thr Arg Val Asn Leu Tyr
        355                 360                 365

Gln Asn Ala Ile Leu Ser Pro Phe Trp Asn Ile Asn Ala His Ser Val
    370                 375                 380

Val Tyr Met Ile Gln Gly His Ala Gln Val Gln Val Val Asn Asn Asn
385                 390                 395                 400

Gly Gln Thr Val Phe Asn Asp Arg Leu Arg Gln Gly Gln Leu Leu Ile
                405                 410                 415

Val Pro Gln His Tyr Val Val Leu Lys Lys Ala Glu Arg Glu Gly Cys
            420                 425                 430

Gln Tyr Ile Ser Phe Lys Thr Asn Pro Asn Ser Met Val Ser His Ile
        435                 440                 445

Ala Gly Lys Ser Ser Ile Leu Arg Ala Leu Pro Val Asp Val Leu Ala
    450                 455                 460

Asn Ala Tyr Arg Ile Ser Arg Gln Glu Ala Arg Asn Leu Lys Asn Asn
465                 470                 475                 480

Arg Gly Gln Glu Ser Gly Val Phe Thr Pro Lys Phe Thr Gln Thr Ser
                485                 490                 495

Phe Gln Pro Tyr Pro Glu Gly Glu Asp Glu Ser Ser Leu Thr Asn Lys
            500                 505                 510

Ala Ser Glu
        515

<210>  239
<211>  493
<212>  PRT

<213>  Brachypodium distachyon

<400>  239

Met Ala His Thr Ser Phe Ser Ser Val Leu Ser Tyr Phe Cys Ile Phe
1               5                   10                  15

Leu Leu Phe His Gly Ser Met Ala Gln Val Pro Gly Gln Gly Ser Thr
            20                  25                  30

Trp Gln Ser Pro Arg Gln Gly Gly Ser Arg Glu Cys Ser Phe Asp Arg
        35                  40                  45

Leu Gln Thr Ile Glu Pro Leu Thr Gln Val Arg Ser Gln Ala Gly Leu
    50                  55                  60

Thr Glu Tyr Phe Asp Glu Gln Asn Glu Gln Phe Arg Cys Ala Gly Val
65                  70                  75                  80

Ser Val Ile Arg Arg Val Ile Glu Pro Arg Gly Leu Leu Leu Pro Arg
                85                  90                  95

Tyr His Asn Thr Pro Gly Leu Val Tyr Ile Leu Glu Gly Ser Gly Phe
            100                 105                 110

Val Gly Leu Ala Phe Pro Gly Cys Pro Glu Thr Phe Leu Glu Gln Phe
        115                 120                 125

Gln Gln Ser Arg Gln Thr Gln Ser Thr Leu Gly Gln Ser Gln Cys Gln
    130                 135                 140

Ser Gln Ser Gln Lys Leu Gly Asp Val His Gln Arg Val His Gln Phe
145                 150                 155                 160

Thr Gln Gly Asp Val Val Ala Leu Pro Ala Gly Val Ala His Trp Phe
                165                 170                 175

Tyr Asn Gly Gly Asp Ala Pro Val Val Ala Val Tyr Val Phe Asp Val
            180                 185                 190

Asn Asn Asn Ala Asn Gln Leu Glu Pro Arg Gln Lys Glu Phe Leu Leu
        195                 200                 205

Ala Gly Asn Tyr Asn Gly Val Leu Gln Ser Gly Arg Asn Ile Leu Asn
    210                 215                 220

Gly Leu Asn Ala Gln Leu Leu Ser Gln Ala Phe Gly Ile Asn Glu Gln
225                 230                 235                 240

Thr Ser Arg Ile Ile Gln Asn Gln Asn Asp Gly Arg Gly Glu Ile Val
                245                 250                 255

Arg Val Glu Tyr Gly Leu Gln Phe Leu Thr Pro Val Val Thr Gln Gln
            260                 265                 270

Gln Gln Lys Gln Pro Phe Leu Pro Ile Glu Pro Gln Glu Gly Gln Ser
        275                 280                 285

Ser Arg Asn Gly Leu Glu Glu Asn Phe Cys Ser Leu Glu Pro Arg Gln
    290                 295                 300

Asn Ile Glu Asp Pro Asn Arg Ala Asp Thr Tyr Asn Pro Arg Ala Gly
305                 310                 315                 320

Ser Ile Ala Arg Leu Asn Gly Gln Asn Phe Pro Ile Leu Asn Leu Val
                325                 330                 335

Gln Met Ser Ala Thr Arg Val Asn Leu Gln Lys Asn Ala Ile Val Ser
            340                 345                 350

Pro Phe Trp Asn Ile Asn Ala His Ser Val Val Tyr Val Ile Gln Gly
        355                 360                 365

Gln Ala Ser Val Gln Val Val Asn Asn Gln Gly Arg Asn Val Phe Asn
    370                 375                 380

Gly Leu Leu Arg Arg Gly Gln Leu Leu Ile Ile Pro Gln Asn Tyr Val
385                 390                 395                 400

Val Leu Lys Lys Ala Glu Ser Glu Gly Tyr Gln Tyr Ile Ala Phe Lys
                405                 410                 415

Thr Asn Ala Asn Ser Met Val Ser His Ile Ala Gly Lys Asn Ser Ile
            420                 425                 430

Leu Arg Ala Leu Pro Val Asp Val Ile Ala Asn Ala Tyr Arg Ile Ser
        435                 440                 445

Arg Gln Glu Ala Gln Asn Leu Lys Asn Asn Arg Gly Glu Glu Ile Gly
    450                 455                 460

Val Leu Thr Pro Asn Phe Pro Gln Ser Ser Cys Gln Ser Tyr Pro Ile
465                 470                 475                 480

Gly Asp Val Asp Ser Ser Ser Thr Pro Lys Ala Gln Glu
                485                 490

<210>  240
<211>  489
<212>  PRT
<213>  Oryza sativa

<400>  240

Met Ala Gln Phe Ser Phe Gly Gly Ser Pro Leu Gln Ser Pro Arg Gly
1               5                   10                  15

Phe Arg Gly Asp Gln Asp Ser Arg His Gln Cys Arg Phe Glu His Leu
            20                  25                  30

Thr Ala Leu Glu Ala Thr His Gln Gln Arg Ser Glu Ala Gly Phe Thr
        35                  40                  45

Glu Tyr Tyr Asn Ile Glu Ala Arg Asn Glu Phe Arg Cys Ala Gly Val
    50                  55                  60

Ser Val Arg Arg Leu Val Val Glu Ser Lys Gly Leu Val Leu Pro Met
65                  70                  75                  80

Tyr Ala Asn Ala His Lys Leu Val Tyr Ile Val Gln Gly Arg Gly Val
                85                  90                  95

Phe Gly Met Ala Leu Pro Gly Cys Pro Glu Thr Phe Gln Ser Val Arg
            100                 105                 110

Ser Pro Phe Glu Gln Glu Val Ala Thr Ala Gly Glu Ala Gln Ser Ser
        115                 120                 125

Ile Gln Lys Met Arg Asp Glu His Gln Gln Leu His Gln Phe His Gln
    130                 135                 140

Gly Asp Val Ile Ala Val Pro Ala Gly Val Ala His Trp Leu Tyr Asn
145                 150                 155                 160

Asn Gly Asp Ser Pro Val Val Ala Phe Thr Val Ile Asp Thr Ser Asn
                165                 170                 175

Asn Ala Asn Gln Leu Asp Pro Lys Arg Arg Glu Phe Phe Leu Ala Gly
            180                 185                 190

Lys Pro Arg Ser Ser Trp Gln Gln Gln Ser Tyr Ser Tyr Gln Thr Glu
        195                 200                 205

Gln Leu Ser Arg Asn Gln Asn Ile Phe Ala Gly Phe Ser Pro Asp Leu
    210                 215                 220

Leu Ser Glu Ala Leu Ser Val Ser Lys Gln Thr Val Leu Arg Leu Gln
225                 230                 235                 240

Gly Leu Ser Asp Pro Arg Gly Ala Ile Ile Arg Val Glu Asn Gly Leu
                245                 250                 255

Gln Ala Leu Gln Pro Ser Leu Gln Val Glu Pro Val Lys Glu Glu Gln
            260                 265                 270

Thr Gln Ala Tyr Leu Pro Thr Lys Gln Leu Gln Pro Thr Trp Leu Arg
        275                 280                 285

Ser Gly Gly Ala Cys Gly Gln Gln Asn Val Leu Asp Glu Ile Met Cys
    290                 295                 300

Ala Phe Lys Leu Arg Lys Asn Ile Asp Asn Pro Gln Ser Ser Asp Ile
305                 310                 315                 320

Phe Asn Pro His Gly Gly Arg Ile Thr Arg Ala Asn Ser Gln Asn Phe
                325                 330                 335

Pro Ile Leu Asn Ile Ile Gln Met Ser Ala Thr Arg Ile Val Leu Gln
            340                 345                 350

Asn Asn Ala Leu Leu Thr Pro His Trp Thr Val Asn Ala His Thr Val
        355                 360                 365

Met Tyr Val Thr Ala Gly Gln Gly His Ile Gln Val Val Asp His Arg
    370                 375                 380

Gly Arg Ser Val Phe Asp Gly Glu Leu His Gln Gln Gln Ile Leu Leu
385                 390                 395                 400

Ile Pro Gln Asn Phe Ala Val Val Val Lys Ala Arg Arg Glu Gly Phe
                405                 410                 415

Ala Trp Val Ser Phe Lys Thr Asn His Asn Ala Val Asp Ser Gln Ile
            420                 425                 430

Ala Gly Lys Ala Ser Ile Leu Arg Ala Leu Pro Val Asp Val Val Ala
        435                 440                 445

Asn Ala Tyr Arg Leu Ser Arg Glu Asp Ser Arg His Val Lys Phe Asn
    450                 455                 460

Arg Gly Asp Glu Met Ala Val Phe Ala Pro Arg Arg Gly Pro Gln Gln

465                 470                 475                 480

Tyr Ala Glu Trp Gln Ile Asn Glu Lys
                485

<210>  241
<211>  489
<212>  PRT
<213>  Oryza sativa

<400>  241

Met Ala Gln Phe Ser Phe Gly Gly Ser Pro Leu Gln Ser Pro Arg Gly
1               5                   10                  15

Phe Arg Gly Asp Gln Asp Ser Arg His Gln Cys Arg Phe Glu His Leu
            20                  25                  30

Thr Ala Leu Glu Ala Thr His Gln Gln Arg Ser Glu Ala Gly Phe Thr
        35                  40                  45

Glu Tyr Tyr Asn Ile Glu Ala Arg Asn Glu Phe Arg Cys Ala Gly Val
    50                  55                  60

Ser Val Arg Arg Leu Val Val Glu Ser Lys Gly Leu Val Leu Pro Met
65                  70                  75                  80

Tyr Ala Asn Ala His Lys Leu Val Tyr Ile Val Gln Gly Arg Gly Val
                85                  90                  95

Phe Gly Met Ala Leu Pro Gly Cys Pro Glu Thr Phe Gln Ser Val Arg
            100                 105                 110

Ser Pro Phe Glu Gln Glu Val Ala Thr Ala Gly Glu Ala Gln Ser Ser
        115                 120                 125

Met Gln Lys Met Arg Asp Glu His Gln Gln Leu His Gln Phe His Gln
    130                 135                 140

Gly Asp Val Ile Ala Val Pro Ala Gly Val Ala His Trp Leu Tyr Asn
145                 150                 155                 160

Asn Gly Asp Ser Pro Val Val Ala Phe Thr Val Ile Asp Thr Ser Asn
                165                 170                 175

Asn Ala Asn Gln Leu Asp Pro Lys Arg Arg Glu Phe Phe Leu Ala Gly
            180                 185                 190

Lys Pro Arg Ser Ser Trp Gln Gln Gln Ser Tyr Ser Tyr Gln Thr Glu

195                 200                 205

Gln Leu Ser Arg Asn Gln Asn Ile Phe Ala Gly Phe Asn Pro Asp Leu
    210                 215                 220

Leu Ser Glu Ala Leu Ser Val Ser Lys Gln Thr Val Leu Arg Leu Gln
225                 230                 235                 240

Gly Leu Ser Asp Pro Arg Gly Ala Ile Ile Arg Val Glu Asn Gly Leu
                245                 250                 255

Gln Ala Leu Gln Pro Ser Leu Gln Val Glu Pro Val Lys Glu Glu Gln
            260                 265                 270

Thr Gln Ala Tyr Leu Pro Thr Lys Gln Leu Gln Pro Thr Trp Ser Arg
        275                 280                 285

Ser Gly Gly Ala Cys Gly Gln Gln Asn Gly Leu Asp Glu Ile Met Cys
    290                 295                 300

Ala Phe Lys Leu Arg Lys Asn Ile Asp Asn Pro Gln Ser Ser Asp Ile
305                 310                 315                 320

Phe Asn Pro His Gly Gly Arg Ile Thr Arg Ala Asn Ser Gln Asn Phe
                325                 330                 335

Pro Ile Leu Asn Ile Ile Gln Met Ser Ala Thr Arg Ile Val Leu Gln
            340                 345                 350

Asn Asn Ala Leu Leu Thr Pro His Trp Thr Val Asn Ala His Thr Val
        355                 360                 365

Met Tyr Val Thr Ala Gly Gln Gly Arg Ile Gln Val Val Asp His Arg
    370                 375                 380

Gly Arg Ser Val Phe Asp Gly Glu Leu His Gln Gln Gln Ile Leu Leu
385                 390                 395                 400

Ile Pro Gln Asn Phe Ala Val Val Val Lys Ala Arg Arg Glu Gly Phe
                405                 410                 415

Ala Trp Val Ser Phe Lys Thr Asn His Asn Ala Val Asp Ser Gln Ile
            420                 425                 430

Ala Gly Lys Ala Ser Ile Leu Arg Ala Leu Pro Val Asp Val Val Ala
        435                 440                 445

Asn Ala Tyr Arg Leu Ser Arg Glu Asp Ser Arg Arg Val Lys Phe Asn
    450                 455                 460

Arg Gly Asp Glu Met Ala Val Phe Ala Pro Arg Arg Gly Pro Gln Gln
465                 470                 475                 480

Tyr Ala Glu Trp Gln Ile Asn Glu Lys
                485

<210>  242
<211>  504
<212>  PRT
<213>  Oryza brachyantha

<400>  242
   Met Val Asp Met Ser Ile Val Val Pro Val Cys Leu Thr Ile Phe Leu
   1               5                   10                  15
   Leu Ser Gln Val Cys Ile Ala Gln Val Ser Phe Asp Gly Ser Pro Leu
               20                  25                  30
   Tyr Ser Ser Arg Gly Phe Arg Gly Gly Ser Ala Ser Gln Gln Gln Cys
           35                  40                  45
   Arg Phe Glu His Leu Ala Ala Leu Glu Val Thr His Gln Glu Lys Ser
       50                  55                  60
   Glu Ala Gly Ser Ile Glu Tyr Tyr Asn Thr Glu Ala Arg Asp Glu Phe
   65                  70                  75                  80
   Arg Cys Ala Arg Val Ser Ala Arg Arg Leu Val Ile Glu Ser Arg Gly
                   85                  90                  95
   Leu Val Leu Pro Val Tyr Ala Asn Ala His Lys Leu Leu Tyr Ile Val
               100                 105                 110
   Gln Gly Arg Gly Val Phe Gly Met Ala Leu Pro Gly Cys Pro Glu Thr
           115                 120                 125
   Phe Gln Ser Val Arg Ser Ala Phe Glu Met Ala Thr Gly Asp Ala Glu
       130                 135                 140
   Ser Ser Thr Arg Lys Leu Arg Asp Glu His Gln Lys Ile His Gln Phe
   145                 150                 155                 160
   Arg Gln Gly Asp Val Ile Ala Val Pro Pro Gly Val Ala His Trp Leu
                   165                 170                 175
   Tyr Asn Asn Gly Asp Ser Pro Val Val Ala Phe Ser Val Ile Asp Phe
                   180                 185                 190
   Gly Asn Asn Ala Asn Gln Leu Asp Pro Lys Pro Arg Glu Phe Phe Leu
               195                 200                 205
   Ala Gly Lys Pro Trp Gly Trp Gln Gln Val Gln Tyr Ser Tyr Gln Ser
       210                 215                 220
   Glu Gln Gln Ser Lys His Gln Asn Ile Phe Ala Gly Phe Asn Pro Asp
   225                 230                 235                 240
   Leu Leu Ala Glu Ala Leu Ser Val Ser Arg Gln Thr Ala Met Arg Leu
                   245                 250                 255
   Gln Glu Leu Asn Asp Gln Arg Gly Ala Ile Ile Arg Val Glu Gln Gly
                   260                 265                 270
   Leu Gln Leu Ala Leu Asp Pro Ser Phe Gln Ala Glu Gln Glu Gln Glu
               275                 280                 285
   Glu Gln Pro Gln Glu Tyr Leu Ser Ser Gln Gln Gln Gln Pro Thr Trp
       290                 295                 300
   Ser Gln Arg Ser Gly Ala Cys Val Gln Asn Asn Gly Leu Asp Glu Ile
   305                 310                 315                 320
   Met Cys Ala Phe Lys Val Ser Lys Asn Ile Asn Ser Ala Gln Ser Thr
                   325                 330                 335
   Asp Ile Phe Asn Pro Arg Gly Gly Arg Ile Thr Arg Ala Asn Ser Gln
                   340                 345                 350
   Asn Phe Pro Val Leu Asn Ile Ile Gln Met Ser Ala Thr Arg Thr Val
               355                 360                 365
   Leu Gln Asn Asn Ala Leu Leu Thr Pro His Trp Thr Val Asn Ala His
       370                 375                 380
   Thr Val Met Tyr Val Thr Ala Gly Gln Gly Arg Ile Gln Val Val Asp
   385                 390                 395                 400
   His Arg Gly Arg Thr Val Phe Asp Gly Glu Leu Arg Gln Gln Gln Ile
                   405                 410                 415
   Leu Leu Ile Pro Gln Asn Phe Ala Val Ala Val Lys Ala Arg His Glu
                   420                 425                 430
   Gly Phe Ser Trp Val Ser Phe Lys Thr Ser His Asn Ala Ile Asp Ser
               435                 440                 445
   Gln Ile Ala Gly Lys Gly Ser Ile Leu Arg Ala Leu Pro Val Asp Val
           450                 455                 460
   Leu Ala Lys Ala Tyr Met Leu Ser Arg Glu Glu Ser Arg Thr Leu Lys
   465                 470                 475                 480
   Tyr Asn Arg Ala Asp Glu Thr Leu Val Phe Ala Pro Arg Pro Glu Ile
                   485                 490                 495
   Gln Leu Tyr Ala Glu Ser Glu Lys
               500

<210>  243
<211>  543
<212>  PRT
<213>  Pisum fulvum

<400>  243

Met Ala Thr Thr Thr Lys Ser Arg Phe Pro Leu Leu Leu Leu Leu Gly
1               5                   10                  15

Ile Ile Phe Leu Ala Ser Val Val Cys Val Thr Tyr Ala Asn Tyr Asp
            20                  25                  30

Glu Gly Ser Glu Pro Arg Val Pro Gly Arg Arg Glu Arg Gly Arg Gln
        35                  40                  45

Glu Gly Glu Lys Glu Glu Lys Arg His Gly Glu Trp Arg Pro Ser Tyr
    50                  55                  60

Glu Lys Glu Glu Asp Glu Glu Glu Gly Gln Arg Glu Arg Gly Arg Gln
65                  70                  75                  80

Glu Gly Glu Lys Glu Glu Lys Arg His Gly Glu Trp Gly Pro Ser Tyr
                85                  90                  95

Glu Lys Gln Glu Asp Glu Glu Glu Lys Gln Lys Tyr Arg Tyr Gln Arg
            100                 105                 110

Glu Lys Glu Asp Glu Glu Glu Lys Gln Lys Tyr Arg Tyr Gln Arg Glu
        115                 120                 125

Lys Lys Glu Gln Lys Glu Val Gln Pro Gly Arg Glu Arg Trp Glu Arg
    130                 135                 140

Glu Glu Asp Glu Glu His Val Asp Glu Glu Trp Arg Gly Ser Gln Arg
145                 150                 155                 160

His Glu Asp Pro Glu Glu Arg Ala Arg Leu Arg Tyr Arg Glu Glu Arg
                165                 170                 175

Thr Lys Arg Asp Arg Arg His Gln Arg Glu Gly Glu Glu Glu Glu Arg
            180                 185                 190

Ser Ser Glu Ser Gln Glu Arg Arg Asn Pro Phe Leu Phe Lys Ser Asn
        195                 200                 205

Lys Phe Gln Thr Leu Phe Glu Asn Glu Asn Gly His Ile Arg Leu Leu
    210                 215                 220

Gln Arg Phe Asp Lys Arg Ser Asp Leu Phe Glu Asn Leu Gln Asn Tyr
225                 230                 235                 240

Arg Leu Val Glu Tyr Arg Ala Lys Pro His Thr Ile Phe Leu Pro Gln
                245                 250                 255

His Ile Asp Ala Asp Leu Ile Leu Val Val Leu Ser Gly Lys Ala Ile
            260                 265                 270

Leu Thr Val Leu Ser Pro Asn Ala Arg Asn Ser Tyr Asn Leu Glu Arg
        275                 280                 285

Gly Asp Thr Ile Lys Leu Pro Ala Gly Thr Thr Ser Tyr Leu Val Asn
    290                 295                 300

Gln Asp Asp Glu Glu Asp Leu Arg Leu Val Asp Leu Val Ile Pro Val
305                 310                 315                 320

Asn Gly Pro Gly Lys Phe Glu Ala Phe Asp Leu Ser Lys Asn Lys Asn
                325                 330                 335

Gln Tyr Leu Arg Gly Phe Ser Lys Asn Ile Leu Glu Ala Ser Tyr Asn
            340                 345                 350

Thr Lys Tyr Glu Thr Ile Glu Lys Val Leu Leu Glu Glu Gln Glu Lys
        355                 360                 365

Thr Asp Ala Ile Val Lys Val Ser Arg Glu Gln Ile Glu Glu Leu Arg
    370                 375                 380

Lys His Ala Lys Ser Ser Ser Lys Lys Ile Phe Pro Ser Glu Phe Glu
385                 390                 395                 400

Pro Ile Asn Leu Arg Asn His Lys Pro Glu Tyr Ser Asn Lys Phe Gly
                405                 410                 415

Lys Leu Phe Glu Ile Thr Pro Glu Lys Lys Tyr Pro Gln Leu Gln Asp
            420                 425                 430

Leu Asp Ile Phe Val Ser Cys Val Glu Ile Asn Glu Gly Ala Leu Met
        435                 440                 445

Leu Pro His Tyr Asn Ser Arg Ala Ile Val Val Leu Leu Val Asn Glu
    450                 455                 460

Gly Lys Gly Asn Leu Glu Leu Leu Gly Leu Glu Asn Glu Gln Gln Glu
465                 470                 475                 480

Arg Glu Asp Arg Lys Glu Arg Asn Asn Glu Val Gln Arg Tyr Glu Ala
                485                 490                 495

Arg Leu Ser Pro Gly Asp Val Val Ile Ile Pro Ala Gly His Pro Val
            500                 505                 510

Ala Ile Thr Ala Ser Ser Asn Leu Asn Leu Leu Ala Phe Gly Ile Asn
        515                 520                 525

Ala Glu Asn Asn Gln Arg Asn Phe Leu Ser Gly Ser Asp Asp Asn
    530                 535                 540

<210>  244
<211>  526
<212>  PRT
<213>  PISUM ABYSSINICUM

<400>  244

Met Ala Thr Thr Val Glu Ser Arg Phe Pro Leu Leu Leu Phe Pro Gly
1               5                   10                  15

Ile Ile Phe Leu Ala Ser Val Cys Val Thr Tyr Ala Asn Tyr Asp Glu
            20                  25                  30

Gly Ser Glu Thr Arg Val Pro Gly Gln Arg Glu Arg Gly Arg Gln Glu
        35                  40                  45

Gly Glu Lys Glu Glu Lys Arg His Gly Glu Trp Arg Pro Ser Tyr Glu
    50                  55                  60

Lys Glu Glu Asp Glu Glu Glu Lys Gln Lys Tyr Arg Tyr Gln Arg Glu
65                  70                  75                  80

Lys Glu Asp Glu Glu Glu Lys Gln Lys Tyr Arg Tyr Gln Arg Glu Lys
                85                  90                  95

Lys Glu Glu Lys Glu Val Gln Pro Gly Arg Glu Arg Trp Glu Arg Glu
            100                 105                 110

Glu Asp Glu Glu Gln Val Asp Glu Glu Trp Arg Gly Ser Gln Arg Arg
        115                 120                 125

Gln Asp Pro Glu Glu Arg Ala Arg Leu Arg His Arg Glu Glu Arg Thr
    130                 135                 140

Lys Arg Asp Arg Arg His Lys Arg Glu Gly Glu Glu Glu Glu Arg Ser
145                 150                 155                 160

Ser Glu Ser Gln Glu Gln Arg Asn Pro Phe Leu Phe Lys Ser Asn Lys
                165                 170                 175

Phe Leu Thr Leu Phe Glu Asn Glu Asn Gly His Ile Arg Arg Leu Gln
            180                 185                 190

Arg Phe Asp Lys Arg Ser Asp Leu Phe Glu Asn Leu Gln Asn Tyr Arg
        195                 200                 205

Leu Val Glu Tyr Arg Ala Lys Pro His Thr Ile Phe Leu Pro Gln His
    210                 215                 220

Ile Asp Ala Asp Leu Ile Leu Val Val Leu Asn Gly Lys Ala Ile Leu
225                 230                 235                 240

Thr Val Leu Ser Pro Asn Asp Arg Asn Ser Tyr Asn Leu Glu Arg Gly
                245                 250                 255

Asp Thr Ile Lys Ile Pro Ala Gly Thr Thr Ser Tyr Leu Val Asn Gln
            260                 265                 270

Asp Asp Glu Glu Asp Leu Arg Val Val Asp Phe Val Ile Pro Val Asn
        275                 280                 285

Arg Pro Gly Lys Phe Glu Ala Phe Gly Leu Ser Glu Asn Lys Asn Gln
    290                 295                 300

Tyr Leu Arg Gly Phe Ser Lys Asn Ile Leu Glu Ala Ser Leu Asn Thr
305                 310                 315                 320

Lys Tyr Glu Thr Ile Glu Lys Val Leu Leu Glu Glu Gln Glu Lys Lys

325                 330                 335

Pro Gln Gln Leu Arg Asp Arg Lys Arg Arg Gln Gln Gly Gly Glu Arg
            340                 345                 350

Asp Ala Ile Ile Lys Val Ser Arg Glu Gln Ile Glu Glu Leu Arg Lys
        355                 360                 365

Leu Ala Lys Ser Ser Ser Lys Lys Ser Leu Pro Ser Glu Phe Glu Pro
    370                 375                 380

Phe Asn Leu Arg Ser His Lys Pro Glu Tyr Ser Asn Lys Phe Gly Lys
385                 390                 395                 400

Leu Phe Glu Ile Thr Pro Glu Lys Lys Tyr Pro Gln Leu Gln Asp Leu
                405                 410                 415

Asp Ile Leu Val Ser Cys Val Glu Ile Asn Lys Gly Ala Leu Met Leu
            420                 425                 430

Pro His Tyr Asn Ser Arg Ala Ile Val Val Leu Leu Val Asn Glu Gly
        435                 440                 445

Lys Gly Asn Leu Glu Leu Leu Gly Leu Lys Asn Glu Gln Gln Glu Arg
    450                 455                 460

Glu Asp Arg Lys Glu Arg Asn Asn Glu Val Gln Arg Tyr Glu Ala Arg
465                 470                 475                 480

Leu Ser Pro Gly Asp Val Val Ile Ile Pro Ala Gly His Pro Val Ala
                485                 490                 495

Ile Ser Ala Ser Ser Asn Leu Asn Leu Leu Gly Phe Gly Thr Asn Ala
            500                 505                 510

Glu Asn Asn Gln Arg Asn Phe Leu Ser Gly Ser Asp Asp Asn
        515                 520                 525

<210>  245
<211>  589
<212>  PRT
<213>  Vicia villosa

<400>  245

Met Ala Thr Thr Ile Lys Ser Arg Phe Pro Val Leu Leu Leu Leu Gly
1               5                   10                  15

Ile Ile Phe Leu Thr Ser Val Cys Val Thr Tyr Ala Asn Tyr Asp Glu

20                  25                  30

Gly Arg Glu Pro Ser Val Pro Gly Gln Arg Glu Arg Gly Arg Gln Glu
        35                  40                  45

Gly Glu Lys Glu Glu Lys Arg His Gly Glu Trp Arg Pro Ser Glu Glu
    50                  55                  60

Asp Glu Glu Glu Lys Tyr Lys Tyr Glu Glu Gly Arg Val Pro Gly Gln
65                  70                  75                  80

Arg Glu Arg Gly Arg Gln Glu Gly Glu Lys Glu Glu Lys Arg His Gly
                85                  90                  95

Lys Trp Arg Pro Ser Glu Glu Glu Asp Glu Glu Glu Lys Tyr Arg Tyr
            100                 105                 110

Glu Glu Gly Ser Glu Pro Arg Gly Pro Gly Gln Arg Glu Thr Gly Arg
        115                 120                 125

Gln Glu Gly Glu Lys Glu Lys Gln Arg Pro Glu Arg Glu Pro Ser Tyr
    130                 135                 140

Glu Lys Glu Glu Asp Glu Glu Glu Lys Gln Lys Tyr Gln Tyr His Arg
145                 150                 155                 160

Glu Lys Lys Glu Gln Arg Glu Val Arg Pro Gly Arg Glu Arg Phe Glu
                165                 170                 175

Arg His Glu Asp Glu Glu Gln Trp Arg Gly Ile Gln Arg His Glu Asp
            180                 185                 190

Pro Glu Glu Arg Ala Arg Glu Arg Tyr Arg Ala Glu Ile Ala Lys Arg
        195                 200                 205

Gln Val Glu Glu Glu Arg Glu Glu Arg Asp Ile Pro His Glu Arg Glu
    210                 215                 220

Gln Arg Asn Pro Phe Leu Phe Lys Ser Asn Lys Phe Gln Thr Leu Phe
225                 230                 235                 240

Gln Asn Glu Asn Gly Tyr Ile Arg Arg Leu Gln Arg Phe Asp Lys Arg
                245                 250                 255

Ser Asp Leu Phe Glu Asn Leu Gln Asn Tyr Arg Leu Val Glu Tyr Arg
            260                 265                 270

Ala Lys Pro His Thr Ile Phe Leu Pro Gln His Ile Asp Ala Asp Leu
        275                 280                 285

Ile Ile Val Val Leu Ser Gly Arg Ala Ile Leu Thr Val Leu Ser Pro
    290                 295                 300

Asp Asp Arg Asn Ser Tyr Asn Leu Glu Arg Gly Asp Thr Ile Lys Leu
305                 310                 315                 320

Pro Ala Gly Thr Thr Ser Tyr Leu Val Asn Gln Asp Asp Glu Glu Asp
                325                 330                 335

Leu Arg Val Val Asp Leu Ala Ile Pro Val Asn Arg Pro Gly Lys Val
            340                 345                 350

Glu Ser Phe Leu Leu Ser Gly Asn Lys Asn Gln Tyr Leu Arg Gly Phe
        355                 360                 365

Ser Lys Asn Ile Leu Glu Ala Ser Phe Asn Thr Asn Tyr Glu Thr Ile
    370                 375                 380

Glu Arg Val Leu Leu Glu Glu Gln Asp Lys Glu Ser Gln Gln Ser Ile
385                 390                 395                 400

Gly Gln Lys Arg Arg Ser Gln Arg Gln Glu Thr Asn Ala Leu Val Lys
                405                 410                 415

Val Ser Arg Glu Gln Leu Glu Asp Leu Lys Arg Leu Ala Lys Ser Ser
            420                 425                 430

Ser Gln Glu Gly Leu Ser Ser Gln Phe Glu Pro Ile Asn Leu Arg Ser
        435                 440                 445

Gln Asn Pro Lys Tyr Ser Asn Lys Phe Gly Lys Val Phe Glu Ile Thr
    450                 455                 460

Pro Glu Lys Lys Tyr Pro Gln Leu Gln Asp Leu Asp Leu Phe Val Ser
465                 470                 475                 480

Ser Val Asp Ile Lys Glu Gly Ala Leu Met Leu Pro His Tyr Asn Ser
                485                 490                 495

Arg Ala Ile Val Val Leu Leu Val Asn Glu Gly Arg Gly Asn Leu Glu
            500                 505                 510

Leu Val Gly Leu Lys Asn Glu Gln Gln Glu Gln Arg Glu Lys Glu Asp
        515                 520                 525

Glu Gln Gln Glu Arg Asn Asn Gln Val Gln Arg Tyr Glu Ala Arg Leu
    530                 535                 540

Ser Pro Gly Asp Val Val Ile Ile Pro Ala Gly His Pro Val Ala Val
545                 550                 555                 560

Arg Ala Ser Ser Asp Leu Asn Leu Leu Ala Phe Gly Ile Asn Ala Glu
                565                 570                 575

Asn Asn Gln Arg Asn Phe Leu Ala Gly Ser Asp Asp Asn
            580                 585

<210>  246
<211>  576
<212>  PRT
<213>  Lathyrus hirsutus

<400>  246

Met Ala Ile Ile Ile Lys Ser Arg Phe Pro Leu Leu Leu Leu Leu Gly
1               5                   10                  15

Ile Ile Phe Leu Ala Ser Val Cys Ala Thr Trp Ala Asn Tyr Asp Glu
            20                  25                  30

Gly Ser Glu Pro Arg Val Pro Gly Gln Arg Glu Arg Gly Arg Gln Glu
        35                  40                  45

Gly Glu Lys Ala Glu Lys Ser His Glu Lys Trp Arg Pro Ser Tyr Glu
    50                  55                  60

Glu Glu Tyr Asp Glu Gly Ser Glu Pro Arg Val Pro Gly Lys Arg Glu
65                  70                  75                  80

Arg Gly Arg Gln Glu Gly Glu Lys Glu Glu Lys Arg His Gly Glu Trp
                85                  90                  95

Arg Pro Ser His Glu Glu Glu Tyr Asp Glu Gly Ser Glu Pro Arg Val
            100                 105                 110

Pro Thr His Gly Glu Arg Gly Arg Gln Glu Gly Glu Lys Glu Glu Lys
        115                 120                 125

Arg His Glu Glu Trp Arg Pro Ser Tyr Glu Lys Glu Glu Asp Glu Glu
    130                 135                 140

Glu Lys Glu Lys Tyr Lys Tyr Gln Arg Glu Lys Lys Glu Gln Lys Glu
145                 150                 155                 160

Val Gln Pro Gly Arg Glu Lys Trp Glu Arg Lys Gln Asp Glu Lys His
                165                 170                 175

Val Glu Glu Asp Glu Asp Gln Glu Glu Glu Gln Trp Arg Gly Ser Lys
            180                 185                 190

Arg Arg Glu Asp Pro Glu Glu Arg Ala Arg Leu Arg Tyr Arg Glu Glu
        195                 200                 205

Arg Thr Lys Ser Asn Val Glu Glu Glu Thr Glu Glu Arg Arg Asn Pro
    210                 215                 220

Phe Leu Phe Lys Ser Asn Lys Phe Leu Thr Leu Phe Glu Asn Glu Asn
225                 230                 235                 240

Gly His Ile Arg Arg Leu Gln Arg Phe Asp Glu Arg Ser Asp Ile Phe
                245                 250                 255

Glu Asn Leu Gln Asn Tyr Arg Leu Val Glu Tyr Lys Ala Lys Pro His
            260                 265                 270

Thr Met Phe Leu Pro Gln His Ile Asp Ala Asp Leu Ile Ile Val Val
        275                 280                 285

Leu Asn Gly Lys Ala Ile Leu Thr Val Leu Ser Pro Asn Asp Arg Asn
    290                 295                 300

Ser Tyr Asn Leu Glu Arg Gly Asp Thr Ile Lys Leu Pro Ala Gly Thr
305                 310                 315                 320

Thr Ser Tyr Leu Val Asn Gln Asp Asp Glu Glu Asp Leu Arg Val Val
                325                 330                 335

Asp Leu Ala Ile Pro Val Asn Arg Pro Gly Lys Phe Glu Ala Phe Gly
            340                 345                 350

Leu Ser Ala Asn Lys Asn Gln Tyr Leu Arg Gly Phe Ser Lys Asn Ile
        355                 360                 365

Leu Glu Ala Phe Leu Asn Thr Lys Tyr Glu Thr Ile Glu Lys Val Leu
    370                 375                 380

Leu Glu Glu Gln Glu Arg Arg Asp Arg Lys Gly Arg Gln Gln Gly Gln
385                 390                 395                 400

Glu Thr Asn Ala Ile Val Lys Val Ser Arg Glu Gln Ile Glu Glu Leu
                405                 410                 415

Arg Lys Leu Ala Lys Ser Ser Ser Lys Lys Ser Leu Leu Ser Glu Ser
            420                 425                 430

Glu Pro Ile Asn Leu Arg Ser Gln Asn Pro Lys Tyr Ser Asn Lys Phe
        435                 440                 445

Gly Lys Leu Phe Glu Ile Thr Pro Glu Lys Lys Tyr Pro Gln Leu Gln
    450                 455                 460

Asp Leu Asp Val Ser Ile Ser Cys Val Glu Ile Asn Glu Gly Ala Pro
465                 470                 475                 480

Leu Leu Pro His Tyr Asn Ser Arg Ala Ile Val Leu Leu Leu Val Asn
                485                 490                 495

Glu Gly Lys Gly Asn Leu Glu Leu Val Gly Phe Lys Asn Glu Gln Gln
            500                 505                 510

Arg Gln Arg Glu Asn Glu Glu Arg Asn Lys Lys Val Gln Arg Tyr Glu
        515                 520                 525

Ala Arg Leu Ser Pro Gly Asp Val Val Val Ile Pro Ala Gly His Pro
    530                 535                 540

Val Ala Ile Ser Ala Ser Leu Asn Leu Asn Leu Val Gly Phe Gly Val
545                 550                 555                 560

Asn Ala Glu Asn Asn Gln Arg Asn Phe Leu Thr Gly Ser Asp Asp Asn
                565                 570                 575

<210>  247
<211>  564
<212>  PRT
<213>  Lathyrus cicera

<400>  247

Met Ala Thr Ile Ile Lys Ser Arg Phe Pro Leu Leu Leu Leu Leu Gly
1               5                   10                  15

Ile Ile Phe Leu Ala Ser Val Cys Val Thr Leu Ala Asn Tyr Asp Glu
            20                  25                  30

Gly Ser Glu Pro Arg Val Pro Ala Gln Arg Glu Arg Gly Arg Gln Glu
        35                  40                  45

Gly Glu Lys Glu Glu Lys Arg His Gly Glu Trp Arg Pro Ser His Glu
    50                  55                  60

Lys Glu Tyr Asp Glu Gly Ser Glu Pro Arg Val Pro Gly Arg Arg Glu
65                  70                  75                  80

Arg Gly Arg Gln Glu Gly Glu Lys Glu Glu Lys Arg His Gly Glu Trp
                85                  90                  95

Arg Pro Ser Tyr Glu Lys Glu Tyr Asp Glu Gly Ser Glu Pro Arg Val
            100                 105                 110

Pro Gly Arg Arg Glu Arg Gly Arg Gln Glu Gly Glu Lys Glu Glu Lys
        115                 120                 125

Arg His Gly Glu Trp Arg Pro Ser Tyr Glu Lys Glu Tyr Asp Glu Glu
    130                 135                 140

Glu Lys Gln Lys Tyr Gln Tyr Glu Arg Glu Lys Glu Glu Gln Lys Glu
145                 150                 155                 160

Val Gln Pro Gly Arg Glu Arg Trp Glu Arg Lys Glu Asp Glu Glu Lys
                165                 170                 175

Glu Glu Asp Gln Trp Arg Gly Ser Gln Arg His Glu Asp Pro Glu Glu
            180                 185                 190

Arg Ala Arg Leu Arg Tyr Arg Lys Glu Arg Thr Lys Lys Tyr Val Glu
        195                 200                 205

Glu Asp Thr Glu Glu Thr Ser Ser Glu Ser Gln Gly Arg Arg Asn Pro
    210                 215                 220

Phe Leu Phe Lys Ser Asn Lys Phe Leu Thr Leu Phe Glu Asn Glu Asn
225                 230                 235                 240

Gly Tyr Ile Arg Arg Leu Gln Arg Phe Asp Glu Arg Ser Asp Ile Phe
                245                 250                 255

Glu Asn Leu Gln Asn Tyr Arg Leu Val Glu Tyr Arg Ala Lys Pro His
            260                 265                 270

Thr Ile Phe Leu Pro Gln His Ile Asp Ala Asp Leu Ile Leu Val Ile
        275                 280                 285

Leu Asn Gly Lys Ala Ile Leu Thr Val Leu Ser Pro Asn Asp Arg Asn
    290                 295                 300

Ser Tyr Asn Leu Glu Arg Gly Asp Thr Ile Lys Leu Pro Ala Gly Thr

305                 310                 315                 320

Thr Ser Tyr Leu Val Asn Glu Asp Asp Glu Glu Asp Leu Arg Val Val
                325                 330                 335

Asp Leu Val Ile Pro Val Asn Arg Pro Gly Lys Phe Glu Ala Phe Asp
            340                 345                 350

Leu Asn Gln Tyr Leu Gly Gly Phe Ser Lys Ser Val Leu Glu Ala Ser
        355                 360                 365

Leu Asn Thr Lys Tyr Glu Thr Ile Glu Lys Val Leu Leu Glu Glu Gln
    370                 375                 380

Gln Lys Gln Gly Gln Glu Thr Asn Ala Ile Val Lys Val Ser Arg Glu
385                 390                 395                 400

Gln Ile Glu Glu Leu Arg Lys Leu Ala Lys Ser Ser Ser Lys Lys Ser
                405                 410                 415

Leu Leu Ser Glu Leu Glu Pro Val Asn Leu Arg Ser His Ser Pro Lys
            420                 425                 430

Tyr Ser Asn Lys Phe Gly Lys Phe Phe Glu Ile Thr Pro Glu Lys Lys
        435                 440                 445

Tyr Pro Gln Leu Gln Asp Leu Asp Val Ser Ile Ser Cys Val Glu Ile
    450                 455                 460

Asn Glu Gly Ala Leu Leu Leu Pro His Tyr Asn Ser Arg Ala Ile Val
465                 470                 475                 480

Val Val Leu Val Asn Glu Gly Lys Gly Asn Leu Glu Leu Leu Gly Val
                485                 490                 495

Gln Asn Glu Asp Glu Gln Gln Glu Arg Lys Glu Arg Asn Lys Glu Val
            500                 505                 510

Gln Arg Tyr Glu Ala Arg Leu Ser Pro Gly Asp Val Val Ile Ile Pro
        515                 520                 525

Ser Gly His Pro Val Ala Val Ser Ala Ser Ser Asn Leu Asn Leu Leu
    530                 535                 540

Gly Phe Gly Ile Asn Ala Glu Asn Asn Gln Arg Asn Phe Leu Ser Gly
545                 550                 555                 560

Ser Asp Asp Asn

<210> 248
<211> 527
<212> PRT
<213> Lathyrus sativus

<400> 248

Met Ala Thr Ile Ile Lys Ser Arg Phe Pro Leu Leu Leu Leu Leu Gly
1               5                   10                  15

Ile Ile Phe Leu Ala Ser Val Cys Val Thr Tyr Ala Asn Tyr Asp Glu
            20                  25                  30

Gly Ser Glu Pro Arg Val Pro Ala Gln Arg Glu Arg Gly Arg Gln Glu
        35                  40                  45

Gly Glu Lys Glu Glu Lys Arg His Gly Glu Trp Arg Pro Ser Ser Glu
    50                  55                  60

Lys Glu Tyr Asp Glu Gly Ser Glu Pro Arg Val Pro Gly Arg Arg Glu
65                  70                  75                  80

Arg Gly Arg Gln Glu Gly Glu Lys Glu Glu Lys Arg His Gly Glu Trp
                85                  90                  95

Arg Pro Ser Tyr Glu Lys Glu Tyr Asp Glu Glu Glu Lys Gln Lys Tyr
            100                 105                 110

Gln Tyr Glu Arg Glu Lys Lys Glu Gln Lys Glu Val Glu Pro Gly Arg
        115                 120                 125

Glu Arg Trp Glu Arg Lys Glu Asp Glu Glu Lys Glu Glu Asp Gln Trp
    130                 135                 140

Arg Gly Ser Gln Arg His Glu Asp Pro Glu Glu Arg Ala Arg Leu Arg
145                 150                 155                 160

Tyr Arg Lys Glu Arg Thr Lys Lys Tyr Val Glu Glu Asp Thr Glu Glu
                165                 170                 175

Thr Ser Ser Glu Ser Gln Gly Arg Arg Asn Pro Phe Leu Phe Lys Ser
            180                 185                 190

Asn Lys Phe Leu Thr Leu Phe Glu Asn Glu Asn Gly Tyr Ile Arg Arg
        195                 200                 205

Leu Gln Arg Phe Asp Glu Arg Ser Asp Leu Phe Glu Asn Leu Gln Asn
    210                 215                 220

Tyr Arg Leu Val Glu Tyr Arg Ala Lys Pro His Thr Ile Phe Leu Pro
225                 230                 235                 240

Gln His Ile Asp Ala Asp Leu Ile Leu Val Ile Leu Asn Gly Lys Ala
                245                 250                 255

Ile Leu Thr Val Leu Ser Pro Asn Asp Arg Asn Ser Tyr Asn Leu Glu
            260                 265                 270

Arg Gly Asp Thr Ile Lys Leu Pro Ala Gly Thr Thr Ser Tyr Leu Val
        275                 280                 285

Asn Glu Asp Asp Glu Glu Asp Leu Arg Val Val Asp Leu Val Ile Pro
    290                 295                 300

Val Asn Arg Pro Gly Lys Phe Glu Ala Phe Asp Leu Asn Gln Tyr Leu
305                 310                 315                 320

Gly Gly Phe Ser Lys Ser Val Leu Lys Ala Ser Leu Asn Thr Lys Tyr
                325                 330                 335

Glu Thr Ile Glu Lys Val Leu Leu Glu Glu Gln Gln Lys Gln Gly Gln
            340                 345                 350

Glu Thr Asn Ala Ile Val Lys Val Ser Arg Glu Gln Ile Glu Glu Leu
        355                 360                 365

Arg Lys Leu Ala Lys Ser Ser Ser Lys Lys Ser Leu Leu Ser Glu Leu
    370                 375                 380

Glu Pro Val Asn Leu Arg Ser His Ser Pro Lys Tyr Ser Asn Lys Phe
385                 390                 395                 400

Gly Lys Phe Phe Glu Ile Thr Pro Glu Lys Lys Tyr Pro Gln Leu Gln
                405                 410                 415

Asp Leu Asp Val Ser Ile Ser Cys Val Glu Ile Asn Glu Gly Ala Leu
            420                 425                 430

Leu Leu Pro His Tyr Asn Ser Arg Ala Ile Val Val Leu Leu Val Asn
        435                 440                 445

Glu Gly Lys Gly Asn Leu Glu Leu Leu Gly Val Gln Asp Glu Asp Glu
    450                 455                 460

Gln Gln Glu Arg Lys Lys Arg Asn Lys Glu Val Gln Arg Tyr Glu Ala
465                 470                 475                 480

Arg Leu Ser Pro Ser Asp Val Val Ile Ile Pro Ala Gly His Pro Val
                485                 490                 495

Ala Val Ser Ala Ser Ser Asn Leu Asn Leu Leu Gly Phe Gly Ile Asn
            500                 505                 510

Ala Glu Asn Asn Glu Arg Asn Phe Leu Ser Gly Ser Asp Asp Asn
        515                 520                 525

<210>  249
<211>  495
<212>  PRT
<213>  Oryza brachyantha

<400>  249

Met Ala Thr Thr Val Phe Ser Arg Phe Ser Thr Tyr Phe Cys Val Leu
1               5                   10                  15

Leu Leu Cys His Gly Ser Met Ala Gln Leu Phe Asn Pro Ser Thr Asn
            20                  25                  30

Pro Trp His Asn Pro Arg Gln Gly Ser Ser Arg Glu Cys Arg Phe Asp
        35                  40                  45

Arg Leu Gln Pro Phe Glu Pro Leu Arg Lys Val Arg Ser Glu Ala Gly
    50                  55                  60

Val Thr Glu Tyr Phe Asp Glu Lys Asn Glu Leu Phe Gln Cys Thr Gly
65                  70                  75                  80

Thr Phe Val Ile Arg Arg Val Ile Gln Pro Gln Gly Leu Leu Val Pro
                85                  90                  95

Arg Tyr Thr Asn Ala Pro Gly Leu Val Tyr Ile Ile Gln Gly Arg Gly
            100                 105                 110

Ser Ile Gly Leu Thr Phe Pro Gly Cys Pro Ala Thr Tyr Gln Gln Gln
        115                 120                 125

Phe Gln Gln Phe Leu Pro Gln Glu Gln Ser Gln Ser Gln Lys Phe Arg
    130                 135                 140

Asp Glu His Gln Lys Ile His Gln Phe Arg Gln Gly Asp Ile Val Ala
145                 150                 155                 160

Leu Pro Ala Gly Val Ala His Trp Phe Tyr Asn Asp Gly Asp Ala Pro
                165                 170                 175

Val Val Ala Val Tyr Val Tyr Asp Val Lys Asn Ser Ala Asn Gln Leu
            180                 185                 190

Glu Pro Arg Gln Arg Glu Phe Leu Leu Gly Gly Asn Asn Met Arg Ala
        195                 200                 205

Gln Gln Val Tyr Gly Ser Ser Ala Glu Gln His Ser Arg Gln Asn Ile
    210                 215                 220

Phe Ser Gly Phe Gly Val Glu Ile Leu Ser Glu Ala Leu Gly Ile Ser
225                 230                 235                 240

Thr Val Thr Thr Lys Arg Leu Gln Ser Gln Asn Asp Gln Arg Gly Glu
                245                 250                 255

Ile Ile His Val Lys Asn Gly Leu Gln Phe Leu Lys Pro Thr Leu Thr
            260                 265                 270

Gln Gln Gln Glu Gln Ala Gln Ala Gln Tyr Gln Glu Val Gln Tyr Ser
        275                 280                 285

Glu Gln Gln Gln Thr Ser Ser Arg Trp Asn Gly Leu Asp Glu Asn Phe
    290                 295                 300

Cys Thr Ile Lys Ala Arg Met Asn Ile Glu Asn Thr Ser Arg Ala Asp
305                 310                 315                 320

Thr Tyr Asn Pro Arg Ala Gly Arg Thr Thr Ser Leu Asn Ser Gln Lys
                325                 330                 335

Phe Pro Ile Leu Asn Leu Val Gln Met Ser Ala Thr Arg Val Asn Leu
            340                 345                 350

Tyr Gln Asn Ala Ile Leu Ser Thr Phe Trp Asn Val Asn Ala His Ser
        355                 360                 365

Leu Val Tyr Thr Ile Gln Gly Arg Ala Arg Val Gln Val Val Ser Asn
    370                 375                 380

Phe Gly Lys Thr Val Phe Asp Gly Glu Leu Arg Pro Gly Gln Leu Leu
385                 390                 395                 400

Ile Ile Pro Gln His Tyr Val Val Leu Lys Lys Ala Gln Arg Glu Gly
                405                 410                 415

Phe Arg Tyr Ile Ala Ile Lys Thr Asn Ala Asn Ala Phe Val Ser Gln
            420                 425                 430

Leu Val Gly Lys Asn Ser Val Phe Arg Ser Leu Pro Val Asp Val Ile
        435                 440                 445

Ala Asn Val Tyr Arg Ile Ser Arg Glu Gln Ala Arg Ser Leu Lys Asn
    450                 455                 460

Asn Arg Gly Glu Glu His Gly Ala Phe Ala Pro Arg Ser Gln Gln Gln
465                 470                 475                 480

Ser Tyr Pro Gly Phe Ser Asn Gln Ser Glu Ser Glu Thr Ser Glu
                485                 490                 495

<210> 250
<211> 494
<212> PRT
<213> Oryza brachyantha

<400> 250

Met Ala Thr Thr Thr Phe Ser Arg Phe Ser Ile Tyr Phe Cys Val Leu
1               5                   10                  15

Leu Leu Cys His Gly Ser Met Ala Gln Leu Phe Ser Pro Thr Leu Asn
            20                  25                  30

Pro Trp His Ser Ser Arg Arg Gly Gly Ser Arg Asp Cys Arg Phe Asp
        35                  40                  45

Arg Leu Gln Ala Phe Glu Pro Leu Arg Arg Val Arg Ser Glu Ala Gly
    50                  55                  60

Val Thr Glu Tyr Phe Asp Glu Arg Asn Glu Gln Phe Gln Cys Thr Gly
65                  70                  75                  80

Thr Phe Val Ile Arg Arg Val Ile Glu Pro Gln Gly Leu Leu Val Pro
                85                  90                  95

Arg Tyr Thr Asn Thr Pro Gly Val Val Tyr Ile Met Gln Gly Arg Gly
            100                 105                 110

Ser Met Gly Leu Thr Phe Pro Gly Cys Pro Ala Thr Tyr Gln Gln Gln
        115                 120                 125

Phe Gln Gln Phe Leu Pro Glu Gly Gln Ser Gln Ser Gln Lys Phe Arg
    130                 135                 140

Asp Glu His Gln Lys Ile His Gln Phe Arg Gln Gly Asp Ile Val Ala
145                 150                 155                 160

Leu Pro Ala Gly Val Ala His Trp Phe Tyr Asn Glu Gly Asp Thr Pro
                165                 170                 175

Val Val Ala Leu Tyr Val Phe Asp Ile Asn Asn Ser Ala Asn Gln Leu
            180                 185                 190

Glu Pro Arg Gln Lys Asp Phe Leu Leu Ala Gly Asn Asn Asn Arg Glu
        195                 200                 205

Gln Gln Val Tyr Gly Arg Ser Ile Glu Lys His Ser Gly Gln Asn Ile
    210                 215                 220

Phe Ser Gly Phe Asn His Glu Leu Leu Ser Glu Ala Leu Gly Ile Ser
225                 230                 235                 240

Thr Leu Ala Ala Lys Arg Leu Gln Gly Gln Asn Asp His Arg Gly Glu
                245                 250                 255

Ile Ile Arg Val Arg Asn Gly Leu Gln Leu Leu Lys Pro Thr Phe Thr
            260                 265                 270

Gln Gln Gln Glu Gln Ala Gln Ser Gln Tyr Gln Val Gln Tyr Ser Glu
        275                 280                 285

Lys Gln Gln Glu Ser Thr Arg Cys Asn Gly Leu Asp Glu Asn Phe Cys
    290                 295                 300

Thr Ile Asn Ala Arg Leu Asn Ile Glu Asn Pro Ser Arg Ala Asp Thr
305                 310                 315                 320

Tyr Asn Pro Arg Ala Gly Arg Ile Thr His Leu Asn Asn Gln Lys Phe
                325                 330                 335

Pro Ile Leu Asn Leu Val Gln Met Ser Ala Thr Arg Val Asn Leu Tyr
            340                 345                 350

Gln Asn Ala Ile Leu Ser Pro Tyr Trp Asn Val Asn Ala His Ser Leu
        355                 360                 365

Val Tyr Met Val Gln Gly His Ala Arg Val Gln Val Val Ser Asn Leu
    370                 375                 380

Gly Lys Thr Val Phe Asn Ser Val Leu Arg Pro Gly Gln Leu Leu Ile

385                 390                 395                 400

Ile Pro Gln His Tyr Val Val Leu Lys Lys Ala Glu Arg Glu Gly Cys
                405                 410                 415

Gln Tyr Ile Ala Phe Lys Thr Asn Ala Asn Ser Ile Val Ser Gln Leu
            420                 425                 430

Ala Gly Lys Asn Ser Ile Leu Arg Ala Met Pro Val Asp Val Val Ala
        435                 440                 445

Asn Ala Tyr Arg Ile Ser Arg Glu Gln Ala Arg Asp Leu Lys Asn Asn
    450                 455                 460

Arg Gly Glu Glu Leu Gly Ala Phe Thr Pro Lys Phe Glu Gln Gln Ser
465                 470                 475                 480

Tyr Pro Gly Leu Ser Asn Glu Ser Glu Ser Glu Ala Ser Glu
                485                 490


<210>  251
<211>  500
<212>  PRT
<213>  Zizania latifolia

<400>  251

Met Asn Met Ala Thr Ile Asn Gly Pro Thr Ile Phe Phe Thr Val Cys
1               5                   10                  15

Leu Phe Leu Leu Cys His Gly Ser Leu Ala Gln Leu Leu Gly Gln Ser
            20                  25                  30

Thr Ser Gln Trp Gln Ser Ser His Arg Gly Ser Ser Arg Gln Cys Arg
        35                  40                  45

Phe Asp Arg Leu Gln Ala Phe Glu Pro Val Arg Ser Val Arg Ser Gln
    50                  55                  60

Ala Gly Thr Thr Glu Phe Phe Asp Ala Ser Asn Glu Leu Phe Gln Cys
65                  70                  75                  80

Ala Gly Val Ser Ile Val Arg Arg Ile Ile Glu Pro Arg Gly Leu Leu
                85                  90                  95

Leu Pro Gln Tyr Thr Asn Gly Ala Thr Ile Met Tyr Ile Ile Gln Gly
            100                 105                 110

Arg Gly Ile Thr Gly Gln Thr Phe Pro Gly Cys Pro Glu Ser Tyr Gln

115                 120                 125

Gln Gln Phe Gln Gln Ser Met Gln Ala Gln Leu Thr Gly Ser Gln Ser
    130                 135                 140

Gln Ser Gln Lys Phe Lys Asp Glu His Gln Lys Ile Asn Arg Phe Arg
145                 150                 155                 160

Gln Gly Asp Val Ile Ala Leu Pro Ala Gly Val Ala His Trp Cys Tyr
                165                 170                 175

Asn Asp Gly Glu Val Pro Val Val Ala Ile Tyr Val Ile Asp Ile Asn
            180                 185                 190

Asn Ala Ala Asn Gln Leu Asp Pro Arg Gln Arg Asp Phe Leu Leu Ala
        195                 200                 205

Gly Asn Met Arg Ser Pro Gln Ala Tyr Arg Arg Glu Val Glu Asn Gln
    210                 215                 220

Ser Gln Asn Ile Phe Ser Gly Phe Ser Ala Glu Leu Leu Ser Glu Ala
225                 230                 235                 240

Leu Gly Ile Ser Thr Gly Val Ala Arg Gln Leu Gln Cys Gln Asn Asp
                245                 250                 255

Gln Arg Gly Glu Ile Val Arg Val Glu His Gly Leu Ser Leu Leu Gln
            260                 265                 270

Pro Tyr Ala Ser Leu Gln Glu Gln Glu Gln Lys Gln Glu Gln Pro Arg
        275                 280                 285

Glu Arg Tyr Gln Val Thr Gln His Gln Gln Ser Gln Tyr Gly Gly Gly
    290                 295                 300

Cys Ser Asn Gly Leu Asp Glu Thr Phe Cys Ala Met Arg Ile Trp Gln
305                 310                 315                 320

Asn Ile Asp Asn Pro Asn Leu Ala Asp Thr Tyr Asn Pro Arg Ala Gly
                325                 330                 335

Arg Val Thr Asn Leu Asn Ser Gln Lys Phe Pro Ile Leu Asn Leu Ile
            340                 345                 350

Gln Met Ser Ala Val Lys Val Asn Leu Tyr Gln Asn Ala Leu Leu Ser
        355                 360                 365

Pro Phe Trp Asn Ile Asn Ser His Ser Val Val Tyr Val Thr Gln Gly
    370                 375                 380

Cys Ala Arg Val Gln Val Val Asn Asn Asn Gly Lys Thr Val Phe Asn
385                 390                 395                 400

Gly Glu Leu Arg Arg Gly Gln Leu Leu Ile Ile Pro Gln His Tyr Val
                405                 410                 415

Val Val Lys Lys Ala Gln Arg Glu Gly Cys Ala Tyr Ile Ala Phe Lys
            420                 425                 430

Thr Asn Pro Asn Ser Met Val Ser His Ile Val Gly Lys Ser Ser Ile
        435                 440                 445

Phe Arg Ala Leu Pro Thr Asp Val Leu Ala Asn Ala Tyr Arg Ile Ser
    450                 455                 460

Arg Glu Asp Ala Gln Arg Leu Lys His Asn Arg Gly Asp Glu Leu Gly
465                 470                 475                 480

Ala Phe Thr Pro Leu Gln Tyr Lys Ser Tyr Gln Asp Val Ser Ser Val
                485                 490                 495

Ala Ala Ser Ser
            500

<210>  252
<211>  454
<212>  PRT
<213>  Oryza sativa

<400>  252

Cys Arg Phe Asp Arg Leu Gln Ala Phe Glu Pro Ile Arg Ser Val Arg
1               5                   10                  15

Ser Gln Ala Gly Thr Thr Glu Phe Phe Asp Val Ser Asn Glu Gln Phe
            20                  25                  30

Gln Cys Thr Gly Val Ser Ala Val Arg Arg Val Ile Glu Pro Arg Gly
        35                  40                  45

Leu Leu Leu Pro His Tyr Thr Asn Gly Ala Ser Leu Val Tyr Ile Ile
    50                  55                  60

Gln Gly Arg Gly Ile Thr Gly Pro Thr Phe Pro Gly Cys Pro Glu Ser
65                  70                  75                  80

Tyr Gln Gln Gln Phe Gln Gln Ser Gly Gln Ala Gln Leu Thr Glu Ser
                85                  90                  95

Gln Ser Gln Ser His Lys Phe Lys Asp Glu His Gln Lys Ile His Arg
            100                 105                 110

Phe Arg Gln Gly Asp Val Ile Ala Leu Pro Ala Gly Val Ala His Trp
        115                 120                 125

Cys Tyr Asn Asp Gly Glu Val Pro Val Val Ala Ile Tyr Val Thr Asp
    130                 135                 140

Leu Asn Asn Gly Ala Asn Gln Leu Asp Pro Arg Gln Arg Asp Phe Leu
145                 150                 155                 160

Leu Ala Gly Asn Lys Arg Asn Pro Gln Ala Tyr Arg Arg Glu Val Glu
                165                 170                 175

Glu Arg Ser Gln Asn Ile Phe Ser Gly Phe Ser Thr Glu Leu Leu Ser
            180                 185                 190

Glu Ala Leu Gly Val Ser Ser Gln Val Ala Arg Gln Leu Gln Cys Gln
        195                 200                 205

Asn Asp Gln Arg Gly Glu Ile Val Arg Val Glu His Gly Leu Ser Leu
    210                 215                 220

Leu Gln Pro Tyr Ala Ser Leu Gln Glu Gln Glu Gln Gly Gln Val Gln
225                 230                 235                 240

Ser Arg Glu Arg Tyr Gln Glu Gly Gln Tyr Gln Gln Ser Gln Tyr Gly
                245                 250                 255

Ser Gly Cys Ser Asn Gly Leu Asp Glu Thr Phe Cys Thr Met Lys Val
            260                 265                 270

Arg Gln Asn Ile Asp Asn Pro Asn Arg Ala Asp Thr Tyr Asn Pro Arg
        275                 280                 285

Ala Gly Arg Val Thr Asn Leu Asn Thr Gln Asn Phe Pro Ile Leu Asn
    290                 295                 300

Leu Val Gln Met Ser Ala Val Lys Val Asn Leu Tyr Gln Asn Ala Leu
305                 310                 315                 320

Leu Ser Pro Phe Trp Asn Ile Asn Ala His Ser Val Val Tyr Ile Thr
                325                 330                 335

Gln Gly Arg Ala Arg Val Gln Val Val Asn Asn Asn Gly Lys Thr Val
            340                 345                 350

Phe Asn Gly Glu Leu Arg Arg Gly Gln Leu Leu Ile Ile Pro Gln His
        355                 360                 365

Tyr Ala Val Val Lys Lys Ala Gln Arg Glu Gly Cys Ala Tyr Ile Ala
    370                 375                 380

Phe Lys Thr Asn Pro Asn Ser Met Val Ser His Ile Ala Gly Lys Ser
385                 390                 395                 400

Ser Ile Phe Arg Ala Leu Pro Asn Asp Val Leu Ala Asn Ala Tyr Arg
                405                 410                 415

Ile Ser Arg Glu Glu Ala Gln Arg Leu Lys His Asn Arg Gly Asp Glu
            420                 425                 430

Phe Gly Ala Phe Thr Pro Ile Gln Tyr Lys Ser Tyr Gln Asp Val Tyr
        435                 440                 445

Asn Ala Ala Glu Ser Ser
    450

<210>  253
<211>  500
<212>  PRT
<213>  Zizania latifolia

<400>  253

Met Asn Met Ala Thr Ile Asn Gly Pro Thr Ile Phe Phe Thr Val Cys
1               5                   10                  15

Leu Phe Leu Leu Cys His Gly Ser Leu Ala Gln Leu Leu Gly Gln Ser
            20                  25                  30

Thr Ser Gln Trp Gln Ser Ser His Arg Gly Ser Ser Arg Gln Cys Arg
        35                  40                  45

Phe Asp Arg Leu Gln Ala Phe Glu Pro Val Arg Ser Val Arg Ser Gln
    50                  55                  60

Ala Gly Thr Thr Glu Phe Phe Asp Ala Ser Asn Glu Leu Phe Gln Cys
65                  70                  75                  80

Ala Gly Val Ser Ile Val Arg Arg Ile Ile Glu Pro Arg Gly Leu Leu
                85                  90                  95

Leu Pro Gln Tyr Thr Asn Gly Ala Thr Ile Met Tyr Ile Ile Gln Gly
        100                 105                 110

Arg Gly Ile Thr Gly Gln Thr Phe Pro Gly Cys Pro Glu Ser Tyr Gln
        115                 120                 125

Gln Gln Phe Gln Gln Ser Met Gln Ala Gln Leu Thr Gly Ser Gln Ser
    130                 135                 140

Gln Ser Gln Lys Phe Lys Asp Glu His Gln Lys Ile Asn Arg Phe Arg
145                 150                 155                 160

Gln Gly Asp Val Ile Ala Leu Pro Ala Gly Val Ala His Trp Cys Tyr
                165                 170                 175

Asn Asp Gly Glu Val Pro Val Val Ala Ile Tyr Val Ile Asp Ile Asn
            180                 185                 190

Asn Ala Ala Asn Gln Leu Asp Pro Arg Gln Arg Asp Phe Leu Leu Ala
        195                 200                 205

Gly Asn Met Arg Ser Pro Gln Ala Tyr Arg Arg Glu Val Glu Asn Gln
    210                 215                 220

Ser Gln Asn Ile Phe Ser Gly Phe Ser Ala Glu Leu Leu Ser Glu Ala
225                 230                 235                 240

Leu Gly Ile Ser Thr Gly Val Ala Arg Gln Leu Gln Cys Gln Asn Asp
                245                 250                 255

Gln Arg Gly Glu Ile Val Arg Val Glu His Gly Leu Ser Leu Leu Gln
            260                 265                 270

Pro Tyr Ala Ser Leu Gln Glu Gln Glu Gln Lys Gln Glu Gln Pro Arg
        275                 280                 285

Glu Arg Tyr Gln Val Thr Gln His Gln Gln Ser Gln Tyr Gly Gly Gly
    290                 295                 300

Cys Ser Asn Gly Leu Asp Glu Thr Phe Cys Ala Met Arg Ile Trp Gln
305                 310                 315                 320

Asn Ile Asp Asn Pro Asn Leu Ala Asp Thr Tyr Asn Pro Arg Ala Gly
                325                 330                 335

Arg Val Thr Asn Leu Asn Ser Gln Lys Phe Pro Ile Leu Asn Leu Ile
            340                 345                 350

Gln Met Ser Ala Val Lys Val Asn Leu Tyr Gln Asn Ala Leu Leu Ser
        355                 360                 365

Pro Phe Trp Asn Ile Asn Ser His Ser Val Val Tyr Val Thr Gln Gly
    370                 375                 380

Cys Ala Arg Val Gln Val Val Asn Asn Asn Gly Lys Thr Val Phe Asn
385                 390                 395                 400

Gly Glu Leu Arg Arg Gly Gln Leu Leu Ile Ile Pro Gln His Tyr Val
                405                 410                 415

Val Val Lys Lys Ala Gln Arg Glu Gly Cys Ala Tyr Ile Ala Phe Lys
            420                 425                 430

Thr Asn Pro Asn Ser Met Val Ser His Ile Val Gly Lys Ser Ser Ile
        435                 440                 445

Phe Arg Ala Leu Pro Thr Asp Val Leu Ala Asn Ala Tyr Arg Ile Ser
    450                 455                 460

Arg Glu Asp Ala Gln Arg Leu Lys His Asn Arg Gly Asp Glu Leu Gly
465                 470                 475                 480

Ala Phe Thr Pro Leu Gln Tyr Lys Ser Tyr Gln Asp Val Ser Ser Val
                485                 490                 495

Ala Ala Ser Ser
            500

<210>  254
<211>  527
<212>  PRT
<213>  Avena sativa

<400>  254

Met Ala Thr Thr Ser Phe Pro Ser Met Leu Phe Tyr Phe Cys Ile Phe
1               5                   10                  15

Leu Leu Phe His Gly Ser Met Ala Gln Leu Phe Gly Gln Ser Ser Thr
            20                  25                  30

Pro Trp Gln Ser Ser Arg Gln Gly Gly Leu Arg Gly Cys Arg Phe Asp
        35                  40                  45

Arg Leu Gln Ala Phe Glu Pro Leu Arg Gln Val Arg Ser Gln Ala Gly
    50                  55                  60

Ile Thr Glu Tyr Phe Asp Glu Gln Asn Glu Gln Phe Arg Cys Thr Gly
65                  70                  75                  80

Val Ser Val Ile Arg Arg Val Ile Glu Pro Gln Gly Leu Val Leu Pro
                85                  90                  95

Gln Tyr His Asn Ala Pro Ala Leu Val Tyr Ile Leu Gln Gly Arg Gly
            100                 105                 110

Phe Thr Gly Leu Thr Phe Pro Gly Cys Pro Ala Thr Phe Gln Gln Gln
        115                 120                 125

Phe Gln Pro Phe Asp Gln Ser Gln Phe Ala Gln Gly Gln Arg Gln Ser
    130                 135                 140

Gln Thr Ile Lys Asp Glu His Gln Arg Val Gln Arg Phe Lys Gln Gly
145                 150                 155                 160

Asp Val Val Ala Leu Pro Ala Gly Ile Val His Trp Cys Tyr Asn Asp
                165                 170                 175

Gly Asp Ala Pro Ile Val Ala Ile Tyr Val Phe Asp Val Asn Asn Asn
            180                 185                 190

Ala Asn Gln Leu Glu Pro Arg Gln Lys Glu Phe Leu Leu Ala Gly Asn
        195                 200                 205

Asn Lys Arg Glu Gln Gln Ser Gly Asn Asn Ile Phe Ser Gly Leu Ser
    210                 215                 220

Val Gln Leu Leu Ser Glu Ala Leu Gly Ile Ser Gln Gln Ala Ala Gln
225                 230                 235                 240

Arg Ile Gln Ser Gln Asn Asp Gln Arg Gly Glu Ile Ile Arg Val Ser
                245                 250                 255

Gln Gly Leu Gln Phe Leu Lys Pro Ile Val Ser Gln Gln Val Pro Gly
            260                 265                 270

Glu Gln Gln Val Tyr Gln Pro Ile Gln Thr Gln Glu Gly Gln Ala Thr
        275                 280                 285

Gln Tyr Gln Val Gly Gln Ser Thr Gln Tyr Gln Val Gly Lys Ser Thr
    290                 295                 300

Pro Tyr Gln Gly Gly Gln Ser Ser Gln Tyr Gln Ala Gly Gln Ser Trp

305                 310                 315                 320

Asp Gln Ser Phe Asn Gly Leu Glu Glu Asn Phe Cys Ser Leu Glu Ala
                325                 330                 335

Arg Lys Asn Ile Glu Asn Pro Gln His Ala Asp Thr Tyr Asn Pro Arg
            340                 345                 350

Ala Gly Arg Ile Thr Arg Leu Asn Ser Lys Asn Phe Pro Ile Leu Asn
        355                 360                 365

Ile Val Gln Met Ser Ala Thr Arg Val Asn Leu Tyr Gln Asn Ala Ile
    370                 375                 380

Leu Ser Pro Phe Trp Asn Ile Asn Ala His Ser Val Ile Tyr Met Ile
385                 390                 395                 400

Gln Gly His Ala Arg Val Gln Val Val Asn Asn Asn Gly Gln Thr Val
                405                 410                 415

Phe Asn Asp Ile Leu Arg Arg Gly Gln Leu Leu Ile Val Pro Gln His
            420                 425                 430

Phe Val Val Leu Lys Lys Ala Glu Arg Glu Gly Cys Gln Tyr Ile Ser
        435                 440                 445

Phe Lys Thr Asn Pro Asn Ser Met Val Ser His Ile Ala Gly Lys Ser
    450                 455                 460

Ser Ile Leu Arg Ala Leu Pro Ile Asp Val Leu Ala Asn Ala Tyr Arg
465                 470                 475                 480

Ile Ser Arg Gln Glu Ala Arg Asn Leu Lys Asn Asn Arg Gly Glu Glu
                485                 490                 495

Phe Gly Ala Phe Thr Pro Lys Leu Thr Gln Lys Gly Phe Gln Ser Tyr
            500                 505                 510

Gln Asp Ile Glu Glu Gly Ser Ser Ser Pro Val Arg Ala Ser Glu
        515                 520                 525

<210>  255
<211>  498
<212>  PRT
<213>  Oryza sativa

<400>  255

Met Ala Ser Thr Asn Arg Pro Ile Val Phe Phe Thr Val Cys Leu Phe

1               5                   10                  15

Leu Leu Cys Asp Gly Ser Leu Ala Gln Gln Leu Leu Gly Gln Ser Thr
            20                  25                  30

Ser Gln Trp Gln Ser Ser Arg Arg Gly Ser Pro Arg Gly Cys Arg Phe
        35                  40                  45

Asp Arg Leu Gln Ala Phe Glu Pro Ile Arg Ser Val Arg Ser Gln Ala
    50                  55                  60

Gly Thr Thr Glu Phe Phe Asp Val Ser Asn Glu Leu Phe Gln Cys Thr
65                  70                  75                  80

Gly Val Ser Val Val Arg Arg Val Ile Glu Pro Arg Gly Leu Leu Leu
                85                  90                  95

Pro His Tyr Thr Asn Gly Ala Ser Leu Val Tyr Ile Ile Gln Gly Arg
            100                 105                 110

Gly Ile Thr Gly Pro Thr Phe Pro Gly Cys Pro Glu Thr Tyr Gln Gln
        115                 120                 125

Gln Phe Gln Gln Ser Gly Gln Ala Gly Leu Thr Glu Ser Gln Ser Gln
    130                 135                 140

Ser His Lys Phe Lys Asp Glu His Gln Lys Ile His Arg Phe Arg Gln
145                 150                 155                 160

Gly Asp Val Ile Ala Leu Pro Ala Gly Val Ala His Trp Cys Tyr Asn
                165                 170                 175

Asp Cys Glu Val Pro Val Val Ala Ile Tyr Val Thr Asp Ile Asn Asn
            180                 185                 190

Gly Ala Asn Gln Leu Asp Pro Arg Gln Arg Asp Phe Leu Leu Ala Gly
        195                 200                 205

Asn Lys Arg Asn Pro Gln Ala Tyr Arg Arg Glu Val Glu Glu Trp Ser
    210                 215                 220

Gln Asn Ile Phe Ser Gly Phe Ser Thr Glu Leu Leu Ser Glu Ala Phe
225                 230                 235                 240

Gly Ile Ser Asn Gln Val Ala Arg Gln Leu Gln Cys Gln Asn Asp Gln
                245                 250                 255

Lys Gly Glu Ile Val Arg Val Glu Arg Gly Leu Ser Leu Leu Gln Pro
            260                 265                 270

Tyr Ala Ser Leu Gln Glu Gln Glu Gln Gly Gln Met Gln Ser Arg Glu
        275                 280                 285

His Tyr Gln Glu Gly Gly Tyr Gln Gln Ser Gln Tyr Gly Ser Gly Cys
    290                 295                 300

Pro Asn Gly Leu Asp Glu Thr Phe Cys Val Asn Lys Val Arg Gln Asn
305                 310                 315                 320

Ile Asp Asn Pro Asn Arg Ala Asp Thr Tyr Asn Pro Arg Ala Gly Arg
                325                 330                 335

Val Thr Asn Leu Ser Gln Asn Phe Pro Ile Leu Asn Leu Val Gln Met
            340                 345                 350

Ser Ala Val Lys Val Asn Leu Tyr Gln Asn Thr Asp Thr Trp Ile Ser
        355                 360                 365

Met Gly Gln Glu Glu Asn Ala Leu Leu Ser Pro Phe Trp Asn Ile Asn
    370                 375                 380

Ala His Ser Ile Val Tyr Ile Thr Gln Gly Arg Ala Gln Val Gln Val
385                 390                 395                 400

Leu Arg Arg Gly Gln Leu Leu Ile Val Pro Gln His Tyr Val Val Val
                405                 410                 415

Lys Lys Ala Gln Arg Glu Gly Cys Ala Tyr Ile Ala Phe Lys Thr Asn
            420                 425                 430

Pro Asn Ser Met Val Ser His Ile Ala Gly Lys Ser Ser Ile Phe Arg
        435                 440                 445

Ala Leu Pro Thr Asp Val Leu Ala Asn Ala Tyr Arg Ile Ser Arg Glu
    450                 455                 460

Glu Ala Gln Arg Leu Lys His Asn Arg Gly Asp Glu Phe Gly Ala Phe
465                 470                 475                 480

Thr Pro Leu Gln Tyr Lys Ser Tyr Gln Asp Val Tyr Asn Val Ala Glu
                485                 490                 495

Ser Ser

<210>  256
<211>  491
<212>  PRT
<213>  Oryza brachyantha

<400>  256

Met Lys Ser Ser Ile Val Phe Ser Thr Ile Cys Leu Val Leu Leu Cys
1               5                   10                  15

His Gly Ser Leu Ala Gln Leu Leu Ser Gln Ser Thr Ser Gln Trp Gln
            20                  25                  30

Ser Ser Arg Arg Gly Ser Pro Arg Gln Cys Arg Phe Asp Gln Leu Gln
        35                  40                  45

Ala Phe Glu Pro Ile Arg Thr Val Arg Ser Gln Ala Gly Val Thr Glu
    50                  55                  60

Phe Tyr Asp Val Ser Asn Glu Leu Phe Gln Cys Thr Gly Val Ser Val
65                  70                  75                  80

Val Arg Arg Val Ile Glu Pro Arg Gly Leu Leu Leu Pro His Tyr Ser
                85                  90                  95

Asn Gly Ala Thr Leu Val Tyr Ile Ile Gln Gly Arg Gly Ile Thr Gly
            100                 105                 110

Pro Thr Phe Pro Gly Cys Pro Glu Thr Tyr Gln Gln Gln Phe Gln Gln
        115                 120                 125

Ser Gly Glu Ala Gln Pro Phe Glu Gly Gln Ser His Lys Phe Arg Asp
    130                 135                 140

Glu His Gln Lys Ile His Arg Phe Arg Gln Gly Asp Val Val Ala Leu
145                 150                 155                 160

Pro Ala Gly Val Ala His Trp Cys Tyr Asn Asp Gly Glu Val Pro Ile
                165                 170                 175

Val Ala Ile Tyr Val Thr Asp Ile Tyr Asn Ser Ala Asn Gln Leu Asp
            180                 185                 190

Pro Arg His Arg Asp Phe Phe Leu Ala Gly Asn Asn Lys Val Ala Gln
        195                 200                 205

Gln Leu Tyr Arg Ser Glu Ala Arg Glu Asn Ser Lys Asn Ile Phe Gly
    210                 215                 220

Gly Phe Ser Val Glu Leu Leu Ser Glu Ala Leu Gly Ile Ser Arg Gly
225                 230                 235                 240

Val Ala Arg Gln Leu Gln Cys Gln Asn Asp Gln Arg Gly Glu Ile Val
                245                 250                 255

Arg Val Glu His Gly Leu Ala Leu Leu Gln Pro Tyr Ala Ser Val Gln
            260                 265                 270

Glu Gln Gln Gln Glu Gln Val Gln Ser Arg Asp Tyr Glu Gln Thr Gln
        275                 280                 285

Tyr Gln Gln Lys Gln Pro Gln Gly Ser Cys Ser Asn Gly Leu Asp Glu
    290                 295                 300

Thr Phe Cys Thr Met Arg Leu Arg Gln Asn Ile Asp Asn Pro Asn Leu
305                 310                 315                 320

Ala Asp Thr Tyr Asn Pro Lys Ala Gly Arg Ile Thr Tyr Leu Asn Gly
                325                 330                 335

Gln Lys Phe Pro Ile Leu Asn Leu Val Gln Met Ser Ala Val Lys Val
            340                 345                 350

Asn Leu Tyr Gln Asn Ala Val Leu Ser Pro Phe Trp Asn Ile Asn Ala
        355                 360                 365

His Ser Val Val Tyr Ile Thr Gln Gly Arg Ala Arg Val Gln Val Val
    370                 375                 380

Asn Asn Asn Gly Lys Thr Val Phe Asp Gly Glu Leu Arg Gln Gly Gln
385                 390                 395                 400

Leu Leu Ile Ile Pro Gln His His Val Val Leu Lys Lys Ala Gln Arg
                405                 410                 415

Glu Gly Cys Ser Tyr Ile Ala Leu Lys Thr Asn Pro Asn Ser Ile Val
            420                 425                 430

Ser His Ile Ala Gly Lys Asn Ser Ile Phe Arg Ala Leu Pro Gly Asp
        435                 440                 445

Val Val Thr Asn Ala Tyr Arg Ile Ser Arg Glu Glu Ala Lys Arg Ile
    450                 455                 460

Lys His Asn Arg Gly Asp Glu Ser Gly Val Phe Ala Pro Ser His Ala
465                 470                 475                 480

Tyr Arg Ser Tyr Gln Asp Met Ser Val Ala Ala
                485                 490

<210>  257
<211>  492
<212>  PRT
<213>  Brachypodium distachyon

<400>  257

Met Ala His Thr Ser Phe Ser Ser Phe Leu Ser Tyr Phe Cys Leu Phe
1               5                   10                  15

Leu Leu Phe His Gly Ser Met Ala Gln Val Leu Gly Gln Val Ser Thr
            20                  25                  30

Trp Gln Ser Ser Arg Gln Gly Gly Ser Arg Asp Cys Ser Phe Asp Arg
        35                  40                  45

Leu Gln Ala Ile Glu Pro Val Thr Gln Val Arg Ser Gln Ala Gly Leu
    50                  55                  60

Thr Glu Tyr Phe Asp Glu Gln Asn Glu Gln Phe Arg Cys Ala Gly Val
65                  70                  75                  80

Phe Val Ile Arg Arg Val Ile Glu Pro Arg Gly Leu Leu Leu Pro Arg
                85                  90                  95

Tyr His Asn Thr Pro Gly Leu Val Tyr Ile Leu Gln Gly Asn Gly Phe
            100                 105                 110

Val Gly Leu Thr Phe Pro Gly Cys Pro Glu Thr Phe Arg Glu Gln Phe
        115                 120                 125

Gln Gln Phe Arg Gln Thr Gln Ser Thr Leu Gly Gln Ser Gln Cys Gln
    130                 135                 140

Ser Gln Lys Leu Gly Asp Val His Gln Arg Val His Gln Phe Thr Gln
145                 150                 155                 160

Gly Asp Val Val Ala Leu Pro Thr Gly Val Ala His Trp Ile Tyr Asn
                165                 170                 175

Gly Gly Asp Ala Pro Val Val Ile Val Tyr Val Phe Asp Val Asn Asn
            180                 185                 190

Asn Ala Asn Gln Leu Glu Pro Arg Gln Lys Glu Phe Leu Leu Gly Gly
        195                 200                 205

Asn Tyr Asn Gly Val Leu Gln Tyr Gly Gln Asn Ile Phe Ser Gly Phe
    210                 215                 220

Asn Ala Gln Leu Leu Ser Gln Ala Phe Gly Ile Asn Glu Gln Thr Ser
225                 230                 235                 240

Gln Arg Ile Gln Asn Gln Asn Asp Gly Arg Gly Asp Ile Ile Arg Val
                245                 250                 255

Asp Asn Gly Leu Gln Phe Leu Lys Pro Val Val Thr Gln Gln Gln Pro
            260                 265                 270

Glu Gln Pro Phe Met Pro Ile Gln His Gln Thr Gly Gln Ser Ser Arg
        275                 280                 285

Asn Gly Leu Glu Glu Asn Phe Cys Ser Leu Glu Pro Arg Gln Asn Ile
    290                 295                 300

Glu Asp Pro Asn Arg Ala Asp Thr Tyr Asn Pro Arg Ala Gly Ser Ile
305                 310                 315                 320

Thr Arg Leu Asn Gly Gln Asn Phe Pro Ile Leu Asn Leu Val Gln Met
                325                 330                 335

Ser Ala Thr Arg Val Asn Leu Gln Lys Asn Ala Ile Leu Ser Pro Phe
            340                 345                 350

Trp Asn Ile Asn Ala His Ser Val Val Tyr Val Ile Gln Gly His Ala
        355                 360                 365

Leu Val Gln Val Val Asn Asn Gln Gly His Asn Val Phe Asn Gly Leu
    370                 375                 380

Leu His Arg Gly Gln Leu Leu Ile Ile Pro Gln Asn Tyr Val Val Leu
385                 390                 395                 400

Lys Lys Ala Glu Ser Glu Gly Tyr Gln Tyr Ile Ala Phe Lys Thr Asn
                405                 410                 415

Ala Asn Ser Met Val Ser His Ile Ala Gly Lys Asn Ser Ile Leu Arg
            420                 425                 430

Ala Leu Pro Val Asp Val Ile Ala Asn Ala Tyr Arg Ile Ser Arg Gln
        435                 440                 445

Glu Ala Gln Asn Leu Lys Asn Asn Arg Gly Glu Glu Thr Gly Val Leu

450                 455                 460

Thr Pro Asn Phe Ser Gln Ser Thr Cys Gln Ser Tyr Gln Thr Glu Asp
465                 470                 475                 480

Val Gln Ser Leu Arg Pro Met Ser His Trp Ser Glu
                485                 490

<210>  258
<211>  166
<212>  PRT
<213>  Oryza sativa

<220>
<221>  misc_feature
<222>  (120)..(120)
<223>  Xaa can be any naturally occurring amino acid

<400>  258

Met Ala Ser Asn Lys Val Val Phe Ser Ala Leu Leu Leu Ile Ile Val
1               5                   10                  15

Ser Val Leu Ala Ala Thr Ala Thr Met Ala Asp His His Lys Asp Gln
            20                  25                  30

Val Val Tyr Ser Leu Gly Glu Arg Cys Gln Pro Gly Met Gly Tyr Pro
        35                  40                  45

Met Tyr Ser Leu Pro Arg Cys Arg Ala Val Val Lys Arg Gln Cys Val
    50                  55                  60

Gly His Gly Ala Pro Gly Gly Ala Val Asp Glu Gln Leu Arg Gln Asp
65                  70                  75                  80

Cys Cys Arg Gln Leu Ala Ala Val Asp Asp Ser Trp Cys Arg Cys Ser
                85                  90                  95

Ala Leu Asn His Met Val Gly Gly Ile Tyr Arg Glu Leu Gly Ala Thr
            100                 105                 110

Asp Val Gly His Pro Met Ala Xaa Val Phe Pro Gly Cys Arg Arg Gly
        115                 120                 125

Asp Leu Glu Arg Ala Ala Ala Ser Leu Pro Ala Phe Cys Asn Val Asp
    130                 135                 140

Ile Pro Asn Gly Thr Gly Gly Val Cys Tyr Trp Leu Gly Tyr Pro Arg
145                 150                 155                 160

Thr Pro Arg Thr Gly His
                165

<210>  259
<211>  165
<212>  PRT
<213>  Oryza sativa

<400>  259

Met Ala Ser Asn Lys Val Val Phe Ser Ala Leu Leu Leu Ile Ile Val
1               5                   10                  15

Ser Val Leu Arg Arg Asp Gly Thr Met Ala Asp His His Lys Asp Gln
            20                  25                  30

Val Val Tyr Ser Leu Gly Glu Arg Cys Gln Pro Gly Met Gly Tyr Pro
        35                  40                  45

Met Tyr Ser Leu Pro Arg Cys Arg Ala Val Val Lys Arg Gln Cys Val
    50                  55                  60

Gly His Gly Ala Pro Gly Ala Val Asp Glu Gln Leu Arg Gln Asp Cys
65                  70                  75                  80

Cys Arg Gln Leu Ala Ala Val Asp Asp Ser Trp Cys Arg Cys Ser Ala
                85                  90                  95

Leu Asn His Met Val Gly Gly Ile Tyr Arg Glu Leu Gly Ala Thr Asp
            100                 105                 110

Val Gly His Pro Met Ala Glu Val Phe Pro Gly Cys Arg Arg Gly Asp
        115                 120                 125

Leu Glu Arg Ala Ala Ala Ser Leu Pro Ala Phe Cys Asn Val Asp Ile
    130                 135                 140

Pro Asn Gly Thr Gly Gly Val Cys Tyr Trp Leu Gly Tyr Pro Arg Thr
145                 150                 155                 160

Pro Arg Thr Gly His
                165

<210>  260
<211>  157
<212>  PRT
<213>  Oryza sativa

<400>  260

Met Ala Ser Asn Lys Val Val Ile Ser Ala Leu Leu Val Val Val Val
1               5                   10                  15

Ser Val Leu Ala Ala Thr Thr Thr Met Ala Asp His His Gln Glu Gln
            20                  25                  30

Val Val Tyr Thr Pro Gly Gln Leu Cys Gln Pro Gly Ile Gly Tyr Pro
        35                  40                  45

Thr Tyr Pro Leu Pro Arg Cys Arg Ala Phe Val Lys Arg Gln Cys Val
    50                  55                  60

Ala Pro Gly Thr Val Asp Glu Gln Val Arg Arg Gly Cys Cys Arg Gln
65                  70                  75                  80

Leu Ala Ala Ile Asp Ser Ser Trp Cys Arg Cys Asp Ala Leu Asn His
                85                  90                  95

Met Leu Arg Ile Ile Tyr Arg Glu Ser Gly Ala Ala Asp Ala Gly His
            100                 105                 110

Pro Met Ala Glu Val Phe Arg Gly Cys Arg Arg Gly Asp Ile Glu Arg
        115                 120                 125

Ala Ala Ala Ser Leu Pro Ala Phe Cys Asn Val Asp Ile Pro Asn Gly
    130                 135                 140

Val Gly Gly Val Cys Tyr Trp Leu Pro Gly Thr Gly Tyr
145                 150                 155

<210>  261
<211>  495
<212>  PRT
<213>  Oryza sativa

<400>  261

Met Thr Ile Ser Val Phe Ser Arg Phe Ser Ile Tyr Phe Cys Val Leu
1               5                   10                  15

Leu Leu Cys Asn Gly Ser Met Ala Gln Leu Phe Asp Pro Ala Thr Asn
            20                  25                  30

Gln Trp Gln Thr His Arg Gln Gly Ser Phe Arg Glu Cys Arg Phe Glu
        35                  40                  45

Arg Leu Gln Ala Phe Glu Pro Leu Gln Asn Val Arg Ser Glu Ala Gly
    50                  55                  60

Val Thr Glu Tyr Phe Asp Glu Thr Asn Glu Leu Phe Gln Cys Thr Gly
65                  70                  75                  80

Thr Phe Val Ile Arg Arg Val Ile Gln Pro Gln Gly Leu Leu Ile Pro
                85                  90                  95

Arg Tyr Ala Asn Thr Pro Gly Met Val Tyr Ile Ile Gln Gly Arg Gly
            100                 105                 110

Ser Met Gly Leu Thr Phe Pro Gly Cys Pro Ala Thr Tyr Gln Gln Gln
        115                 120                 125

Ser Gln Gln Phe Leu Phe Gln Gly Glu Ser Gln Ser Gln Lys Phe Ile
    130                 135                 140

Asp Glu His Gln Lys Ile His Gln Phe Arg Gln Gly Asp Ile Val Val
145                 150                 155                 160

Leu Pro Thr Gly Val Ala His Trp Phe Tyr Asn Asp Gly Asp Thr Pro
                165                 170                 175

Val Val Ala Leu Tyr Val Tyr Asp Ile Asn Asn Ser Ala Asn Gln Leu
            180                 185                 190

Glu Pro Arg His Arg Glu Phe Leu Leu Ala Gly Lys Asn Asn Arg Val
        195                 200                 205

Gln Gln Val Tyr Gly Arg Ser Ile Gln Gln His Ser Gly Gln Asn Ile
    210                 215                 220

Phe Asn Gly Phe Ser Val Glu Pro Leu Ser Glu Ala Leu Asn Ile Asn
225                 230                 235                 240

Thr Val Thr Thr Lys Arg Leu Gln Ser Gln Asn Asp Gln Arg Gly Glu
                245                 250                 255

Ile Ile His Val Lys Asn Gly Leu Gln Leu Leu Lys Pro Thr Leu Thr
            260                 265                 270

Gln Arg Gln Glu Gln Glu Gln Ala Gln Tyr Gln Glu Val Gln Tyr Ser
        275                 280                 285

Glu Lys Pro Gln Thr Ser Ser Arg Trp Asn Gly Leu Glu Glu Asn Leu
    290                 295                 300

Cys Thr Ile Lys Thr Arg Leu Asn Ile Glu Asn Pro Ser Arg Ala Asp
305                 310                 315                 320

Ser Tyr Asp Pro Arg Ala Gly Arg Ile Thr Ser Leu Asp Ser Gln Lys
                325                 330                 335

Phe Pro Ile Leu Asn Ile Ile Gln Met Ser Ala Thr Arg Val Asn Leu
            340                 345                 350

Tyr Gln Asn Ala Ile Leu Thr Pro Phe Trp Asn Val Asn Ala His Ser
        355                 360                 365

Leu Met Tyr Val Ile Arg Gly Arg Ala Arg Val Gln Val Val Ser Asn
    370                 375                 380

Phe Gly Lys Thr Val Phe Asp Gly Val Leu Arg Pro Glu Gln Leu Leu
385                 390                 395                 400

Ile Ile Pro Gln Asn Tyr Val Val Leu Lys Lys Ala Gln His Glu Gly
                405                 410                 415

Cys Gln Tyr Ile Ala Ile Asn Thr Asn Ala Asn Ala Phe Val Ser His
            420                 425                 430

Leu Ala Gly Val Asp Ser Val Phe His Ala Leu Pro Val Asp Val Ile
        435                 440                 445

Ala Asn Ala Tyr Cys Ile Ser Arg Glu Glu Ala Arg Arg Leu Lys Asn
    450                 455                 460

Asn Arg Gly Asp Glu Tyr Gly Pro Phe Pro Pro Arg Leu Gln Gln Gln
465                 470                 475                 480

Ile Tyr Pro Glu Phe Ser Asn Glu Ser Lys Gly Glu Thr Ser Glu
                485                 490                 495

<210>  262
<211>  480
<212>  PRT
<213>  Oryza sativa

<400>  262

Leu Leu Cys His Gly Ser Met Ala Gln Ile Phe Ser Leu Gly Ile Asn
1               5                   10                  15

Pro Trp Gln Asn Pro Arg Gln Gly Gly Ser Arg Glu Cys Arg Phe Asp
            20                  25                  30

Arg Leu Gln Ala Phe Glu Pro Leu Arg Lys Val Arg His Glu Ala Gly
        35                  40                  45

Val Thr Glu Tyr Phe Asp Glu Lys Asn Glu Gln Phe Gln Cys Thr Gly
    50                  55                  60

Thr Leu Val Ile Arg Arg Ile Ile Glu Pro Gln Gly Leu Leu Leu Pro
65                  70                  75                  80

Arg Tyr Ser Asn Thr Pro Gly Leu Val Tyr Ile Ile Gln Gly Thr Gly
                85                  90                  95

Val Leu Gly Leu Thr Phe Pro Gly Cys Pro Ala Thr Tyr Gln Lys Gln
            100                 105                 110

Phe Arg His Phe Gly Leu Glu Gly Gly Ser Gln Arg Gln Gly Lys Lys
        115                 120                 125

Leu Arg Asp Glu Asn Gln Lys Ile His Gln Phe Arg Gln Gly Asp Val
    130                 135                 140

Val Ala Leu Pro Ser Gly Ile Pro His Trp Phe Tyr Asn Glu Gly Asp
145                 150                 155                 160

Thr Pro Val Val Ala Leu Phe Val Phe Asp Val Asn Asn Asn Ala Asn
                165                 170                 175

Gln Leu Glu Pro Arg Gln Lys Glu Phe Leu Leu Ala Gly Asn Asn Ile
            180                 185                 190

Glu Gln Gln Val Ser Asn Pro Ser Ile Asn Lys His Ser Gly Gln Asn
        195                 200                 205

Ile Phe Asn Gly Phe Asn Thr Lys Leu Leu Ser Glu Ala Leu Gly Val
    210                 215                 220

Asn Ile Glu Val Thr Arg Arg Leu Gln Ser Gln Asn Asp Arg Arg Gly
225                 230                 235                 240

Asp Ile Ile Arg Val Lys Asn Gly Leu Arg Leu Ile Lys Pro Thr Ile
                245                 250                 255

Thr Gln Gln Gln Glu Gln Thr Gln Asp Gln Tyr Gln Gln Ile Gln Tyr
            260                 265                 270

His Arg Glu Gln Arg Ser Thr Ser Lys Tyr Asn Gly Leu Asp Glu Asn
        275                 280                 285

Phe Cys Ala Ile Arg Ala Arg Leu Asn Ile Glu Asn Pro Asn His Ala
    290                 295                 300

Asp Thr Tyr Asn Pro Arg Ala Gly Arg Ile Thr Asn Leu Asn Ser Gln
305                 310                 315                 320

Lys Phe Ser Ile Leu Asn Leu Val Gln Met Ser Ala Thr Arg Val Asn
                325                 330                 335

Leu Tyr Gln Asn Ala Ile Leu Ser Pro Phe Trp Asn Ile Asn Ala His
            340                 345                 350

Ser Leu Val Tyr Thr Ile Gln Gly Arg Ala Arg Val Gln Val Val Ser
        355                 360                 365

Asn His Gly Lys Ala Val Phe Asn Gly Val Leu Arg Pro Gly Gln Leu
    370                 375                 380

Leu Ile Ile Pro Gln Asn Tyr Val Val Met Lys Lys Ala Glu Leu Glu
385                 390                 395                 400

Gly Phe Gln Phe Ile Ala Phe Lys Thr Asn Pro Asn Ala Met Val Asn
                405                 410                 415

His Ile Ala Gly Lys Asn Ser Val Leu Arg Ala Met Pro Val Asp Val
            420                 425                 430

Ile Ala Asn Ala Tyr Arg Ile Ser Arg Gln Glu Ala Arg Ser Leu Lys
        435                 440                 445

Asn Asn Arg Gly Glu Glu Ile Gly Ala Phe Thr Pro Arg Tyr Gln Gln
    450                 455                 460

Gln Lys Ile His Gln Glu Tyr Ser Asn Pro Asn Glu Ser Glu Thr Gln
465                 470                 475                 480

<210>  263
<211>  518
<212>  PRT
<213>  Avena sativa

<400>  263

Met Ala Thr Thr Arg Phe Pro Ser Leu Leu Phe Tyr Ser Cys Ile Phe
1               5                   10                  15

Leu Leu Cys Asn Gly Ser Met Ala Gln Leu Phe Gly Gln Ser Phe Thr
            20                  25                  30

Pro Trp Gln Ser Ser Arg Gln Gly Gly Leu Arg Gly Cys Arg Phe Asp
        35                  40                  45

Arg Leu Gln Ala Phe Glu Pro Leu Arg Gln Val Arg Ser Gln Ala Gly
    50                  55                  60

Ile Thr Glu Tyr Phe Asp Glu Gln Asn Glu Gln Phe Arg Cys Ala Gly
65                  70                  75                  80

Val Ser Val Ile Arg Arg Val Ile Glu Pro Gln Gly Leu Leu Leu Pro
                85                  90                  95

Gln Tyr His Asn Ala Pro Gly Leu Val Tyr Ile Leu Gln Gly Arg Gly
            100                 105                 110

Phe Thr Gly Leu Thr Phe Pro Gly Cys Pro Ala Thr Phe Gln Gln Gln
        115                 120                 125

Phe Gln Pro Phe Asp Gln Ala Arg Phe Ala Gln Gly Gln Ser Lys Ser
    130                 135                 140

Gln Asn Leu Lys Asp Glu His Gln Arg Val His His Ile Lys Gln Gly
145                 150                 155                 160

Asp Val Val Ala Leu Pro Ala Gly Ile Val His Trp Cys Tyr Asn Asp
                165                 170                 175

Gly Asp Ala Pro Ile Val Ala Val Tyr Val Phe Asp Val Asn Asn Asn
            180                 185                 190

Ala Asn Gln Leu Glu Pro Arg Gln Lys Glu Phe Leu Leu Ala Gly Asn
        195                 200                 205

Asn Lys Arg Glu Gln Gln Phe Gly Gln Asn Ile Phe Ser Gly Phe Ser
    210                 215                 220

Val Gln Leu Leu Ser Glu Ala Leu Gly Ile Ser Gln Gln Ala Ala Gln
225                 230                 235                 240

Lys Ile Gln Ser Gln Asn Asp Gln Arg Gly Glu Ile Ile Arg Val Ser
                245                 250                 255

Gln Gly Leu Gln Phe Leu Lys Pro Phe Val Ser Gln Gln Gly Pro Val
            260                 265                 270

Glu His Gln Ala Tyr Gln Pro Ile Gln Ser Gln Gln Glu Gln Ser Thr
        275                 280                 285

Gln Tyr Gln Val Gly Gln Ser Pro Gln Tyr Gln Glu Gly Gln Ser Thr

290                 295                 300

Gln Tyr Gln Ser Gly Gln Ser Trp Asp Gln Ser Phe Asn Gly Leu Glu
305                 310                 315                 320

Glu Asn Phe Cys Ser Leu Glu Ala Arg Gln Asn Ile Glu Asn Pro Lys
                325                 330                 335

Arg Ala Asp Thr Tyr Asn Pro Arg Ala Gly Arg Ile Thr His Leu Asn
            340                 345                 350

Ser Lys Asn Phe Pro Thr Leu Asn Leu Val Gln Met Ser Ala Thr Arg
        355                 360                 365

Val Asn Leu Tyr Gln Asn Ala Ile Leu Ser Pro Tyr Trp Asn Ile Asn
    370                 375                 380

Ala His Ser Val Met His Met Ile Gln Gly Arg Ala Arg Val Gln Val
385                 390                 395                 400

Val Asn Asn His Gly Gln Thr Val Phe Asn Asp Ile Leu Arg Arg Gly
                405                 410                 415

Gln Leu Leu Ile Ile Pro Gln His Tyr Val Val Leu Lys Lys Ala Glu
            420                 425                 430

Arg Glu Gly Cys Gln Tyr Ile Ser Phe Lys Thr Thr Pro Asn Ser Met
        435                 440                 445

Val Ser Tyr Ile Ala Gly Lys Thr Ser Ile Leu Arg Ala Leu Pro Val
    450                 455                 460

Asp Val Leu Ala Asn Ala Tyr Arg Ile Ser Arg Gln Glu Ser Gln Asn
465                 470                 475                 480

Leu Lys Asn Asn Arg Gly Glu Glu Phe Gly Ala Phe Thr Pro Lys Phe
                485                 490                 495

Ala Gln Thr Gly Ser Gln Ser Tyr Gln Asp Glu Gly Glu Ser Ser Ser
            500                 505                 510

Thr Glu Lys Ala Ser Glu
        515

<210>  264
<211>  605
<212>  PRT
<213>  Oryza rufipogon

<400> 264

Met Ser Ala Leu Thr Thr Ser Gln Leu Ala Thr Ser Ala Thr Gly Phe
1               5                   10                  15

Gly Ile Ala Asp Arg Ser Ala Pro Ser Ser Leu Leu Arg His Gly Phe
            20                  25                  30

Gln Gly Leu Lys Pro Arg Ser Pro Ala Gly Gly Asp Ala Thr Ser Leu
        35                  40                  45

Ser Val Thr Thr Ser Ala Arg Ala Thr Pro Lys Gln Gln Arg Ser Val
    50                  55                  60

Gln Arg Gly Ser Arg Arg Phe Pro Ser Val Val Val Tyr Ala Thr Gly
65                  70                  75                  80

Ala Gly Met Asn Val Val Phe Val Gly Ala Glu Met Ala Pro Trp Ser
                85                  90                  95

Lys Thr Gly Gly Leu Gly Asp Val Leu Gly Gly Leu Pro Pro Ala Met
            100                 105                 110

Ala Ala Asn Gly His Arg Val Met Val Ile Ser Pro Arg Tyr Asp Gln
        115                 120                 125

Tyr Lys Asp Ala Trp Asp Thr Ser Val Val Ala Glu Ile Lys Val Ala
    130                 135                 140

Asp Arg Tyr Glu Arg Val Arg Phe Phe His Cys Tyr Lys Arg Gly Val
145                 150                 155                 160

Asp Arg Val Phe Val Asp His Pro Ser Phe Leu Glu Lys Val Trp Gly
                165                 170                 175

Lys Thr Gly Glu Lys Ile Tyr Gly Pro Asp Thr Gly Val Asp Tyr Lys
            180                 185                 190

Asp Asn Gln Met Arg Phe Ser Leu Leu Cys Gln Ala Pro Arg Ile Leu
        195                 200                 205

Asn Leu Asn Asn Asn Pro Tyr Phe Lys Gly Thr Tyr Gly Glu Asp Val
    210                 215                 220

Val Phe Val Cys Asn Asp Trp His Thr Gly Pro Leu Ala Ser Tyr Leu
225                 230                 235                 240

Lys Asn Asn Tyr Gln Pro Asn Gly Ile Tyr Arg Asn Ala Lys Val Ala
                245                 250                 255

Phe Cys Ile His Asn Ile Ser Tyr Gln Gly Arg Phe Ala Phe Glu Asp
            260                 265                 270

Tyr Pro Glu Leu Asn Leu Ser Glu Arg Phe Arg Ser Ser Phe Asp Phe
        275                 280                 285

Ile Asp Gly Tyr Asp Thr Pro Val Glu Gly Arg Lys Ile Asn Trp Met
    290                 295                 300

Lys Ala Gly Ile Leu Glu Ala Asp Arg Val Leu Thr Val Ser Pro Tyr
305                 310                 315                 320

Tyr Ala Glu Glu Leu Ile Ser Gly Ile Ala Arg Gly Cys Glu Leu Asp
                325                 330                 335

Asn Ile Met Arg Leu Thr Gly Ile Thr Gly Ile Val Asn Gly Met Asp
            340                 345                 350

Val Ser Glu Trp Asp Pro Ser Lys Asp Lys Tyr Ile Thr Ala Lys Tyr
        355                 360                 365

Asp Ala Thr Thr Ala Ile Glu Ala Lys Ala Leu Asn Lys Glu Ala Leu
    370                 375                 380

Gln Ala Glu Ala Gly Leu Pro Val Asp Arg Lys Ile Pro Leu Ile Ala
385                 390                 395                 400

Phe Ile Gly Arg Leu Glu Glu Gln Lys Gly Pro Asp Val Met Ala Ala
                405                 410                 415

Ala Ile Pro Glu Leu Met Gln Glu Asp Val Gln Ile Val Leu Leu Gly
            420                 425                 430

Thr Gly Lys Lys Lys Phe Glu Lys Leu Leu Lys Ser Met Glu Glu Lys
        435                 440                 445

Tyr Pro Gly Lys Val Arg Ala Val Val Lys Phe Asn Ala Pro Leu Ala
    450                 455                 460

His Leu Ile Met Ala Gly Ala Asp Val Leu Ala Val Pro Ser Arg Phe
465                 470                 475                 480

Glu Pro Cys Gly Leu Ile Gln Leu Gln Gly Met Arg Tyr Gly Thr Pro
                485                 490                 495

Cys Ala Cys Ala Ser Thr Gly Gly Leu Val Asp Thr Val Ile Glu Gly
        500                 505                 510

Lys Thr Gly Phe His Met Gly Arg Leu Ser Val Asp Cys Lys Val Val
        515                 520                 525

Glu Pro Ser Asp Val Lys Lys Val Ala Ala Thr Leu Lys Arg Ala Ile
    530                 535                 540

Lys Val Val Gly Thr Pro Ala Tyr Glu Glu Met Val Arg Asn Cys Met
545                 550                 555                 560

Asn Gln Asp Leu Ser Trp Lys Gly Pro Ala Lys Asn Trp Glu Asn Val
                565                 570                 575

Leu Leu Gly Leu Gly Val Ala Gly Ser Ala Pro Gly Ile Glu Gly Asp
        580                 585                 590

Glu Ile Ala Pro Leu Ala Lys Glu Asn Val Ala Ala Pro
        595                 600                 605

<210>  265
<211>  609
<212>  PRT
<213>  ORYZA OFFICINALIS

<400>  265

Met Ser Ala Leu Thr Thr Ser Gln Leu Ala Thr Ser Ala Thr Gly Phe
1               5                   10                  15

Gly Ile Ala Asp Arg Ser Ala Pro Ser Ser Leu Leu Arg His Gly Phe
        20                  25                  30

Gln Gly Leu Lys Pro Arg Ser Pro Ala Gly Gly Asp Ala Ser Ser Leu
        35                  40                  45

Ser Val Thr Thr Ser Ala Arg Ala Thr Pro Lys Gln Gln Arg Ser Val
    50                  55                  60

Gln Arg Gly Ser Arg Arg Phe Pro Ser Val Val Val Tyr Ala Thr Gly
65                  70                  75                  80

Ala Gly Met Asn Val Val Phe Val Gly Ala Glu Met Ala Pro Trp Ser
                85                  90                  95

Lys Thr Gly Gly Leu Gly Asp Val Leu Gly Gly Leu Pro Pro Ala Met
        100                 105                 110

Ala Ala Asn Gly His Arg Val Met Val Ile Ser Pro Arg His Asp Gln
        115                 120                 125

Tyr Lys Asp Ala Trp Asp Thr Ser Val Val Ala Glu Ile Lys Val Ala
    130                 135                 140

Asp Arg Tyr Glu Arg Val Arg Phe Phe His Cys Tyr Lys Arg Gly Val
145                 150                 155                 160

Asp Arg Val Phe Ile Asp His Pro Ser Phe Leu Glu Lys Val Trp Gly
                165                 170                 175

Lys Thr Gly Glu Lys Ile Tyr Gly Pro Asp Thr Gly Val Asp Tyr Lys
            180                 185                 190

Asp Asn Gln Met Arg Phe Ser Leu Leu Cys Gln Ala Ala Leu Glu Ala
        195                 200                 205

Pro Arg Ile Leu Asn Leu Asn Asn Asn Pro Tyr Phe Lys Gly Thr Tyr
    210                 215                 220

Gly Glu Asp Val Val Phe Val Cys Asn Asp Trp His Thr Gly Pro Leu
225                 230                 235                 240

Pro Ser Tyr Leu Lys Asn Asn Tyr Gln Pro Asn Gly Ile Tyr Arg Asn
                245                 250                 255

Ala Lys Val Ala Phe Cys Ile His Asn Ile Ser Tyr Gln Gly Arg Phe
            260                 265                 270

Ala Phe Glu Asp Tyr Pro Glu Leu Asn Leu Ser Glu Arg Phe Arg Ser
        275                 280                 285

Ser Phe Asp Phe Ile Asp Gly Tyr Asp Thr Pro Val Glu Gly Arg Lys
    290                 295                 300

Ile Asn Trp Met Lys Ala Gly Ile Leu Glu Ser Asp Arg Val Leu Thr
305                 310                 315                 320

Val Ser Pro Tyr Tyr Ala Glu Glu Leu Ile Ser Gly Ile Ala Arg Gly
                325                 330                 335

Cys Glu Leu Asp Asn Ile Met Arg Leu Thr Gly Ile Thr Gly Ile Val
            340                 345                 350

Asn Gly Met Asp Val Ser Glu Trp Asp Pro Ser Lys Asp Lys Tyr Ile
        355                 360                 365

Ala Ala Lys Tyr Asp Ala Thr Thr Ala Ile Glu Ala Lys Ala Leu Asn
    370                 375                 380

Lys Glu Ala Leu Gln Ala Glu Ala Gly Leu Pro Val Asp Arg Lys Ile
385                 390                 395                 400

Pro Leu Ile Ala Phe Ile Gly Arg Leu Glu Glu Gln Lys Gly Pro Asp
                405                 410                 415

Val Met Ala Ala Ala Ile Pro Glu Leu Met Gln Glu Asn Val Gln Ile
            420                 425                 430

Val Leu Leu Gly Thr Gly Lys Lys Lys Phe Glu Lys Leu Leu Lys Ser
        435                 440                 445

Met Glu Glu Lys Tyr Pro Gly Lys Val Arg Ala Val Val Lys Phe Asn
    450                 455                 460

Ala Pro Leu Ala His Leu Ile Met Ala Gly Ala Asp Val Leu Ala Val
465                 470                 475                 480

Pro Ser Arg Phe Glu Pro Cys Gly Leu Ile Gln Leu Gln Gly Met Arg
                485                 490                 495

Tyr Gly Thr Pro Cys Ala Cys Ala Ser Thr Gly Gly Leu Val Asp Thr
            500                 505                 510

Val Ile Glu Gly Lys Thr Gly Phe His Met Gly Arg Leu Ser Val Asp
        515                 520                 525

Cys Lys Val Val Glu Pro Ser Asp Val Gln Lys Val Ala Thr Thr Leu
    530                 535                 540

Lys Arg Ala Ile Lys Ile Val Gly Thr Pro Ala Tyr Asn Glu Met Val
545                 550                 555                 560

Arg Asn Cys Met Asn Gln Asp Leu Ser Trp Lys Gly Pro Ala Lys Asn
                565                 570                 575

Trp Glu Asn Val Leu Leu Gly Leu Gly Val Ala Gly Ser Ala Pro Gly
            580                 585                 590

Val Glu Gly Glu Glu Ile Ala Pro Leu Ala Lys Glu Asn Val Ala Ala
        595                 600                 605

Pro

<210>  266
<211>  603
<212>  PRT
<213>  Hordeum vulgare

<400>  266

Met Ala Ala Leu Ala Thr Ser Gln Leu Ala Thr Ser Gly Thr Val Leu
1               5                   10                  15

Gly Val Thr Asp Arg Phe Arg Arg Pro Gly Phe Gln Gly Leu Arg Pro
            20                  25                  30

Arg Asn Pro Ala Asp Ala Ala Leu Gly Met Arg Thr Ile Gly Ala Ser
        35                  40                  45

Ala Ala Pro Lys Gln Ser Arg Lys Ala His Arg Gly Ser Arg Arg Cys
    50                  55                  60

Leu Ser Val Val Val Arg Ala Thr Gly Ser Gly Met Asn Leu Val Phe
65                  70                  75                  80

Val Gly Ala Glu Met Ala Pro Trp Ser Lys Thr Gly Gly Leu Gly Asp
                85                  90                  95

Val Leu Gly Gly Leu Pro Pro Ala Met Ala Ala Asn Gly His Arg Val
            100                 105                 110

Met Val Val Ser Pro Arg Tyr Asp Gln Tyr Lys Asp Ala Trp Asp Thr
        115                 120                 125

Ser Val Ile Ser Glu Ile Lys Val Ala Asp Glu Tyr Glu Arg Val Arg
    130                 135                 140

Phe Phe His Cys Tyr Lys Arg Gly Val Asp Arg Val Phe Ile Asp His
145                 150                 155                 160

Pro Trp Phe Leu Glu Lys Val Arg Gly Lys Thr Lys Glu Lys Ile Tyr
                165                 170                 175

Gly Pro Asp Ala Gly Thr Asp Tyr Glu Asp Asn Gln Gln Arg Phe Ser
            180                 185                 190

Leu Leu Cys Gln Ala Ala Leu Glu Ala Pro Arg Ile Leu Asn Leu Asn
        195                 200                 205

Asn Asn Pro Tyr Phe Ser Gly Pro Tyr Gly Glu Asp Val Val Phe Val

210                 215                 220

Cys Asn Asp Trp His Thr Gly Leu Leu Ala Cys Tyr Leu Lys Ser Asn
225                 230                 235                 240

Tyr Gln Ser Asn Gly Ile Tyr Arg Thr Ala Lys Val Ala Phe Cys Ile
                245                 250                 255

His Asn Ile Ser Tyr Gln Gly Arg Phe Ser Phe Asp Asp Phe Ala Gln
            260                 265                 270

Leu Asn Leu Pro Asp Arg Phe Lys Ser Ser Phe Asp Phe Ile Asp Gly
        275                 280                 285

Tyr Asp Lys Pro Val Glu Gly Arg Lys Ile Asn Trp Met Lys Ala Gly
    290                 295                 300

Ile Leu Gln Ala Asp Lys Val Leu Thr Val Ser Pro Tyr Tyr Ala Glu
305                 310                 315                 320

Glu Leu Ile Ser Asp Glu Ala Arg Gly Cys Glu Leu Asp Asn Ile Met
                325                 330                 335

Arg Leu Thr Gly Ile Thr Gly Ile Val Asn Gly Met Asp Val Ser Glu
            340                 345                 350

Trp Asp Pro Thr Lys Asp Lys Phe Leu Ala Val Asn Tyr Asp Ile Thr
        355                 360                 365

Thr Ala Leu Glu Ala Lys Ala Leu Asn Lys Glu Ala Leu Gln Ala Glu
    370                 375                 380

Val Gly Leu Pro Val Asp Arg Lys Val Pro Leu Val Ala Phe Ile Gly
385                 390                 395                 400

Arg Leu Glu Glu Gln Lys Gly Pro Asp Val Met Ile Ala Ala Ile Pro
                405                 410                 415

Glu Ile Leu Lys Glu Glu Asp Val Gln Ile Ile Leu Leu Gly Thr Gly
            420                 425                 430

Lys Lys Lys Phe Glu Lys Leu Leu Lys Ser Met Glu Glu Lys Phe Pro
        435                 440                 445

Gly Lys Val Arg Ala Val Val Arg Phe Asn Ala Pro Leu Ala His Gln
    450                 455                 460

Met Met Ala Gly Ala Asp Leu Leu Ala Val Thr Ser Arg Phe Glu Pro
465                 470                 475                 480

Cys Gly Leu Ile Gln Leu Gln Gly Met Arg Tyr Gly Thr Pro Cys Val
                485                 490                 495

Cys Ala Ser Thr Gly Gly Leu Val Asp Thr Ile Val Glu Gly Lys Thr
            500                 505                 510

Gly Phe His Met Gly Arg Leu Ser Val Asp Cys Asn Val Val Glu Pro
        515                 520                 525

Ala Asp Val Lys Lys Val Ala Thr Thr Leu Lys Arg Ala Val Lys Val
    530                 535                 540

Val Gly Thr Pro Ala Tyr Gln Glu Met Val Lys Asn Cys Met Ile Gln
545                 550                 555                 560

Asp Leu Ser Trp Lys Gly Pro Ala Lys Asn Trp Glu Asp Val Leu Leu
                565                 570                 575

Glu Leu Gly Val Glu Gly Ser Glu Pro Gly Ile Val Gly Glu Glu Ile
            580                 585                 590

Ala Pro Leu Ala Met Glu Asn Val Ala Ala Pro
        595                 600

<210>  267
<211>  336
<212>  PRT
<213>  Staphylococcus aureus

<400>  267

Met Lys Gly Lys Phe Leu Lys Val Ser Ser Leu Phe Val Ala Thr Leu
1               5                   10                  15

Thr Thr Ala Thr Leu Val Ser Ser Pro Ala Ala Asn Ala Leu Ser Ser
            20                  25                  30

Lys Ala Met Asp Asn His Pro Gln Gln Ser Gln Ser Ser Lys Gln Gln
        35                  40                  45

Thr Pro Lys Ile Gln Lys Gly Gly Asn Leu Lys Pro Leu Glu Gln Arg
    50                  55                  60

Glu His Ala Asn Val Ile Leu Pro Asn Asn Asp Arg His Gln Ile Thr
65                  70                  75                  80

Asp Thr Thr Asn Gly His Tyr Ala Pro Val Thr Tyr Ile Gln Val Glu
                85                  90                  95

Ala Pro Thr Gly Thr Phe Ile Ala Ser Gly Val Val Val Gly Lys Asp
            100                 105                 110

Thr Leu Leu Thr Asn Lys His Val Val Asp Ala Thr His Gly Asp Pro
        115                 120                 125

His Ala Leu Lys Ala Phe Pro Ser Ala Ile Asn Gln Asp Asn Tyr Pro
    130                 135                 140

Asn Gly Gly Phe Thr Ala Glu Gln Ile Thr Lys Tyr Ser Gly Glu Gly
145                 150                 155                 160

Asp Leu Ala Ile Val Lys Phe Ser Pro Asn Glu Gln Asn Lys His Ile
                165                 170                 175

Gly Glu Val Val Lys Pro Ala Thr Met Ser Asn Asn Ala Glu Thr Gln
            180                 185                 190

Val Asn Gln Asn Ile Thr Val Thr Gly Tyr Pro Gly Asp Lys Pro Val
        195                 200                 205

Ala Thr Met Trp Glu Ser Lys Gly Lys Ile Thr Tyr Leu Lys Gly Glu
    210                 215                 220

Ala Met Gln Tyr Asp Leu Ser Thr Thr Gly Gly Asn Ser Gly Ser Pro
225                 230                 235                 240

Val Phe Asn Glu Lys Asn Glu Val Ile Gly Ile His Trp Gly Gly Val
                245                 250                 255

Pro Asn Glu Phe Asn Gly Ala Val Phe Ile Asn Glu Asn Val Arg Asn
            260                 265                 270

Phe Leu Lys Gln Asn Ile Glu Asp Ile His Phe Ala Asn Asp Asp Gln
        275                 280                 285

Pro Asn Asn Pro Asp Asn Pro Asp Asn Pro Asn Asn Pro Asp Asn Pro
    290                 295                 300

Asn Asn Pro Asp Glu Pro Asn Asn Pro Asp Asn Pro Asn Asn Pro Asp
305                 310                 315                 320

Asn Pro Asp Asn Gly Asp Asn Asn Asn Ser Asp Asn Pro Asp Ala Ala
                325                 330                 335

## Description

### Background to the Invention

It is estimated that a staggering 2 billion people worldwide suffer from some type of inflammation. Inflammation is a vast biological process and an integral part of our immune response. The inflammatory response may be acute (short lived) or chronic (longer lasting) and can occur in almost every part of the body whether it be internal or external. Interestingly as well, whatever the cause of inflammation, the biological changes that occur within the body due to the inflammation response are the same throughout the body, meaning the modes of reducing inflammation, which different anti-inflammatory agents carry are the same in all parts of the body.
Typically, inflammation is a natural response and a necessary one that rids the cells of injury causes, foreign attacks, or removes dead cells. However, excess inflammation has drastic and sometimes detrimental effects on the human body. Indeed, an inflammatory response can have long-lasting, negative consequences such as tissue damage. During an inflammatory response, the body releases lysosomal enzymes which can damage tissue and can even lead to a life-threatening hypersensitivity reaction. These conditions can have long-lasting effects both externally, with the development of acute skin rashes and eczema, and internally, triggering diseases such as inflammatory bowel disease. As a result, maintaining a normal level of inflammation is very important for our health and wellbeing, both inside and out. Unfortunately, inflammation is on the rise. One of the major factors of this increase comes from our exposure to an increasing variety of external agents that our bodies are not accustomed to.

Most anti-inflammatory treatments used today are drugs. There are two major types of anti-inflammatory drugs, corticosteroids and non-steroidal anti-inflammatory drugs (NSAIDs). These include: Immunosuppressives (methotrexate, ciclosporin); Specific biological drugs (mostly TNF-alpha inhibitors, but also inhibitors of cyclooxygenase enzyme for example); Cytotoxic drugs; and Oral retinoids (acitretin). Furthermore, topical treatments exist to specifically reduce skin inflammation. Examples include creams and ointment (mostly cortisone based), and physical treatments like UV radiations. These drugs however have drastic side-effects such as gastrointestinal toxicity and anaphylactoid reactions. They can even suppress the immune system to such a point that it is made vulnerable to other diseases and pathogens.

Therefore, there is a clear need for the identification of agents having anti-inflammatory activity that are not immunosuppressive and/or cause other undesirable side effects. To that end, very specific types of food are known to reduce inflammation (Kiecolt-Glaser J.K. *et al.* 2010, Middleton E. *et al.* 2000, Chatterjee M. *et al.* 2005). Indeed, particular components of these specific foods are in low concentrations, hidden, or locked away, and once identified and unlocked can be scaled to specifically target inflammation in a recognized way, as our bodies understand the components of food and are able to readily process these molecules. Indeed, these particular food molecules can reduce inflammation without completely blocking this immune system response which puts the body in a vulnerable state. For those with many food allergies as well, identifying and unlocking the particular components of a food that may reduce inflammation would allow for these individuals to gain the anti-inflammatory benefits of a food they would otherwise be allergic to.

It is an object of the invention to overcome at least one of the above-referenced problems.

### Statements of Invention

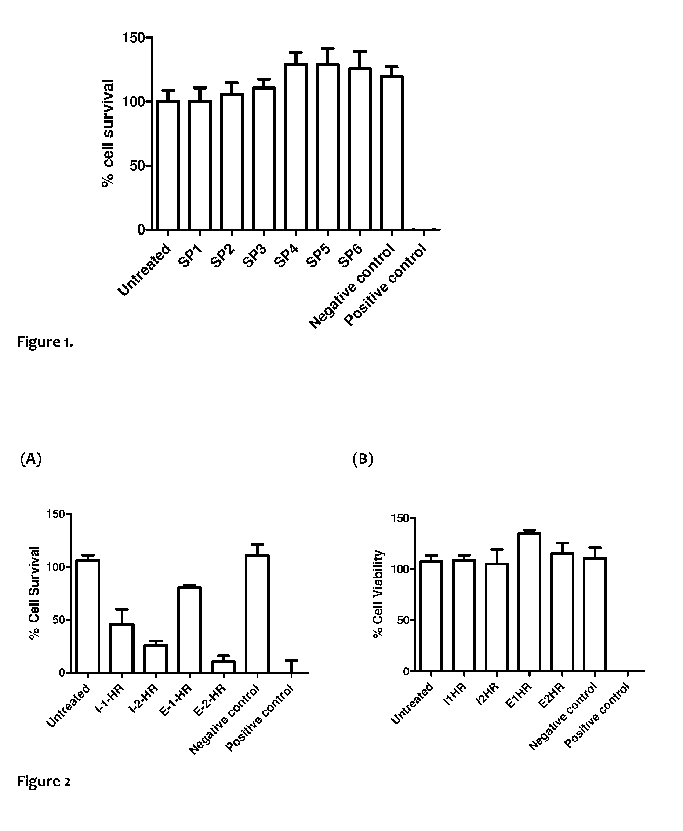

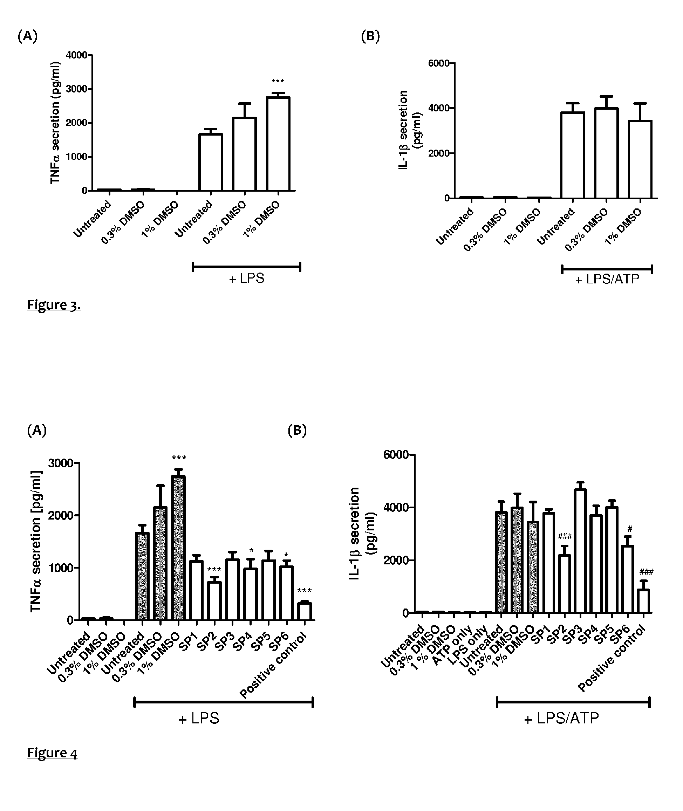

The pea genome codes for over 70,000 different proteins. The Applicant has identified seven of these proteins, each of which contains one or more anti-inflammatory fragments. Likewise, out of the more than 60,000 proteins encoded by the rice genome, the Applicant has identified eight proteins, each of which contains one or more anti-inflammatory fragments. The anti-inflammatory fragments of the sixteen identified proteins have been shown to have anti-inflammatory activity when incubated with LPS-stimulated human cells (Figs 4-6), do not cause human cell viability issues (Fig. 1) and are not toxic to human cells (Fig. 2). The specific plant proteins from which the natural peptides are derived are provided in SEQUENCE ID NO: 1-15, especially from regions of the proteins provided in SEQUENCE ID NO: 17-69. The specific pea proteins from which the peptides are derived are provided in SEQUENCE ID NO: 1 to 5, 8 and 9, and the specific rice proteins from which the peptides are derived are provided in SEQUENCE ID NO: 6, 7 and 10 to 15. Homologs of these proteins are described in SEQUENCE ID NO: 222-267. The specific peptides initially identified in the pea proteins are shown in SEQUENCE ID NO's: 71-107 and 110-111. The specific peptides initially identified in the rice proteins are shown in SEQUENCE ID NO's: 108-109 and 112-220.

In a first aspect, the invention provides an anti-inflammatory peptide and comprising an anti-inflammatory fragment of a protein selected from SEQUENCE ID NO's: 1 to 16, or a homolog thereof, or a variant of the anti-inflammatory fragment..

In one embodiment, the fragment has between 7 and 37 amino acids and a charge of between -9 and +3.

Preferably, the c-terminal amino acid is not cysteine (C) or methionine (M).

Preferably, the n-terminal amino acid is not cysteine (C), histidine (H), proline (P) or threonine (T)

Preferably, the c-terminal domain of the fragment does not contain cysteine (C).

Preferably, the n-terminal domain of the fragment does not contain cysteine (C).

Preferably, the fragment does not contain cysteine (C).

Preferably, the peptide does not contain cysteine (C).

Preferably, the fragment is from a region of the proteins of SEQUENCE ID NO's: 1 to 16, which regions are characterised by the following features:
- 17 to 109 amino acids in length;
- a charge of between -6 and +4;
- the c-terminal amino acid is not aspartic acid (D), phenylalanine (F), methionine (M) or tryptophan (W);
- the n-terminal amino acid is not aspartic acid (D), histidine (H), methionine (M), proline (P) or tryptophan (W).

Preferably, the c-terminal domain of the region does not contain tryptophan (W).

Preferably, the regions of the proteins of SEQUENCE ID NO's: 1 to 7 are selected from the SEQUENCE ID NO's: 17-33.

Preferably, the regions of the proteins of SEQUENCE ID NO's: 8 to 16 are selected from the SEQUENCE ID NO's: 34-70.

Preferably, the regions of the proteins of SEQUENCE ID NO's: 1 to 16 are selected from the SEQUENCE ID NO's: 17-70.

Preferably, the anti-inflammatory fragment is selected from SEQUENCE ID NO's: 71 to 221, or an anti-inflammatory variant of the fragment.

Preferably, the peptide consists of a fragment selected from SEQUENCE ID NO's: 71 to 221, or an anti-inflammatory variant of the fragment.

Preferably, the peptide consists of a sequence selected from SEQUENCE ID NO's: 71 to 221.

### [SEQUENCE ID NO: 1 (Pea Protein 1)]

Preferably, the peptide comprises an anti-inflammatory fragment of the protein of SEQUENCE ID NO: 1, or a homolog thereof.

Preferably, the peptide comprises a fragment of one of seven regions of SEQUENCE ID NO: 1, namely SEQUENCE ID NO'S: 17 to 23, or an anti-inflammatory variant of the fragment.

Preferably, the peptide comprises an anti-inflammatory fragment selected from SEQUENCE ID NO'S: 71 to 91, or an anti-inflammatory variant of the fragment.

Preferably, the peptide comprises a fragment of the region of SEQUENCE ID NO:17, for example SEQUENCE ID NO: 71, or an anti-inflammatory variant of the fragment.

Preferably, the peptide comprises a fragment of the region of SEQUENCE ID NO:18, for example SEQUENCE ID NO: 72, or an anti-inflammatory variant of the fragment.

Preferably, the peptide comprises a fragment of the region of SEQUENCE ID NO:19, for example either or both of SEQUENCE ID NO: 73 or 74, or an anti-inflammatory variant of the fragment.

Preferably, the peptide comprises a fragment of the region of SEQUENCE ID NO:20, for example either or both of SEQUENCE ID NO: 75 or 76. or an anti-inflammatory variant of the fragment.

Preferably, the peptide comprises a fragment of the region of SEQUENCE ID NO:21, for example one or more or all of SEQUENCE ID NO: 77 to 84, or an anti-inflammatory variant of the fragment.

Preferably, the peptide comprises a fragment of the region of SEQUENCE ID NO:22, for example one or more or all of SEQUENCE ID NO: 85 to 89, or an anti-inflammatory variant of the fragment.

Preferably, the peptide comprises a fragment of the region of SEQUENCE ID NO:23, for example SEQUENCE ID NO: 90 or an anti-inflammatory variant of the fragment.

Preferably, the peptide comprises SEQUENCE ID NO: 91, or an anti-inflammatory variant of the fragment.

The invention also provides a composition comprising at least one anti-inflammatory peptides of the invention, which peptide includes an anti-inflammatory fragment of SEQUENCE ID NO: 1 or a homolog thereof. The invention also provides a composition comprising at least 2, preferably 3, preferably 4, preferably 5, preferably 6, preferably 7, preferably 8, preferably 9, or preferably 10 anti-inflammatory peptides of the invention, each of which comprises a different anti-inflammatory fragment of SEQUENCE ID NO: 1 or a homolog thereof.. Preferably, the composition comprises a first anti-inflammatory peptide of the invention that comprises an anti-inflammatory fragment of a first region selected from SEQUENCE ID NO'S: 17 to 23, and a second anti-inflammatory peptide of the invention that comprises an anti-inflammatory fragment of a second region selected from SEQUENCE ID NO'S: 17 to 23. Preferably, the composition comprises a first anti-inflammatory peptide comprising a first anti-inflammatory fragment selected from SEQUENCE ID NO: 71 to 91 (or an anti-inflammatory variant of the fragment), and a second anti-inflammatory peptide comprising a second anti-inflammatory fragment selected from SEQUENCE ID NO: 71 to 91 (or an anti-inflammatory variant of the fragment).

Homologs of Pea Protein 1 (SEQUENCE ID NO: 1) include Vicia fabia, Cicer arietinum and Lens culinaris homologs (SEQ ID NO: 222-224).

### [SEQUENCE ID NO: 2 (Pea Protein 2)]

Preferably, the peptide comprises an anti-inflammatory fragment of the protein of SEQUENCE ID NO: 2, or a homolog thereof.

Preferably, the peptide comprises an anti-inflammatory fragment of one of two regions of SEQUENCE ID NO: 2, namely SEQUENCE ID NO'S: 24 or 25.

Preferably, the peptide comprises an anti-inflammatory fragment of the region of SEQUENCE ID NO:24, for example SEQUENCE ID NO: 92, or an anti-inflammatory variant of the fragment.

Preferably, the peptide comprises a fragment of the region of SEQUENCE ID NO:25, for example SEQUENCE ID NO: 93, or an anti-inflammatory variant of the fragment.

The invention also provides a composition comprising at least one anti-inflammatory peptides of the invention, which peptide includes an anti-inflammatory fragment of SEQUENCE ID NO: 2 or a homolog thereof. The invention also provides a composition comprising at least two anti-inflammatory peptides of the invention, each of which comprises a different anti-inflammatory fragment of SEQUENCE ID NO: 2 or a homolog thereof. Preferably, the composition comprises a first anti-inflammatory peptide comprising an anti-inflammatory fragment of the region of SEQUENCE ID NO 24, and a second anti-inflammatory peptide comprising an anti-inflammatory fragment of the region of SEQUENCE ID NO 25. Preferably, the composition comprises a first anti-inflammatory peptide comprising an anti-inflammatory fragment of SEQUENCE ID NO 92, and a second anti-inflammatory peptide comprising an anti-inflammatory fragment of SEQUENCE ID NO 93.

Homologs of Pea Protein 2 (SEQUENCE ID NO: 2) include Lens culinaris, Vicia narbonensis and Glycine max (SEQ ID NO: 225-227).

### [SEQUENCE ID NO: 3 (Pea Protein 3)]

Preferably, the peptide comprises an anti-inflammatory fragment of the protein of SEQUENCE ID NO: 3, or a homolog thereof.

Preferably, the peptide comprises an anti-inflammatory fragment of one of two regions of SEQUENCE ID NO: 3, namely SEQUENCE ID NO'S: 26 or 27.

Preferably, the peptide comprises an anti-inflammatory fragment of the region of SEQUENCE ID NO:26, for example SEQUENCE ID NO: 94, or an anti-inflammatory variant the fragment.

Preferably, the peptide comprises an anti-inflammatory fragment of the region of SEQUENCE ID NO:27, for example SEQUENCE ID NO: 95, or an anti-inflammatory variant of the fragment.

The invention also provides a composition comprising at least one anti-inflammatory peptides of the invention, which peptide includes an anti-inflammatory fragment of SEQUENCE ID NO: 3 or a homolog thereof. The invention also provides a composition comprising at least two anti-inflammatory peptides of the invention, each of which comprises a different anti-inflammatory fragment of SEQUENCE ID NO: 3 or a homolog thereof. Preferably, the composition comprises a first peptide comprising an anti-inflammatory fragment of the region of SEQUENCE ID NO 26, and a second peptide comprising an anti-inflammatory fragment of SEQUENCE ID NO 27. Preferably, the composition comprises a first anti-inflammatory peptide comprising an anti-inflammatory fragment of SEQUENCE ID NO 94 (or an anti-inflammatory variant of the fragment), and a second anti-inflammatory peptide comprising an anti-inflammatory fragment of SEQUENCE ID NO 95 (or an anti-inflammatory variant of the fragment).

Homologs of Pea Protein 3 (SEQUENCE ID NO: 3) include Vicia sativa, Medicago truncatula, and Lotus japonicas (SEQ ID NO: 228-230).

### [SEQUENCE ID NO: 4 (Pea Protein 4)]

Preferably, the peptide comprises an anti-inflammatory fragment of the protein of SEQUENCE ID NO: 4, or a homolog thereof.

Preferably, the peptide comprises an anti-inflammatory fragment of one of five regions of SEQUENCE ID NO: 4, namely SEQUENCE ID NO'S: 28 to 32. Preferably, the region is SEQUENCE ID NO: 28, preferably SEQUENCE ID NO: 29, preferably SEQUENCE ID NO: 30, preferably SEQUENCE ID NO: 31, preferably SEQUENCE ID NO: 32.

Preferably, the peptide comprises an anti-inflammatory fragment of the region of SEQUENCE ID NO:28, for example SEQUENCE ID NO: 96, or an anti-inflammatory variant of the fragment.

Preferably, the peptide comprises an anti-inflammatory fragment of the region of SEQUENCE ID NO:29, for example SEQUENCE ID NO: 97 to 103, or an anti-inflammatory variant of the fragment.

Preferably, the peptide comprises an anti-inflammatory fragment of the region of SEQUENCE ID NO:30, for example SEQUENCE ID NO: 104, or an anti-inflammatory variant of the fragment.

Preferably, the peptide comprises an anti-inflammatory fragment of the region of SEQUENCE ID NO:31, for example SEQUENCE ID NO: 105, or an anti-inflammatory variant of the fragment.

Preferably, the peptide comprises an anti-inflammatory fragment of the region of SEQUENCE ID NO:32, for example SEQUENCE ID NO: 106, or an anti-inflammatory variant of the fragment.

The invention also provides a composition comprising at least one anti-inflammatory peptides of the invention, which peptide includes an anti-inflammatory fragment of SEQUENCE ID NO: 4 or a homolog thereof. The invention also provides a composition comprising at least 2, preferably 3, preferably 4, preferably 5, preferably 6, preferably 7, preferably 8, preferably 9, or preferably 10 peptides of the invention that comprise different anti-inflammatory fragments of SEQUENCE ID NO: 4 or a homolog thereof. Preferably, the composition comprises a first peptide comprising an anti-inflammatory fragment of a first region selected from SEQUENCE ID NO'S: 28 to 32, and a second peptide that comprises an anti-inflammatory fragment of a second region selected from SEQUENCE ID NO'S: 28 to 32. Preferably, the composition comprises a first anti-inflammatory peptide comprising a first anti-inflammatory fragment selected from SEQUENCE ID NO: 96 to 106 (or an anti-inflammatory variant of the fragment), and a second anti-inflammatory peptide comprising a second anti-inflammatory fragment selected from SEQUENCE ID NO 96 to 106 (or an anti-inflammatory variant of the fragment).

Homologs of Pea Protein 4 (SEQUENCE ID NO: 4) include Pisum abyssinicum, Lathyrus annuus, and Vicia villosa (SEQ ID 231-233).

### [SEQUENCE ID NO: 5 (Pea Protein 5)]

Preferably, the peptide comprises an anti-inflammatory fragment of the protein of SEQUENCE ID NO: 5, or a homolog thereof.

Preferably, the peptide comprises an anti-inflammatory fragment of a region of SEQUENCE ID NO: 5, namely the region of SEQUENCE ID NO 33, for example SEQUENCE ID NO: 107, or an anti-inflammatory variant of the fragment.

The invention also provides a composition comprising at least one anti-inflammatory peptides of the invention, which peptide includes an anti-inflammatory fragment of SEQUENCE ID NO: 5 or a homolog thereof. The invention also provides a composition comprising at least two peptides of the invention that comprise at least one fragment of SEQUENCE ID NO: 5 or a homolog thereof, for example SEQUENCE ID NO: 107.

Homologs of Pea Protein 5 (SEQUENCE ID NO: 5) include Medicago truncatula, Vicia peregrine, and Vicia lutea (SEQ ID NO: 234-236).

### [SEQUENCE ID NO: 6 (Rice Protein 7 - Q6K508)]

Preferably, the peptide comprises an anti-inflammatory fragment of the protein of SEQUENCE ID NO: 6 or a homolog thereof.

Preferably, the peptide comprises an anti-inflammatory fragment of SEQUENCE ID NO: 108, or an anti-inflammatory variant of the fragment.

The invention also provides a composition comprising at least one anti-inflammatory peptides of the invention, which peptide includes an anti-inflammatory fragment of SEQUENCE ID NO: 6 or a homolog thereof. The invention also provides a composition comprising at least two peptides of the invention including at least one anti-inflammatory fragment of SEQUENCE ID NO: 6 or a homolog thereof, for example SEQUENCE ID NO: 108 (SP1).

Homologs of Rice Protein 7 (SEQUENCE ID NO: 6) include Oryza, brachyantha, Avena sativa, and Brachypodium distachyon (SEQ ID NO: 237-239).

### [SEQUENCE ID NO: 7 (Rice Protein 8 - Q6K7K6)]

Preferably, the peptide comprises an anti-inflammatory fragment of the protein of SEQUENCE ID NO: 7 or a homolog thereof.

Preferably, the peptide comprises an anti-inflammatory fragment of SEQUENCE ID NO: 109 (SP2), or an anti-inflammatory variant of the fragment.

The invention also provides a composition comprising at least one anti-inflammatory peptides of the invention, which peptide includes an anti-inflammatory fragment of SEQUENCE ID NO: 7 or a homolog thereof. The invention also provides a composition comprising at least two peptides of the invention including at least one anti-inflammatory fragment of SEQUENCE ID NO: 7 or a homolog thereof, for example SEQUENCE ID NO: 109 (SP2).

Homologs of Rice Protein 8 (SEQUENCE ID NO: 7) include Oryza sativa Japonica Group, Oryza sativa Indica Group, and Oryza brachyantha (SEQ ID NO: 240-242).

### [SEQUENCE ID NO: 8 (Pea Protein 6 - P13919)]

Preferably, the peptide comprises an anti-inflammatory fragment of the protein of SEQUENCE ID NO: 8 or a homolog thereof.

Preferably, the peptide comprises an anti-inflammatory fragment of SEQUENCE ID NO: 110 (SP3), or an anti-inflammatory variant of the fragment.

The invention also provides a composition comprising at least one anti-inflammatory peptides of the invention, which peptide includes an anti-inflammatory fragment of SEQUENCE ID NO: 8 or a homolog thereof. The invention also provides a composition comprising at least two peptides of the invention including at least one anti-inflammatory fragment of SEQUENCE ID NO: 8 or a homolog thereof, for example SEQUENCE ID NO: 110 (SP3).

Homologs of Pea Protein 8 (SEQUENCE ID NO: 8) include Pisum fulvum, Pisum abyssinicum and Vicia villosa (SEQ ID NO: 243-245).

### [SEQUENCE ID NO: 9 (Pea Protein 7 - P02855)]

Preferably, the peptide comprises an anti-inflammatory fragment of the protein of SEQUENCE ID NO: 9 or a homolog thereof.

Preferably, the peptide comprises an anti-inflammatory fragment of SEQUENCE ID NO: 111 (SP4), or an anti-inflammatory variant of the fragment.

The invention also provides a composition comprising at least one anti-inflammatory peptides of the invention, which peptide includes an anti-inflammatory fragment of SEQUENCE ID NO: 9 or a homolog thereof. The invention also provides a composition comprising at least two peptides of the invention including at least one anti-inflammatory fragment of SEQUENCE ID NO: 9 or a homolog thereof, for example SEQUENCE ID NO: 111 (SP4).

Homologs of Pea Protein 9 (SEQUENCE ID NO: 9) include Lathyrus hirsutus, Lathyrus cicero, Lathyrus sativus (SEQ ID NO: 246-248).

### [SEQUENCE ID NO: 10 (Rice Protein 1 - P07728)]

Preferably, the peptide comprises an anti-inflammatory fragment of the protein of SEQUENCE ID NO: 10 or a homolog thereof.

Preferably, the peptide comprises a fragment of one of nine regions of SEQUENCE ID NO: 10, namely SEQUENCE ID NO'S: 34 to 42. Preferably, the region is SEQUENCE ID NO: 34, preferably SEQUENCE ID NO: 35, preferably SEQUENCE ID NO: 36, preferably SEQUENCE ID NO: 37, preferably SEQUENCE ID NO: 38, preferably SEQUENCE ID NO: 39, preferably SEQUENCE ID NO: 40, preferably SEQUENCE ID NO: 41, preferably SEQUENCE ID NO: 42.

Preferably, the peptide comprises an anti-inflammatory fragment of the region of SEQUENCE ID NO:34, for example SEQUENCE ID NO: 112 or SEQUENCE ID NO: 113 or an anti-inflammatory variant of the fragment.

Preferably, the peptide comprises an anti-inflammatory fragment of the region of SEQUENCE ID NO:35, for example SEQUENCE ID NO: 114, or an anti-inflammatory variant of the fragment.

Preferably, the peptide comprises an anti-inflammatory fragment of the region of SEQUENCE ID NO:36, for example SEQUENCE ID NO: 115 or SEQUENCE ID NO: 116, or an anti-inflammatory variant of the fragment.

Preferably, the peptide comprises an anti-inflammatory fragment of the region of SEQUENCE ID NO:37, for example SEQUENCE ID NO: 117 or SEQUENCE ID NO: 118, or an anti-inflammatory variant of the fragment.

Preferably, the peptide comprises an anti-inflammatory fragment of the region of SEQUENCE ID NO:38, for example SEQUENCE ID NO: 119, or an anti-inflammatory variant of the fragment.

Preferably, the peptide comprises an anti-inflammatory fragment of the region of SEQUENCE ID NO:39, for example SEQUENCE ID NO: 120, 121 or 122, or an anti-inflammatory variant of the fragment.

Preferably, the peptide comprises an anti-inflammatory fragment of the region of SEQUENCE ID NO:40, for example SEQUENCE ID NO: 123, or an anti-inflammatory variant of the fragment.

Preferably, the peptide comprises an anti-inflammatory fragment of the region of SEQUENCE ID NO:41, for example SEQUENCE ID NO: 124, 125, 126, 127, 128 or 129, or an anti-inflammatory variant of the fragment.

Preferably, the peptide comprises a fragment of the region of SEQUENCE ID NO:42, for example SEQUENCE ID NO: 130, 131 or 132, or an anti-inflammatory variant of the fragment.

The invention also provides a composition comprising at least one anti-inflammatory peptides of the invention, which peptide includes an anti-inflammatory fragment of SEQUENCE ID NO: 10 or a homolog thereof. The invention also provides a composition comprising at least 2, preferably 3, preferably 4, preferably 5, preferably 6, preferably 7, preferably 8, preferably 9, or preferably 10 peptides of the invention, each of which comprises a different anti-inflammatory fragment of SEQUENCE ID NO: 10 or a homolog thereof. Preferably, the composition comprises a first peptide that comprises an anti-inflammatory fragment of a first region selected from SEQUENCE ID NO'S: 34 to 42, and a second peptide that comprises an anti-inflammatory fragment of a second region selected from SEQUENCE ID NO'S: 34 to 42. Preferably, the composition comprises a first anti-inflammatory peptide comprising a first anti-inflammatory fragment selected from SEQUENCE ID NO: 112 to 132 (or an anti-inflammatory variant of the fragment), and a second anti-inflammatory peptide comprising a second anti-inflammatory fragment selected from SEQUENCE ID NO 112 to 132 (or an anti-inflammatory variant of the fragment).

Homologs of Rice Protein 1 (SEQUENCE ID NO: 10) include Oryza brachyantha, and Zizania latifolia (SEQ ID NO: 249-251).

### [SEQUENCE ID NO: 11 (Rice Protein 2 - P07728)]

Preferably, the peptide comprises an anti-inflammatory fragment of the protein of SEQUENCE ID NO: 11 or a homolog thereof.

Preferably, the peptide comprises an anti-inflammatory fragment of one of four regions of SEQUENCE ID NO: 11, namely SEQUENCE ID NO'S: 43 to 46. Preferably, the region is SEQUENCE ID NO: 43, preferably SEQUENCE ID NO: 44, preferably SEQUENCE ID NO: 45, preferably SEQUENCE ID NO: 46.

Preferably, the peptide comprises an anti-inflammatory fragment of the region of SEQUENCE ID NO:43, for example SEQUENCE ID NO: 133 or an anti-inflammatory variant of the fragment.

Preferably, the peptide comprises an anti-inflammatory fragment of the region of SEQUENCE ID NO:44, for example SEQUENCE ID NO: 134 to 137, or an anti-inflammatory variant of the fragment.

Preferably, the peptide comprises an anti-inflammatory fragment of the region of SEQUENCE ID NO:45, for example SEQUENCE ID NO: 138 to 144, or an anti-inflammatory variant of the fragment.

Preferably, the peptide comprises an anti-inflammatory fragment of the region of SEQUENCE ID NO:46, for example SEQUENCE ID NO:145, or an anti-inflammatory variant of the fragment.

The invention also provides a composition comprising at least one anti-inflammatory peptides of the invention, which peptide includes an anti-inflammatory fragment of SEQUENCE ID NO:11 or a homolog thereof. The invention also provides a composition comprising at least 2, preferably 3, preferably 4, preferably 5, preferably 6, preferably 7, preferably 8, preferably 9, or preferably 10 peptides of the invention, each of which comprises an anti-inflammatory fragment of SEQUENCE ID NO: 11 or a homolog thereof. Preferably, the composition comprises a first peptide that comprises an anti-inflammatory fragment of a first region selected from SEQUENCE ID NO'S: 43 to 46, and a second peptide that comprises an anti-inflammatory fragment of a second region selected from SEQUENCE ID NO'S: 43 to 46. Preferably, the composition comprises a first anti-inflammatory peptide comprising a first anti-inflammatory fragment selected from SEQUENCE ID NO: 133 to 145 (or an anti-inflammatory variant of the fragment), and a second anti-inflammatory peptide comprising a second anti-inflammatory fragment selected from SEQUENCE ID NO 133 to 145 (or an anti-inflammatory variant of the fragment).

Homologs of Rice Protein 2 (SEQUENCE ID NO: 11) include Oryza sativa Indica Group, Zizania latifolia, Avena sativa (SEQ ID NO: 252-254).

### [SEQUENCE ID NO: 12 (Rice Protein 3 - P07730)]

Preferably, the peptide comprises an anti-inflammatory fragment of the protein of SEQUENCE ID NO: 12 or a homolog thereof.

Preferably, the peptide comprises an anti-inflammatory fragment of one of eight regions of SEQUENCE ID NO: 12, namely SEQUENCE ID NO'S: 47 to 54. Preferably, the region is SEQUENCE ID NO: 47, preferably SEQUENCE ID NO: 48, preferably SEQUENCE ID NO: 49, preferably SEQUENCE ID NO: 50, preferably SEQUENCE ID NO: 51, preferably SEQUENCE ID NO: 52, preferably SEQUENCE ID NO: 53, preferably SEQUENCE ID NO: 54.

Preferably, the peptide comprises an anti-inflammatory fragment of the region of SEQUENCE ID NO:47, for example one of SEQUENCE ID NO: 146 to 150, or an anti-inflammatory variant of the fragment.

Preferably, the peptide comprises an anti-inflammatory fragment of the region of SEQUENCE ID NO:48, for example one of SEQUENCE ID NO: 151 to 157, or an anti-inflammatory variant of the fragment.

Preferably, the peptide comprises an anti-inflammatory fragment of the region of SEQUENCE ID NO:49, for example one of SEQUENCE ID NO: 158 or SEQUENCE ID NO: 159, or an anti-inflammatory variant of the fragment.

Preferably, the peptide comprises an anti-inflammatory fragment of the region of SEQUENCE ID NO:50, for example one of SEQUENCE ID NO: 160 to 162, or an anti-inflammatory variant of the fragment.

Preferably, the peptide comprises an anti-inflammatory fragment of the region of SEQUENCE ID NO:51, for example one of SEQUENCE ID NO: 163 or 164, or an anti-inflammatory variant of the fragment.

Preferably, the peptide comprises an anti-inflammatory fragment of the region of SEQUENCE ID NO:52, for example SEQUENCE ID NO: 165, or an anti-inflammatory variant of the fragment.

Preferably, the peptide comprises an anti-inflammatory fragment of the region of SEQUENCE ID NO:53, for example one of SEQUENCE ID NO: 166 to 171, or an anti-inflammatory variant of the fragment.

Preferably, the peptide comprises an anti-inflammatory fragment of the region of SEQUENCE ID NO:54, for example one of SEQUENCE ID NO: 172 to 176, or an anti-inflammatory variant of the fragment.

The invention also provides a composition comprising at least one anti-inflammatory peptides of the invention, which peptide includes an anti-inflammatory fragment of SEQUENCE ID NO: 12 or a homolog thereof. The invention also provides a composition comprising at least 2, preferably 3, preferably 4, preferably 5, preferably 6, preferably 7, preferably 8, preferably 9, or preferably 10 peptides of the invention, each of which comprises an anti-inflammatory fragment of SEQUENCE ID NO: 12 or a homolog thereof. Preferably, the composition comprises a first peptide that comprises an anti-inflammatory fragment of a first region selected from SEQUENCE ID NO'S: 47 to 54, and a second peptide that comprises an anti-inflammatory fragment of a second region selected from SEQUENCE ID NO'S: 47 to 54. Preferably, the composition comprises a first anti-inflammatory peptide comprising a first anti-inflammatory fragment selected from SEQUENCE ID NO: 146 to 172 (or an anti-inflammatory variant of the fragment), and a second anti-inflammatory peptide comprising a second anti-inflammatory fragment selected from SEQUENCE ID NO 146 to 172 (or an anti-inflammatory variant of the fragment).

Homologs of Rice Protein 3 (SEQUENCE ID NO: 12) include Oryza brachyantha, Brachipodium distachyon (SEQ ID NO: 255-257).

### [SEQUENCE ID NO: 13 (Rice Protein 4 - Q0D7S0)]

Preferably, the peptide comprises an anti-inflammatory fragment of the protein of SEQUENCE ID NO: 13 or a homolog thereof.

Preferably, the peptide comprises an anti-inflammatory fragment of a region of SEQUENCE ID NO: 13, namely SEQUENCE ID NO 55, for example SEQUENCE ID NO: 177, or an anti-inflammatory variant of the fragment.

The invention also provides a composition comprising at least one anti-inflammatory peptides of the invention, which peptide includes an anti-inflammatory fragment of SEQUENCE ID NO: 13 or a homolog thereof. The invention also provides a composition comprising at least 2 peptides of the invention, at least one of which comprises an anti-inflammatory fragment of SEQUENCE ID NO: 13 or a homolog thereof, for example SEQUENCE ID NO: 177, or an anti-inflammatory variant of the fragment.

Homologs of Rice Protein 4 (SEQUENCE ID NO: 13) include Oryza sativa Indica Group, Zizania latifolia, Avena sativa (SEQ ID NO: 252-254).

### [SEQUENCE ID NO: 14 (Rice Protein 5 - P14614)]

Preferably, the peptide comprises a fragment of the protein of SEQUENCE ID NO: 14 or a homolog thereof.

Preferably, the peptide comprises an anti-inflammatory fragment of one of seven regions of SEQUENCE ID NO: 14, namely SEQUENCE ID NO'S: 56 to 62. Preferably, the region is SEQUENCE ID NO: 56, preferably SEQUENCE ID NO: 57, preferably SEQUENCE ID NO: 58, preferably SEQUENCE ID NO: 59, preferably SEQUENCE ID NO: 60, preferably SEQUENCE ID NO: 61, preferably SEQUENCE ID NO: 62.

Preferably, the peptide comprises an anti-inflammatory fragment of the region of SEQUENCE ID NO:56, for example one of SEQUENCE ID NO: 178 to 180 or an anti-inflammatory variant of the fragment.

Preferably, the peptide comprises an anti-inflammatory fragment of the region of SEQUENCE ID NO:57, for example one of SEQUENCE ID NO: 181 to 182, or an anti-inflammatory variant of the fragment.

Preferably, the peptide comprises an anti-inflammatory fragment of the region of SEQUENCE ID NO:58, for example SEQUENCE ID NO: 183, or an anti-inflammatory variant of the fragment.

Preferably, the peptide comprises an anti-inflammatory fragment of the region of SEQUENCE ID NO:59, for example one of SEQUENCE ID NO: 184 to 190, or an anti-inflammatory variant of the fragment.

Preferably, the peptide comprises an anti-inflammatory fragment of the region of SEQUENCE ID NO:60, for example SEQUENCE ID NO:191, or an anti-inflammatory variant of the fragment.

Preferably, the peptide comprises an anti-inflammatory fragment of the region of SEQUENCE ID NO:61, for example one of SEQUENCE ID NO: 192 to 195, or an anti-inflammatory variant of the fragment.

Preferably, the peptide comprises an anti-inflammatory fragment of the region of SEQUENCE ID NO:62, for example one of SEQUENCE ID NO: 196 to 197, or an anti-inflammatory variant of the fragment.

The invention also provides a composition comprising at least one anti-inflammatory peptides of the invention, which peptide includes an anti-inflammatory fragment of SEQUENCE ID NO: 14 or a homolog thereof. The invention also provides a composition comprising at least 2, preferably 3, preferably 4, preferably 5, preferably 6, preferably 7, preferably 8, preferably 9, or preferably 10 peptides of the invention, each of which comprises an anti-inflammatory fragment of SEQUENCE ID NO: 14 or a homolog thereof. Preferably, the composition comprises a first peptide that comprises an anti-inflammatory fragment of a first region selected from SEQUENCE ID NO'S: 56 to 62, and a second peptide that comprises an anti-inflammatory fragment of a second region selected from SEQUENCE ID NO'S: 56 to 62. Preferably, the composition comprises a first anti-inflammatory peptide comprising a first anti-inflammatory fragment selected from SEQUENCE ID NO: 178 to 197 (or an anti-inflammatory variant of the fragment), and a second anti-inflammatory peptide comprising a second anti-inflammatory fragment selected from SEQUENCE ID NO 178 to 197 (or an anti-inflammatory variant of the fragment).

Homologs of Rice Protein 5 (SEQUENCE ID NO: 14) include Oryza sativa Japonica Group, Brachipodium distachyon (SEQ ID NO: 261-263).

### [SEQUENCE ID NO: 15 (Rice Protein 6 - QODEV5)]

Preferably, the peptide comprises an anti-inflammatory fragment of the protein of SEQUENCE ID NO: 15 or a homolog thereof.

Preferably, the peptide comprises an anti-inflammatory fragment of one of seven regions of SEQUENCE ID NO: 15, namely SEQUENCE ID NO'S: 63 to 67. Preferably, the region is SEQUENCE ID NO: 63, preferably SEQUENCE ID NO: 64, preferably SEQUENCE ID NO: 65, preferably SEQUENCE ID NO: 66, preferably SEQUENCE ID NO: 67.

Preferably, the peptide comprises a fragment of the region of SEQUENCE ID NO:63, for example one of SEQUENCE ID NO: 198 to 200, or an anti-inflammatory variant of the fragment.

Preferably, the peptide comprises a fragment of the region of SEQUENCE ID NO:64, for example one of SEQUENCE ID NO: 201 to 203 or an anti-inflammatory variant of the fragment.

Preferably, the peptide comprises a fragment of the region of SEQUENCE ID NO:65, for example SEQUENCE ID NO: 204 or an anti-inflammatory variant of the fragment.

Preferably, the peptide comprises a fragment of the region of SEQUENCE ID NO:66, for example one of SEQUENCE ID NO: 205 to 208 or an anti-inflammatory variant of the fragment.

Preferably, the peptide comprises a fragment of the region of SEQUENCE ID NO:67, for example one of SEQUENCE ID NO:209 to 215 or an anti-inflammatory variant of the fragment.

Preferably, the peptide comprises a fragment of the region of SEQUENCE ID NO:68, for example one of SEQUENCE ID NO: 216 to 219, or an anti-inflammatory variant of the fragment.

Preferably, the peptide comprises a fragment of the region of SEQUENCE ID NO:69, for example SEQUENCE ID NO: 220, or an anti-inflammatory variant of the fragment.

The invention also provides a composition comprising at least one anti-inflammatory peptides of the invention, which peptide includes an anti-inflammatory fragment of SEQUENCE ID NO: 15 or a homolog thereof. The invention also provides a composition comprising at least 2, preferably 3, preferably 4, preferably 5, preferably 6, preferably 7, preferably 8, preferably 9, or preferably 10 peptides of the invention, each of which comprises an anti-inflammatory fragment of SEQUENCE ID NO: 15 or a homolog thereof. Preferably, the composition comprises a first peptide that comprises an anti-inflammatory fragment of a first region selected from SEQUENCE ID NO'S: 63 to 69, and a second peptide that comprises an anti-inflammatory fragment of a second region selected from SEQUENCE ID NO'S: 63 to 69. Preferably, the composition comprises a first anti-inflammatory peptide comprising a first anti-inflammatory fragment selected from SEQUENCE ID NO: 198 to 220 (or an anti-inflammatory variant of the fragment), and a second anti-inflammatory peptide comprising a second anti-inflammatory fragment selected from SEQUENCE ID NO 198 to 220 (or an anti-inflammatory variant of the fragment). Homologs of Rice Protein 6 (SEQUENCE ID NO: 15) include Oryza rufipogon, Oryza officinalis, Hordeum vulgare subsp. vulgare (SEQ ID NO: 264-266).

### [SEQUENCE ID NO: 16 (Bacterial Protein 1 - P0C1U8)]

Preferably, the peptide comprises an anti-inflammatory fragment of the protein of SEQUENCE ID NO: 16 or a homolog thereof.

Preferably, the peptide comprises an anti-inflammatory fragment of a region of SEQUENCE ID NO: 16, namely the region of SEQUENCE ID NO 70. Typically, the peptide is SEQUENCE ID NO 221, or an anti-inflammatory variant of the fragment.

The invention also provides a composition comprising at least one anti-inflammatory peptides of the invention, which peptide includes an anti-inflammatory fragment of SEQUENCE ID NO: 16 or a homolog thereof. The invention also provides a composition comprising at least 2 peptides of the invention, at least one of which comprises a fragment of SEQUENCE ID NO: 16 or a homolog thereof, for example SEQUENCE ID NO: 212 or an anti-inflammatory variant of the fragment.

Homologs of Bacterial Protein 1 (SEQUENCE ID NO: 16) include:
>gi|580560623|gb|EVF84961.1| glutamyl endopeptidase [Staphylococcus aureus COAS6020]
>gi|580687002|gb|EVH10169.1| glutamyl endopeptidase [Staphylococcus aureus UCIM6080]
>gi|751815683|gb|KIN24957.1| glutamyl endopeptidase [Staphylococcus aureus MRSA_CVM43477]
>gi|781884797|dbj|BAR08486.1| glutamyl endopeptidase precursor [Staphylococcus aureus subsp. aureus]
>gi|781887762|dbj|BAR11210.1| glutamyl endopeptidase precursor [Staphylococcus aureus subsp. aureus]

The invention also provides an anti-inflammatory composition comprising at least one and preferably a plurality of anti-inflammatory peptides of the invention, wherein each of the peptides of the invention comprises an anti-inflammatory fragment of a protein selected from SEQUENCE ID NO: 1 to 16, or an anti-inflammatory variant of the fragment. Typically, the or each peptide of the invention is selected from, or comprises an anti-inflammatory fragment selected from, SEQUENCE ID NO: 71-220, or an anti-inflammatory variant of the fragment. Typically, the or each peptide of the invention is selected from, or comprises an anti-inflammatory fragment selected from, SEQUENCE ID NO: 71-107 and 110-111, or an anti-inflammatory variant of the fragment.

Typically, the or each peptide of the invention is selected from, or comprises an anti-inflammatory fragment selected from, SEQUENCE ID NO: 108-109 and 112 to 220, or an anti-inflammatory variant of the fragment.

Preferably, the composition comprises at least two distinct peptides of the invention.

Preferably, the composition comprises at least three distinct peptides of the invention.

Preferably, the composition comprises at least four distinct peptides of the invention.

Preferably, the composition comprises at least five distinct peptides of the invention.

Preferably, the composition comprises at least six distinct peptides of the invention.

Preferably, the composition comprises at least seven distinct peptides of the invention.

Preferably, the composition comprises at least eight distinct peptides of the invention.

Preferably, the composition comprises at least nine distinct peptides of the invention.

Preferably, the composition comprises at least ten distinct peptides of the invention.

In one embodiment, the invention comprises a composition comprising substantially all of fragments SEQUENCE ID NO: 71-107 and 110-111, or anti-inflammatory variants of the fragments, or a mixture of the anti-inflammatory fragments and variants.

In one embodiment, the invention comprises a composition comprising substantially all of fragments SEQUENCE ID NO: 108-109 and 112 to 220, or anti-inflammatory variants of the fragments, or a mixture of the anti-inflammatory fragments and variants.

In one embodiment the composition is edible (comestible). In one embodiment, the composition is a food or beverage. In one embodiment, the composition is a personal care composition. In one embodiment, the composition is a pharmaceutical composition. In one embodiment, the composition is nutritional supplement. In one embodiment, the composition is solid. In one embodiment, the composition is semi-solid (i.e. a cream, gel or lotion). In one embodiment, the composition is liquid.

The invention also relates to a comestible product comprising a peptide of the invention. Preferably the comestible product is man-made.

The invention also relates to a comestible product comprising a composition of peptides of the invention. Preferably the comestible product is man-made.

Preferably, the comestible product is a food product for human or animal (mammalian) consumption.

In one embodiment the man-made comestible product is a beverage. In one embodiment the man-made comestible product is a bakery product. In one embodiment the man-made comestible product is a dairy product. In one embodiment the man-made comestible product is a snack product. In one embodiment the man-made comestible product is a baked extruded food product. In one embodiment the man-made comestible product is powdered milk. In one embodiment the man-made comestible product is an infant formula product. In one embodiment the man-made comestible product is a confectionary product. In one embodiment the man-made comestible product is a yoghurt. In one embodiment the man-made comestible product is a yoghurt drink. In one embodiment the man-made comestible product is an ice cream product. In one embodiment the man-made comestible product is a frozen food product. In one embodiment the man-made comestible product is a breakfast cereal. In one embodiment the man-made comestible product is a bread. In one embodiment the man-made comestible product is a flavoured milk drink. In one embodiment the man-made comestible product is a confectionary bar. In one embodiment the man-made comestible product is a tea or tea product. In one embodiment the man-made comestible product is a based extruded snack product. In one embodiment the man-made comestible product is a fried snack product. In one embodiment the man-made comestible product is a nutritional supplement. In one embodiment the man-made comestible product is a sports nutritional product. In one embodiment the man-made comestible product is a baby food product. In one embodiment the man-made comestible product is a speciality food product for immunocompromised individuals. In one embodiment the man-made comestible product is a food for geriatric patients.

The invention also relates to a man-made personal care composition comprising a peptide of the invention.

The invention also relates to a man-made personal care composition comprising a composition of peptides of the invention.

In one embodiment the personal care composition is a skincare product. In one embodiment the personal care composition is a haircare product. In one embodiment the personal care composition is a dentrifice product. In one embodiment the personal care composition is a perfumery product. In one embodiment the personal care composition is a deodorant product. In one embodiment the personal care composition is an anti-perspirant product. In one embodiment the personal care composition is a soap. In one embodiment the personal care composition is a liquid soap. In one embodiment the personal care composition is a cream. In one embodiment the personal care composition is a lotion. In one embodiment the personal care composition is a gel. In one embodiment the personal care composition is a powder.

The invention also relates to a peptide of the invention for use in treatment or prevention of inflammation in a mammal.

The invention also relates to a composition of peptides of the invention for use in treatment or prevention of inflammation in a mammal.

The invention also relates to a peptide of the invention for use in treatment or prevention of an inflammatory disorder in a mammal.

The invention also relates to a composition of peptides of the invention for use in treatment or prevention of an inflammatory disorder in a mammal.

In one embodiment the inflammation is symptomatic inflammation.

In one embodiment the inflammatory disorder is an inflammatory disorder of the joints. In one embodiment the inflammatory disorder is an inflammatory disorder of the cardiovascular system. In one embodiment the inflammatory disorder is an autoimmune disease. In one embodiment the inflammatory disorder is a lung and airway inflammatory disorder. In one embodiment the inflammatory disorder is an intestinal inflammatory disorder. In one embodiment the inflammatory disorder is dermatitis. In one embodiment the inflammatory disorder is acne vulgaris. In one embodiment the inflammatory disorder is psoriasis. In one embodiment the inflammatory disorder is rheumatoid arthritis. In one embodiment the inflammatory disorder is cardiovascular disease. In one embodiment the inflammatory disorder is atherosclerosis. In one embodiment the inflammatory disorder is Type I diabetes. In one embodiment the inflammatory disorder is Graves disease. In one embodiment the inflammatory disorder is Guillain-Barre disease. In one embodiment the inflammatory disorder is Lupus. In one embodiment the inflammatory disorder is Psoriatic arthritis. In one embodiment the inflammatory disorder is Ulcerative colitis. In one embodiment the inflammatory disorder is asthma. In one embodiment the inflammatory disorder is cystic fibrosis. In one embodiment the inflammatory disorder is COPD. In one embodiment the inflammatory disorder is emphysema. In one embodiment the inflammatory disorder is acute respiratory distress syndrome. In one embodiment the inflammatory disorder is colitis. In one embodiment the inflammatory disorder is inflammatory bowel disease.

The invention also relates to a peptide of the invention for use in treatment or prevention of pain in a mammal.

The invention also relates to a composition of peptides of the invention for use in treatment or prevention of pain in a mammal.

The invention also relates to a peptide of the invention for use in treatment or prevention of a metabolic disorder in a mammal.

The invention also relates to a composition of peptides of the invention for use in treatment or prevention of a metabolic disorder in a mammal.

In one embodiment, the metabolic disorder is pre-diabetes. In one embodiment, the metabolic disorder is diabetes. In one embodiment, the metabolic disorder is Type-1 diabetes. In one embodiment, the metabolic disorder is Type-2 diabetes. In one embodiment, the metabolic disorder is metabolic syndrome. In one embodiment, the metabolic disorder is obesity. In one embodiment, the metabolic disorder is diabetic dyslipidemia. In one embodiment, the metabolic disorder is hyperlipidemia. In one embodiment, the metabolic disorder is hypertension. In one embodiment, the metabolic disorder is hypertriglyceridemia. In one embodiment, the metabolic disorder is hyperfattyacidemia. In one embodiment, the metabolic disorder is hypercholerterolemia. In one embodiment, the metabolic disorder is hyperinsulinemia. In one embodiment, the metabolic disorder is MODY

The invention also relates to a peptide of the invention for use in maintaining or restoring gut health in a mammal.

The invention also relates to a composition of peptides of the invention for use in maintaining or restoring gut health in in a mammal.

The invention also relates to a peptide of the invention for use in maintaining or restoring muscle health (for example lean tissue mass) in a mammal.

The invention also relates to a composition of peptides of the invention for use in maintaining or restoring muscle health (for example lean tissue mass) in in a mammal.

The invention also relates to a pharmaceutical composition comprising a peptide of the invention in combination with a pharmaceutically acceptable carrier.

The invention also relates to a pharmaceutical composition comprising a composition of peptides of the invention in combination with a pharmaceutically acceptable carrier.

Such peptides can be used in personal care, supplement, food and pharmaceutical products to treat and maintain healthy levels of inflammation throughout the body. The present invention meets the huge need for food-derived specific peptides and peptide compositions that reduces inflammation in a way that is able to be processed by the body without completely blocking the immune response and causing autoimmune issues and other undesirable side effects. The invention is ultimately helping the 2 billion people suffering from inflammation.

### Definitions

The term "mammal" should be understood to mean a higher mammal, especially a human. However, the term also includes non-mammalian animals such as fish.

The term "peptide" used herein refers to a polymer composed of 5 to 50 amino acid monomers typically via peptide bond linkage. Peptides (including fragments and variants thereof) of and for use in the invention may be generated wholly or partly by chemical synthesis or by expression from nucleic acid. For example, the peptides of and for use in the present invention can be readily prepared according to well-established, standard liquid or, preferably, solid-phase peptide synthesis methods known in the art (see, for example, J. M. Stewart and J. D. Young, Solid Phase Peptide Synthesis, 2nd edition, Pierce Chemical Company, Rockford, Illinois (1984), in M. Bodanzsky and A. Bodanzsky, The Practice of Peptide Synthesis, Springer Verlag, New York (1984). When necessary, any of the peptides employed in the invention can be chemically modified to increase their stability. A chemically modified peptide or a peptide analog includes any functional chemical equivalent of the peptide characterized by its increased stability and/or efficacy in vivo or in vitro in respect of the practice of the invention. The term peptide analog also refers to any amino acid derivative of a peptide as described herein. A peptide analog can be produced by procedures that include, but are not limited to, modifications to side chains, incorporation of unnatural amino acids and/or their derivatives during peptide synthesis and the use of crosslinkers and other methods that impose conformational constraint on the peptides or their analogs. Examples of side chain modifications include modification of amino groups, such as by reductive alkylation by reaction with an aldehyde followed by reduction with NaBH₄; amidation with methylacetimidate; acetylation with acetic anhydride; carbamylation of amino groups with cyanate; trinitrobenzylation of amino groups with 2, 4, 6, trinitrobenzene sulfonic acid (TNBS); alkylation of amino groups with succinic anhydride and tetrahydrophthalic anhydride; and pyridoxylation of lysine with pyridoxa-5'-phosphate followed by reduction with NABH₄. The guanidino group of arginine residues may be modified by the formation of heterocyclic condensation products with reagents such as 2,3-butanedione, phenylglyoxal and glyoxal. The carboxyl group may be modified by carbodiimide activation via o-acylisourea formation followed by subsequent derivatization, for example, to a corresponding amide. Sulfhydryl groups may be modified by methods, such as carboxymethylation with iodoacetic acid or iodoacetamide; performic acid oxidation to cysteic acid; formation of mixed disulphides with other thiol compounds; reaction with maleimide; maleic anhydride or other substituted maleimide; formation of mercurial derivatives using 4-chloromercuribenzoate, 4-chloromercuriphenylsulfonic acid, phenylmercury chloride, 2-chloromercuric-4-nitrophenol and other mercurials; carbamylation with cyanate at alkaline pH. Tryptophan residues may be modified by, for example, oxidation with N-bromosuccinimide or alkylation of the indole ring with 2-hydroxy-5-nitrobenzyl bromide or sulphonyl halides. Tryosine residues may be altered by nitration with tetranitromethane to form a 3-nitrotyrosine derivative. Modification of the imidazole ring of a histidine residue may be accomplished by alkylation with iodoacetic acid derivatives or N-carbethoxylation with diethylpyrocarbonate. Examples of incorporating unnatural amino acids and derivatives during peptide synthesis include, but are not limited to, use of norleucine, 4-amino butyric acid, 4-amino-3-hydroxy-5-phenylpentanoic acid, 6-aminohexanoic acid, t-butylglycine, norvaline, phenylglycine, ornithine, sarcosine, 4-amino-3-hydroxy-6-methylheptanoic acid, 2-thienyl alanine and/or D-isomers of amino acids. Peptide structure modification includes the generation of retro-inverso peptides comprising the reversed sequence encoded by D-amino acids.

"Fragment" means a segment of a protein selected from SEQUENCE ID NO's: 1 to 16, and particularly from a region of those proteins selected SEQUENCE ID NO'S: 17 to 70, the fragment typically being 7 to 37 contiguous amino acids in length, and generally having a charge of between -9 and +3; a c-terminal amino acid that typcially is not cysteine (C) or methionine (M); and an n-terminal amino acid that typcially is not cysteine (C), histidine (H), proline (P) or threonine (T). The charge of a peptide, fragment or region is determined using the method of Cameselle, J.C., Ribeiro, J.M., and Sillero, A. (1986). Derivation and use of a formula to calculate the net charge of acid-base compounds. Its application to amino acids, proteins and nucleotides. Biochem. Educ. 14, 131-136.

The term "natural" as applied to an anti-inflammatory peptide means an anti-inflammatory peptide that includes (a) an anti-inflammatory fragment of a plant protein, typically rice or pea protein, or variants of pea protein including lentil, sweet pea, or chick pea or variants of rice protein including oat, grass, corn, wild rice and bananas, or (b) an anti-inflammatory variant of the fragment of a plant protein, for example an anti-inflammatory fragment of a homolog of the plant protein. The peptides or fragments of the invention may be isolated from plant protein hydrolysates or made synthetically.

"C-terminal domain" as applied to a fragment means the first three amino acids at the c-terminus of the fragment.

"N-terminal domain" as applied to a fragment means the last three amino acids at the n-terminus of the fragment.

"Anti-inflammatory" or "anti-inflammatory activity" as applied to a peptide or fragment means a peptide or fragment that is capable of significantly reducing the secretion of TNFα by LPS-stimulated J774.2 macrophages (compared with untreated LPS-stimulated J774.2 macrophages) when the macrophages are treated with 100µM of the peptide or fragment as described in the experimental section below.

"Homolog" of a reference protein should be understood to mean a protein from a different species of plant having at least 60% sequence homology with the reference protein. Thus, for example, homologs of pea protein P13918 include:
>gi|137584|sp|P08438.1|VCL_VICFARecName:Full=Vicilin;Flags: Precursor [Vicia faba]
>gi|22057|emb|CAA68559.l| vicilin [Vicia faba var. minor]>gi|383931031|gb|AFH56916.1| vicilin **[Vicia faba]**
>gi|502105333|ref|XP_004492829.1| PREDICTED: vicilin-like isoform X1 **[Cicer arietinum] ChickPea**
>gi|29539109|emb|CAD87730.1| allergen Len c 1.0101 **[Lens culinaris] Lentil**

A "variant" of an anti-inflammatory fragment shall be taken to mean a fragment having an amino acid sequence that is substantially identical to the anti-inflammatory fragment, and which has anti-inflammatory activity as defined above. Thus, for example, the term should be taken to include fragments that are altered in respect of one or more amino acid residues. Preferably such alterations involve the insertion, addition, deletion and/or substitution of 5 or fewer amino acids, more preferably of 4 or fewer, even more preferably of 3 or fewer, most preferably of 1 or 2 amino acids only. Insertion, addition and substitution with natural and modified amino acids is envisaged. The variant may have conservative amino acid changes, wherein the amino acid being introduced is similar structurally, chemically, or functionally to that being substituted. Generally, the variant will have at least 70% amino acid sequence homology, preferably at least 80% sequence homology, more preferably at least 90% sequence homology, and ideally at least 95%, 96%, 97%, 98% or 99% sequence homology with the parent anti-inflammatory fragment. In this specification, the term "sequence homology" should be understand to comprise both sequence identity and similarity, i.e. a variant (or homolog) that shares 70% sequence homology with a reference sequence is one in which any 70% of aligned residues of the variant (or homolog) are either identical to, or conservative substitutions of, the corresponding residues in the reference sequence.

The invention also relates to a comestible product, for example a food product comprising a composition of the invention, for example a dairy or non-dairy product, a solid food or a beverage, a food additive or supplement. The dairy product may be a milk, a cheese, or yoghurt. In one embodiment, the food product is a snack bar. The food product may comprise any amount of the composition of the invention, for example from 0.1% to 30% (w/w).

The food product may be a Food for Specific Medicinal Purposes (FSMP) which is defined as foods that are specifically formulated, processed and intended for the dietary management of diseases, disorders or medical conditions of individuals who are being treated under medical supervision. These foods are intended for the exclusive or partial feeding of people whose nutritional requirements cannot be met by normal foods.

"Inflammatory disorder" means an immune-mediated inflammatory condition that affects humans and is generally characterised by dysregulated expression of one or more cytokines. Examples of inflammatory disorders include skin inflammatory disorders, inflammatory disorders of the joints, inflammatory disorders of the cardiovascular system, certain autoimmune diseases, lung and airway inflammatory disorders, intestinal inflammatory disorders. Examples of skin inflammatory disorders include dermatitis, for example atopic dermatitis and contact dermatitis, acne vulgaris, and psoriasis. Examples of inflammatory disorders of the joints include rheumatoid arthritis. Examples of inflammatory disorders of the cardiovascular system are cardiovascular disease and atherosclerosis. Examples of autoimmune diseases include Type 1 diabetes, Graves disease, Guillain-Barre disease, Lupus, Psoriatic arthritis, and Ulcerative colitis. Examples of lung and airway inflammatory disorders include asthma, cystic fibrosis, COPD, emphysema, and acute respiratory distress syndrome. Examples of intestinal inflammatory disorders include colitis and inflammatory bowel disease. Other inflammatory disorders include cancer, hay fever, periodontitis, allergies, hypersensitivity, ischemia, depression, systemic diseases, post infection inflammation and bronchitis.

The peptides and compositions of the invention may also be employed in the non-therapeutic treatment of inflammation. Examples of non-therapeutic treatment of inflammation include use to relieve normal, non-pathological, inflammation, for example inflammation in the muscles and joints following exercise.

In this specification, the term "Metabolic disorder" should be understood to include pre-diabetes, diabetes; Type-1 diabetes; Type-2 diabetes; metabolic syndrome; obesity; diabetic dyslipidemia; hyperlipidemia; hypertension; hypertriglyceridemia; hyperfattyacidemia; hypercholerterolemia; hyperinsulinemia, and MODY.

A further aspect of the invention relates to a pharmaceutical composition comprising a peptide of the invention or a composition of peptides of the invention, admixed with one or more pharmaceutically acceptable diluents, excipients or carriers. Even though the peptides and compositions of the present invention can be administered alone, they will generally be administered in admixture with a pharmaceutical carrier, excipient or diluent, particularly for human therapy. The pharmaceutical compositions may be for human or animal usage in human and veterinary medicine. Examples of such suitable excipients for the various different forms of pharmaceutical compositions described herein may be found in the "Handbook of Pharmaceutical Excipients, 2nd Edition, (1994), Edited by A Wade and PJ Weller. In particular, formulations for topical delivery are described in Topical drug delivery formulations edited by David Osborne and Antonio Aman, Taylor & Francis, the complete contents of which are incorporated herein by reference. Acceptable carriers or diluents for therapeutic use are well known in the pharmaceutical art, and are described, for example, in Remington's Pharmaceutical Sciences, Mack Publishing Co. (A. R. Gennaro edit. 1985). Examples of suitable carriers include lactose, starch, glucose, methyl cellulose, magnesium stearate, mannitol, sorbitol and the like. Examples of suitable diluents include ethanol, glycerol and water. The choice of pharmaceutical carrier, excipient or diluent can be selected with regard to the intended route of administration and standard pharmaceutical practice. The pharmaceutical compositions may comprise as, or in addition to, the carrier, excipient or diluent any suitable binder(s), lubricant(s), suspending agent(s), coating agent(s), solubilising agent(s). Examples of suitable binders include starch, gelatin, natural sugars such as glucose, anhydrous lactose, free-flow lactose, beta-lactose, corn sweeteners, natural and synthetic gums, such as acacia, tragacanth or sodium alginate, carboxymethyl cellulose and polyethylene glycol. Examples of suitable lubricants include sodium oleate, sodium stearate, magnesium stearate, sodium benzoate, sodium acetate, sodium chloride and the like. Preservatives, stabilizers, dyes and even flavouring agents may be provided in the pharmaceutical composition. Examples of preservatives include sodium benzoate, sorbic acid and esters of p-hydroxybenzoic acid. Antioxidants and suspending agents may be also used.

The peptide or composition of the invention may be adapted for topical, oral, rectal, parenteral, intramuscular, intraperitoneal, intra-arterial, intrabronchial, subcutaneous, intradermal, intravenous, nasal, vaginal, buccal or sublingual routes of administration. For oral administration, particular use is made of compressed tablets, pills, tablets, gellules, drops, and capsules. Preferably, these compositions contain from 1 to 250 mg and more preferably from 10-100 mg, of active ingredient per dose. Other forms of administration comprise solutions or emulsions which may be injected intravenously, intra-arterial, subcutaneously, intradermally, intraperitoneally or intramuscularly, and which are prepared from sterile or sterilisable solutions. The pharmaceutical compositions of the present invention may also be in form of suppositories, vaginal rings, pessaries, suspensions, emulsions, lotions, ointments, creams, gels, sprays, solutions or dusting powders. The composition of the invention may be formulated for topical delivery. Topical delivery generally means delivery to the skin, but can also mean delivery to a body lumen lined with epithelial cells, for example the lungs or airways, the gastrointestinal tract, the buccal cavity. In particular, formulations for topical delivery are described in Topical drug delivery formulations edited by David Osborne and Antonio Aman, Taylor & Francis, the complete contents of which are incorporated herein by reference. Compositions or formulations for delivery to the airways are described in O'Riordan et al (Respir Care, 2002, Nov. 47), EP2050437, WO2005023290, US2010098660, and US20070053845. Composition and formulations for delivering active agents to the iluem, especially the proximal iluem, include microparticles and microencapsulates where the active agent is encapsulated within a protecting matrix formed of polymer or dairy protein that is acid resistant but prone to dissolution in the more alkaline environment of the ileum. Examples of such delivery systems are described in EP1072600.2 and EP13171757.1. An alternative means of transdermal administration is by use of a skin patch. For example, the active ingredient can be incorporated into a cream consisting of an aqueous emulsion of polyethylene glycols or liquid paraffin. The active ingredient can also be incorporated, at a concentration of between 1 and 10% by weight, into an ointment consisting of a white wax or white soft paraffin base together with such stabilisers and preservatives as may be required.
Injectable forms may contain between 10-1000 mg, preferably between 10-250 mg, of active ingredient per dose.

Compositions may be formulated in unit dosage form, i.e., in the form of discrete portions containing a unit dose, or a multiple or sub-unit of a unit dose.

A person of ordinary skill in the art can easily determine an appropriate dose of one of the instant compositions to administer to a subject without undue experimentation. Typically, a physician will determine the actual dosage which will be most suitable for an individual patient and it will depend on a variety of factors including the activity of the specific compound employed, the metabolic stability and length of action of that compound, the age, body weight, general health, sex, diet, mode and time of administration, rate of excretion, drug combination, the severity of the particular condition, and the individual undergoing therapy. The dosages disclosed herein are exemplary of the average case. There can of course be individual instances where higher or lower dosage ranges are merited, and such are within the scope of this invention. Depending upon the need, the agent may be administered at a dose of from 0.01 to 30 mg/kg body weight, such as from 0.1 to 10 mg/kg, more preferably from 0.1 to 1 mg/kg body weight. In an exemplary embodiment, one or more doses of 10 to 300 mg/day or more preferably, 10 to 150 mg/day, will be administered to the patient for the treatment of an inflammatory disorder.

In a particularly preferred embodiment, the methods and uses of the invention involve administration of a peptide or composition of the invention in combination with one or more other active agents, for example, existing anti-inflammatory drugs or pharmacological enhancers available on the market. In such cases, the compounds of the invention may be administered consecutively, simultaneously or sequentially with the one or more other active agents.

"Man-made" as applied to comestible products should be understood to mean made by a human being and not existing in nature.

"Maintaining or restoring gut health": means reducing and/or regulating the pro-inflammatory response in the gut and more specifically the epithelial cells. The healthy microbiome offers some protection against pathogenic viruses and bacteria, and their presence is needed to guide the development of our immune system. It has been shown that these bacteria can react to human signals of stress, sickness, or age which can be manifested by inflammation and as a consequence switch on their virulence genes and cause or contribute to disease. Having the ability to reduce and maintain at healthy levels the inflammatory response can help maintain the healthy bacteria. Digestive problems, which comprise the number one health problem in North America, appear to be occurring with more frequency in recent years. One way to maintain digestive health is to maintain proper inflammation and intestinal flora.

"Maintaining or restoring muscle health" means helping retain or restore mammalian muscle health resulting from damage incurred during exercise. By lowering inflammation the peptides promote recovery from injuries during exercise, and relieve muscle soreness/pain and injury connected with exercise. They can also be used to decrease and prevent muscle cramping, and to allow a faster recovery from muscle cramping. Cramping can result from physical stress, mental stress, and or Repetitive Strain Injury stress. By lowering inflammation the peptides help reduce Myopathy of the muscle, and help prevent Sarcopenia in mammals, promote recovery from injuries during exercise, and relieve muscle soreness/pain and injury connected with exercise. They can also be used to decrease and prevent muscle cramping, and to allow a faster recovery from muscle cramping. Cramping can result from physical stress, mental stress, and or Repetitive Strain Injury stress. By lowering inflammation the peptides help reduce Myopathy of the muscle, and help prevent Sarcopenia in mammals.

In this specification, the term "composition" should be understood to mean something made by the hand of man, and not excludes naturally occurring compositions. Exemplary compositions include foods, beverages, nutritional supplements, personal care compositions, and pharmaceutical compositions.

In this specification, the term "substantially all" as applied to a list of peptides or fragments should be understood to mean at least 60%, 70%, 80%, 90% or 95% of the peptides or fragments.

In this specification, the term "personal care composition" should be understood to mean a composition formulated for use by humans in cleaning or treating the human body, particularly the skin, teeth, nails, feet and hair. Examples include shampoo, conditioner, skin creams and lotions, powders, dentrifice, shower gel or creams, body lotion, deodorant, and anti-perspirant.

In this specification, the term "nutritional supplement" should be understood to mean a product formulated for ingestion by a mammal and intended to confer a health benefit on the recipient. The supplement can take any form, for example a solid, liquid, or powder. Examples of supplements include powders, tablets, capsules, and drinks.

In the specification the terms "comprise, comprises, comprised and comprising" or any variation thereof and the terms "include, includes, included and including" or any variation thereof are considered to be totally interchangeable and they should all be afforded the widest possible interpretation and vice versa.

### Brief Description of the Figures

**Figure 1**. *Viability of J774.2 macrophages after treatment with synthetic peptides.* J774.2 macrophages were treated 100µM of synthetic peptide for 24 hours before an alamar blue assay was performed. Data are presented as an average of n=3 +/- SEM.
**Figure 2****.** *The effects of hydrolysates on cell survival.* J774.2 macrophages were treated with (A) 1 mg/ml or (B) 0.5mg/ml of hydrolysates for 24 hours before an alamar blue assay was performed. Data is shown as (A) n=1 +/- SEM and (B) n=3 +/- SEM.
**Figure 3****.** *The effect of DMSO vehicle on TNFα and IL-1β secretion from J774.2 macrophages.* J774.2 macrophages were treated with a final concentration of 0.3% and 1% DMSO (equivalent to the amounts used to dissolve the peptides) for 24 hours and the effect on TNFα and IL-1β after stimulation was established. Data are presented as an average of n=3 +/- SEM. (***p<0.001 w.r.t LPS).
**Figure 4****.** *The effect on synthetic peptides 1-6 on TNFα and IL-1β secretion from J774.2 macrophages*. J774.2 macrophages were treated with 100µM of synthetic peptide for 24 hours and then stimulated with (A) LPS (10ng/ml) for five hours or (B) LPS (10ng/ml) for 5 hours followed by ATP (5mM) for one hour. Supernatant was collected and levels of (A) TNFα and (B) IL-1β were determined by ELISA. (***p<0.001 w.r.t LPS, **p<0.01 w.r.t LPS, *p<0.05, ^{###}p<0.001 w.r.t. LPS/ATP, ^{##}p<0.01 w.r.t LPS/ATP and ^{#}p<0.05 w.r.t LPS/ATP). Final concentration of DMSO in well: SP1 - 0.3%, SP2 - 0%, SP3 - 0.3%, SP4-1%, SP5 - 1%, SP6 - 0.3%, Positive Control - 0%. Data are presented as an average of n=3 +/- SEM.

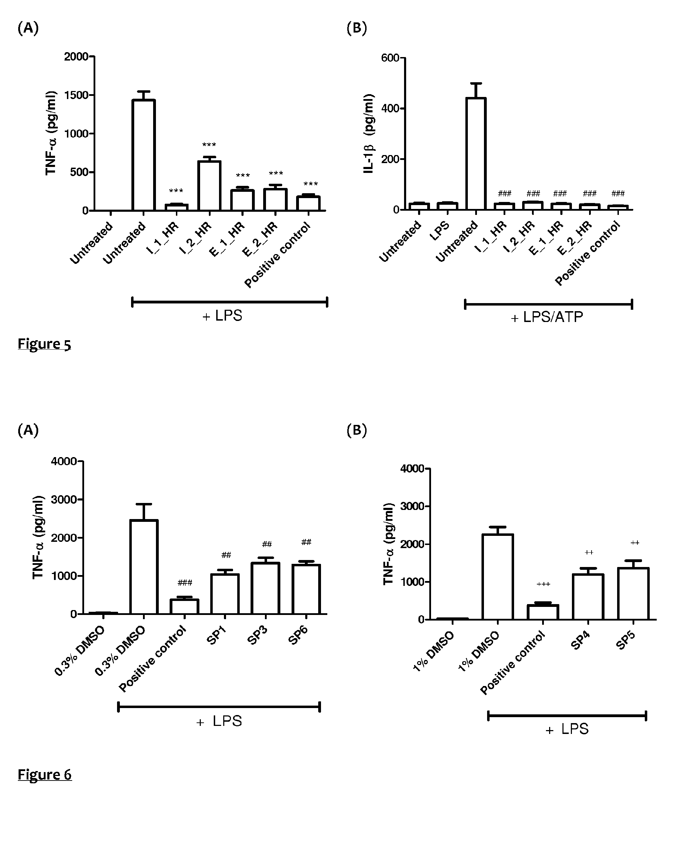

**Figure 5****.** *The effect on hydrolysates on TNFα and IL-1β secretion.* J774.2 macrophages were treated with 0.5mg/ml of hydrolysate for 24 hours and then stimulated with (A) LPS (10ng/ml) for five hours or (B) LPS (10ng/ml) for 5 hours followed by ATP (5mM) for one hour. Supernatant was collected and levels of (A) TNFα and (B) IL-1β were determined by ELISA. (***p<0.001 w.r.t untreated+LPS, ^{###}p<0.001 w.r.t. untreated+LPS/ATP). Data are presented as an average of n=3 +/- SEM.
**Figure 6****.** *The effects of synthetic peptides with DMSO vehicle on TNFα* J774.2 macrophages were treated with 100µM of synthetic peptide for 24 hours and then LPS (10ng/ml) for five hours. Supernatant was collected and levels of TNFα were determined by ELISA. ^{###}p<0.001 w.r.t 0.3% DMSO+LPS, ^{##}p<0.01 w.r.t. 0.3% DMSO+LPS,+++p<0.001 w.r.t 1% DMSO+LPS, ++p<0.01 w.r.t 1% DMSO+LPS/ATP). Final concentration of DMSO in well: positive control - 0% , SP1 - 0.3%, SP2 - 0%, SP3 - 0.3%, SP4 - 1%, SP5 - 1%, SP6-0.3%. Data are pre

### Detailed Description of the Invention

### Study Description

The effect of six synthetic peptides of the invention, SP1 to SP6 (SEQUENCE ID NO: 108, 109, 110, 111, 85 and 91) and four peptide compositions on the inflammatory response *in vitro* using a cell line was determined.
Peptide composition I_1_HR (Rice) contained the followings peptides (identified by SEQ ID): 116, 197, 207, 112, 211, 158, 201, 203, 114, 183, 130, 113, 182, 167, 166, 152, 220, 213, 215, 154, 219, 218, 165, 123, 185, 190, 209, 181, 198, 200, 147, 172, 184, 124, 153, 205, 115, 196, 151, 161, 160, 216, 210, 208, 146, 133, 204, 212, 206.
Peptide composition I_2_HR (Rice) contained the followings peptides (identified by SEQ ID): 189, 177, 174, 129, 176, 202, 193, 195, 194, 192, 182, 128, 220, 127, 134, 136, 135, 180, 179, 178, 219, 218, 145, 120, 175, 190, 149, 126, 187, 191, 121, 122, 159, 132, 162, 137, 150, 186, 188, 164, 118, 125, 163, 157, 156, 117.
Peptide composition E_1_HR (Pea) contained the followings peptides (identified by SEQ ID):74, 76, 106, 102, 101, 100, 92, 96, 83, 89, 90, 104, 82, 75, 79, 78, 77, 99, 103, 72, 86, 105, 94, 93, 81, 97, 80, 88, 85, 87, 71, 107, 73, 84, 98, 95.
Peptide composition E_2_HR contained homologs of the peptides of the invention.

A J774.2 mouse macrophage cell line was treated with 100µM of each synthetic peptide (SP) and 0.5 mg/ml of each peptide composition and the effect on two pro-inflammatory markers - tumour necrosis factor α (TNFα) and interleukin-1β (IL-1β) was determined after inflammation was induced using lipopolysaccharide (LPS) as an inflammatory stimulus. A one way anova was used with the dunnett test which is a multiple comparison and compares every mean with a single control mean.

### Synthetic Peptides: Cell viability

Synthetic peptides were first diluted in a suitable solvent. Dimethyl sulfoxide (DMSO) was the solvent of choice for peptides with poor predicted water solubility. Final concentration of DMSO in each well: SP1 (1_155_HR) - 0.3%, SP2 (1_374_HR) - 0%, SP3 (E_155_HR) - 0.3%, SP4 (E_54_HR) - 1%, SP5 (E_41_HR) - 1%, SP6 (E_788_HR) - 0.3%, positive Control - 0%. Cells were first treated with 100µM of each SP for 24 hours before an alamar blue assay was performed. No viability issues were seen with any of the peptides (Fig. 1).

### Peptide compositions: Preparation and Toxicity

The peptide compositions were prepared by adjusting the pH to between 6-7 and sterile filtering. The effects of the peptide compositions on cell viability was determined. J774.2 macrophages were treated with lmg/ml and 0.5mg/ml of each peptide composition, hydrogen peroxide to induce cell death as a positive control, and a peptide known to be non-toxic as a negative control. An alamar blue assay was then performed and cell survival is shown in Fig. 2 as a percentage of untreated (100%). As cell survival was compromised with lmg/ml of peptide, 0.5mg/ml of hydrolysate was used for further assays.

### Inflammatory Markers

The effect of the DMSO on TNFα and IL-1β secretion was determined (Fig. 3A and 3B). 1% DMSO significantly increased levels of TNFα (Fig. 3A. ***p<0.001 w.r.t LPS) and this was taken into account when analysing the effect of the peptides on TNFα. No significant effect was seen with regards DMSO and IL-1β secretion (Fig. 3B).

### Results: Synthetic Peptides

The effect of the synthetic peptides on TNFα and IL-1β secretion following LPS stimulation was then investigated. J774.2 macrophages were treated with 100 µM of each synthetic peptide for 24 hours before treatment with 10ng/ml of LPS for 5 hours to stimulate TNFα secretion (Fig. 4A) and 10ng/ml of LPS for 5 hours followed by 1 hour of 5mM ATP to stimulate IL-1β secretion (Fig. 4B).
The positive control reduced both TNFα (***p<0.001 w.r.t LPS - Fig.4A) and IL-1β (^{###} p<0.0001 w.r.t LPS/ATP - Fig.4B) significantly. SP2 reduced both TNFα (***p<0.001 w.r.t LPS - Fig4A) and IL-1β (^{###} p<0.0001 w.r.t LPS/ATP - Fig.4B) significantly. SP6 also reduced TNFα (*p<0.05 w.r.t LPS - Fig.4A) and IL-1β (^{#}p<0.05 w.r.t. LPS/ATP - Fig.4B) significantly. SP4 reduced TNFα (*p<0.05 w.r.t LPS - Fig.4A) significantly, but no significant reduction in IL-1β levels was seen.

These results demonstrate that SP2 has the most potent anti-inflammatory properties out of the six synthetic peptides tested.

### Results: Peptide Compositions

The effect of the peptide compositions of the invention on TNFα and IL-1β secretion was then determined (Fig. 5). A peptide known to reduce both TNFα and IL-1β secretion was used as a positive control to ensure the assay was working and to allow comparison between assays. J774.2 macrophages were treated with 0.5mg/ml of hydrolysates for 24 hours before inflammation was induced using LPS. I_1_HR, I_2_HR, E_1_HR and E_2_HR all reduced both TNFα and IL-1β significantly (***p<0.001 w.r.t untreated+LPS, ^{###}p<0.001 w.r.t. untreated+LPS/ATP).

### Potency relative to the vehicle control (synthetic peptides)

Importantly, as shown in figure 2, the vehicle control DMSO (0.3% and 1 % final concentrations) did not reduce either TNFα or IL-1β secretion. However, there was a trend towards increased TNFα secretion at 0.3% DMSO and a significant increase with 1% DMSO after 5 hour LPS stimulation (***p<0.001 w.r.t LPS). Therefore, in order to ensure that peptide activity was not being masked by increased levels of TNFα secretion due to the DMSO vehicle, we compared peptides to their corresponding vehicle control and in each case different significance levels were seen. SP1, SP3 and SP6 all reduced TNFα compared to the vehicle control (^{##}p<0.01 w.r.t. 0.3% DMSO+LPS - Fig.6A). SP4 and SP5 both reduced TNFα with respect to the vehicle control (⁺⁺p<0.01 w.r.t LPS - Fig. 6B).

The invention is not limited to the embodiments hereinbefore described which may be varied in construction and detail without departing from the spirit of the invention.

### SEQUENCE INFORMATION

PEA PROTEIN 1 : P13918 - 1 –
MAATTMKASFPLLMLMGISFLASVCVSSRSDPQNPFIFKSNKFQTLFENENGHIRLLQKFDQRSKIFENLQNYRLLEYKSKPHTIFLPQHTDA
DYILVVLSGKAILTVLKPDDRNSFNLERGDTIKLPAGTIAYLVNRDDNEELRVLDLAIPVNRPGQLQSFLLSGNQNQQNYLSGFSKNILEASFN
TDYEEIEKVLLEEHEKETQHRRSLKDKRQQSQEENVIVKLSRGQIEELSKNAKSTSKKSVSSESEPFNLRSRGPIYSNEFGKFFEITPEKNPQLQ
DLDIFVNSVEIKEGSLLLPHYNSRAIVIVTVNEGKGDFELVGQRNENQQEQRKEDDEEEEQGEEEINKQVQNYKAKLSSGDVFVIPAGHPVA
VKASSNLDLLGFGINAENNQRNFLAGDEDNVISQIQRPVKELAFPGSAQEVDRILENQKQSHFADAQPQQRERGSRETRDRLSSV [SEQ
ID 1]

| | |
|---|---|
| REGION : | 253 to 274 - PFNLRSRGPIYSNEFGKFFEIT [SEQ ID 17] |
| | PEPTIDE : SRGPIYSNEFGK [SEQ ID 71] |
| REGION : | 110 to 126 - KPDDRNSFNLERGDTIK [SEQ ID 18] |
| | PEPTIDE : NSFNLER [SEQ ID 72] |
| REGION : | 134 to 160 - YLVNRDDNEELRVLDLAIPVNRPGQLQ [SEQ ID 19] |
| | PEPTIDE : VLDLAIPVNR [SEQ ID 73] |
| | PEPTIDE : DDNEELR [SEQ ID 74] |
| REGION : | 331 to 381 - QEQRKEDDEEEEQGEEEINKQVQNYKAKLSSGDVFVIPAGHPVAVKASSNL [SEQ ID 20] |
| | PEPTIDE : LSSGDVFVIPAGHPVAVK [SEQ ID 75] |
| | PEPTIDE : EDDEEEEQGEEEINK [SEQ ID 76] |
| REGION : | 175 to 242 - SGFSKNILEASFNTDYEEIEKVLLEEHEKETQHRRSLKDKRQQSQEENVIVKLSRGQIEELSKNAKST [SEQ ID 21] |
| | PEPTIDE : NILEASFNTDYEEIEKVLLEEHEKETQHR [SEQ ID 77] |
| | PEPTIDE : NILEASFNTDYEEIEKVLLEEHEK [SEQ ID 78] |
| | PEPTIDE : NILEASFNTDYEEIEK [SEQ ID 79] |
| | PEPTIDE : RQQSQEENVIVK [SEQ ID 80] |
| | PEPTIDE : QQSQEENVIVK [SEQ ID 81] |
| | PEPTIDE : LSRGQIEELSK [SEQ ID 82] |
| | PEPTIDE : GQIEELSK [SEQ ID 83] |
| | PEPTIDE : VLLEEHEK [SEQ ID 84] |
| REGION : | 35 to 108 - PFIFKSNKFQTLFENENGHIRLLQKFDQRSKIFENLQNYRLLEYKSKPHTIFLPQHTDADYILVVLSGKAILTV [SEQ ID 22] |
| | PEPTIDE : SKPHTIFLPQHTDADYILVVLSGK [SEQ ID 85] |
| | PEPTIDE : PHTIFLPQHTDADYILVVLSGK [SEQ ID 86] |
| | PEPTIDE : SNKFQTLFENENGHIR [SEQ ID 87] |
| | PEPTIDE : SKIFENLQNYR [SEQ ID 88] |
| | PEPTIDE : IFENLQNYR [SEQ ID 89] |
| REGION : | 423 to 450 - QEVDRILENQKQSHFADAQPQQRERGSR [SEQ ID 23] |
| | PEPTIDE : ILENQKQSHFADAQPQQR [SEQ ID 90] |
| | |
| | PEPTIDE PGQLQSFLLSGNQNQQNYLSGFSK [SEQ ID 91] |

PEA PROTEIN 2: P02856 - 2 –
DRRQELSNENVLVKVSRRQLEELSKNAKSSSRRSVSSESGPFNLRSEDPLYSNNSGKFFELTPEKNQQLQDLDLFVNSVDLKEGSLLLPNYNS
RALLVLVLVVNEGKGDFELVGQRNENQGKEN [SEQ ID 2]

| | |
|---|---|
| REGION : | 53 to 87 - NNSGKFFELTPEKNQQLQDLDLFVNSVDLKEGSLL [SEQ ID 24] |
| | PEPTIDE : FFELTPEKNQQLQDLDLFVNSVDLK [SEQ ID 92] |
| REGION : | 14 to 30 - KVSRRQLEELSKNAKSS [SEQ ID 25] |
| | PEPTIDE : QLEELSK [SEQ ID 93] |

PEA PROTEIN 3 : P15838 - 3 –
MATKLLALSLSFCFLLLGGCFALREQPEQNECQLERLNALEPDNRIESEGGLIETWNPNNKQFRCAGVALSRATLQHNALRRPYYSNAPQEI
FIQQGNGYFGMVFPGCPETFEEPQESEQGEGRRYRDRHQKVNRFREGDIIAVPTGIVFWMYNDQDTPVIAVSLTDIRSSNNQLDQMPRR
FYLAGNHEQEFLRYQHQQGGKQEQENEGNNIFSGFKRDFLEDAFNVNRHIVDRLQGRNEDEEKGAIVKVKGGLSIISPPEKQARHQRGSR
QEEDEDEDEERQPRHQRGSRQEEEEDEDEERQPRHQRRRGEEEEEDKKERRGSQKGKSRRQGDNGLEETVCTAKLRLNIGPSSSPDIYNPE
AGRIKTVTSLDLPVLRWLKLSAEHGSLHKNAMFVPHYNLNANSIIYALKGRARLQVVNCNGNTVFDGELEAGRALTVPQNYAVAAKSLSD
RFSYVAFKTNDRAGIARLAGTSSVINNLPLDVVAATFNLQRNEARQLKSNNPFKFLVPARQSENRASA [SEQ ID 3]

| | |
|---|---|
| REGION : | 267 to 287 - QRGSRQEEDEDEDEERQPRHQ [SEQ ID 26] |
| | PEPTIDE : QEEDEDEDEER [SEQ ID 94] |
| REGION : | 190 to 222 - QEFLRYQHQQGGKQEQENEGNNIFSGFKRDFLE [SEQ ID 27] |
| | PEPTIDE : YQHQQGGKQEQENEGNNIFSGFK [SEQ ID 95] |

| | |
|---|---|
| PEA PROTEIN 4 : Q9M3X6 - 4 – MATTIKSRFPLLLLLGIIFLASVVCVTYANYDEGSEPRVPAQRERGRQEGEKEEKRHGEWRPSYEKEEDEEEGQRERGRQEGEKEEKRHGE WRPSYEKQEDEEEKQKYRYQREKEDEEEKQKYQYQREKKEQKEVQPGRERWEREEDEEQVDEEWRGSQRREDPEERARLRHREERTKR DRRHQREGEEEEERSSESQERRNPFLFKSNKFLTLFENENGHIRLLQRFDKRSDLFENLQNYRLVEYRAKPHTIFLPQHIDADLILVVLSGKAILT VLSPNDRNSYNLERGDTIKLPAGTTSYLVNQDDEEDLRLVDLVIPVNGPGKFEAFDLAKNKNQYLRGFSKNILEASYNTRYETIEKVLLEEQE KDRKRRQQGEETDAIVKVSREQIEELKKLAKSSSKKSLPSEFEPINLRSHKPEYSNKFGKLFEITPEKKYPQLQDLDLFVSCVEINEGALMLPHY NSRAIVVLLVNEGKGNLELLGLKNEQQEREDRKERNNEVQRYEARLSPGDVVIIPAGHPVAITASSNLNLLGFGINAENNERNFLSGSDDNV ISQIENPVKELTFPGSVQEINRLIKNQKQSHFANAEPEQKEQGSQGKRSPLSSILGTFY [SEQ ID 4] | |
| REGION : | 284 to 317 - YNLERGDTIKLPAGTTSYLVNQDDEEDLRLVDLV [SEQ ID 28] |
| | PEPTIDE : GDTIKLPAGTTSYLVNQDDEEDLR [SEQ ID 96] |
| REGION : | 321 to 400-NGPGKFEAFDLAKNKNQYLRGFSKNILEASYNTRYETIEKVLLEEQEKDRKRRQQGEETDAIVKVSREQIEELKKLAKSS [SEQ ID 29] |
| | PEPTIDE : RQQGEETDAIVK [SEQ ID 97] |
| | PEPTIDE : VLLEEQEKDRK [SEQ ID 98] |
| | PEPTIDE : NILEASYNTR [SEQ ID 99] |
| | PEPTIDE : FEAFDLAK [SEQ ID 100] |
| | PEPTIDE : EQIEELKK [SEQ ID 101] |
| | PEPTIDE : EQIEELK [SEQ ID 102] |
| | PEPTIDE : NKNQYLR [SEQ ID 103] |
| REGION : | 503 to 549 - RYEARLSPGDVVIIPAGHPVAITASSNLNLLGFGINAENNERNFLSG [SEQ ID 30] |
| | PEPTIDE : LSPGDVVIIPAGHPVAITASSNLNLLGFGINAENNER [SEQ ID 104] |
| REGION : | 89 to 112 - HGEWRPSYEKQEDEEEKQKYRYQR [SEQ ID 31] |
| | PEPTIDE : PSYEKQEDEEEKQK [SEQ ID 105] |
| REGION : | 62 to 80 - PSYEKEEDEEEGQRERGRQ [SEQ ID 32] |
| | PEPTIDE : EEDEEEGQR [SEQ ID 106] |

| | |
|---|---|
| PEA PROTEIN 5 : D3VNE1 - 5 – MAATPIKPLMLLAIAFLASVCVSSRSDQENPFIFKSNRFQTLYENENGHIRLLQKFDKRSKIFENLQNYRLLEYKSKPRTLFLPQYTDADFILVV LSGKATLTVLKSNDRNSFNLERGDTIKLPAGTIAYLANRDDNEDLRVLDLTIPVNKPGQLQSFLLSGTQNQPSLLSGFSKNILEAAFNTNYEEI EKVLLEQQEQEPQHRRSLKDRRQEINEENVIVKVSREQIEELSKNAKSSSKKSVSSESGPFNLRSRNPIYSNKFGKFFEITPEKNQQLQDLDIF VNSVDIKEGSLLLPNYNSRAIVIVTVTEGKGDFELVGQRNENQGKENDKEEEQEEETSKQVQLYRAKLSPGDVFVIPAGHPVAINASSDLNLI GFGINAENNERNFLAGEEDNVISQVERPVKELAFPGSSHEVDRLLKNQKQSYFANAQPLQRE [SEQ ID 5] | |
| REGION : | 75 to 104 - KSKPRTLFLPQYTDADFILVVLSGKATLTV [SEQ ID 33] |
| | PEPTIDE : TLFLPQYTDADFILVVLSGK [SEQ ID 107] |

RICE PROTEIN 7: Q6K508
MATTTSLLSSCLCALLLAPLFSQGVDAWESRQGASRQCRFDRLQAFEPLRKVRSEAGDTE
YFDERNEQFRCAGVFVIRRVIEPQGLVVPRYSNTPALAYIIQGKGYVGLTFPGCPATHQQ
QFQLFEQRQSDQAHKFRDEHQKIHEFRQGDVVALPASVAHWFYNGGDTPAVVVYVYDIKS
FANQLEPRQKEFLLAGNNQRGQQIFEHSIFQHSGQNIFSGFNTEVLSEALGINTEASKRL
QSQNDQRGDIIRVKHGLQLLKPTLTQRQEEHRQYQQVQYREGQYNGLDENFCTIKARVNI
ENPSRADYYNPRAGRITLLNNQKFPILNLIGMGAARVNLYQNALLSPFWNINAHSVVYII
QGSVRVQVANNQGRSVFNGVLHQGQLLIIPQNHAVIKKAEHNGCQYVAIKTISDPTVSWV
AGKNSILRALPVDVIANAYRISRDEARRLKNNRADEIGPFTPRFPQKSQRGYQFLTEGLS
LIGM [SEQ ID 6]

### PEPTIDE: GYVGLTFPGCPATHQQQFQLFEQR [SEQ ID 108]

RICE PROTEIN 8: Q6K7K6
MASMSTILPLCLGLLLFFQVSMAQFSFGGSPLQSPRGFRGDQDSRHQCRFEHLTALEATH
QQRSEAGFTEYYNIEARNEFRCAGVSVRRLVVESKGLVLPMYANAHKLVYIVQGRGVFGM
ALPGCPETFQSVRSPFEQEVATAGEAQSSIQKMRDEHQQLHQFHQGDVIAVPAGVAHWLY
NNGDSPVVAFTVIDTSNNANQLDPKRREFFLAGKPRSSWQQQSYSYQTEQLSRNQNIFAG
FSPDLLSEALSVSKQTVLRLQGLSDPRGAIIRVENGLQALQPSLQVEPVKEEQTQAYLPT
KQLQPTWLRSGGACGQQNVLDEIMCAFKLRKNIDNPQSSDIFNPHGGRITRANSQNFPIL
NIIQMSATRIVLQNNALLTPHWTVNAHTVMYVTAGQGHIQVVDHRGRSVFDGELHQQQIL
LIPQNFAVVVKARREGFAWVSFKTNHNAVDSQIAGKASILRALPVDVVANAYRLSREDSR
HVKFNRGDEMAVFAPRRGPQQYAEWQINEK [SEQ ID 7]

PEPTIDE: RGPQQYAEWQINEK [SEQ ID 109]

PEA PROTEIN 6: P13919
MATTIKSRFPLLLLLGIIFLASVVSVTYANYDEGSEPRVPAQRERGRQEGEKEEKRHGEW
RPSYEKEEDEEEGQRERGRQEGEKEEKRHGEWGPSYEKQEDEEEKQKYRYQREKEDEEEK
QKYQYQREKKEQKEVQPGRERWEREEDEEQVDEEWRGSQRREDPEERARLRHREERTKRD
RRHQREGEEEERSSESQERRNPFLFKSNKFLTLFENENGHIRLLQRFDKRSDLFENLQNY
RLVEYRAKPHTIFLPQHIDADLILVVLSGKAILTVLSPNDRNSYNLERGDTIKLPAGTTS
YLVNQDDEEDLRLVDLVIPVNGPGKFEAFDLAKNKNQYLRGFSKNILEASYNTRYETIEK
VLLEEQEKDRKRRQQGEETDAIVKVS [SEQ ID 8]

### PEPTIDE: LVDLVIPVNGPGKFEAFDLAK [SEQ ID 110]

PEA PROTEIN 7: P02855
MATTIKSRFPLLLLLGIIFLASVVSVTYANYDEGSEPRVPAQRERGRQEGEKEEKRHGEW
RPSYEKEEDEEEGQRERGRQEGEKEEKRHGEWGPSYEKQEDEEEKQKYRYQREKEDEEEK
QKYQYQREKKEQKEVQPGRERWEREEDEEQVDEEWRGSQRREDPEERARLRHREERTKRD
RRHQREGEEEERSSESQERRNPFLFKSNKFLTLFENENGHIRLLQRFDKRSDLFENLQNY
RLVEYRAKPHTIFLPQHIDADLILVVLSGKAILTVLSPNDRNSYNLERGDTIKLPAGTTS
YLVNQDDEEDLRLVDLVIPVNGPGKFEAFDLAKNKNQYLRGFSKNILEASYNTRYETIEK
VLLEEQEKDRKRRQQGEETDAIVKVS [SEQ ID 9]

### PEPTIDE: KNPQLQDLDIFVNYVEIK [SEQ ID 111]

RICE PROTEIN 1 : P14323 - 1 –
MASSVFSRFSIYFCVLLLCHGSMAQLFNPSTNPWHSPRQGSFRECRFDRLQAFEPLRKVRSEAGVTEYFDEKNELFQCTGTFVIRRVIQPQG
LLVPRYTNIPGVVYIIQGRGSMGLTFPGCPATYQQQFQQFSSQGQSQSQKFRDEHQKIHQFRQGDIVALPAGVAHWFYNDGDAPIVAVY
VYDVNNNANQLEPRQKEFLLAGNNNRAQQQQVYGSSIEQHSGQNIFSGFGVEMLSEALGINAVAAKRLQSQNDQRGEIIHVKNGLQLLK
PTLTQQQEQAQAQDQYQQVQYSERQQTSSRWNGLEENFCTIKVRVNIENPSRADSYNPRAGRITSVNSQKFPILNLIQMSATRVNLYQN
AILSPFWNVNAHSLVYMIQGRSRVQVVSNFGKTVFDGVLRPGQLLIIPQHYAVLKKAEREGCQYIAIKTNANAFVSHLAGKNSVFRALPVD
VVANAYRISREQARSLKNNRGEEHGAFTPRFQQQYYPGLSNESESETSE [SEQ ID 10]

| | |
|---|---|
| REGION : | 138 to 159 - SQSQKFRDEHQKIHQFRQGDIV [SEQ ID 34] |
| | PEPTIDE : DEHQKIHQFR [SEQ ID 112] |
| | PEPTIDE : FRDEHQK [SEQ ID 113] |
| REGION : | 336 to 358 - VNSQKFPILNLIQMSATRVNLYQ [SEQ ID 35] |
| | PEPTIDE : FPILNLIQMSATR [SEQ ID 114] |
| REGION : | 423 to 462 - YIAIKTNANAFVSHLAGKNSVFRALPVDVVANAYRISREQ [SEQ ID 36] |
| | PEPTIDE : TNANAFVSHLAGK [SEQ ID 115] |
| | PEPTIDE : ALPVDVVANAYR [SEQ ID 116] |
| REGION : | 175 to 205 - APIVAVYVYDVNNNANQLEPRQKEFLLAGNN [SEQ ID 37] |
| | PEPTIDE : YVYDVNNNANQLEPRQKEFL [SEQ ID 117] |
| | PEPTIDE : VYVYDVNNNANQLEPRQKEFL [SEQ ID 118] |
| REGION : | 318 to 334 - ENPSRADSYNPRAGRIT [SEQ ID 38] |
| | PEPTIDE : ADSYNPR [SEQ ID 119] |
| REGION : | 265 to 296 - GLQLLKPTLTQQQEQAQAQDQYQQVQYSERQQ [SEQ ID 39] |
| | PEPTIDE : KPTLTQQQEQAQAQDQ [SEQ ID 120] |
| | PEPTIDE : QAQAQDQYQQVQY [SEQ ID 121] |
| | PEPTIDE : QAQDQYQQVQY [SEQ ID 122] |
| REGION : | 45 to 62 - CRFDRLQAFEPLRKVRSE [SEQ ID 40] |
| | PEPTIDE : LQAFEPLR [SEQ ID 123] |
| REGION : | 361 to 408 - ILSPFWNVNAHSLVYMIQGRSRVQVVSNFGKTVFDGVLRPGQLLIIPQ [SEQ ID 41] |
| | PEPTIDE : SRVQVVSNFGK [SEQ ID 124] |
| | PEPTIDE : WNVNAHSLVY [SEQ ID 125] |
| | PEPTIDE : NVNAHSLVY [SEQ ID 126] |
| | PEPTIDE : IQGRSRVQVVSNFGK [SEQ ID 127] |
| | PEPTIDE : GKTVFDGVLRPGQL [SEQ ID 128] |
| | PEPTIDE : FGKTVFDGVLRPGQL [SEQ ID 129] |
| REGION : | 476 to 499 - AFTPRFQQQYYPGLSNESESETSE [SEQ ID 42] |
| | PEPTIDE : FQQQYYPGLSNESESETSE [SEQ ID 130] |
| | PEPTIDE : QQYYPGLSN [SEQ ID 131] |
| | PEPTIDE : QQQYYPGLSN [SEQ ID 132] |

RICE PROTEIN 2 : P07728 - 2 –
MASINRPIVFFTVCLFLLCNGSLAQQLLGQSTSQWQSSRRGSPRECRFDRLQAFEPIRSVRSQAGTTEFFDVSNEQFQCTGVSVVRRVIEPR

GLLLPHYTNGASLVYIIQGRGITGPTFPGCPESYQQQFQQSGQAQLTESQSQSQKFKDEHQKIHRFRQGDVIALPAGVAHWCYNDGEVPV
VAIYVTDLNNGANQLDPRQRDFLLAGNKRNPQAYRREVEERSQNIFSGFSTELLSEALGVSSQVARQLQCQNDQRGEIVRVEHGLSLLQPY
ASLQEQEQGQVQSRERYQEGQYQQSQYGSGCSNGLDETFCTLRVRQNIDNPNRADTYNPRAGRVTNLNTQNFPILSLVQMSAVKVNLY
QNALLSPFWNINAHSVVYITQGRARVQVVNNNGKTVFNGELRRGQLLIIPQHYAVVKKAQREGCAYIAFKTNPNSMVSHIAGKSSIFRAL
PNDVLANAYRISREEAQRLKHNRGDEFGAFTPIQYKSYQDVYNAAESS [SEQ ID 11]

| | |
|---|---|
| REGION : | 332 to 362 - PRAGRVTNLNTQNFPILSLVQMSAVKVNLYQ [SEQ ID 43] |
| | PEPTIDE : VTNLNTQNFPILSLVQMSAVK [SEQ ID 133] |
| REGION : | 375 to 406 - HSVVYITQGRARVQVVNNNGKTVFNGELRRGQ [SEQ ID 44] |
| | PEPTIDE : ITQGRARVQVVNNNGKTVF [SEQ ID 134] |
| | PEPTIDE : ITQGRARVQVVNNNGKTVFNGE [SEQ ID 135] |
| | PEPTIDE : ITQGRARVQVVNNNGKTVFNG [SEQ ID 136] |
| | PEPTIDE : RVQVVNNNGKTVF [SEQ ID 137] |
| REGION : | 440 to 471 - HIAGKSSIFRALPNDVLANAYRISREEAQRLK [SEQ ID 45] |
| | PEPTIDE : RALPNDVLANAYRISREE [SEQ ID 138] |
| | PEPTIDE : SIFRALPNDVLANAYR [SEQ ID 139] |
| | PEPTIDE : SIFRALPNDVLANAY [SEQ ID 140] |
| | PEPTIDE : SIFRALPNDVLAN [SEQ ID 141] |
| | PEPTIDE : SSIFRALPNDVLANAYR [SEQ ID 142] |
| | PEPTIDE : SIFRALPNDVLANAYRISREE [SEQ ID 143] |
| | PEPTIDE : SIFRALPND [SEQ ID 144] |
| REGION : | 180 to 208 - VPVVAIYVTDLNNGANQLDPRQRDFLLAG [SEQ ID 46] |
| | PEPTIDE : IYVTDLNNGANQLDPRQRD [SEQ ID 145] |

RICE PROTEIN 3 : P07730 - 2 –
MASINRPIVFFTVCLFLLCDGSLAQQLLGQSTSQWQSSRRGSPRGCRFDRLQAFEPIRSVRSQAGTTEFFDVSNELFQCTGVSVVRRVIEPR
GLLLPHYTNGASLVYIIQGRGITGPTFPGCPETYQQQFQQSGQAQLTESQSQSHKFKDEHQKIHRFRQGDVIALPAGVAHWCYNDGEVPV
VAIYVTDINNGANQLDPRQRDFLLAGNKRNPQAYRREVEEWSQNIFSGFSTELLSEAFGISNQVARQLQCQNDQRGEIVRVERGLSLLQPY
ASLQEQEQGQMQSREHYQEGGYQQSQYGSGCPNGLDETFCTMRVRQNIDNPNRADTYNPRAGRVTNLNSQNFPILNLVQMSAVKVN
LYQNALLSPFWNINAHSIVYITQGRAQVQVVNNNGKTVFNGELRRGQLLIVPQHYVVVKKAQREGCAYIAFKTNPNSMVSHIAGKSSIFR
ALPTDVLANAYRISREEAQRLKHNRGDEFGAFTPLQYKSYQDVYNVAESS [SEQ ID 12]

| | |
|---|---|
| REGION : | : 311 to 362 - TFCTMRVRQNIDNPNRADTYNPRAGRVTNLNSQNFPILNLVQMSAVKVNLYQ [SEQ ID 47] |
| | PEPTIDE : VTNLNSQNFPILNLVQMSAVK [SEQ ID 146] |
| | PEPTIDE : QNIDNPNR [SEQ ID 147] |
| | PEPTIDE : ADTYNPR [SEQ ID 148] |
| | PEPTIDE : NIDNPNRADTYNPRAGRVTNL [SEQ ID 149] |
| | PEPTIDE : RVRQNIDNPNRADTYNPRAGRVTNL [SEQ ID 150] |
| REGION : | 427 to 499-YIAFKTNPNSMVSHIAGKSSIFRALPTDVLANAYRISREEAQRLKHNRGDEFGAFTPLQYKSYQDVYNVAESS[SEQ ID 48] |
| | PEPTIDE : TNPNSMVSHIAGKSSIFR [SEQ ID 151] |
| | PEPTIDE : HNRGDEFGAFTPLQYK [SEQ ID 152] |
| | PEPTIDE : SYQDVYNVAESS [SEQ ID 153] |
| | PEPTIDE : ISREEAQR [SEQ ID 154] |
| | PEPTIDE : SIFRALPTDVLANAYRISREE [SEQ ID 155] |
| | PEPTIDE : YRISREEAQRLKHNRGDEF [SEQ ID 156] |
| | PEPTIDE : YRISREEAQRLKHNRGDE [SEQ ID 157] |
| REGION : | 143 to 178 - SQSHKFKDEHQKIHRFRQGDVIALPAGVAHWCYNDG [SEQ ID 49] |
| | PEPTIDE : FKDEHQKIHR [SEQ ID 158] |
| | PEPTIDE : QGDVIALPAGVAHW [SEQ ID 159] |
| REGION : | 381 to 409 TQGRAQVQVVNNNGKTVFNGELRRGQLLI [SEQ ID 50] |
| | PEPTIDE : TVFNGELRR [SEQ ID 160] |
| | PEPTIDE : TVFNGELR [SEQ ID 161] |
| | PEPTIDE : QVQVVNNNGKTVF [SEQ ID 162] |
| REGION : | 101 to 124 - NGASLVYIIQGRGITGPTFPGCPE [SEQ ID 51] |
| | PEPTIDE : YIIQGRGITGPTF [SEQ ID 163] |
| | PEPTIDE : VYIIQGRGITGPTF [SEQ ID 164] |
| REGION : | 46 to 66 - CRFDRLQAFEPIRSVRSQAGT [SEQ ID 52] |
| | PEPTIDE : LQAFEPIRSVR [SEQ ID 165] |
| REGION : | 253 to 292 - QNDQRGEIVRVERGLSLLQPYASLQEQEQGQMQSREHYQE [SEQ ID 53] |
| | PEPTIDE : GLSLLQPYASLQEQEQGQMQSR [SEQ ID 166] |
| | PEPTIDE : GEIVRVER [SEQ ID 167] |
| | PEPTIDE : RGLSLLQPYASLQ [SEQ ID 168] |
| | PEPTIDE : RGLSLLQPYASLQEQ [SEQ ID 169] |
| | PEPTIDE : RGLSLLQPYASLQEQE [SEQ ID 170] |
| | PEPTIDE : RGLSLLQPYASLQE [SEQ ID 171] |
| REGION : | 199 to 233 - PRQRDFLLAGNKRNPQAYRREVEEWSQNIFSGFST [SEQ ID 54] |
| | PEPTIDE : RNPQAYR [SEQ ID 172] |
| | PEPTIDE : FLLAGNKRNPQAY [SEQ ID 173] |
| | PEPTIDE : EVEEWSQNIF [SEQ ID 174] |
| | PEPTIDE : LAGNKRNPQAYR [SEQ ID 175] |
| | PEPTIDE : FLLAGNKRNPQA [SEQ ID 176] |

RICE PROTEIN 4 : Q0D7S0 - 3 –
MASNKVVFSVLLLAVVSVLAATATMAEYHHQDQVVYTPGPLCQPGMGYPMYPLPRCRALVKRQCVGRGTAAAAEQVRRDCCRQLAAV
DDSWCRCEAISHMLGGIYRELGAPDVGHPMSEVFRGCRRGDLERAAASLPAFCNVDIPNGGGGVCYWLARSGY [SEQ ID 13]

| | |
|---|---|
| REGION : | 102 to 124 - GGIYRELGAPDVGHPMSEVFRGC [SEQ ID 55] |
| | PEPTIDE : ELGAPDVGHPMSE [SEQ ID 177] |

RICE PROTEIN 5 : P14614 - 4 –
MATIAFSRLSIYFCVLLLCHGSMAQLFGPNVNPWHNPRQGGFRECRFDRLQAFEPLRRVRSEAGVTEYFDEKNEQFQCTGTFVIRRVIEPQ
GLLVPRYSNTPGMVYIIQGRGSMGLTFPGCPATYQQQFQQFLPEGQSQSQKFRDEHQKIHQFRQGDIVALPAGVAHWFYNEGDAPVVA
LYVFDLNNNANQLEPRQKEFLLAGNNNREQQMYGRSIEQHSGQNIFSGFNNELLSEALGVNALVAKRLQGQNDQRGEIIRVKNGLKLLRP
AFAQQQEQAQQQEQAQAQYQVQYSEEQQPSTRCNGLDENFCTIKARLNIENPSHADTYNPRAGRITRLNSQKFPILNLVQLSATRVNLY
QNAILSPFWNVNAHSLVYIVQGHARVQVVSNLGKTVFNGVLRPGQLLIIPQHYVVLKKAEHEGCQYISFKTNANSMVSHLAGKNSIFRAM
PVDVIANAYRISREQARSLKNNRGEELGAFTPRYQQQTYPGFSNESENEALE [SEQ ID 14]

| | |
|---|---|
| REGION : | 372 to 397 - HSLVYIVQGHARVQVVSNLGKTVFNG [SEQ ID 56] |
| | PEPTIDE : IVQGHARVQVVSNLGK [SEQ ID 178] |
| | PEPTIDE : IVQGHARVQVVSNL [SEQ ID 179] |
| | PEPTIDE : IVQGHARVQVVSN [SEQ ID 180] |
| REGION : | 464 to 486 - ARSLKNNRGEELGAFTPRYQQQT [SEQ ID 57] |
| | PEPTIDE : NNRGEELGAFTPR [SEQ ID 181] |
| | PEPTIDE : GEELGAFTPR [SEQ ID 182] |
| REGION : | 337 to 359 - LNSQKFPILNLVQLSATRVNLYQ [SEQ ID 58] |
| | PEPTIDE : FPILNLVQLSATR [SEQ ID 183] |
| REGION : | 210 to 293 - QMYGRSIEQHSGQNIFSGFNNELLSEALGVNALVAKRLQGQNDQRGEIIRVKNGLKLLRPAFAQQQEQAQQQEQAQAQYQVQYS [SEQ ID 59] |
| | PEPTIDE : SIEQHSGQNIFSGFNNELLSEALGVNALVAK [SEQ ID 184] |
| | PEPTIDE : LQGQNDQR [SEQ ID 185] |
| | PEPTIDE : SGFNNELLSEALGVNALVAK [SEQ ID 186] |
| | PEPTIDE : PAFAQQQEQAQQQEQAQAQY [SEQ ID 187] |
| | PEPTIDE : VAKRLQGQNDQRGEI [SEQ ID 188] |
| | PEPTIDE : ALVAKRLQGQNDQRGEI [SEQ ID 189] |
| | PEPTIDE : LQGQNDQRGEIIR [SEQ ID 190] |
| REGION : | 24 to 47 - AQLFGPNVNPWHNPRQGGFRECRF [SEQ ID 60] |
| | PEPTIDE : PNVNPWHNPRQGGF [SEQ ID 191] |
| REGION : | 164 to 186 - GVAHWFYNEGDAPVVALYVFDLN [SEQ ID 61] |
| | PEPTIDE : FYNEGDAPVVALY [SEQ ID 192] |
| | PEPTIDE : FYNEGDAPVV [SEQ ID 193] |
| | PEPTIDE : FYNEGDAPVVAL [SEQ ID 194] |
| | PEPTIDE : FYNEGDAPVVA [SEQ ID 195] |
| REGION : | 424 to 463 - YISFKTNANSMVSHLAGKNSIFRAMPVDVIANAYRISREQ [SEQ ID 62] |
| | PEPTIDE : TNANSMVSHLAGK [SEQ ID 196] |
| | PEPTIDE : AMPVDVIANAYR [SEQ ID 197] |

RICE PROTEIN 6 : Q0DEV5 - 5 –
MSALTTSQLATSATGFGIADRSAPSSLLRHGFQGLKPRSPAGGDATSLSVTTSARATPKQQRSVQRGSRRFPSVVVYATGAGMNVVFVGA
EMAPWSKTGGLGDVLGGLPPAMAANGHRVMVISPRYDQYKDAWDTSVVAEIKVADRYERVRFFHCYKRGVDRVFIDHPSFLEKVWGK
TGEKIYGPDTGVDYKDNQMRFSLLCQAALEAPRILNLNNNPYFKGTYGEDVVFVCNDWHTGPLASYLKNNYQPNGIYRNAKVAFCIHNIS
YQGRFAFEDYPELNLSERFRSSFDFIDGYDTPVEGRKINWMKAGILEADRVLTVSPYYAEELISGIARGCELDNIMRLTGITGIVNGMDVSE
WDPSKDKYITAKYDATTAIEAKALNKEALQAEAGLPVDRKIPLIAFIGRLEEQKGPDVMAAAIPELMQEDVQIVLLGTGKKKFEKLLKSMEE
KYPGKVRAVVKFNAPLAHLIMAGADVLAVPSRFEPCGLIQLQGMRYGTPCACASTGGLVDTVIEGKTGFHMGRLSVDCKVVEPSDVKKV
AATLKRAIKVVGTPAYEEMVRNCMNQDLSWKGPAKNWENVLLGLGVAGSAPGIEGDEIAPLAKENVAAP [SEQ ID 15]

| | |
|---|---|
| REGION : | 571 to 608 - KGPAKNWENVLLGLGVAGSAPGIEGDEIAPLAKENVAA [SEQ ID 63] |
| | PEPTIDE : NWENVLLGLGVAGSAPGIEGDEIAPLAK [SEQ ID 198] |
| | PEPTIDE : NVLLGLGVAGSAPGIEGDE [SEQ ID 199] |
| | PEPTIDE : NWENVLLGLGVAGSAPGIEGDEIAPLAK [SEQ ID 200] |
| REGION : | 458 to 488 - RAVVKFNAPLAHLIMAGADVLAVPSRFEPCG [SEQ ID 64] |
| | PEPTIDE : FNAPLAHLIMAGADVLAVPSR [SEQ ID 201] |
| | PEPTIDE : FNAPLAHLIM [SEQ ID 202] |
| | PEPTIDE : FNAPLAHLIMAGADVLAVPSR [SEQ ID 203] |
| REGION : | 545 to 566 - KRAIKVVGTPAYEEMVRNCMNQ [SEQ ID 65] |
| | PEPTIDE : VVGTPAYEEMVR [SEQ ID 204] |
| REGION : | 93 to 147 - APWSKTGGLGDVLGGLPPAMAANGHRVMVISPRYDQYKDAWDTSVVAEIKVADRY [SEQ ID 66] |
| | PEPTIDE : TGGLGDVLGGLPPAMAANGHR [SEQ ID 205] |
| | PEPTIDE : YDQYKDAWDTSVVAEIK [SEQ ID 206] |
| | PEPTIDE : DAWDTSVVAEIK [SEQ ID 207] |
| | PEPTIDE : VMVISPR [SEQ ID 208] |
| REGION : | 305 to 413 - INWMKAGILEADRVLTVSPYYAEELISGIARGCELDNIMRLTGITGIVNGMDVSEWDPSKDKYITAKYDATTAIEAKALNKEALQAEAGLPVDR KIPLIAFIGRLEEQK [SEQ ID 67] |
| | PEPTIDE : LTGITGIVNGMDVSEWDPSKDK [SEQ ID 209] |
| | PEPTIDE : VLTVSPYYAEELISGIAR [SEQ ID 210] |
| | PEPTIDE : EALQAEAGLPVDRK [SEQ ID 211] |
| | PEPTIDE : YDATTAIEAK [SEQ ID 212] |
| | PEPTIDE : IPLIAFIGR [SEQ ID 213] |
| | PEPTIDE : AGILEADR [SEQ ID 214] |
| | PEPTIDE : IPLIAFIGR [SEQ ID 215] |
| REGION : | 158 to 202 - RGVDRVFIDHPSFLEKVWGKTGEKIYGPDTGVDYKDNQMRFSLLC [SEQ ID 68] |
| | PEPTIDE : VFIDHPSFLEK [SEQ ID 216] |
| | PEPTIDE : GPDTGVDYKDNQM [SEQ ID 217] |
| | PEPTIDE : IYGPDTGVDYKDNQMR [SEQ ID 218] |
| | PEPTIDE : IYGPDTGVDYK [SEQ ID 219] |
| REGION : | 206 to 226 - LEAPRILNLNNNPYFKGTYGE [SEQ ID 69] |
| | PEPTIDE : ILNLNNNPYFK [SEQ ID 220] |

BACTERIAL PROTEIN 1 : P0C1U8 - 5 –
MKGKFLKVSSLFVATLTTATLVSSPAANALSSKAMDNHPQQTQSSKQQTPKIQKGGNLKPLEQREHANVILPNNDRHQITDTTNGHYAPV
TYIQVEAPTGTFIASGVVVGKDTLLTNKHVVDATHGDPHALKAFPSAINQDNYPNGGFTAEQITKYSGEGDLAIVKFSPNEQNKHIGEVVK
PATMSNNAETQVNQNITVTGYPGDKPVATMWESKGKITYLKGEAMQYDLSTTGGNSGSPVFNEKNEVIGIHWGGVPNEFNGAVFINEN
VRNFLKQNIEDIHFANDDQPNNPDNPDNPNNPDNPNNPDEPNNPDNPNNPDNPDNGDNNNSDNPDAA [SEQ ID 16]

| | |
|---|---|
| REGION : | 92 to 117 - YIQVEAPTGTFIASGVVVGKDTLLTN [SEQ ID 70] |
| | PEPTIDE : APTGTFIASGVVVGKD [SEQ ID 221] |

Homologs of Pea protein 1 (SEQ ID 1)
   >gi|137584|sp|P08438.1|VCL_VICFA RecName: Full=Vicilin; Flags: Precursor [Vicia faba] >gi|22057|emb|CAA68559.1| vicilin [Vicia faba var. minor] >gi|383931031|gb|AFH56916.1| vicilin **[Vicia faba]** 
MAATTLKDSFPLLTLLGIAFLASVCLSSRSDQDNPFVFESNRFQTLFENENGHIRLLQKFDQHSKLLENLQNYRLLEYKSKPHTIFLPQQTDADFIL
VVLSGKAILTVLLPNDRNSFSLERGDTIKLPAGTIGYLVNRDDEEDLRVLDLVIPVNRPGEPQSFLLSGNQNQPSILSGFSKNILEASFNTDYKEIEK
VLLEEHGKEKYHRRGLKDRRQRGQEENVIVKISRKQIEELNKNAKSSSKKSTSSESEPFNLRSREPIYSNKFGKFFEITPKRNPQLQDLNIFVNYVEI
NEGSLLLPHYNSRAIVIVTVNEGKGDFELVGQRNENQQGLREEYDEEKEQGEEEIRKQVQNYKAKLSPGDVLVIPAGYPVAIKASSNLNLVGFGI
NAENNQRYFLAGEEDNVISQIHKPVKELAFPGSAQEVDTLLENQKQSHFANAQPRERERGSQEIKDHLYSILGSF [SEQ ID 222]
 >gi|502105533|ref|XP_004492829.1| PREDICTED: vicilin-like isoform X1 [Cicer **arietinum] ChickPea** 
MAIKARFPLLVLLGIVFLASVCAKSDKENPFFFKSNNCQTLFENENGHVRLLQRFDKRSQLFENLQNYRLMEYNSKPHTLFLPQHNDADFILVVL
RGRAILTVLNPNDRNTFKLERGDTIKLPAGTIAYLANRDDNEDLRVLDLAIPVNRPGQFQSFSLSGNENQQSYFQGFSKKILEASFNSDYEEIERV
LLEEQEQKPEQRRGHKGRQQSQETDVIVKISREQIEELSKNAKSNCKKSVSSESEPFNLRSRSPIYSNRFGNFFEITPEKNPQLKDLDIFVNSVEIK
EGSLLLPHFNSRATVILVVNEGKGEVELVGLRNENEQENKKEDEEEEEDRNVQVQRFQSKLSSGDVVVIPASHPFSINASSDLFLLGFGINAQN
NQRNFLAGEEDNVISQIQRPVKEVAFPGSAEEVDRLLKNQRQSHFANAQPQQKRKGSQRIRSPF [SEQ ID 223]
 >gi|29539109|emb|CAD87730.1| allergen Len c 1.0101 **[Lens culinaris] Lentil** 
SRSDQENPFIFKSNRFQTIYENENGHIRLLQRFDKRSKIFENLQNYRLLEYKSKPHTIFLPQFTDADFILVVLSGKAILTVLNSNDRNSFNLERGDTI
KLPAGTIAYLANRDDNEDLRVLDLAIPVNRPGQLQSFLLSGTQNQPSFLSGFSKNILEAAFNTEYEEIEKVLLEEQEQKSQHRRSLRDKRQEITNE
DVIVKVSREQIEELSKNAKSSSKKSVSSESEPFNLRSRNPIYSNKFGKFFEITPEKNPQLQDLDIFVNSVEIKEGSLLLPNYNSRAIVIVTVNEGKGDF
ELVGQRNENQQEQREENDEEEGQEEETTKQVQRYRARLSPGDVLVIPAGHPVAINASSDLNLIGFGINAKNNQRNFLAGEEDNVISQIQRPV
KELAFPGSSREVDRLLTNQKQSHFANAQPLQIE [SEQ ID 224]
Homologs of Pea protein 2 (SEQ ID 2)
   >gi |29539111|emb|CAD87731.1| allergen Len c 1.0102 **[Lens culinaris]** 
SRSDQENPFIFKSNRFQTIYENENGHIRLLQKFDKRSKIFENLQNYRLLEYKSKPHTLFLPQYTDADFILVVLSGKAVLTVLNSNDRNSFNLERGDT
IKLPAGTIAYLANRDDNEDLRVLDLAIPVNNPGQLESFLLSGTQNQPSFLSGFNKSILEAAFNTDYEEIEKVLLEDQEQEPQHRRSLRDRRQEINK
ENVIVKVSREQIKELSKNAKSSSKKSVSSESEPFNLRSRNPIYSNKFGKFFEITPEKNPQLQDLDIFVNSVEIKEGSLLLPNYNSRAIVIVTVNEGKGY
FELVGQRNENQREENDDEEEQEEETSTQVQRYRAKLSPGDVFVVPAGHPVAINASSDLNLIGFGINAKNNQRNFLAGEEDNVISQIQRPVKEL
AFPGSSREVDRLLTNQKQSHFANAQPLQIE [SEQ ID 225]
 >gi|1297072|emb||CAA96514.1| vicilin precursor **[Vicia narbonensis]** 
MAAITMKVSFPLLMLLGISFLASVCVSSRSDQENPFIFKSNKFQTLFENDNGHIRLLQKFDERSKILENLQNYRLLEYKSKPRTIFLPQQTNADFIL
VVLSGKAILTVLKPDDRNSFNLERGDTIKLPAGTIAYLVNKDDNEDLRVLDLAIPVNGPDQLQSFLLSGSENQQSILSGFSKSVLEASFNTGYEEIE
KVLLEEREKETQHRRSLRDKRQHSQDEDVIVKLSRGQIEELSRNAKSSSKKSVSSESEPFNLRSRNPIYSNKFGKFFEITPEKNPQLQDLDVLVNSV
EIKEGSLLLPHYNSRAIVIVTVNDGKGDFEIVGQRNENRQGQRKEDDEEEEQGDENTNTQVQNYKAKLSRGDVFVIPAGHPVSIKASSNLDLLG
FGINAKNNQRNFLAGEEDNVISQIDRPVKELAFPGSAQEVDRLLENQKQSHFANAQPQQRERGSHETRDHLSSILDAF [SEQ ID 226]
 >gi|28629838|gb|AAO45103.1| beta-conglycinin alpha' subunit **[Glycine max]** 
QYGHVRVLQRFNKRSQQLQNLRDYRILEFNSKPNTLLLPHHADADYLIVILNGTAILTLVNNDDRDSYNLQSGDALRVPAGTTYYVVNPDNDE
NLRMITLAIPVNKPGRFESFFLSSTQAQQSYLQGFSKNILEASYDTKFEEINKVLFGREEGQQQGEERLQESVIVEISKKQIRELSKHAKSSSRKTIS
SEDKPFNLRSRDPIYSNKLGKLFEITPEKNPQLRDLDVFLSVVDMNEGALFLPHFNSKAIVVLVINEGEANIELVGIKEQQQRQQQEEQPLEVRK
YRAELSEQDIFVIPAGYPVVVNATSDLNFFAFGINAENNQRNFLAGSKDNVISQIPSQVQELAFPGSAKDIENLIKSQSESYFVDAQPQQKEEGN
KGRKGPLSSILRAFY [SEQ ID 227]
Homologs of Pea protein 3 (SEQ ID 3)
   >gi|483449|emb|CAA83677.1|legumin A [Vicia sativa] 
MAKLLALSLSFCFLLFSSCFALREQSQQNECQLERINALEPDNRIESEGGLIETWNPNNRQFRCARVALSRATLQRNALRRPYYSNAPQEIYIQQ
GNGYFGMVFPGCPETHEEPQQSEQGEGRRYRDSHQKVNRFREGDIIAVPTGIAFWMYNDQDTPVIAISLTDTGSSNNQLDQMPRRFYLAG
NQEQEFLRYQHQQGGKQEQDNDGNNIFSGFKRDFLEDAFNVNRHIVDRLQGRNEDEEKGAIVKVKGGLSIIAPPERQARHERGSRQEEDED
EKEERQPSHHKSRRDEDEDDKEKRHSQKGQSRRQGDNGLEETVCTAKLRANIGSSPSPDIYNPQAGRIKTVTSLDLPVLRWLKLSAEHGSLHK
NAMFVPHYNLNANSVIYALKGRARLQVVNCNGNTVFDGELEAGRALTVPQNYAVAAKSLSERFTYVAFKTDDRASIARLAGTSSVIDDLPLDV
VAATFNMQRNEARQLKSNNPFKFLVPPRQSEMRASA [SEQ ID 228]
 >gi|657379551|gb|KEH23931.1| legumin A2 [Medicago truncatula] 
MAKLLALSLSLCFLLFSGCFAIREHQPHQKQQPQQNECQLEQLNALEPDNRIESEGGIIETWNPNNRQFRCAGVALSRCTLQRNSLRRPFYSNA
PQEIFIQQGSGYFGMVFPGCPETFEEPQESEQRESRRIRESEQGESRRIRESEQGEGRRFRDSHQKVNRFREGDLIAVPTGTVFWMYNDQDTP
VIAVSLIDTGSFQNQLDEMPRRFYLAGNQEQEFLQYQQQQVRGRGEQRRGREQQENEGGNIFSGFKRDFLEDALNVNRHIVDRLQGRNEDE
EKGAIVKVRGGLSFVTPPERQSRHQGGSIIEEDEDEEDEWRRPHHQKSRRGEEEERPCRRGQKCERSNGLEETICTARLRQNIGSSSSPDIYNPE
AGRIKTVTSFDLPALRWLRLSAEHGTLHRNAMFVPHYNLNANSAIYALRGRARLQVVNCNGNTVFDGELEAGRVLIVPQNFAVAAKSMSDRF
QYVSFKTNDNAAIARLAGTQSTLSGVPMDVLAATYNMDRNEARQLKNNNLYKFLVPPRESERRAAA [SEQ ID 229]
 >gi||206712292|emb|CAR78996.1| legumin storage protein 5 [Lotus japonicus] 
MAYKLFALSLSFCFLLFGGCFAIRQQSQQQNECQLERLNALKPDNRIESEAGYIETWNPTNNQFRCAGVALSRCTLRRNGLKRPSYSNAPQEIFI
QQGSGIFGMIFPGCPETVEEPFESDQQGRRDRHQKVNRFREGDVIAVPPGVVFWMYNEEETPVIAVSLIDTGSYLNQLDQMPRRFYLSGNQ
EQEFLQYQRQEVRGREEENQGGNIFSGFGGEFLEDALNIDRNIVHKLQGRDEEQDKGAIVRVKGGLSVITPPERQSHRRGSEEEEDEEEDRPS
RHQSRGGSRRNGLEETICTVRLRMNIGKSSSPDIFNPQAGRIKTATGFDFPALRFLKLSAEHGSLNRNAMVVPHYNLNANSIIYALRGRAWIQV
VNCKGNRIFDGELEEGQVLIVPQNFVVAARSMSDKFNYVAFKTNDMPTMAKLAGATSEIQAMPLEVIQNAFNLEREQAKQVKFNNRFNFLV
PPREQSQRRASA [SEQ ID 230]
Homologs of Pea protein 4 (SEQ ID 4)
   >gi|164512526|emb|CAP06312.1| cvc [Pisum abyssinicum] 
MATTVESRFPLLLFPGIIFLASVCVTYANYDEGSETRVPGQRERGRQEGEKEEKRHGEWRPSYEKEEDEEEKQKYRYQREKEDEEEKQKYRYQR
EKKEEKEVQPGRERWEREEDEEQVDEEWRGSQRRQDPEERARLRHREERTKRDRRHKREGEEEERSSESQEQRNPFLFKSNKFLTLFENENG
HIRRLQRFDKRSDLFENLQNYRLVEYRAKPHTIFLPQHIDADLILVVLNGKAILTVLSPNDRNSYNLERGDTIKIPAGTTSYLVNQDDEEDLRVVD
FVIPVNRPGKFEAFGLSENKNQYLRGFSKNILEASLNTKYETIEKVLLEEQEKKPQQLRDKRRQQGGERDAIIKVSREQIEELRKLAKSSSKKSLPS
EFEPFNLRSHKPEYSNKFGKLFEITPEKKYPQLQDLDILVSCVEINKGALMLPHYNSRAIVVLLVNEGKGNLELLGLKNEQQEREDRKERNNEVQ
RYEARLSPGDVVIIPAGHPVAISASSNLNLLGFGTNAENNQRNFLSGSDDN [SEQ ID 231]
 >gi|164512538| emb|CAP06318.1|cvc [Lathyrus annuus] 
MATTIKSRFPLLLLLGIIFLASVCVTWANYDEGSEPRVPGQRERGRQEGEKEEKRHGEWRPSYEEEYDEGLEPKVPGKRERGRQEGEKEEKRHE
EWRPSYEKEEDEEEKQKYNYQREKKEHKEVQPGRERWERKQDEKQVEEDEEPGEEQWRGSKRHEDPEERARLRHREEKTKSYVEDNEETSS
KEGRNPFLFKSNKFLTLFENENGHIRRLQRFDERSDIFENLQNYRLVEYRAKPHTMFLPQHIDADLILVVLNGKAILTVLSPNDRNSYNLERGDT
VKLPAGTTSYLVNQDDEEDLRVVDLAIPVNRPGKFEAFGLSANKNQYLRGFSKNILEASLNTKYETIEKVLLEERRDQKGRQQGQETNAIVKVSR
EQIEELRKLAKSSSKKSLLSESEPLNLRSQNPKYSNKFGKFFEITPQKKYPQLQDLDVSISCVEINKGALLLPHYNSRSIGILLVNEGKGNLELVGFKN
EQQRQRENEETNKKLQRYEARLSSGDVVVIPEGHPVAISASSNLNLLGFGINAANNQRNFLTGSDDN [SEQ ID 232]
 >gi|164512558|emb|CAP06328.1|cvc [Vicia villosa] 
MATTIKSRFPVLLLLGIIFLTSVCVTYANYDEGREPSVPGQRERGRQEGEKEEKRHGEWRPSEEDEEEKYKYEEGRVPGQRERGRQEGEKEEKR
HGKWRPSEEEDEEEKYRYEEGSEPRGPGQRETGRQEGEKEKQRPEREPSYEKEEDEEEKQKYQYHREKKEQREVRPGRERFERHEDEEQWRG
IQRHEDPEERARERYRAEIAKRQVEEEREERDIPHEREQRNPFLFKSNKFQTLFQNENGYIRRLQRFDKRSDLFENLQNYRLVEYRAKPHTIFLPQ
HIDADLIIVVLSGRAILTVLSPDDRNSYNLERGDTIKLPAGTTSYLVNQDDEEDLRVVDLAIPVNRPGKVESFLLSGNKNQYLRGFSKNILEASFNT
NYETIERVLLEEQDKESQQSIGQKRRSQRQETNALVKVSREQLEDLKRLAKSSSQEGLSSQFEPINLRSQNPKYSNKFGKVFEITPEKKYPQLQDL
DLFVSSVDIKEGALMLPHYNSRAIVVLLVNEGRGNLELVGLKNEQQEQREKEDEQQERNNQVQRYEARLSPGDVVIIPAGHPVAVRASSDLNL
LAFGINAENNQRNFLAGSDDN [SEQ ID 233]
Homologs of Pea protein 5 (SEQ ID 5)
   >gi|357507721|ref|XP_003624149.1| Provicilin [Medicago truncatula] >gi|87162569|gb|ABD28364.1| Cupin, RmIC-type [Medicago truncatula] >gi|355499164|gb|AES80367.1| vicilin 47 kDa protein [Medicago truncatula] 
MAIKAPFQLLMLLGIFFLASVCVSSRDDRHDQENPFFFNANHFQTLFENENGHIRLLQRFDKRSKIFENLQNYRLLEYHSKPHTLFLPQHNDAD
FILAVLSGKAILTVLNPDNRNSFNLERGDTIKLPAGSIAYLANRDDNEDLRVLDLAIPVNRPGKFQSFSLSGSQNQQSFFSGFSKNILEAAFNANY
EEIERVLIEEHEQEPQHRRGLRKDRRQQSQDSNVIVKVSREQIEELSRHAKSSSRRSGSSESAPFNLRSREPIYSNEFGNFFEITPEKNPQLKDLDIL
VNYAEIREGSLLLPHFNSRATVIVVVDEGKGEFELVGQRNENQQEQREEDEQQEERSQQVQRYRARLSPGDVYVIPAGHPTVVSASSDLSLL
GFGINAENNERNFLAGEEDNVISQIERPVKEVAFPGSAQDVESLLKNQRQSYFANAQPQQREREEGRSQRQRELISSILGVF [SEQ ID 234]
 >gi|164512560|emb|CAP06329.1|convicilin [Vicia peregrina] 
MATTFKSRFSLLLLLGIIFLAFVCVTCANYDEGSEPRVPGQRERGRQEGEKEEQSRERHPQREPSREKEEDEEEKQKYDEGTEPRVPGQRERGR
QEGEKEEQRRERHPGQREPSQEEDEEREESDRRQEGSSKSEEQRNPFLFKSNKFLTLFQNGNGHIRLLQRFDKRSDLFENLQNYRLLEYRAKPH
TIFLPQHIDADLILVVLSGRAILTVLSPDDRNSYNLERGDTIKLPAGTTSYPLNQDDEEDLRVVDLAISVNRPGKVESFNLSGNKNQYLRGFSENIL
EASFNTKYETIEKVLLEEQDKESQQPRGQRLQRQETNALVKVSREQVEELKRLARTSSKKGVSSEFEPFNLRSHGPKYSNKFGKFFEITPEKKYPQ
LQDLDISVSSVEINEGALFLPHYNSRAIVVVLVDEGKGNLELVGFKNEQQEQREKEDEQEERNKQVQRYEAKLSPGDVVIIPAGHPVAVSASSN
LNLLGFGINAENNQRNFLTGSDDN [SEQ ID 235]
 >gi|164512562| emb|CAP06330.1| convicilin [Vicia lutea] 
MATTIKLRFPLLLLLGVILLASVCVTCANYDEGSEPRVPGRPEGEKEEKHRGKLRPSYEKEEDEGEKQRYHYEKKEQKEAQPRREKKEQKEEEKQ
VEEESRESQRYEDPGERARERYRAEIIKRQVEKEREERDRRHQREGEEEEGSSKSRNPFLFKSNNFLTLFENENGHIRLLQRFDKRSDLFENLQNY
RLVEYRAKPHTIFLPQHIDADLILVVLSGKAILTVLSPNNRNSYNLKRGDTIKLPAGTTSYLLNSDDEEDLRMVDLAISVNRPGKVESFNLSGNKN
QYLRGFSKNILEASFNTKYETIEKVLLEEQDKESQQSIGQKRISQRQETNALVKVSREQIEEPKRLARSSSRKGVSSEFEPINLRSQRPKYSNKFGKF
YEISPEKKYPQLQDLDVSVSSVEINEGALLLPHYNSRAIVTVLVNEGKGNLELIGFQNEQQGQREKEDEQQHERNKQVQRYDARLSSGDVVIIP
AGHPVAVSASSNLDLLGFGINAENSQRNFLTGSDDN [SEQ ID 236]
Homologs of Rice protein 1 (SEQ ID 6)
   >gi|573919041|ref|XP_006647142.1| PREDICTED: glutelin type-B 4-like [Oryza brachyantha] 
MATTTFSRFSIYFCVLLLCHGSMAQLFSPTLNPWHSSRRGGSRDCRFDRLQAFEPLRRVRSEAGVTEYFDERNEQFQCTGTFVIRRVIEPQGLL
VPRYTNTPGVVYIMQGRGSMGLTFPGCPATYQQQFQQFLPEGQSQSQKFRDEHQKIHQFRQGDIVALPAGVAHWFYNEGDTPVVALYVFD
INNSANQLEPRQKDFLLAGNNNREQQVYGRSIEKHSGQNIFSGFNHELLSEALGISTLAAKRLQGQNDHRGEIIRVRNGLQLLKPTFTQQQEQ
AQSQYQVQYSEKQQESTRCNGLDENFCTINARLNIENPSRADTYNPRAGRITHLNNQKFPILNLVQMSATRVNLYQNAILSPYWNVNAHSLV
YMVQGHARVQVVSNLGKTVFNSVLRPGQLLIIPQHYVVLKKAEREGCQYIAFKTNANSIVSQLAGKNSILRAMPVDVVANAYRISREQARDLK
NNRGEELGAFTPKFEQQSYPGLSNESESEASE [SEQ ID 237]
 >gi|2764800|emb|CAA54153.1| 12s globulin [Avena sativa] 
MATTSFPSVLFYSCIFLLYNGSMAQLFGQSFTPWQSSRQGGLKGCKFDRLQAFEPLRQVRSQAGVTEYFDEQNEQFRCTGVFVIRRVIEPQGL
LLPQYHNAPGLVYILQGRGYTGLTFPGCPATFQQQFQPFDQAQDQSQSHLKDEHQRVHRFKQGDVIALPAGIVHWGYNDGDAPVVAIYVF
DVNNNANQLEPRQKEFLLAGNNKEDQQFGQNIFSGFNIQLLSEALGISQQAAQRIQSQKEQRGEIIRVTQALQFLKPTMSQQELVEHQAYQP
IQSQEGQSTQYQVGQSTQYQEGQSTQYQAGQSQDRSFNGLEENFCSLEARQNIGNPKRADTHNPRAGRITRLHGQNFPILNLVQMSATRV
NLYQNAILSPFWNINAHSVVYMIQGHAQVQVVNNNGQTVFNDRLRQGQLLIVPQHYVVLKKAEREGCQYISFKTNPNSMVSHIAGKSSILRA
LPVDVLANAYRISRQEARNLKNNRGQESGVFTPKFTQTSFQPYPEGEDESSLTNKASE [SEQ ID 238]
 >gi|357130026|ref|XP_003566659.1| PREDICTED: 12S seed storage globulin 1-like [Brachypodium distachyon]
   >gi|357130028|ref|XP_003566660.1| PREDICTED: 12S seed storage globulin 1-like [Brachipodium distachyon] 
MAHTSFSSVLSYFCIFLLFHGSMAQVPGQGSTWQSPRQGGSRECSFDRLQTIEPLTQVRSQAGLTEYFDEQNEQFRCAGVSVIRRVIEPRGLLL
PRYHNTPGLVYILEGSGFVGLAFPGCPETFLEQFQQSRQTQSTLGQSQCQSQSQKLGDVHQRVHQFTQGDVVALPAGVAHWFYNGGDAPV
VAVYVFDVNNNANQLEPRQKEFLLAGNYNGVLQSGRNILNGLNAQLLSQAFGINEQTSRIIQNQNDGRGEIVRVEYGLQFLTPVVTQQQQK
QPFLPIEPQEGQSSRNGLEENFCSLEPRQNIEDPNRADTYNPRAGSIARLNGQNFPILNLVQMSATRVNLQKNAIVSPFWNINAHSVVYVIQG
QASVQVVNNQGRNVFNGLLRRGQLLIIPQNYVVLKKAESEGYQYIAFKTNANSMVSHIAGKNSILRALPVDVIANAYRISRQEAQNLKNNRGE
EIGVLTPNFPQSSCQSYPIGDVDSSSTPKAQE [SEQ ID 239]
Homologs of Rice protein 2 (SEQ ID 7)
   >gi|222622792|gb|EEE56924.1| hypothetical protein OsJ_06602 [Oryza sativa Japonica Group] 
MAQFSFGGSPLQSPRGFRGDQDSRHQCRFEHLTALEATHQQRSEAGFTEYYNIEARNEFRCAGVSVRRLVVESKGLVLPMYANAHKLVYIVQ
GRGVFGMALPGCPETFQSVRSPFEQEVATAGEAQSSIQKMRDEHQQLHQFHQGDVIAVPAGVAHWLYNNGDSPVVAFTVIDTSNNANQL
DPKRREFFLAGKPRSSWQQQSYSYQTEQLSRNQNIFAGFSPDLLSEALSVSKQTVLRLQGLSDPRGAIIRVENGLQALQPSLQVEPVKEEQTQA
YLPTKQLQPTWLRSGGACGQQNVLDEIMCAFKLRKNIDNPQSSDIFNPHGGRITRANSQNFPILNIIQMSATRIVLQNNALLTPHWTVNAHT
VMYVTAGQGHIQVVDHRGRSVFDGELHQQQILLIPQNFAVVVKARREGFAWVSFKTNHNAVDSQIAGKASILRALPVDVVANAYRLSREDS
RHVKFNRGDEMAVFAPRRGPQQYAEWQINEK [SEQ ID 240]
 >gi|218190679|gb|EEC73106.1| hypothetical protein Osl_07091 [Oryza sativa Indica Group] 
MAQFSFGGSPLQSPRGFRGDQDSRHQCRFEHLTALEATHQQRSEAGFTEYYNIEARNEFRCAGVSVRRLVVESKGLVLPMYANAHKLVYIVQ
GRGVFGMALPGCPETFQSVRSPFEQEVATAGEAQSSMQKMRDEHQQLHQFHQGDVIAVPAGVAHWLYNNGDSPVVAFTVIDTSNNANQ
LDPKRREFFLAGKPRSSWQQQSYSYQTEQLSRNQNIFAGFNPDLLSEALSVSKQTVLRLQGLSDPRGAIIRVENGLQALQPSLQVEPVKEEQTQ
AYLPTKQLQPTWSRSGGACGQQNGLDEIMCAFKLRKNIDNPQSSDIFNPHGGRITRANSQNFPILNIIQMSATRIVLQNNALLTPHWTVNAH
TVMYVTAGQGRIQVVDHRGRSVFDGELHQQQILLIPQNFAVVVKARREGFAWVSFKTNHNAVDSQIAGKASILRALPVDVVANAYRLSREDS
RRVKFNRGDEMAVFAPRRGPQQYAEWQINEK [SEQ ID 241]
 >gi|573922051|ref|XP_006648611.1| PREDICTED: glutelin type-A 1-like [Oryza brachyantha] 
MVDMSIVVPVCLTIFLLSQVCIAQVSFDGSPLYSSRGFRGGSASQQQCRFEHLAALEVTHQEKSEAGSIEYYNTEARDEFRCARVSARRLVIESR
GLVLPVYANAHKLLYIVQGRGVFGMALPGCPETFQSVRSAFEMATGDAESSTRKLRDEHQKIHQFRQGDVIAVPPGVAHWLYNNGDSPVVA
FSVIDFGNNANQLDPKPREFFLAGKPWGWQQVQYSYQSEQQSKHQNIFAGFNPDLLAEALSVSRQTAMRLQELNDQRGAIIRVEQGLQLAL
DPSFQAEQEQEEQPQEYLSSQQQQPTWSQRSGACVQNNGLDEIMCAFKVSKNINSAQSTDIFNPRGGRITRANSQNFPVLNIIQMSATRTV
LQNNALLTPHWTVNAHTVMYVTAGQGRIQVVDHRGRTVFDGELRQQQILLIPQNFAVAVKARHEGFSWVSFKTSHNAIDSQIAGKGSILRA
LPVDVLAKAYMLSREESRTLKYNRADETLVFAPRPEIQLYAESEK [SEQ ID 242]
Homologs of Pea protein 6 (SEQ ID 8)
   >gi|164512534|emb|CAP06316.1| cvc [Pisum fulvum] 
MATTTKSRFPLLLLLGIIFLASVVCVTYANYDEGSEPRVPGRRERGRQEGEKEEKRHGEWRPSYEKEEDEEEGQRERGRQEGEKEEKRHGEWG
PSYEKQEDEEEKQKYRYQREKEDEEEKQKYRYQREKKEQKEVQPGRERWEREEDEEHVDEEWRGSQRHEDPEERARLRYREERTKRDRRHQ
REGEEEERSSESQERRNPFLFKSNKFQTLFENENGHIRLLQRFDKRSDLFENLQNYRLVEYRAKPHTIFLPQHIDADLILVVLSGKAILTVLSPNAR
NSYNLERGDTIKLPAGTTSYLVNQDDEEDLRLVDLVIPVNGPGKFEAFDLSKNKNQYLRGFSKNILEASYNTKYETIEKVLLEEQEKTDAIVKVSRE
QIEELRKHAKSSSKKIFPSEFEPINLRNHKPEYSNKFGKLFEITPEKKYPQLQDLDIFVSCVEINEGALMLPHYNSRAIVVLLVNEGKGNLELLGLEN
EQQEREDRKERNNEVQRYEARLSPGDVVIIPAGHPVAITASSNLNLLAFGINAENNQRNFLSGSDDN [SEQ ID 243]
 >gi|164512526|emb|CAP06312.1| cvc [Pisum abyssinicum] 
MATTVESRFPLLLFPGIIFLASVCVTYANYDEGSETRVPGQRERGRQEGEKEEKRHGEWRPSYEKEEDEEEKQKYRYQREKEDEEEKQKYRYQR
EKKEEKEVQPGRERWEREEDEEQVDEEWRGSQRRQDPEERARLRHREERTKRDRRHKREGEEEERSSESQEQRNPFLFKSNKFLTLFENENG
HIRRLQRFDKRSDLFENLQNYRLVEYRAKPHTIFLPQHIDADLILVVLNGKAILTVLSPNDRNSYNLERGDTIKIPAGTTSYLVNQDDEEDLRVVD
FVIPVNRPGKFEAFGLSENKNQYLRGFSKNILEASLNTKYETIEKVLLEEQEKKPQQLRDRKRRQQGGERDAIIKVSREQIEELRKLAKSSSKKSLPS
EFEPFNLRSHKPEYSNKFGKLFEITPEKKYPQLQDLDILVSCVEINKGALMLPHYNSRAIVVLLVNEGKGNLELLGLKNEQQEREDRKERNNEVQ
RYEARLSPGDVVIIPAGHPVAISASSNLNLLGFGTNAENNQRNFLSGSDDN [SEQ ID 244]
 >gi|164512558| emb|CAP06328.1|cvc [Vicia villosa] 
MATTIKSRFPVLLLLGIIFLTSVCVTYANYDEGREPSVPGQRERGRQEGEKEEKRHGEWRPSEEDEEEKYKYEEGRVPGQRERGRQEGEKEEKR
HGKWRPSEEEDEEEKYRYEEGSEPRGPGQRETGRQEGEKEKQRPEREPSYEKEEDEEEKQKYQYHREKKEQREVRPGRERFERHEDEEQWRG
IQRHEDPEERARERYRAEIAKRQVEEEREERDIPHEREQRNPFLFKSNKFQTLFQNENGYIRRLQRFDKRSDLFENLQNYRLVEYRAKPHTIFLPQ
HIDADLIIVVLSGRAILTVLSPDDRNSYNLERGDTIKLPAGTTSYLVNQDDEEDLRVVDLAIPVNRPGKVESFLLSGNKNQYLRGFSKNILEASFNT
 
NYETIERVLLEEQDKESQQSIGQKRRSQRQETNALVKVSREQLEDLKRLAKSSSQEGLSSQFEPINLRSQNPKYSNKFGKVFEITPEKKYPQLQDL
DLFVSSVDIKEGALMLPHYNSRAIVVLLVNEGRGNLELVGLKNEQQEQREKEDEQQERNNQVQRYEARLSPGDVVIIPAGHPVAVRASSDLNL
LAFGINAENNQRNFLAGSDDN [SEQ ID 245]
Homologs of Pea protein 7 (SEQ ID 9)
   >gi|164512536| emb |CAP06317.1| cvc [Lathyrus hirsutus] 
MAIIIKSRFPLLLLLGIIFLASVCATWANYDEGSEPRVPGQRERGRQEGEKAEKSHEKWRPSYEEEYDEGSEPRVPGKRERGRQEGEKEEKRHGE
WRPSHEEEYDEGSEPRVPTHGERGRQEGEKEEKRHEEWRPSYEKEEDEEEKEKYKYQREKKEQKEVQPGREKWERKQDEKHVEEDEDQEEE
QWRGSKRREDPEERARLRYREERTKSNVEEETEERRNPFLFKSNKFLTLFENENGHIRRLQRFDERSDIFENLQNYRLVEYKAKPHTMFLPQHID
ADLIIVVLNGKAILTVLSPNDRNSYNLERGDTIKLPAGTTSYLVNQDDEEDLRVVDLAIPVNRPGKFEAFGLSANKNQYLRGFSKNILEAFLNTKY
ETIEKVLLEEQERRDRKGRQQGQETNAIVKVSREQIEELRKLAKSSSKKSLLSESEPINLRSQNPKYSNKFGKLFEITPEKKYPQLQDLDVSISCVEI
NEGAPLLPHYNSRAIVLLLVNEGKGNLELVGFKNEQQRQRENEERNKKVQRYEARLSPGDVVVIPAGHPVAISASLNLNLVGFGVNAENNQR
NFLTGSDDN [SEQ ID 246]
 >gi|164512542| emb|CAP06320.1| cvc [Lathyrus cicera] 
MATIIKSRFPLLLLLGIIFLASVCVTLANYDEGSEPRVPAQRERGRQEGEKEEKRHGEWRPSHEKEYDEGSEPRVPGRRERGRQEGEKEEKRHGE
WRPSYEKEYDEGSEPRVPGRRERGRQEGEKEEKRHGEWRPSYEKEYDEEEKQKYQYEREKEEQKEVQPGRERWERKEDEEKEEDQWRGSQ
RHEDPEERARLRYRKERTKKYVEEDTEETSSESQGRRNPFLFKSNKFLTLFENENGYIRRLQRFDERSDIFENLQNYRLVEYRAKPHTIFLPQHIDA
DLILVILNGKAILTVLSPNDRNSYNLERGDTIKLPAGTTSYLVNEDDEEDLRVVDLVIPVNRPGKFEAFDLNQYLGGFSKSVLEASLNTKYETIEKVL
LEEQQKQGQETNAIVKVSREQIEELRKLAKSSSKKSLLSELEPVNLRSHSPKYSNKFGKFFEITPEKKYPQLQDLDVSISCVEINEGALLLPHYNSRA
IVVVLVNEGKGNLELLGVQNEDEQQERKERNKEVQRYEARLSPGDVVIIPSGHPVAVSASSNLNLLGFGINAENNQRNFLSGSDDN [SEQ ID
247]
 >gi|164512544| emb|CAP06321.1| convicilin [Lathyrus sativus] 
MATIIKSRFPLLLLLGIIFLASVCVTYANYDEGSEPRVPAQRERGRQEGEKEEKRHGEWRPSSEKEYDEGSEPRVPGRRERGRQEGEKEEKRHGE
WRPSYEKEYDEEEKQKYQYEREKKEQKEVEPGRERWERKEDEEKEEDQWRGSQRHEDPEERARLRYRKERTKKYVEEDTEETSSESQGRRNP
FLFKSNKFLTLFENENGYIRRLQRFDERSDLFENLQNYRLVEYRAKPHTIFLPQHIDADLILVILNGKAILTVLSPNDRNSYNLERGDTIKLPAGTTS
YLVNEDDEEDLRVVDLVIPVNRPGKFEAFDLNQYLGGFSKSVLKASLNTKYETIEKVLLEEQQKQGQETNAIVKVSREQIEELRKLAKSSSKKSLLS
ELEPVNLRSHSPKYSNKFGKFFEITPEKKYPQLQDLDVSISCVEINEGALLLPHYNSRAIVVLLVNEGKGNLELLGVQDEDEQQERKKRNKEVQRY
EARLSPSDVVIIPAGHPVAVSASSNLNLLGFGINAENNERNFLSGSDDN [SEQ ID 248]
Homologs of Rice protein 3 (SEQ ID 10)
   >gi|573918992|ref|XP_006647120.1| PREDICTED: glutelin type-B 2-like [Oryza brachyantha] 
MATTVFSRFSTYFCVLLLCHGSMAQLFNPSTNPWHNPRQGSSRECRFDRLQPFEPLRKVRSEAGVTEYFDEKNELFQCTGTFVIRRVIQPQGLL
VPRYTNAPGLVYIIQGRGSIGLTFPGCPATYQQQFQQFLPQEQSQSQKFRDEHQKIHQFRQGDIVALPAGVAHWFYNDGDAPVVAVYVYDV
KNSANQLEPRQREFLLGGNNMRAQQVYGSSAEQHSRQNIFSGFGVEILSEALGISTVTTKRLQSQNDQRGEIIHVKNGLQFLKPTLTQQQEQA
QAQYQEVQYSEQQQTSSRWNGLDENFCTIKARMNIENTSRADTYNPRAGRTTSLNSQKFPILNLVQMSATRVNLYQNAILSTFWNVNAHSL
VYTIQGRARVQVVSNFGKTVFDGELRPGQLLIIPQHYVVLKKAQREGFRYIAIKTNANAFVSQLVGKNSVFRSLPVDVIANVYRISREQARSLKN
NRGEEHGAFAPRSQQQSYPGFSNQSESETSE [SEQ ID 249]
 >gi|573919041|ref|XP_006647142.1| PREDICTED: glutelin type-B 4-like [Oryza brachyantha] 
MATTTFSRFSIYFCVLLLCHGSMAQLFSPTLNPWHSSRRGGSRDCRFDRLQAFEPLRRVRSEAGVTEYFDERNEQFQCTGTFVIRRVIEPQGLL
VPRYTNTPGVVYIMQGRGSMGLTFPGCPATYQQQFQQFLPEGQSQSQKFRDEHQKIHQFRQGDIVALPAGVAHWFYNEGDTPVVALYVFD
INNSANQLEPRQKDFLLAGNNNREQQVYGRSIEKHSGQNIFSGFNHELLSEALGISTLAAKRLQGQNDHRGEIIRVRNGLQLLKPTFTQQQEQ
AQSQYQVQYSEKQQESTRCNGLDENFCTINARLNIENPSRADTYNPRAGRITHLNNQKFPILNLVQMSATRVNLYQNAILSPYWNVNAHSLV
 
YMVQGHARVQVVSNLGKTVFNSVLRPGQLLIIPQHYVVLKKAEREGCQYIAFKTNANSIVSQLAGKNSILRAMPVDVVANAYRISREQARDLK
NNRGEELGAFTPKFEQQSYPGLSNESESEASE [SEQ ID 250]
 >gi|109894635|gb|ABG47337.1| glutelin precursor [Zizania latifolia] 
MNMATINGPTIFFTVCLFLLCHGSLAQLLGQSTSQWQSSHRGSSRQCRFDRLQAFEPVRSVRSQAGTTEFFDASNELFQCAGVSIVRRIIEPRG
LLLPQYTNGATIMYIIQGRGITGQTFPGCPESYQQQFQQSMQAQLTGSQSQSQKFKDEHQKINRFRQGDVIALPAGVAHWCYNDGEVPVVA
IYVIDINNAANQLDPRQRDFLLAGNMRSPQAYRREVENQSQNIFSGFSAELLSEALGISTGVARQLQCQNDQRGEIVRVEHGLSLLQPYASLQE
QEQKQEQPRERYQVTQHQQSQYGGGCSNGLDETFCAMRIWQNIDNPNLADTYNPRAGRVTNLNSQKFPILNLIQMSAVKVNLYQNALLSP
FWNINSHSVVYVTQGCARVQVVNNNGKTVFNGELRRGQLLIIPQHYVVVKKAQREGCAYIAFKTNPNSMVSHIVGKSSIFRALPTDVLANAY
RISREDAQRLKHNRGDELGAFTPLQYKSYQDVSSVAASS [SEQ ID 251]
Homologs of Rice protein 4 (SEQ ID 11)
   >gi|531874314|gb|AGT59174.1| glutelin, partial [Oryza sativa Indica Group] 
CRFDRLQAFEPIRSVRSQAGTTEFFDVSNEQFQCTGVSAVRRVIEPRGLLLPHYTNGASLVYIIQGRGITGPTFPGCPESYQQQFQQSGQAQLT
ESQSQSHKFKDEHQKIHRFRQGDVIALPAGVAHWCYNDGEVPVVAIYVTDLNNGANQLDPRQRDFLLAGNKRNPQAYRREVEERSQNIFSG
FSTELLSEALGVSSQVARQLQCQNDQRGEIVRVEHGLSLLQPYASLQEQEQGQVQSRERYQEGQYQQSQYGSGCSNGLDETFCTMKVRQNI
DNPNRADTYNPRAGRVTNLNTQNFPILNLVQMSAVKVNLYQNALLSPFWNINAHSVVYITQGRARVQVVNNNGKTVFNGELRRGQLLIIPQ
HYAVVKKAQREGCAYIAFKTNPNSMVSHIAGKSSIFRALPNDVLANAYRISREEAQRLKHNRGDEFGAFTPIQYKSYQDVYNAAESS [SEQ ID
252]
 >gi|109894635|gb|ABG47337.1| glutelin precursor [Zizania latifolia] 
MNMATINGPTIFFTVCLFLLCHGSLAQLLGQSTSQWQSSHRGSSRQCRFDRLQAFEPVRSVRSQAGTTEFFDASNELFQCAGVSIVRRIIEPRG
LLLPQYTNGATIMYIIQGRGITGQTFPGCPESYQQQFQQSMQAQLTGSQSQSQKFKDEHQKINRFRQGDVIALPAGVAHWCYNDGEVPVVA
IYVIDINNAANQLDPRQRDFLLAGNMRSPQAYRREVENQSQNIFSGFSAELLSEALGISTGVARQLQCQNDQRGEIVRVEHGLSLLQPYASLQE
QEQKQEQPRERYQVTQHQQSQYGGGCSNGLDETFCAMRIWQNIDNPNLADTYNPRAGRVTNLNSQKFPILNLIQMSAVKVNLYQNALLSP
FWNINSHSVVYVTQGCARVQVVNNNGKTVFNGELRRGQLLIIPQHYVVVKKAQREGCAYIAFKTNPNSMVSHIVGKSSIFRALPTDVLANAY
RISREDAQRLKHNRGDELGAFTPLQYKSYQDVSSVAASS [SEQ ID 253]
 >gi|472867| emb|CAA52764.1| 11S globulin [Avena sativa] 
MATTSFPSMLFYFCIFLLFHGSMAQLFGQSSTPWQSSRQGGLRGCRFDRLQAFEPLRQVRSQAGITEYFDEQNEQFRCTGVSVIRRVIEPQGL
VLPQYHNAPALVYILQGRGFTGLTFPGCPATFQQQFQPFDQSQFAQGQRQSQTIKDEHQRVQRFKQGDVVALPAGIVHWCYNDGDAPIVA
IYVFDVNNNANQLEPRQKEFLLAGNNKREQQSGNNIFSGLSVQLLSEALGISQQAAQRIQSQNDQRGEIIRVSQGLQFLKPIVSQQVPGEQQV
YQPIQTQEGQATQYQVGQSTQYQVGKSTPYQGGQSSQYQAGQSWDQSFNGLEENFCSLEARKNIENPQHADTYNPRAGRITRLNSKNFPIL
NIVQMSATRVNLYQNAILSPFWNINAHSVIYMIQGHARVQVVNNNGQTVFNDILRRGQLLIVPQHFVVLKKAEREGCQYISFKTNPNSMVSH
IAGKSSILRALPIDVLANAYRISRQEARNLKNNRGEEFGAFTPKLTQKGFQSYQDIEEGSSSPVRASE [SEQ ID 254]
Homologs of Rice protein 5 (SEQ ID 12)
   >gi|225959|prf||1404367A glutelin 
MASTNRPIVFFTVCLFLLCDGSLAQQLLGQSTSQWQSSRRGSPRGCRFDRLQAFEPIRSVRSQAGTTEFFDVSNELFQCTGVSVVRRVIEPRGL
LLPHYTNGASLVYIIQGRGITGPTFPGCPETYQQQFQQSGQAGLTESQSQSHKFKDEHQKIHRFRQGDVIALPAGVAHWCYNDCEVPVVAIYV
TDINNGANQLDPRQRDFLLAGNKRNPQAYRREVEEWSQNIFSGFSTELLSEAFGISNQVARQLQCQNDQKGEIVRVERGLSLLQPYASLQEQ
EQGQMQSREHYQEGGYQQSQYGSGCPNGLDETFCVNKVRQNIDNPNRADTYNPRAGRVTNLSQNFPILNLVQMSAVKVNLYQNTDTWIS
MGQEENALLSPFWNINAHSIVYITQGRAQVQVLRRGQLLIVPQHYVVVKKAQREGCAYIAFKTNPNSMVSHIAGKSSIFRALPTDVLANAYRIS
REEAQRLKHNRGDEFGAFTPLQYKSYQDVYNVAESS [SEQ ID 255]
 >gi|573943558|ref|XP_006654150.1| PREDICTED: glutelin type-A 3-like [Oryza brachyantha] 
MKSSIVFSTICLVLLCHGSLAQLLSQSTSQWQSSRRGSPRQCRFDQLQAFEPIRTVRSQAGVTEFYDVSNELFQCTGVSVVRRVIEPRGLLLPHY
SNGATLVYIIQGRGITGPTFPGCPETYQQQFQQSGEAQPFEGQSHKFRDEHQKIHRFRQGDVVALPAGVAHWCYNDGEVPIVAIYVTDIYNS
ANQLDPRHRDFFLAGNNKVAQQLYRSEARENSKNIFGGFSVELLSEALGISRGVARQLQCQNDQRGEIVRVEHGLALLQPYASVQEQQQEQV
QSRDYEQTQYQQKQPQGSCSNGLDETFCTMRLRQNIDNPNLADTYNPKAGRITYLNGQKFPILNLVQMSAVKVNLYQNAVLSPFWNINAHS
VVYITQGRARVQVVNNNGKTVFDGELRQGQLLIIPQHHVVLKKAQREGCSYIALKTNPNSIVSHIAGKNSIFRALPGDVVTNAYRISREEAKRIK
HNRGDESGVFAPSHAYRSYQDMSVAA [SEQ ID 256]
 >gi|721641733|ref|XP_010231907.1| PREDICTED: 12S seed storage globulin 1-like [Brachypodium distachyon] 
MAHTSFSSFLSYFCLFLLFHGSMAQVLGQVSTWQSSRQGGSRDCSFDRLQAIEPVTQVRSQAGLTEYFDEQNEQFRCAGVFVIRRVIEPRGLL
LPRYHNTPGLVYILQGNGFVGLTFPGCPETFREQFQQFRQTQSTLGQSQCQSQKLGDVHQRVHQFTQGDVVALPTGVAHWIYNGGDAPVV
IVYVFDVNNNANQLEPRQKEFLLGGNYNGVLQYGQNIFSGFNAQLLSQAFGINEQTSQRIQNQNDGRGDIIRVDNGLQFLKPVVTQQQPEQ
PFMPIQHQTGQSSRNGLEENFCSLEPRQNIEDPNRADTYNPRAGSITRLNGQNFPILNLVQMSATRVNLQKNAILSPFWNINAHSVVYVIQG
HALVQVVNNQGHNVFNGLLHRGQLLIIPQNYVVLKKAESEGYQYIAFKTNANSMVSHIAGKNSILRALPVDVIANAYRISRQEAQNLKNNRGE
ETGVLTPNFSQSTCQSYQTEDVQSLRPMSHWSE [SEQ ID 257]
Homologs of Rice protein 6 (SEQ ID 13)
   >gi|169244463|gb|ACA50505.1| seed allergenic protein RAG2 [Oryza sativa Japonica Group] 
MASNKVVFSALLLIIVSVLAATATMADHHKDQVVYSLGERCQPGMGYPMYSLPRCRAVVKRQCVGHGAPGGAVDEQLRQDCCRQLAAVD
DSWCRCSALNHMVGGIYRELGATDVGHPMAXVFPGCRRGDLERAAASLPAFCNVDIPNGTGGVCYWLGYPRTPRTGH [SEQ ID 258]
 >gi|5777592|emb|CAA44001.1| low molecular weight globulin [Oryza sativa] 
MASNKVVFSALLLIIVSVLRRDGTMADHHKDQVVYSLGERCQPGMGYPMYSLPRCRAVVKRQCVGHGAPGAVDEQLRQDCCRQLAAVDD
SWCRCSALNHMVGGIYRELGATDVGHPMAEVFPGCRRGDLERAAASLPAFCNVDIPNGTGGVCYWLGYPRTPRTGH [SEQ ID 259]
 >gi|115471175|ref|NP_001059186.1| Os07g0214600 [Oryza sativa Japonica Group] >gi|23616954|dbj|BAC20657.1| allergen RA16 [Oryza sativa Japonica Group] >gi|113610722|dbj|BAF21100.1| Os07g0214600 [Oryza sativa Japonica Group] >gi|125557687|gb|EAZ03223.1| hypothetical protein Osl_25372 [Oryza sativa Indica Group] 
MASNKVVISALLVVVVSVLAATTTMADHHQEVVYTPGQLCQPGIGYPTYPLPRCRAFVKRQCVAPGTVDEQVRRGCCRQLAAIDSSWCRC
DALNHMLRIIYRESGAADAGHPMAEVFRGCRRGDIERAAASLPAFCNVDIPNGVGGVCYWLPGTGY [SEQ ID 260]
Homologs of Rice protein 7 (SEQ ID 14)
   >gi|115445309|ref|NP_001046434.1|Os02g0248800[Oryza sativa Japonica Group] >gi|37993738|gb|AAR06952.1| glutelin type-B [Oryza sativa Japonica Group] >gi|47477729|dbj|BAD19794.1| glutelin type-B [Oryza sativa Japonica Group] >gi|113535965|dbj|BAF08348.1|Os02g0248800 [Oryza sativa Japonica Group] >gi|215768942|dbj|BAH01171.1| unnamed protein product [Oryza sativa Japonica Group] >gi|284431772|gb|ADB84627.1| glutelin [Oryza sativa Japonica Group] 
MTISVFSRFSIYFCVLLLCNGSMAQLFDPATNQWQTHRQGSFRECRFERLQAFEPLQNVRSEAGVTEYFDETNELFQCTGTFVIRRVIQPQGLL
IPRYANTPGMVYIIQGRGSMGLTFPGCPATYQQQSQQFLFQGESQSQKFIDEHQKIHQFRQGDIVVLPTGVAHWFYNDGDTPVVALYVYDI
NNSANQLEPRHREFLLAGKNNRVQQVYGRSIQQHSGQNIFNGFSVEPLSEALNINTVTTKRLQSQNDQRGEIIHVKNGLQLLKPTLTQRQEQE
QAQYQEVQYSEKPQTSSRWNGLEENLCTIKTRLNIENPSRADSYDPRAGRITSLDSQKFPILNIIQMSATRVNLYQNAILTPFWNVNAHSLMYV
IRGRARVQVVSNFGKTVFDGVLRPEQLLIIPQNYVVLKKAQHEGCQYIAINTNANAFVSHLAGVDSVFHALPVDVIANAYCISREEARRLKNNR
GDEYGPFPPRLQQQIYPEFSNESKGETSE [SEQ ID 261]
 >gi|428674402|gb|AFZ41188.1| glutelin, partial [Oryza sativa Japonica Group] 
LLCHGSMAQIFSLGINPWQNPRQGGSRECRFDRLQAFEPLRKVRHEAGVTEYFDEKNEQFQCTGTLVIRRIIEPQGLLLPRYSNTPGLVYIIQGT
GVLGLTFPGCPATYQKQFRHFGLEGGSQRQGKKLRDENQKIHQFRQGDVVALPSGIPHWFYNEGDTPVVALFVFDVNNNANQLEPRQKEFL
LAGNNIEQQVSNPSINKHSGQNIFNGFNTKLLSEALGVNIEVTRRLQSQNDRRGDIIRVKNGLRLIKPTITQQQEQTQDQYQQIQYHREQRSTS
KYNGLDENFCAIRARLNIENPNHADTYNPRAGRITNLNSQKFSILNLVQMSATRVNLYQNAILSPFWNINAHSLVYTIQGRARVQVVSNHGKA
VFNGVLRPGQLLIIPQNYVVMKKAELEGFQFIAFKTNPNAMVNHIAGKNSVLRAMPVDVIANAYRISRQEARSLKNNRGEEIGAFTPRYQQQ
KIHQEYSNPNESETQ [SEQ ID 262]
 >gi|226510|prf||1515394A seed storage globulin 
MATTRFPSLLFYSCIFLLCNGSMAQLFGQSFTPWQSSRQGGLRGCRFDRLQAFEPLRQVRSQAGITEYFDEQNEQFRCAGVSVIRRVIEPQGLL
LPQYHNAPGLVYILQGRGFTGLTFPGCPATFQQQFQPFDQARFAQGQSKSQNLKDEHQRVHHIKQGDVVALPAGIVHWCYNDGDAPIVAV
YVFDVNNNANQLEPRQKEFLLAGNNKREQQFGQNIFSGFSVQLLSEALGISQQAAQKIQSQNDQRGEIIRVSQGLQFLKPFVSQQGPVEHQA
YQPIQSQQEQSTQYQVGQSPQYQEGQSTQYQSGQSWDQSFNGLEENFCSLEARQNIENPKRADTYNPRAGRITHLNSKNFPTLNLVQMSA
TRVNLYQNAILSPYWNINAHSVMHMIQGRARVQVVNNHGQTVFNDILRRGQLLIIPQHYVVLKKAEREGCQYISFKTTPNSMVSYIAGKTSIL
RALPVDVLANAYRISRQESQNLKNNRGEEFGAFTPKFAQTGSQSYQDEGESSSTEKASE [SEQ ID 263]
Homologs of Rice protein 8 (SEQ ID 15)
   >gi|83375868|gb|ABC17777.1| waxy [Oryza rufipogon] 
MSALTTSQLATSATGFGIADRSAPSSLLRHGFQGLKPRSPAGGDATSLSVTTSARATPKQQRSVQRGSRRFPSVVVYATGAGMNVVFVGAEM
APWSKTGGLGDVLGGLPPAMAANGHRVMVISPRYDQYKDAWDTSVVAEIKVADRYERVRFFHCYKRGVDRVFVDHPSFLEKVWGKTGEKI
YGPDTGVDYKDNQMRFSLLCQAPRILNLNNNPYFKGTYGEDVVFVCNDWHTGPLASYLKNNYQPNGIYRNAKVAFCIHNISYQGRFAFEDYP
ELNLSERFRSSFDFIDGYDTPVEGRKINWMKAGILEADRVLTVSPYYAEELISGIARGCELDNIMRLTGITGIVNGMDVSEWDPSKDKYITAKYD
ATTAIEAKALNKEALQAEAGLPVDRKIPLIAFIGRLEEQKGPDVMAAAIPELMQEDVQIVLLGTGKKKFEKLLKSMEEKYPGKVRAVVKFNAPLA
HLIMAGADVLAVPSRFEPCGLIQLQGMRYGTPCACASTGGLVDTVIEGKTGFHMGRLSVDCKVVEPSDVKKVAATLKRAIKVVGTPAYEEMV
RNCMNQDLSWKGPAKNWENVLLGLGVAGSAPGIEGDEIAPLAKENVAAP [SEQ ID 264]
 >gi|297614332|gb-ADI48504.1| glycogen synthetase [Oryza officinalis] 
MSALTTSQLATSATGFGIADRSAPSSLLRHGFQGLKPRSPAGGDASSLSVTTSARATPKQQRSVQRGSRRFPSVVVYATGAGMNVVFVGAEM
APWSKTGGLGDVLGGLPPAMAANGHRVMVISPRHDQYKDAWDTSVVAEIKVADRYERVRFFHCYKRGVDRVFIDHPSFLEKVWGKTGEKI
YGPDTGVDYKDNQMRFSLLCQAALEAPRILNLNNNPYFKGTYGEDVVFVCNDWHTGPLPSYLKNNYQPNGIYRNAKVAFCIHNISYQGRFAF
EDYPELNLSERFRSSFDFIDGYDTPVEGRKINWMKAGILESDRVLTVSPYYAEELISGIARGCELDNIMRLTGITGIVNGMDVSEWDPSKDKYIA
AKYDATTAIEAKALNKEALQAEAGLPVDRKIPLIAFIGRLEEQKGPDVMAAAIPELMQENVQIVLLGTGKKKFEKLLKSMEEKYPGKVRAVVKF
NAPLAHLIMAGADVLAVPSRFEPCGLIQLQGMRYGTPCACASTGGLVDTVIEGKTGFHMGRLSVDCKVVEPSDVQKVATTLKRAIKIVGTPAY
NEMVRNCMNQDLSWKGPAKNWENVLLGLGVAGSAPGVEGEEIAPLAKENVAAP [SEQ ID 265]
 >gi|389620054|gb|AFK93486.1| granule-bound starch synthase [Hordeum vulgare subsp. vulgare] 
MAALATSQLATSGTVLGVTDRFRRPGFQGLRPRNPADAALGMRTIGASAAPKQSRKAHRGSRRCLSVVVRATGSGMNLVFVGAEMAPWS
KTGGLGDVLGGLPPAMAANGHRVMVVSPRYDQYKDAWDTSVISEIKVADEYERVRFFHCYKRGVDRVFIDHPWFLEKVRGKTKEKIYGPDA
GTDYEDNQQRFSLLCQAALEAPRILNLNNNPYFSGPYGEDVVFVCNDWHTGLLACYLKSNYQSNGIYRTAKVAFCIHNISYQGRFSFDDFAQL
NLPDRFKSSFDFIDGYDKPVEGRKINWMKAGILQADKVLTVSPYYAEELISDEARGCELDNIMRLTGITGIVNGMDVSEWDPTKDKFLAVNYDI
TTALEAKALNKEALQAEVGLPVDRKVPLVAFIGRLEEQKGPDVMIAAIPEILKEEDVQIILLGTGKKKFEKLLKSMEEKFPGKVRAVVRFNAPLAH
QMMAGADLLAVTSRFEPCGLIQLQGMRYGTPCVCASTGGLVDTIVEGKTGFHMGRLSVDCNVVEPADVKKVATTLKRAVKVVGTPAYQEM
VKNCMIQDLSWKGPAKNWEDVLLELGVEGSEPGIVGEEIAPLAMENVAAP [SEQ ID 266]
Homologs of GLUC(Staphylococcus aureus) protein 1 (SEQ ID 16)
   >gi|446599182|ref|WP_000676528.1| glutamyl endopeptidase [Staphylococcus aureus] >gi|253729369|gb|EES98098.1|
   trypsin [Staphylococcus aureus subsp. aureus TCH130] >gi|341844549|gb|EGS85761.1| glutamyl endopeptidase
   [Staphylococcus aureus subsp. aureus 21259] >gi|537390486|gb|AGU61109.1| Glutamyl endopeptidase precursor
   [Staphylococcus aureus subsp. aureus CN1] >gi|564714561|gb|ETD14665.1| glutamyl endopeptidase [Staphylococcus
   aureus subsp. aureus KPL1845] >gi|577466329|gb|EUG79766.1| glutamyl endopeptidase [Staphylococcus aureus M0139]
   >gi|580560623|gb|EVF84961.1| glutamyl endopeptidase [Staphylococcus aureus COAS6020]
   >gi|580687002|gb|EVH10169.1| glutamyl endopeptidase [Staphylococcus aureus UCIM6080]
   >gi|751815683|gb-KIN24957.1| glutamyl endopeptidase [Staphylococcus aureus MRSA_CVM43477]
   >gi|781884797|dbj|BAR08486.1| glutamyl endopeptidase precursor [Staphylococcus aureus subsp. aureus]
   >gi 17818877621 dbj I BAR11210.1| glutamyl endopeptidase precursor [Staphylococcus aureus subsp. aureus]

MKGKFLKVSSLFVATLTTATLVSSPAANALSSKAMDNHPQQSQSSKQQTPKIQKGGNLKPLEQREHANVILPNNDRHQITDTTNGHYAPVTYI
QVEAPTGTFIASGVVVGKDTLLTNKHVVDATHGDPHALKAFPSAINQDNYPNGGFTAEQITKYSGEGDLAIVKFSPNEQNKHIGEVVKPATMS
NNAETQVNQNITVTGYPGDKPVATMWESKGKITYLKGEAMQYDLSTTGGNSGSPVFNEKNEVIGIHWGGVPNEFNGAVFINENVRNFLKQ
NIEDIHFANDDQPNNPDNPDNPNNPDNPNNPDEPNNPDNPNNPDNPDNGDNNNSDNPDAA [SEQ ID 267]

## Claims

**1.** A natural anti-inflammatory peptide comprising an anti-inflammatory fragment of a protein selected from SEQUENCE ID NO's: 1 to 15, or an anti-inflammatory variant of the fragment, wherein the anti-inflammatory fragment is selected from SEQUENCE ID NO's: 71 to 220, and wherein:
- the anti-inflammatory fragment has 7 to 37 contiguous amino acids;
- the anti-inflammatory fragment has a change of -9 to +3;
- the c-terminal amino acid of the anti-inflammatory fragment is not cysteine (C) or methionine (M); and
- the n-terminal amino acid of the anti-inflammatory fragment is not cysteine (C), histidine (H), proline (P) or threonine (T).

**2.** A natural anti-inflammatory peptide of Claim 1 in which the c-terminal domain and n-terminal domain of the anti-inflammatory fragment does not contain cysteine (C).

**3.** A natural anti-inflammatory peptide of any preceding Claim in which the anti-inflammatory fragment does not contain cysteine (C).

**4.** A natural anti-inflammatory peptide of any preceding Claim, wherein the protein is a pea protein selected from SEQUENCE ID NO's: 1 to 5 and 8 to 9, and wherein the growth promoting fragment is selected from SEQUENCE ID NO's: 71 to 107 and 110 to 111.

**5.** A natural growth promoting peptide of any preceding Claim, wherein the protein is a rice protein selected from SEQUENCE ID NO's: 6 to 7 and 10 to 15, and wherein the growth promoting fragment is selected from SEQUENCE ID NO's: 108 to 109 and 112 to 220.

**6.** A composition comprising one or more natural anti-inflammatory peptides according to any of Claims 1 to 5.

**7.** A composition according to Claim 6 and having a peptide profile comprising substantially all of the peptides of SEQUENCE ID NO's: 71 to 107 and 110 to 111.

**8.** A composition according to Claim 6 having a peptide profile comprising substantially all of the peptides of SEQUENCE ID NO's: 108 to 109 and 112 to 220.

**9.** A composition according to any of Claims 6 to 8 selected from a food or beverage, nutritional supplement, personal care composition, or pharmaceutical composition.

**10.** A non-therapeutic method of preventing or inhibiting inflammation in a mammal comprising a step of topically administering to the mammal a natural growth promoting peptide of any of Claims 1 to 5 or a composition of any of Claims 6 to 9.

**11.** A non-therapeutic method of maintaining gut health in a mammal comprising a step of orally administering to the mammal a natural growth promoting peptide of any of Claims 1 to 6 or a composition of any of Claims 7 to 9.

**13.** A natural growth promoting peptide of any of Claims 1 to 5, or composition of any of Claims 6 to 8, **for use in** a method for treatment or prevention of inflammation or an inflammatory disorder in a mammal.

**14.** A natural growth promoting peptide of any of Claims 1 to 5, or composition of any of Claims 6 to 8, for use of Claim 13, in which the inflammatory disorder is selected from a skin inflammatory disorder, an inflammatory disorder of the joints, an inflammatory disorder of the cardiovascular system, an inflammatory disorder of the lung or airways, and a gut inflammatory disorder.
